(19) Europäisches Patentamt
European Patent Office
Office européen des brevets

(11) EP 3 473 620 B1

(12) EUROPEAN PATENT SPECIFICATION

(45) Date of publication and mention of the grant of the patent:
**26.02.2020 Bulletin 2020/09**

(51) Int Cl.:
***C07D 403/10*** *(2006.01)* ***C09K 11/06*** *(2006.01)*
***H01L 51/50*** *(2006.01)*

(21) Application number: **17197056.9**

(22) Date of filing: **18.10.2017**

(54) **ORGANIC MOLECULES, IN PARTICULAR FOR USE IN OPTOELECTRONIC DEVICES**

ORGANISCHE MOLEKÜLE, INSBESONDERE ZUR VERWENDUNG IN OPTO-ELEKTRONISCHEN VORRICHTUNGEN

MOLÉCULES ORGANIQUES, EN PARTICULIER DESTINÉES À ÊTRE UTILISÉES DANS DES DISPOSITIFS OPTOÉLECTRONIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.04.2019 Bulletin 2019/17**

(73) Proprietor: **CYNORA GMBH**
**76646 Bruchsal (DE)**

(72) Inventors:
- **SEIFERMANN, Stefan**
  **77815 Bühl (DE)**
- **DANZ, Michael**
  **76344 Eggenstein-Leopoldshafen (DE)**
- **BERGMANN, Larissa**
  **76199 Karlsruhe (DE)**

(74) Representative: **Hoppe, Georg Johannes**
**Darani Anwaltskanzlei**
**Beuckestrasse 20**
**14163 Berlin (DE)**

(56) References cited:
**WO-A1-2014/146752** **US-A1- 2015 349 273**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**Description**

The invention relates to organic light-emitting molecules and their use in organic light-emitting diodes (OLEDs) and in other optoelectronic devices.
US 2015 0349273 A1 describes luminescent materials including donor-acceptor compounds. WO 2014/146752 A1 discloses materials for optoelectronic devices.

**Description**

The object of the present invention is to provide molecules which are suitable for use in optoelectronic devices.
This object is achieved by the invention which provides a new class of organic molecules.
According to the invention, the organic molecules are purely organic molecules, i.e. they do not contain any metal ions in contrast to metal complexes known for use in organic optoelectronic devices.
According to the present invention, the organic molecules exhibit emission maxima in the blue, sky-blue or green spectral range. The organic molecules exhibit in particular emission maxima between 420 nm and 520 nm, preferably between 440 nm and 495 nm, more preferably between 450 nm and 470 nm. The photoluminescence quantum yields of the organic molecules according to the invention are, in particular, 70 % or more. The molecules according to the invention exhibit in particular thermally activated delayed fluorescence (TADF). The use of the molecules according to the invention in an optoelectronic device, for example an organic light-emitting diode (OLED), leads to higher efficiencies of the device.
The organic light-emitting molecules according to the invention consist of one first chemical moiety comprising or consisting of a structure of Formula I,

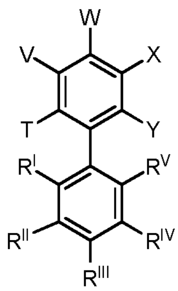

Formula I

and

- one second chemical moiety consisting of a structure of Formula II,

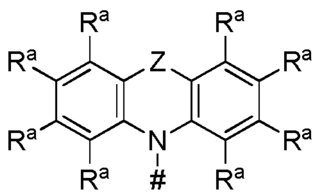

Formula II

wherein the first chemical moiety is linked to the second chemical moiety via a single bond.

T is the binding site of a single bond linking the first chemical moiety to the second chemical moiety, or is selected from the group consisting of $R^1$ and $R^T$.
V is the binding site of a single bond linking the first chemical moiety to the second chemical moiety or is hydrogen (H).
W is the binding site of a single bond linking the first chemical moiety to the second chemical moiety, or is selected from the group consisting of $R^1$ and $R^T$.
X is selected from the group consisting of $R^1$ and $R^T$.
Y is selected from the group consisting of $R^1$ and $R^T$.

# represents the binding site of a single bond linking the first chemical moiety to the second chemical moiety.

Z is a direct bond.

$R^T$ is 1,3,5-triazinyl, substituted with two substituents $R^N$:

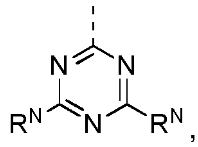

wherein the dotted bond represents the binding site of $R^T$ to the single bond linking the first chemical moiety and $R^T$.
$R^N$ is at each occurrence independently from another selected from the group consisting of hydrogen, deuterium, $C_1$-$C_5$-alkyl,

wherein one or more hydrogen atoms are optionally substituted by deuterium; $C_2$-$C_8$-alkenyl,
wherein one or more hydrogen atoms are optionally substituted by deuterium; $C_2$-$C_8$-alkynyl,
wherein one or more hydrogen atoms are optionally substituted by deuterium; $C_6$-$C_{18}$-aryl,
which is optionally substituted with one or more substituents $R^6$; and
$C_3$-$C_{17}$-heteroaryl,
which is optionally substituted with one or more substituents $R^6$.

$R^I$, $R^{II}$, $R^{III}$, $R^{IV}$, and $R^V$ is at each occurrence independently from another selected from the group consisting of
hydrogen,
deuterium,
F,
$C_1$-$C_5$-alkyl,

wherein one or more hydrogen atoms are optionally substituted by deuterium; $C_2$-$C_8$-alkenyl,
wherein one or more hydrogen atoms are optionally substituted by deuterium; $C_2$-$C_8$-alkynyl,
wherein one or more hydrogen atoms are optionally substituted by deuterium; and

$C_6$-$C_{18}$-aryl,
which is optionally substituted with one or more substituents $R^6$.

$R^1$ is at each occurrence independently from another selected from the group consisting of hydrogen,
deuterium,
$C_1$-$C_5$-alkyl,

wherein one or more hydrogen atoms are optionally substituted by deuterium; $C_2$-$C_8$-alkenyl,
wherein one or more hydrogen atoms are optionally substituted by deuterium; $C_2$-$C_8$-alkynyl,
wherein one or more hydrogen atoms are optionally substituted by deuterium; and

$C_6$-$C_{18}$-aryl,
which is optionally substituted with one or more substituents independently from another selected from the group consisting of

$C_1$-$C_5$-alkyl,
wherein optionally one or more hydrogen atoms are independently from each other substituted by deuterium, CN, $CF_3$, or F;
and
$C_6$-$C_{18}$-aryl,
which is optionally substituted with one or more $C_1$-$C_5$-alkyl substituents.

$R^a$, $R^3$ and $R^4$ is at each occurrence independently from another selected from the group consisting of hydrogen,
deuterium,
$N(R^5)_2$,
$OR^5$,
$Si(R^5)_3$,
$B(OR^5)_2$,

$OSO_2R^5$,
$CF_3$,
CN,
F,
Br,
I,
$C_1$-$C_{40}$-alkyl,
which is optionally substituted with one or more substituents $R^5$ and wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;
$C_1$-$C_{40}$-alkoxy,
which is optionally substituted with one or more substituents $R^5$ and wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;
$C_1$-$C_{40}$-thioalkoxy,
which is optionally substituted with one or more substituents $R^5$ and wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;
$C_2$-$C_{40}$-alkenyl,
which is optionally substituted with one or more substituents $R^5$ and wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;
$C_2$-$C_{40}$-alkynyl, which is optionally substituted with one or more substituents $R^5$ and wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;
$C_6$-$C_{60}$-aryl,
which is optionally substituted with one or more substituents $R^5$; and
$C_3$-$C_{57}$-heteroaryl,
which is optionally substituted with one or more substituents $R^5$.

$R^5$ is at each occurrence independently from another selected from the group consisting of hydrogen,
deuterium,
$N(R^6)_2$,
$OR^6$,
$Si(R^6)_3$,
$B(OR^6)_2$,
$OSO_2R^6$,
$CF_3$,
CN,
F,
Br,
I,
$C_1$-$C_{40}$-alkyl,
which is optionally substituted with one or more substituents $R^6$ and wherein one or more non-adjacent $CH_2$-groups are optionally substituted by $R^6C$=$CR^6$, C≡C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_1$-$C_{40}$-alkoxy,
which is optionally substituted with one or more substituents $R^6$ and wherein one or more non-adjacent $CH_2$-groups are optionally substituted by $R^6C$=$CR^6$, C≡C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_1$-$C_{40}$-thioalkoxy,
which is optionally substituted with one or more substituents $R^6$ and wherein one or more non-adjacent $CH_2$-groups are optionally substituted by $R^6C$=$CR^6$, C≡C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_2$-$C_{40}$-alkenyl,
which is optionally substituted with one or more substituents $R^6$ and wherein one or more non-adjacent $CH_2$-groups are optionally substituted by $R^6C$=$CR^6$, C≡C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_2$-$C_{40}$-alkynyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, $C\equiv O$, C=S, C=Se, $C=NR^6$, $P(=O)(R^6)$, SO, $SO_2$, $NR^6$, O, S or $CONR^6$;
$C_6$-$C_{60}$-aryl,
which is optionally substituted with one or more substituents $R^6$; and
$C_3$-$C_{57}$-heteroaryl,
which is optionally substituted with one or more substituents $R^6$.

$R^6$ is at each occurrence independently from another selected from the group consisting of hydrogen, deuterium,
OPh,
$CF_3$,
CN,
F,
$C_1$-$C_5$-alkyl,
wherein optionally one or more hydrogen atoms are independently from each other substituted by deuterium, CN, $CF_3$, or F;
$C_1$-$C_5$-alkoxy,
wherein optionally one or more hydrogen atoms are independently from each other substituted by deuterium, CN, $CF_3$, or F;
$C_1$-$C_5$-thioalkoxy,
wherein optionally one or more hydrogen atoms are independently from each other substituted by deuterium, CN, $CF_3$, or F;
C2-C5-alkenyl,
wherein optionally one or more hydrogen atoms are independently from each other substituted by deuterium, CN, $CF_3$, or F;
C2-C5-alkynyl, wherein optionally one or more hydrogen atoms are independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$; and
$N(C_3$-$C_{17}$-heteroaryl$)(C_6$-$C_{18}$-aryl$)$.

The substituents $R^a$, $R^3$, $R^4$ and/or $R^5$ independently from each other can optionally form a mono- or polycyclic, aliphatic, aromatic and/or benzo-fused ring system with one or more substituents $R^a$, $R^3$, $R^4$ and/or $R^5$.

According to the invention, exactly one (one and only one) substituent selected from the group consisting of T, W, X, and Y is $R^T$, and exactly one substituent selected from the group consisting of T, V and W represents the binding site of a single bond linking the first chemical moiety to the second chemical moiety.

According to the invention, T is hydrogen in case W is $R^T$ and V represents the binding site of a single bond linking the first chemical moiety and the second chemical moiety; and W is hydrogen in case T is $R^T$ and V represents the binding site of a single bond linking the first chemical moiety and the second chemical moiety.

According to the invention, exactly two substituents selected from the group consisting of $R^I$, $R^{II}$, $R^{III}$, $R^{IV}$ and $R^V$ are F.

In one embodiment, $R^1$ and $R^N$ is at each occurrence independently from another selected from the group consisting of H, methyl and phenyl.

In one embodiment, $R^N$ is Ph, which is optionally substituted with one or more substituents independently from each other selected from the group consisting of Me, $^iPr$, $^tBu$, CN, $CF_3$, and Ph.

In one embodiment, $R^N$ is Ph.

In one embodiment, X is $R^T$ and W represents the binding site of a single bond linking the first chemical moiety and the second chemical moiety.

In one embodiment of the invention, $R^{II}$ and $R^{IV}$ is F.

In one embodiment of the invention, $R^I$ and $R^{III}$ is F.

In one embodiment of the invention, $R^{III}$ and $R^V$ is F.

In one embodiment of the invention, $R^I$ and $R^{IV}$ is F.

In one embodiment of the invention, $R^{II}$ and $R^V$ is F.

In one embodiment of the invention, $R^I$ and $R^{II}$ is F.

In one embodiment of the invention, $R^{IV}$ and $R^V$ is F.

In one embodiment of the invention, $R^{II}$ and $R^{III}$ is F.

In one embodiment of the invention, $R^{III}$ and $R^{IV}$ is F.

In one embodiment of the invention, $R^{I}$ and $R^{V}$ is F.

In one embodiment of the invention, $R^{II}$ and $R^{V}$ is F.

In one embodiment of the invention, T is $R^{T}$.

In one embodiment of the invention, W is $R^{T}$.

In one embodiment of the invention, X is $R^{T}$.

In one embodiment of the invention, Y is $R^{T}$.

In one embodiment of the invention, exactly three substituents selected from the group consisting of $R^{I}$, $R^{II}$, $R^{III}$, $R^{IV}$ and $R^{V}$ are H.

In one embodiment of the invention, Y is $R^{T}$.

In one embodiment of the invention, exactly three substituents selected from the group consisting of $R^{I}$, $R^{II}$, $R^{III}$, $R^{IV}$ and $R^{V}$ are H.

According to the invention, the second chemical moiety consists of a structure of Formula IIa:

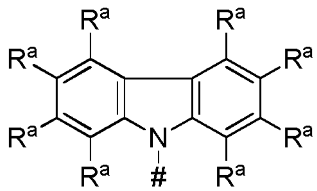

Formula IIa

wherein # and $R^a$ are defined as above.

In a further embodiment of the invention, $R^a$ is at each occurrence independently from another selected from the group consisting of
hydrogen,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph,
carbazolyl, which is optionally substituted with one or more substituents independently from each other selected from the group consisting of Me, $^{i}Pr$, $^{t}Bu$, CN, $CF_3$, and Ph,
triazinyl, which is optionally substituted with one or more substituents independently from each other selected from the group consisting of Me, $^{i}Pr$, $^{t}Bu$, CN, $CF_3$, and Ph,
and $N(Ph)_2$.

In a further embodiment of the invention, $R^a$ is at each occurrence independently from another selected from the group consisting of
hydrogen,
Me,
$^{i}Pr$,
$^{t}Bu$,
CN,
$CF_3$,
Ph, which is optionally substituted with one or more substituents independently from each other selected from the group consisting of Me, $^{i}Pr$, $^{t}Bu$, CN, $CF_3$, and Ph,
pyridinyl, which is optionally substituted with one or more substituents independently from each other selected from the group consisting of Me, $^{i}Pr$, $^{t}Bu$, CN, $CF_3$, and Ph,
pyrimidinyl, which is optionally substituted with one or more substituents independently from each other selected from the group consisting of Me, $^{i}Pr$, $^{t}Bu$, CN, $CF_3$, and Ph, and

triazinyl, which is optionally substituted with one or more substituents independently from each other selected from the group consisting of Me, $^{i}$Pr, $^{t}$Bu, CN, $CF_3$, and Ph.

In a further embodiment of the invention, the second chemical moiety consists of a structure of Formula IIb, a structure of Formula IIb-2, a structure of Formula IIb-3 or a structure of Formula IIb-4:

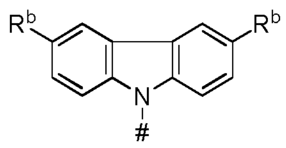

Formula IIb

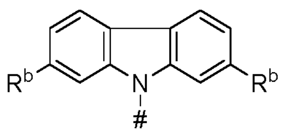

Formula IIb-2

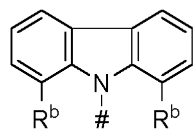

Formula IIb-3

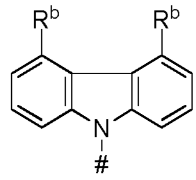

Formula IIb-4

wherein

$R^b$ is at each occurrence independently from another selected from the group consisting of deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,
which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C≡O, C=S, C=Se, $C=NR^5$, $P(=O)(R^5)$, SO, $SO_2$, $NR^5$, O, S or $CONR^5$;
$C_1$-$C_{40}$-alkoxy,
which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C≡O, C=S, C=Se, $C=NR^5$, $P(=O)(R^5)$, SO, $SO_2$, $NR^5$, O, S or $CONR^5$;
$C_1$-$C_{40}$-thioalkoxy,
which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C≡O, C=S, C=Se, $C=NR^5$, $P(=O)(R^5)$, SO, $SO_2$, $NR^5$, O, S or $CONR^5$;
$C_2$-$C_{40}$-alkenyl,
which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C≡O, C=S, C=Se, $C=NR^5$, $P(=O)(R^5)$, SO, $SO_2$, $NR^5$, O, S or $CONR^5$;
$C_2$-$C_{40}$-alkynyl,
which is optionally substituted with one or more substituents $R^5$ and wherein one or more non-adjacent $CH_2$-groups are optionally substituted by $R^5C=CR^5$, C≡C, $Si(R^5)_2$, $Ge(R^5)_2$, $Sn(R^5)_2$, C≡O, C=S, C=Se, $C=NR^5$, $P(=O)(R^5)$, SO, $SO_2$, $NR^5$, O, S or $CONR^5$;
$C_6$-$C_{60}$-aryl,
which is optionally substituted with one or more substituents $R^5$; and
$C_3$-$C_{57}$-heteroaryl,
which is optionally substituted with one or more substituents $R^5$.

Apart from that the aforementioned definitions apply.

In one additional embodiment of the invention, the second chemical moiety consists of a structure of Formula IIc, a structure of Formula IIc-2, a structure of Formula IIc-3 or a structure of Formula IIc-4:
wherein the aforementioned definitions apply.

In a further embodiment of the invention, $R^b$ is at each occurrence independently from another selected from the group consisting of

Me,
${}^{i}Pr$,
${}^{t}Bu$,
CN,
$CF_3$,
Ph, which is optionally substituted with one or more substituents independently from each other selected from the group consisting of Me, ${}^{i}Pr$, ${}^{t}Bu$, CN, $CF_3$, and Ph,
pyridinyl, which is optionally substituted with one or more substituents independently from each other selected from the group consisting of Me, ${}^{i}Pr$, ${}^{t}Bu$, CN, $CF_3$, and Ph,
pyrimidinyl, which is optionally substituted with one or more substituents independently from each other selected from the group consisting of Me, ${}^{i}Pr$, ${}^{t}Bu$, CN, $CF_3$, and Ph,
carbazolyl, which is optionally substituted with one or more substituents independently from each other selected from the group consisting of Me, ${}^{i}Pr$, ${}^{t}Bu$, CN, $CF_3$, and Ph,
triazinyl, which is optionally substituted with one or more substituents independently from each other selected from the group consisting of Me, ${}^{i}Pr$, ${}^{t}Bu$, CN, $CF_3$, and Ph,
and $N(Ph)_2$.

In a further embodiment of the invention, $R^b$ is at each occurrence independently from another selected from the group consisting of
Me,
${}^{i}Pr$,
${}^{t}Bu$,
CN,
$CF_3$,
Ph, which is optionally substituted with one or more substituents independently from each other selected from the group consisting of Me, ${}^{i}Pr$, ${}^{t}Bu$, CN, $CF_3$, and Ph,
pyridinyl, which is optionally substituted with one or more substituents independently from each other selected from the group consisting of Me, ${}^{i}Pr$, ${}^{t}Bu$, CN, $CF_3$, and Ph,
pyrimidinyl, which is optionally substituted with one or more substituents independently from each other selected from the group consisting of Me, ${}^{i}Pr$, ${}^{t}Bu$, CN, $CF_3$, and Ph, and
triazinyl, which is optionally substituted with one or more substituents independently from each other selected from the group consisting of Me, ${}^{i}Pr$, ${}^{t}Bu$, CN, $CF_3$, and Ph.
pyridinyl, which is optionally substituted with one or more substituents independently from each other selected from the group consisting of Me, ${}^{i}Pr$, ${}^{t}Bu$, CN, $CF_3$, and Ph,
pyrimidinyl, which is optionally substituted with one or more substituents independently from each other selected from the group consisting of Me, ${}^{i}Pr$, ${}^{t}Bu$, CN, $CF_3$, and Ph, and
triazinyl, which is optionally substituted with one or more substituents independently from each other selected from the group consisting of Me, ${}^{i}Pr$, ${}^{t}Bu$, CN, $CF_3$, and Ph.

In the following, exemplary embodiments of the second chemical moiety are shown:

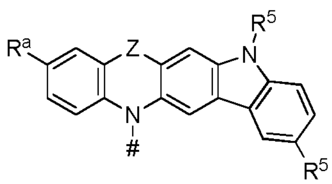
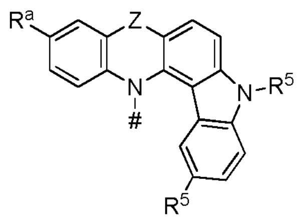
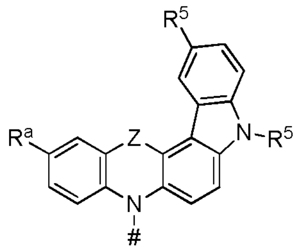
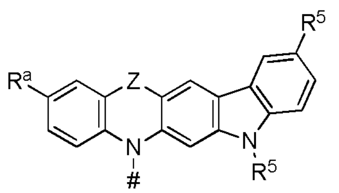
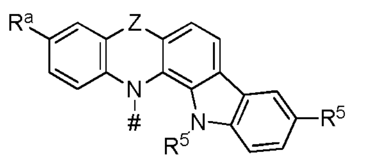
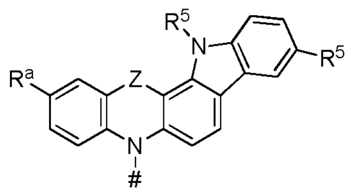

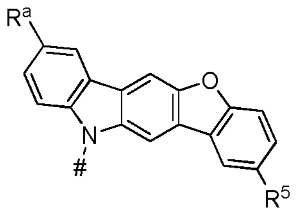 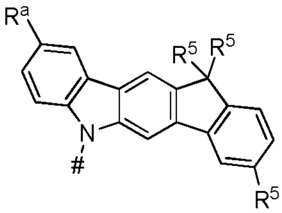 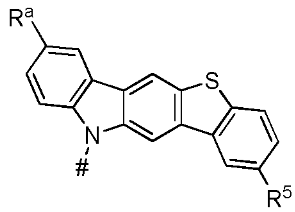

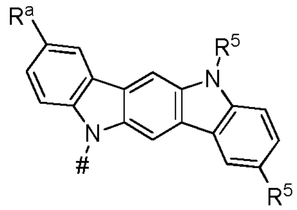

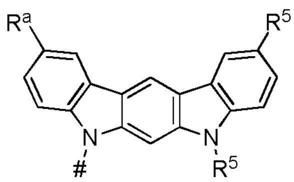 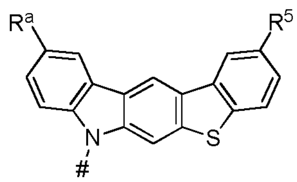 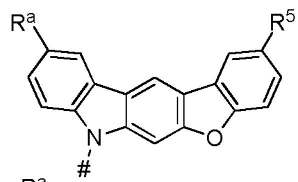

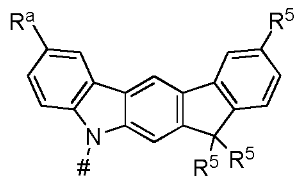 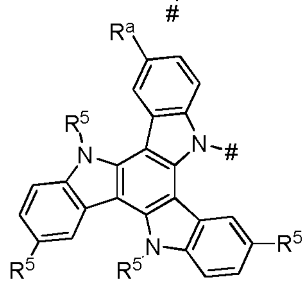

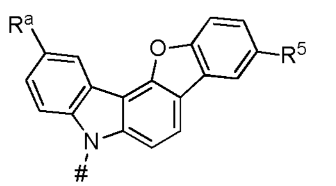 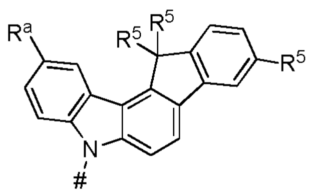 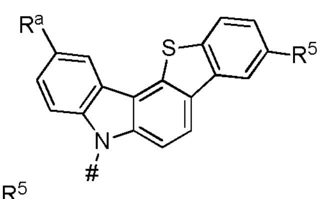

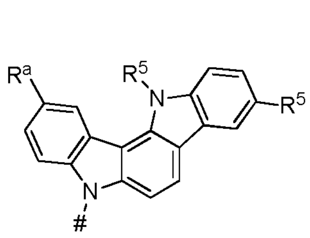 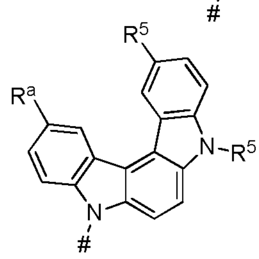

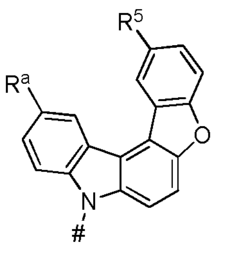 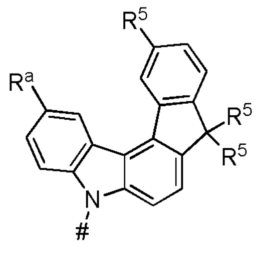 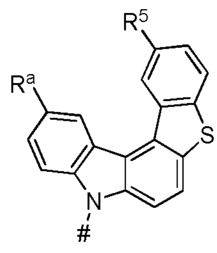

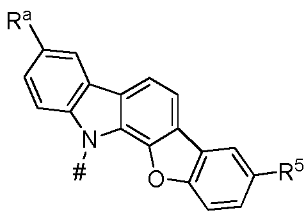
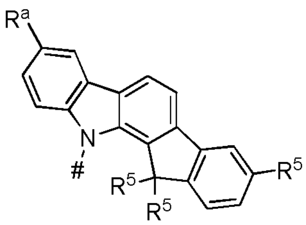
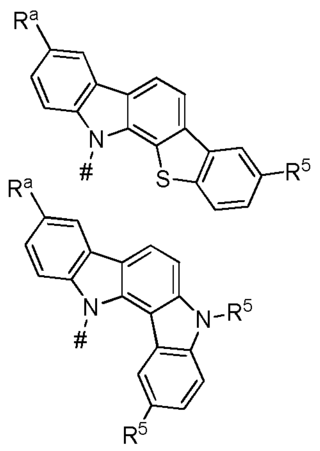
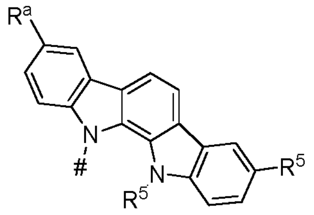

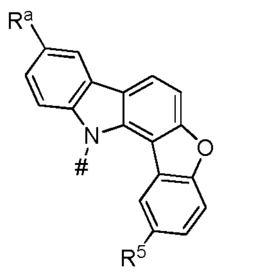
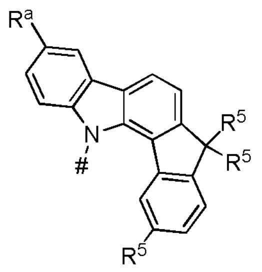
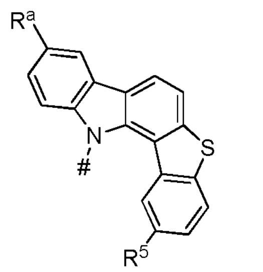

wherein for #, Z, $R^a$, $R^3$, $R^4$ and $R^5$ the aforementioned definitions apply.

In one embodiment, $R^a$ and $R^5$ is at each occurrence independently from another selected from the group consisting of hydrogen (H), methyl (Me), i-propyl ($CH(CH_3)_2$) ($^iPr$), t-butyl ($^tBu$), phenyl (Ph),
triazinyl, which is optionally substituted with one or more substituents independently from each other selected from the group consisting of Me, $^iPr$, $^tBu$, CN, $CF_3$, and Ph,
and diphenylamine ($NPh_2$).

In one embodiment of the invention, the organic molecules comprise or consist of a structure of Formula III-1, Formula III-2, Formula III-3, Formula III-4 or Formula III-5:

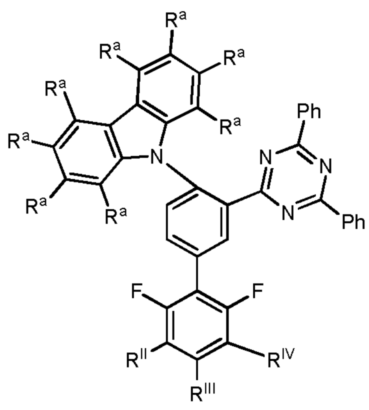
Formula III-1

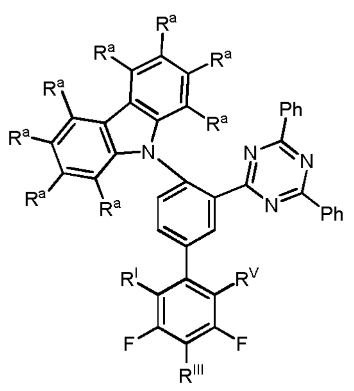
Formula III-2

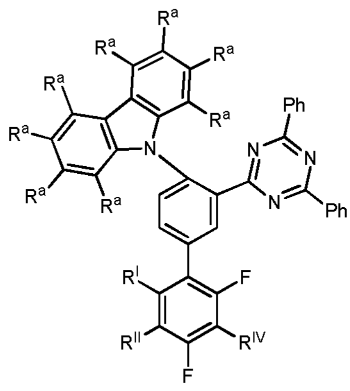
Formula III-3

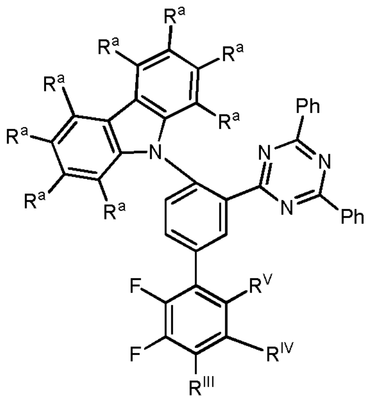 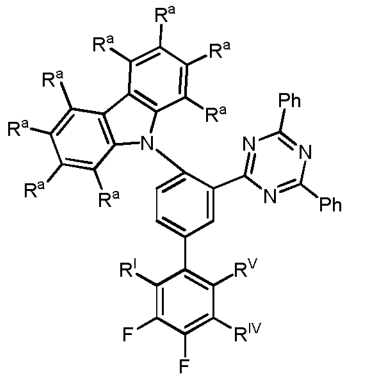

Formula III-4 Formula III-5

wherein the aforementioned definitions apply.

In a preferred embodiment, the organic molecules comprise or consist of a structure of Formula III-1.

In a further embodiment of the invention, the organic molecules comprise or consist of a structure of Formula IIIa-1, Formula IIIa-2, Formula IIIa-3, Formula IIIa-4 or Formula IIIa-5:

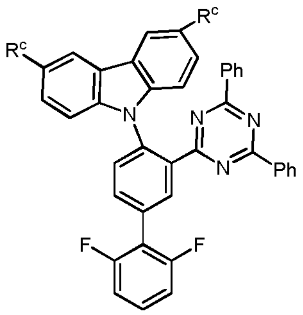 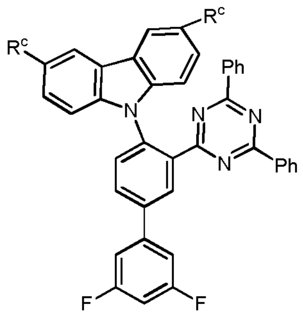 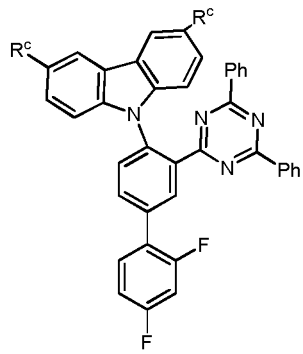

Formula IIIa-1 Formula IIIa-2 Formula IIIa-3

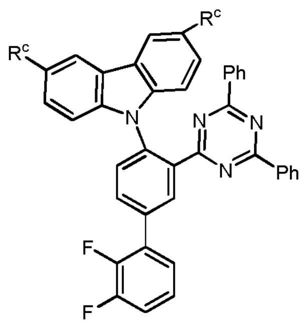 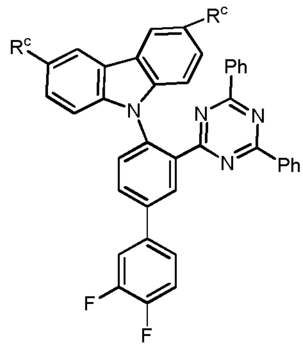

Formula IIIa-4 Formula IIIa-5

wherein

$R^c$ is at each occurrence independently from another selected from the group consisting of Me,
$^i$Pr,
$^t$Bu,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,

pyrimidinyl, which is optionally substituted with one or more substituents independently from each other selected from the group consisting of Me, $^{i}$Pr, $^{t}$Bu, CN, $CF_3$, and Ph,
carbazolyl, which is optionally substituted with one or more substituents independently from each other selected from the group consisting of Me, $^{i}$Pr, $^{t}$Bu, CN, $CF_3$, and Ph,
triazinyl, which is optionally substituted with one or more substituents independently from each other selected from the group consisting of Me, $^{i}$Pr, $^{t}$Bu, CN, $CF_3$, and Ph,
and $N(Ph)_2$.

In a preferred embodiment, the organic molecules comprise or consist of a structure of Formula IIIa-1.

In one additional embodiment of the invention, the organic molecules comprise or consist of a structure of Formula IIIb-1, Formula IIIb-2, Formula IIIb-3, Formula IIIb-4 or Formula IIIb-5:

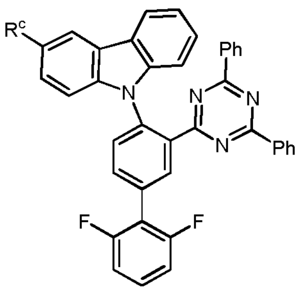

Formula IIIb-1

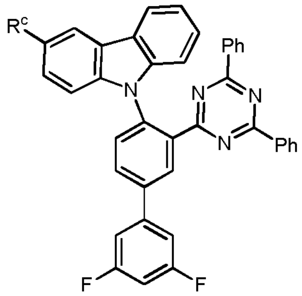

Formula IIIb-2

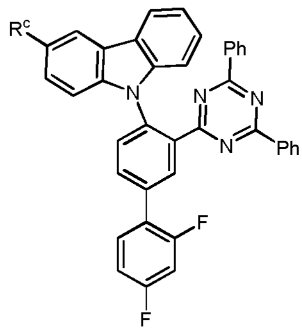

Formula IIIb-3

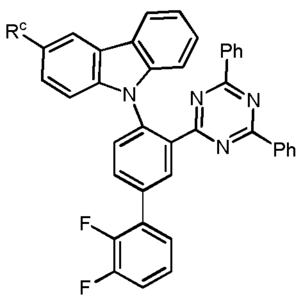

Formula IIIb-4

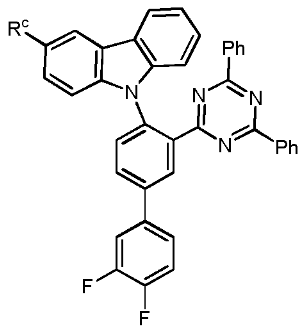

Formula IIIb-5

wherein the aforementioned definitions apply.

In a preferred embodiment, the organic molecules comprise or consist of a structure of Formula IIIb-1.

In one embodiment of the invention, the organic molecules comprise or consist of a structure of Formula IV-1, Formula IV-2, Formula IV-3, Formula IV-4 or Formula IV-5:

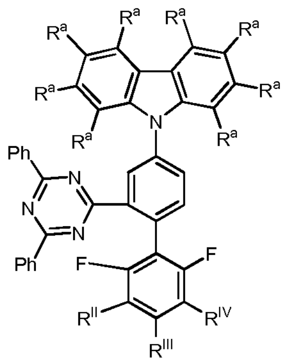

Formula IV-1

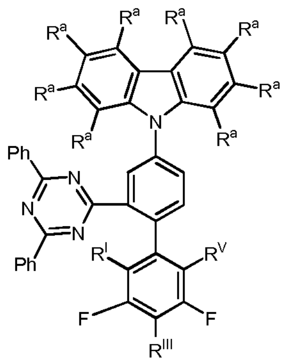

Formula IV-2

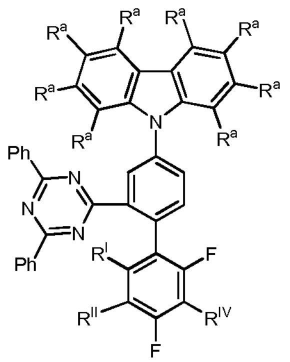

Formula IV-3

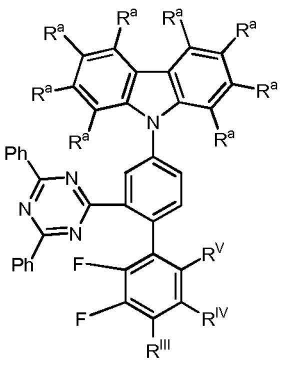

Formula IV-4

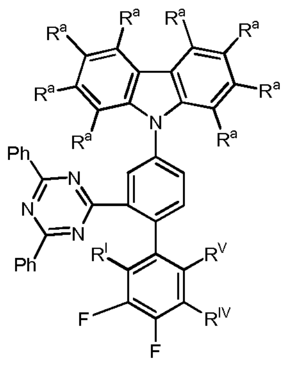

Formula IV-5

wherein the aforementioned definitions apply.

In a preferred embodiment, the organic molecules comprise or consist of a structure of Formula IV-1.

In a further embodiment of the invention, the organic molecules comprise or consist of a structure of Formula IVa-1, Formula IVa-2, Formula IVa-3, Formula IVa-4 or Formula IVa-5:

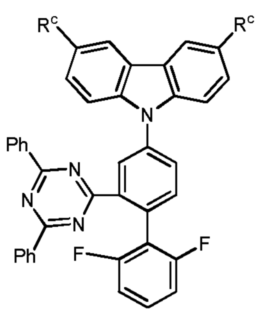

Formula IVa-1

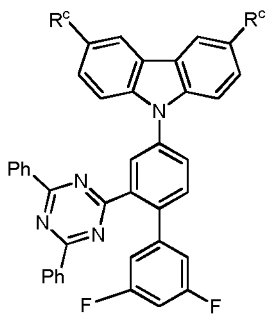

Formula IVa-2

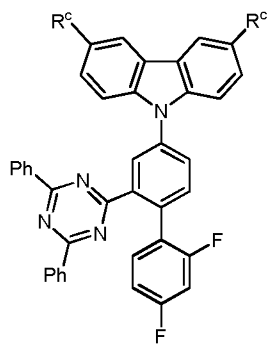

Formula IVa-3

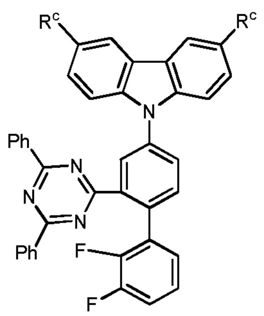

Formula IVa-4

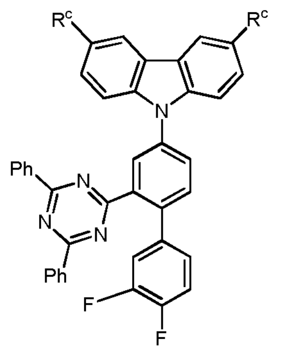

Formula IVa-5

wherein the aforementioned definitions apply.

In a preferred embodiment, the organic molecules comprise or consist of a structure of Formula IVa-1.

In one additional embodiment of the invention, the organic molecules comprise or consist of a structure of Formula IVb-1, Formula IVb-2, Formula IVb-3, Formula IVb-4 or Formula IVb-5:

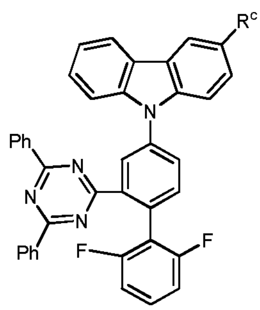

Formula IVb-1

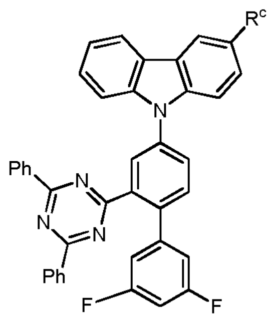

Formula IVb-2

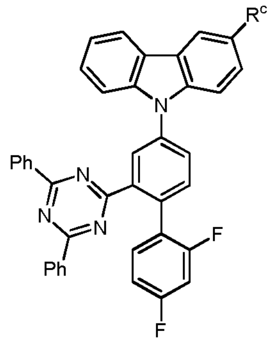

Formula IVb-3

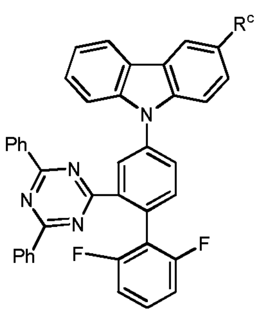

Formula IVb-4

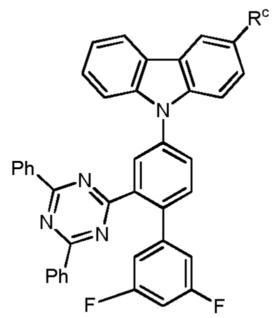

Formula IVb-5

wherein the aforementioned definitions apply.

In a preferred embodiment, the organic molecules comprise or consist of a structure of Formula IVb-1.

In one embodiment of the invention, the organic molecules comprise or consist of a structure of Formula V-1, Formula V-2, Formula V-3, Formula V-4 or Formula V-5:

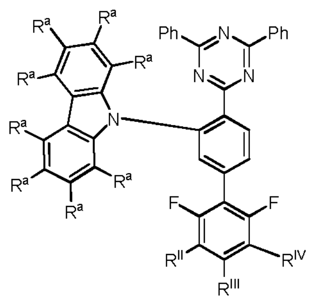

Formula V-1

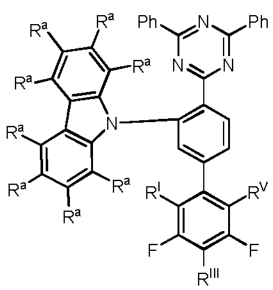

Formula V-2

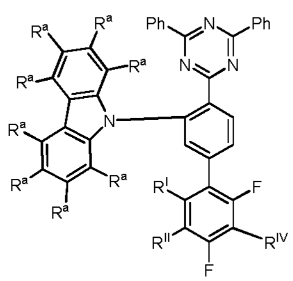

Formula V-3

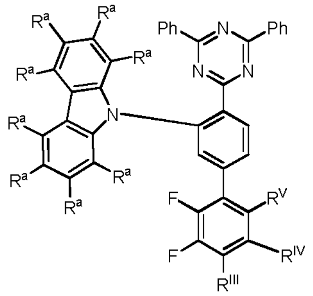

Formula V-4

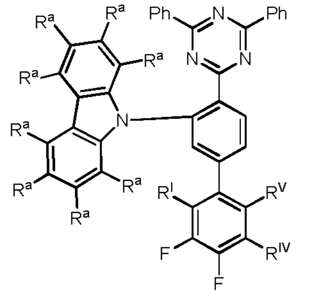

Formula V-5

wherein the aforementioned definitions apply.

In a preferred embodiment, the organic molecules comprise or consist of a structure of Formula V-1.

In a further embodiment of the invention, the organic molecules comprise or consist of a structure of Formula Va-1, Formula Va-2, Formula Va-3, Formula Va-4 or Formula Va-5:

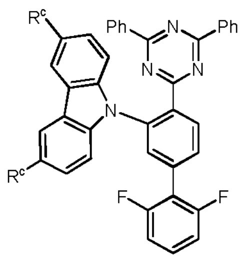
Formula Va-1

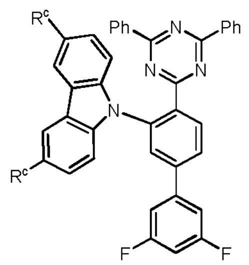
Formula Va-2

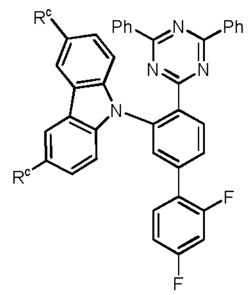
Formula Va-3

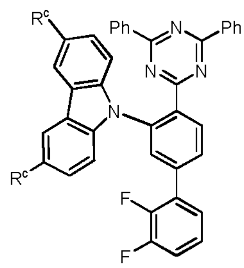
Formula Va-4

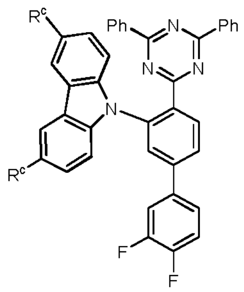
Formula Va-5

wherein the aforementioned definitions apply.

In a preferred embodiment, the organic molecules comprise or consist of a structure of Formula Va-1.

In one additional embodiment of the invention, the organic molecules comprise or consist of a structure of Formula Vb-1, Formula Vb-2, Formula Vb-3, Formula Vb-4 or Formula Vb-5:

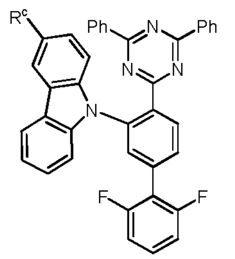
Formula Vb-1

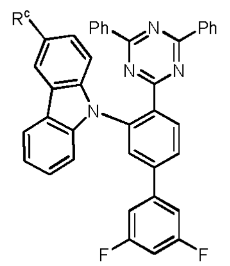
Formula Vb-2

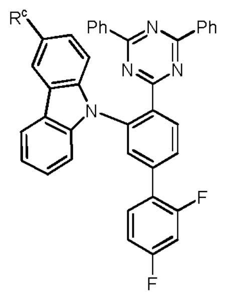
Formula Vb-3

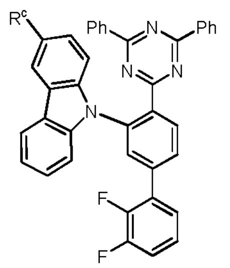
Formula Vb-4

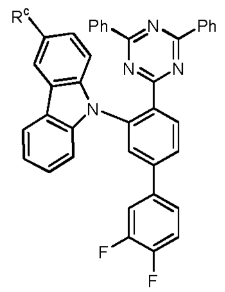
Formula Vb-5

wherein the aforementioned definitions apply.

In a preferred embodiment, the organic molecules comprise or consist of a structure of Formula Vb-1.

In one embodiment of the invention, the organic molecules comprise or consist of a structure of Formula VI-1, Formula VI-2, Formula VI-3, Formula VI-4 or Formula VI-5:

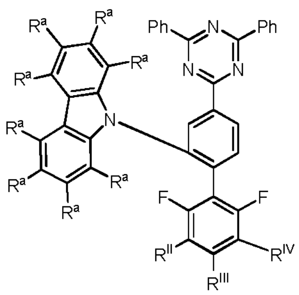

Formula VI-1

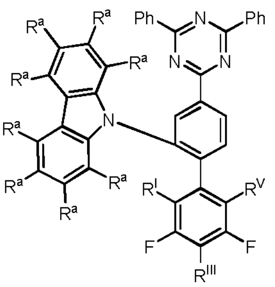

Formula VI-2

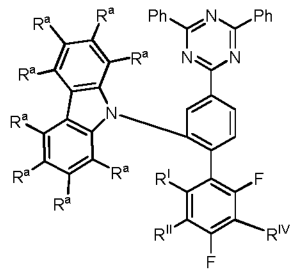

Formula VI-3

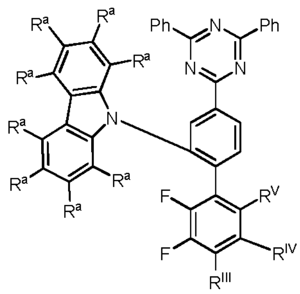

Formula VI-4

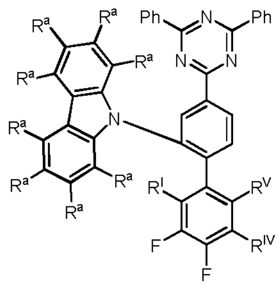

Formula VI-5

wherein the aforementioned definitions apply.

In a preferred embodiment, the organic molecules comprise or consist of a structure of Formula VI-1.

In a further embodiment of the invention, the organic molecules comprise or consist of a structure of Formula VIa-1, Formula VIa-2, Formula VIa-3, Formula VIa-4 or Formula VIa-5:

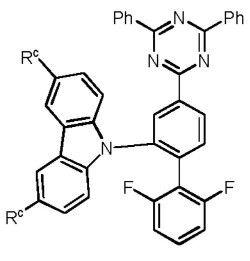

Formula VIa-1

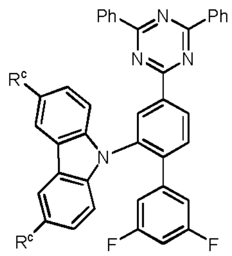

Formula VIa-2

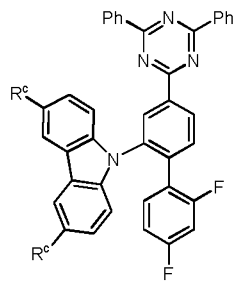

Formula VIa-3

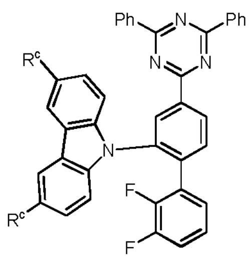

Formula VIa-4

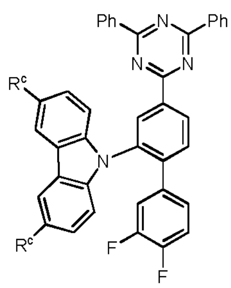

Formula VIa-5

wherein the aforementioned definitions apply.

In a preferred embodiment, the organic molecules comprise or consist of a structure of Formula VIa-1.

In one additional embodiment of the invention, the organic molecules comprise or consist of a structure of Formula VIb-1, Formula VIb-2, Formula VIb-3, Formula VIb-4 or Formula VIb-5:

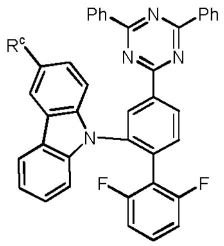

Formula VIb-1

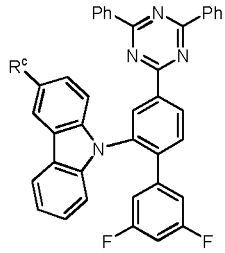

Formula VIb-2

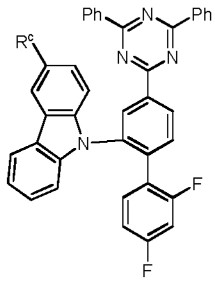

Formula VIb-3

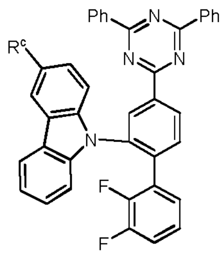

Formula VIb-4

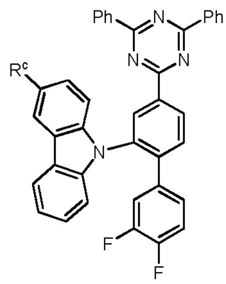

Formula VIb-5

wherein the aforementioned definitions apply.

In a preferred embodiment, the organic molecules comprise or consist of a structure of Formula VIb-1.

In one embodiment of the invention, the organic molecules comprise or consist of a structure of Formula VII-1, Formula VII-2, Formula VII-3, Formula VII-4 or Formula VII-5:

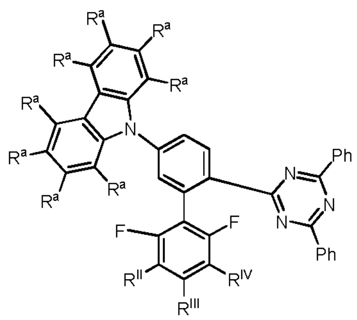

Formula VII-1

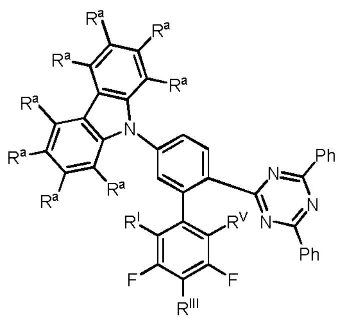

Formula VII-2

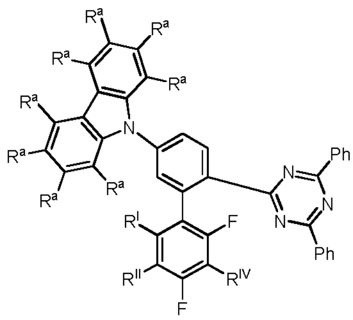

Formula VII-3

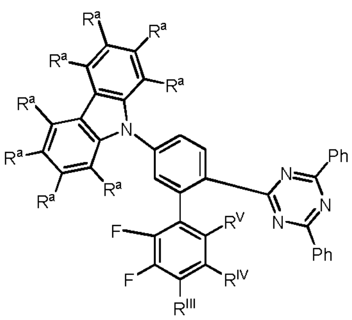

Formula VII-4

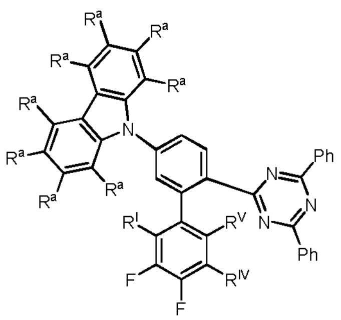

Formula VII-5

wherein the aforementioned definitions apply.

In a preferred embodiment, the organic molecules comprise or consist of a structure of Formula VII-1.

In a further embodiment of the invention, the organic molecules comprise or consist of a structure of Formula VIIa-1, Formula VIIa-2, Formula VIIa-3, Formula VIIa-4 or Formula VIIa-5:

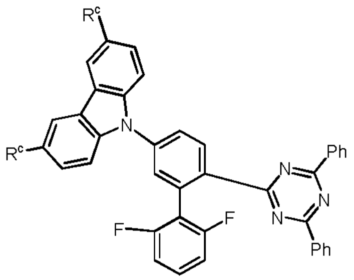

Formula VIIa-1

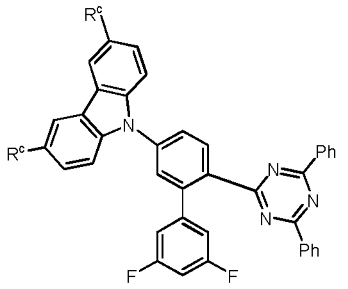

Formula VIIa-2

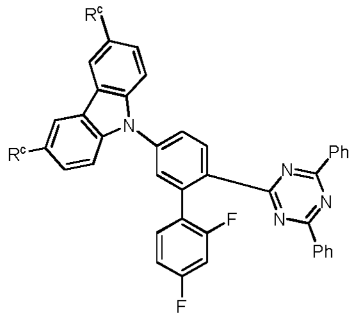

Formula VIIa-3

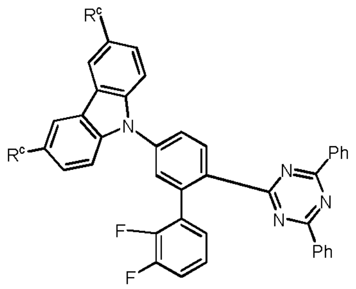

Formula VIIa-4

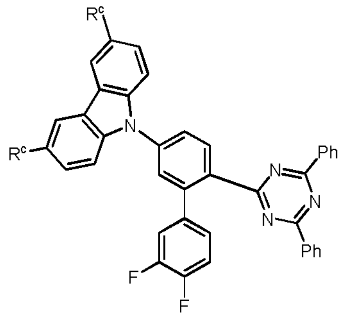

Formula VIIa-5

wherein the aforementioned definitions apply.

In a preferred embodiment, the organic molecules comprise or consist of a structure of Formula VIIa-1.

In one additional embodiment of the invention, the organic molecules comprise or consist of a structure of Formula VIIb-1, Formula VIIb-2, Formula VIIb-3, Formula VIIb-4 or Formula VIIb-5:

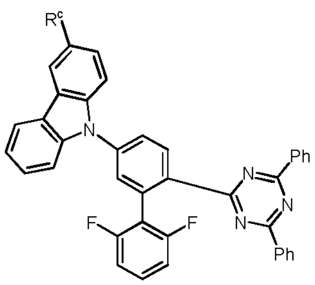

Formula VIIb-1

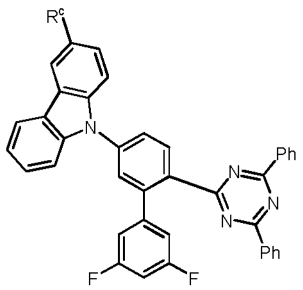

Formula VIIb-2

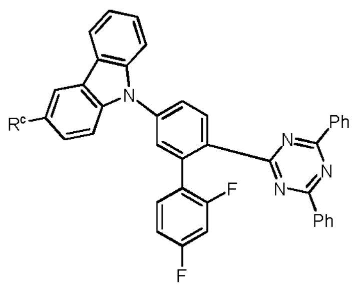

Formula VIIb-3

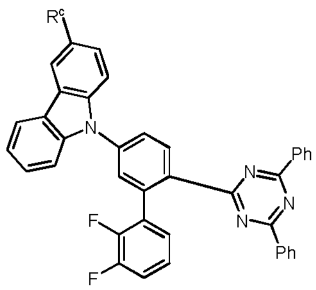

Formula VIIb-4

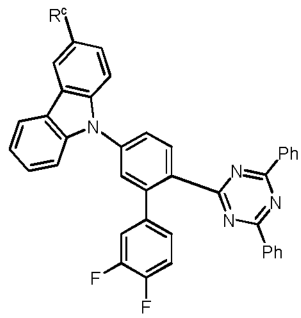

Formula VIIb-5

wherein the aforementioned definitions apply.

In a preferred embodiment, the organic molecules comprise or consist of a structure of Formula VIIb-1.

In one embodiment of the invention, the organic molecules comprise or consist of a structure of Formula VIII-1, Formula VIII-2, Formula VIII-3, Formula VIII-4 or Formula VIII-5:

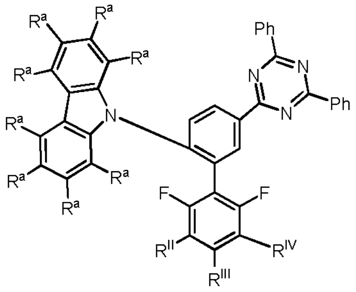

Formula VIII-1

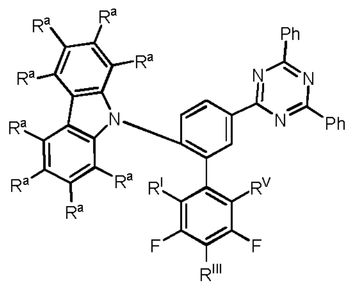

Formula VIII-2

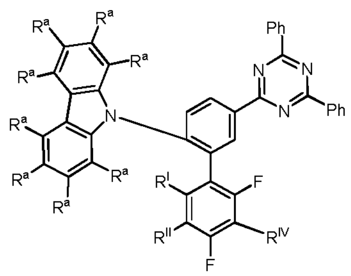

Formula VIII-3

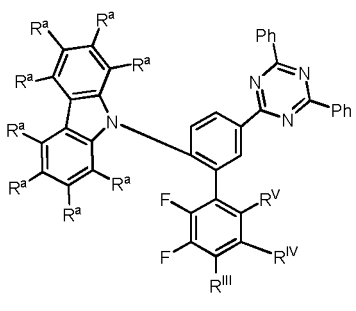

Formula VIII-4

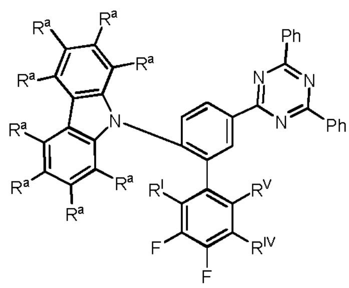

Formula VIII-5

wherein the aforementioned definitions apply.

In a preferred embodiment, the organic molecules comprise or consist of a structure of Formula VIII-1.

In a further embodiment of the invention, the organic molecules comprise or consist of a structure of Formula VIIIa-1, Formula VIIIa-2, Formula VIIIa-3, Formula VIIIa-4 or Formula VIIIa-5:

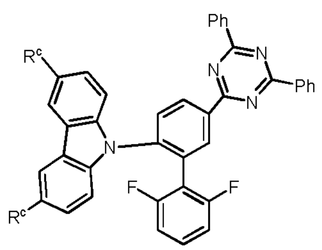

Formula VIIIa-1

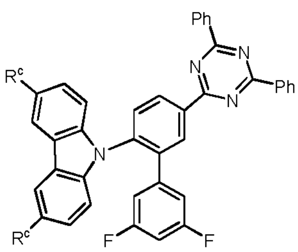

Formula VIIIa-2

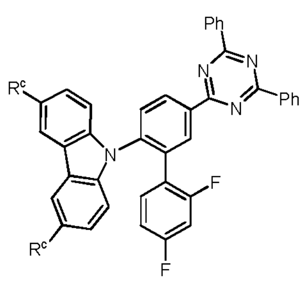

Formula VIIIa-3

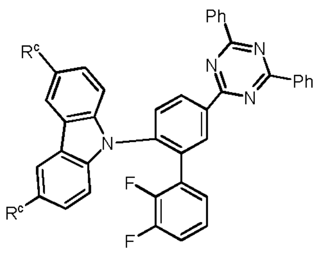

Formula VIIIa-4

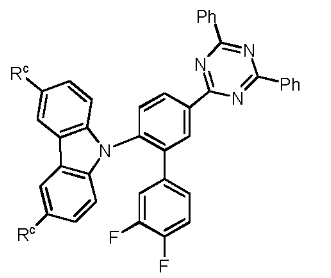

Formula VIIIa-5

wherein the aforementioned definitions apply.

In a preferred embodiment, the organic molecules comprise or consist of a structure of Formula VIIIa-1.

In one additional embodiment of the invention, the organic molecules comprise or consist of a structure of Formula VIIIb-1, Formula VIIIb-2, Formula VIIIb-3, Formula VIIIb-4 or Formula VIIIb-5:

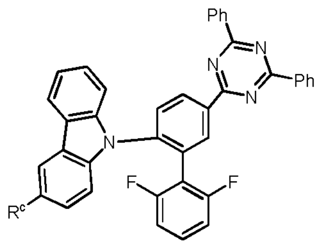

Formula VIIIb-1

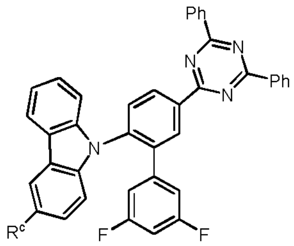

Formula VIIIb-2

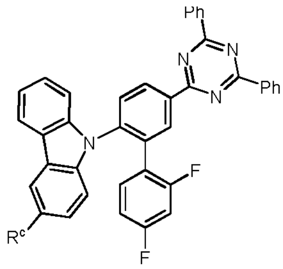

Formula VIIIb-3

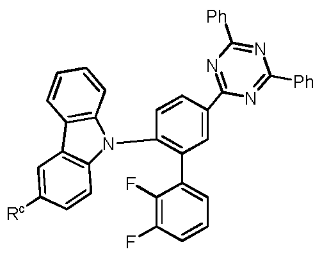

Formula VIIIb-4

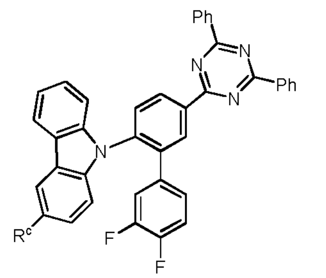

Formula VIIIb-5

wherein the aforementioned definitions apply.

In a preferred embodiment, the organic molecules comprise or consist of a structure of Formula VIIIb-1.

In one embodiment of the invention, the organic molecules comprise or consist of a structure of Formula IX-1, Formula IX-2, Formula IX-3, Formula IX-4 or Formula IX-5:

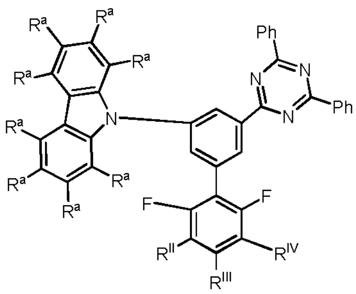

Formula IX-1

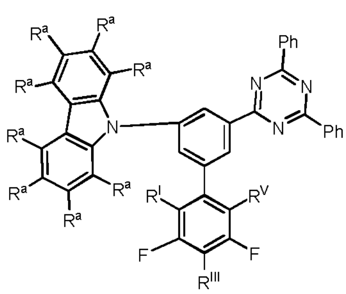

Formula IX-2

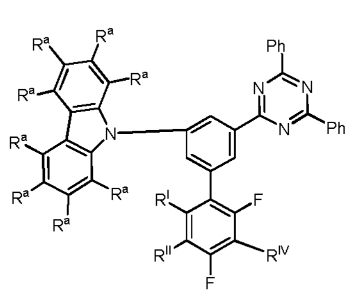

Formula IX-3

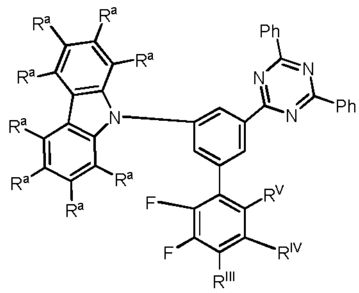

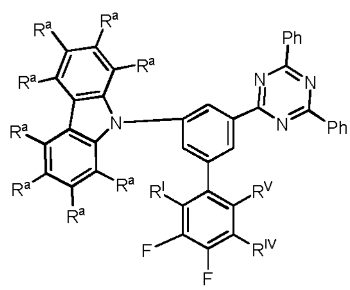

Formula IX-4 Formula IX-5

wherein the aforementioned definitions apply.

In a preferred embodiment, the organic molecules comprise or consist of a structure of Formula IX-1.

In a further embodiment of the invention, the organic molecules comprise or consist of a structure of Formula IXa-1, Formula IXa-2, Formula IXa-3, Formula IXa-4 or Formula IXa-5:

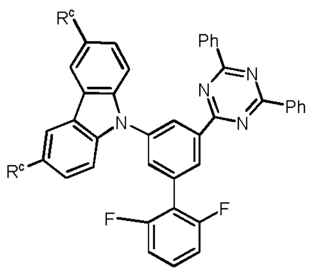

Formula IXa-1

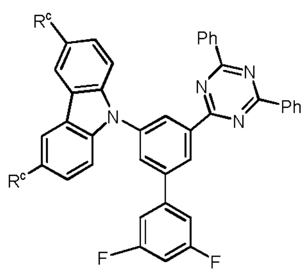

Formula IXa-2

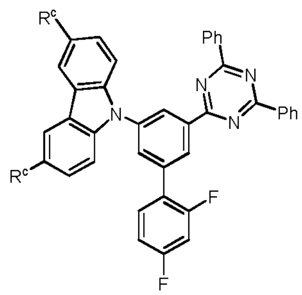

Formula IXa-3

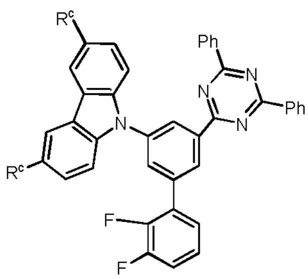

Formula IXa-4

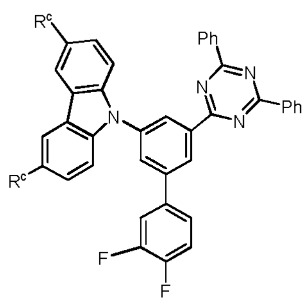

Formula IXa-5

wherein the aforementioned definitions apply.

In a preferred embodiment, the organic molecules comprise or consist of a structure of Formula IXa-1.

In one additional embodiment of the invention, the organic molecules comprise or consist of a structure of Formula IXb-1, Formula IXb-2, Formula IXb-3, Formula IXb-4 or Formula IXb-5:

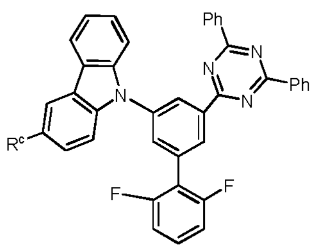

Formula IXb-1

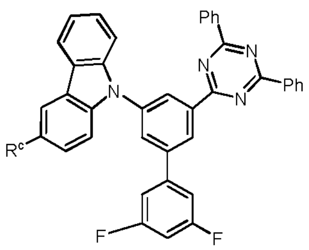

Formula IXb-2

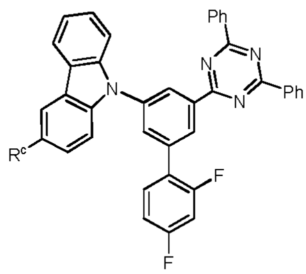

Formula IXb-3

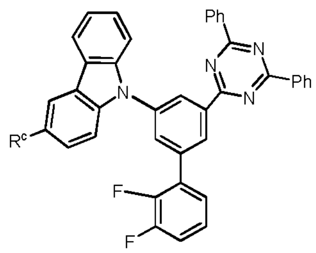

Formula IXb-4

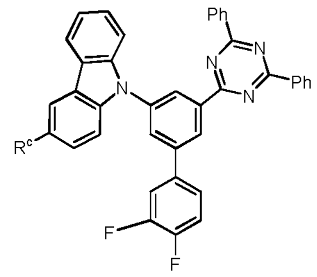

Formula IXb-5

wherein the aforementioned definitions apply.

In a preferred embodiment, the organic molecules comprise or consist of a structure of Formula IXb-1.

In one embodiment of the invention, the organic molecules comprise or consist of a structure of Formula X-1, Formula X-2, Formula X-3, Formula X-4 or Formula X-5:

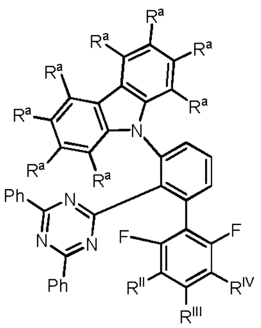

Formula X-1

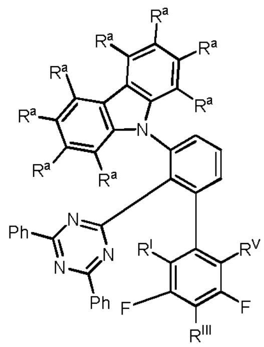

Formula X-2

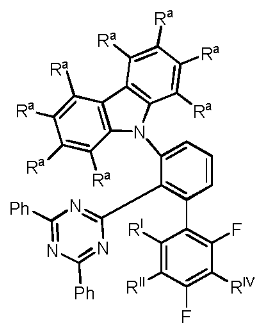

Formula X-3

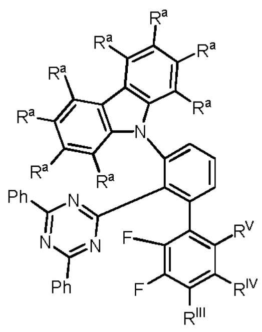

Formula X-4

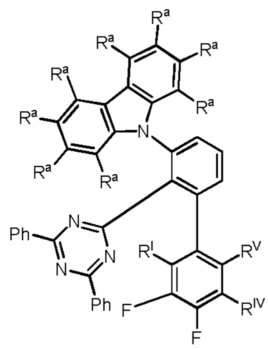

Formula X-5

wherein the aforementioned definitions apply.

In a preferred embodiment, the organic molecules comprise or consist of a structure of Formula X-1.

In a further embodiment of the invention, the organic molecules comprise or consist of a structure of Formula Xa-1, Formula Xa-2, Formula Xa-3, Formula Xa-4 or Formula Xa-5:

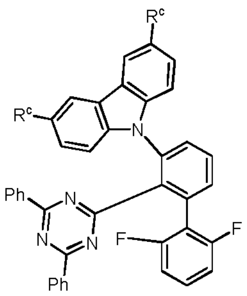

Formula Xa-1

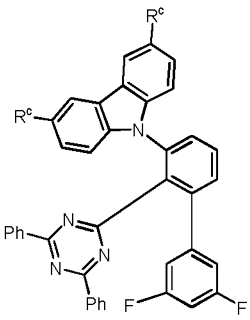

Formula Xa-2

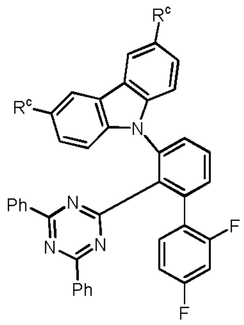

Formula Xa-3

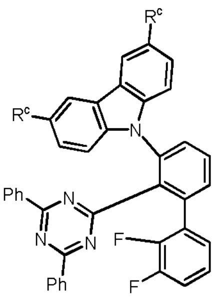

Formula Xa-4

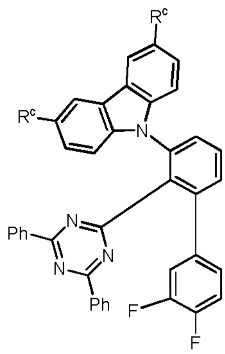

Formula Xa-5

wherein the aforementioned definitions apply.

In a preferred embodiment, the organic molecules comprise or consist of a structure of Formula Xa-1.

In one additional embodiment of the invention, the organic molecules comprise or consist of a structure of Formula Xb-1, Formula Xb-2, Formula Xb-3, Formula Xb-4 or Formula Xb-5:

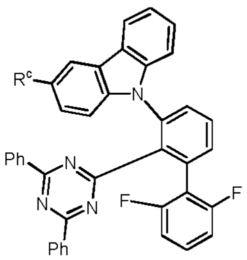

Formula Xb-1

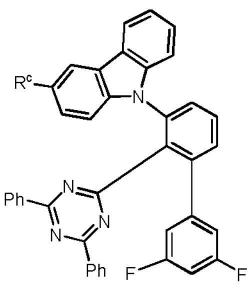

Formula Xb-2

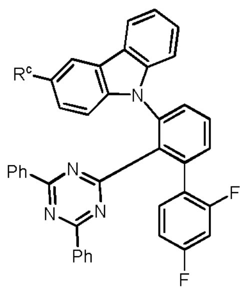

Formula Xb-3

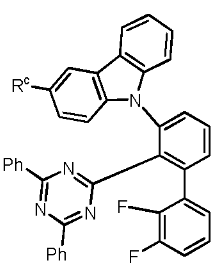

Formula Xb-4

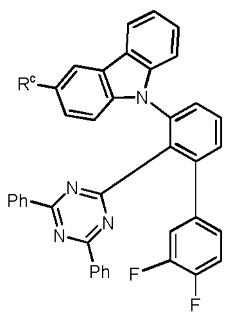

Formula Xb-5

wherein the aforementioned definitions apply.

In a preferred embodiment, the organic molecules comprise or consist of a structure of Formula Xb-1.

In one embodiment of the invention, the organic molecules comprise or consist of a structure of Formula XI-1, Formula XI-2, Formula XI-3, Formula XI-4 or Formula XI-5:

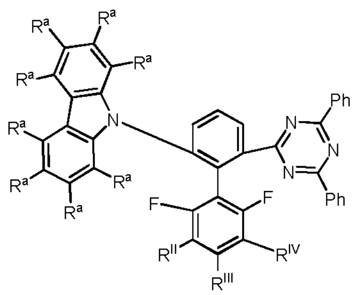

Formula XI-1

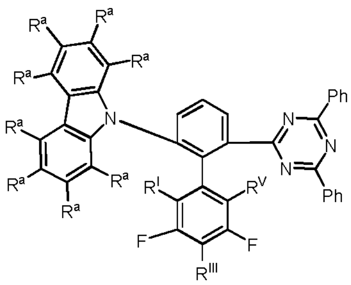

Formula XI-2

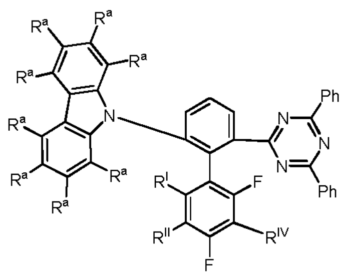

Formula XI-3

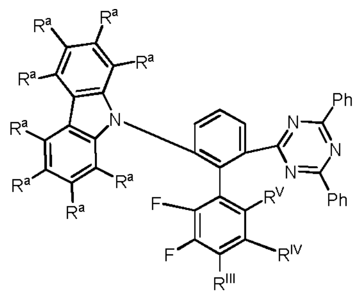

Formula XI-4

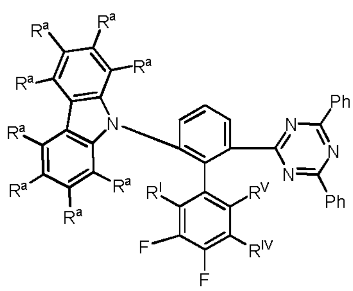

Formula XI-5

wherein the aforementioned definitions apply.

In a preferred embodiment, the organic molecules comprise or consist of a structure of Formula XI-1.

In a further embodiment of the invention, the organic molecules comprise or consist of a structure of Formula XIa-1, Formula XIa-2, Formula XIa-3, Formula XIa-4 or Formula XIa-5:

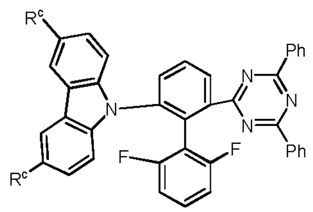

Formula XIa-1

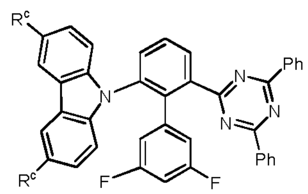

Formula XIa-2

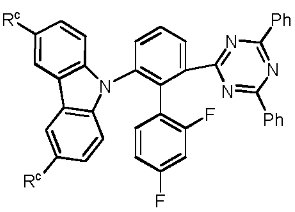

Formula XIa-3

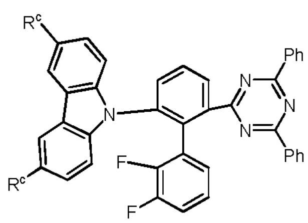

Formula XIa-4

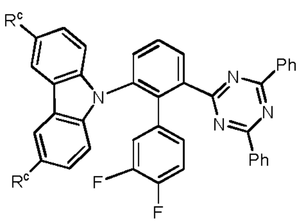

Formula XIa-5

wherein the aforementioned definitions apply.

In a preferred embodiment, the organic molecules comprise or consist of a structure of Formula XIa-1.

In one additional embodiment of the invention, the organic molecules comprise or consist of a structure of Formula XIb-1, Formula XIb-2, Formula XIb-3, Formula XIb-4 or Formula XIb-5:

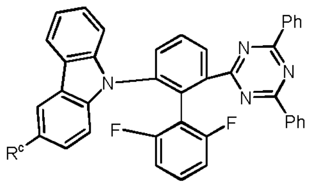

Formula XIb-1

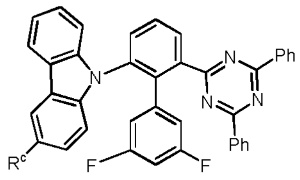

Formula XIb-2

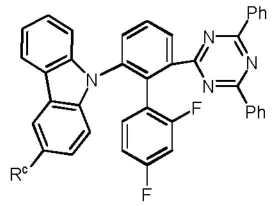

Formula XIb-3

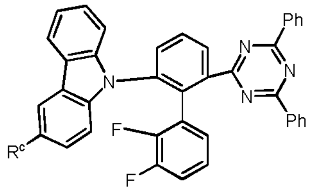

Formula XIb-4

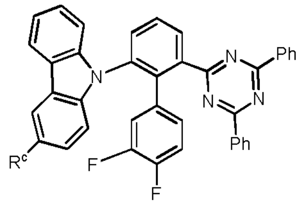

Formula XIb-5

wherein the aforementioned definitions apply.

In a preferred embodiment, the organic molecules comprise or consist of a structure of Formula XIb-1.

In one embodiment of the invention, $R^c$ is at each occurrence independently from another selected from the group consisting of

Me,
$^i$Pr,
$^t$Bu,
CN,
$CF_3$,
Ph, which is optionally substituted with one or more substituents independently from each other selected from the group consisting of Me, $^i$Pr, $^t$Bu, CN, $CF_3$, and Ph,
pyridinyl, which is optionally substituted with one or more substituents independently from each other selected from the group consisting of Me, $^i$Pr, $^t$Bu, CN, $CF_3$, and Ph,
pyrimidinyl, which is optionally substituted with one or more substituents independently from each other selected from the group consisting of Me, $^i$Pr, $^t$Bu, CN, $CF_3$, and Ph, and
triazinyl, which is optionally substituted with one or more substituents independently from each other selected from the group consisting of Me, $^i$Pr, $^t$Bu, CN, $CF_3$, and Ph.

In one embodiment of the invention, $R^c$ is at each occurrence independently from another selected from the group consisting of

Me,
$^i$Pr,
$^t$Bu,
Ph, which is optionally substituted with one or more substituents independently from each other selected from the group consisting of Me, $^i$Pr, $^t$Bu, CN, $CF_3$ and Ph; and
triazinyl, which is optionally substituted with one or more substituents independently from each other selected from the group consisting of Me, $^i$Pr, $^t$Bu, CN, $CF_3$ and Ph.

As used throughout the present application, the terms "aryl" and "aromatic" may be understood in the broadest sense as any mono-, bi- or polycyclic aromatic moieties. Accordingly, an aryl group contains 6 to 60 aromatic ring atoms, and a heteroaryl group contains 5 to 60 aromatic ring atoms, of which at least one is a heteroatom. Notwithstanding, throughout the application the number of aromatic ring atoms may be given as subscripted number in the definition of certain substituents. In particular, the heteroaromatic ring includes one to three heteroatoms. Again, the terms "heteroaryl" and "heteroaromatic" may be understood in the broadest sense as any mono-, bi- or polycyclic hetero-aromatic moieties that include at least one heteroatom. The heteroatoms may at each occurrence be the same or different and be individually selected from the group consisting of N, O and S. Accordingly, the term "arylene" refers to a divalent substituent that bears two binding sites to other molecular structures and thereby serving as a linker structure. In case, a group in the exemplary embodiments is defined differently from the definitions given here, for example, the number of aromatic ring atoms or number of heteroatoms differs from the given definition, the definition in the exemplary embodiments is to be applied. According to the invention, a condensed (annulated) aromatic or heteroaromatic polycycle is built of two or more

single aromatic or heteroaromatic cycles, which formed the polycycle via a condensation reaction.

In particular, as used throughout the present application the term aryl group or heteroaryl group comprises groups which can be bound via any position of the aromatic or heteroaromatic group, derived from benzene, naphthaline, anthracene, phenanthrene, pyrene, dihydropyrene, chrysene, perylene, fluoranthene, benzanthracene, benzphenanthrene, tetracene, pentacene, benzpyrene, furan, benzofuran, isobenzofuran, dibenzofuran, thiophene, benzothiophene, isobenzothiophene, dibenzothiophene; pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthoimidazole, phenanthroimidazole, pyridoimidazole, pyrazinoimidazole, quinoxalinoimidazole, oxazole, benzoxazole, napthooxazole, anthroxazol, phenanthroxazol, isoxazole, 1,2-thiazole, 1,3-thiazole, benzothiazole, pyridazine, benzopyridazine, pyrimidine, benzopyrimidine, 1,3,5-triazine, quinoxaline, pyrazine, phenazine, naphthyridine, carboline, benzocarboline, phenanthroline, 1,2,3-triazole, 1,2,4-triazole, benzotriazole, 1,2,3-oxadiazole, 1,2,4-oxadiazole, 1,2,5-oxadiazole, 1,2,3,4-tetrazine, purine, pteridine, indolizine und benzothiadiazole or combinations of the abovementioned groups.

As used throughout the present application the term cyclic group may be understood in the broadest sense as any mono-, bi- or polycyclic moieties.

As used throughout the present application the term alkyl group may be understood in the broadest sense as any linear, branched, or cyclic alkyl substituent. In particular, the term alkyl comprises the substituents methyl (Me), ethyl (Et), n-propyl ($^{n}$Pr), i-propyl ($^{i}$Pr), cyclopropyl, n-butyl ($^{n}$Bu), i-butyl ($^{i}$Bu), s-butyl ($^{s}$Bu), t-butyl($^{t}$Bu), cyclobutyl, 2-methylbutyl, n-pentyl, s-pentyl, t-pentyl, 2-pentyl, neo-pentyl, cyclopentyl, n-hexyl, s-hexyl, t-hexyl, 2-hexyl, 3-hexyl, neohexyl, cyclohexyl, 1-methylcyclopentyl, 2-methylpentyl, n-heptyl, 2-heptyl, 3-heptyl, 4-heptyl, cycloheptyl, 1-methylcyclohexyl, n-octyl, 2-ethylhexyl, cyclooctyl, 1-bicyclo[2,2,2]octyl, 2-bicyclo[2,2,2]-octyl, 2-(2,6-dimethyl)octyl, 3-(3,7-dimethyl)octyl, adamantyl, 2,2,2-trifluorethyl, 1,1-dimethyl-n-hex-1-yl, 1,1-dimethyl-n-hept-1-yl, 1,1-dimethyl-n-oct-1-yl, 1,1-dimethyl-n-dec-1-yl, 1,1-dimethyl-n-dodec-1-yl, 1,1-dimethyl-n-tetradec-1-yl, 1,1-dimethyl-n-hexadec-1-yl, 1,1-dimethyl-n-octadec-1-yl, 1,1-diethyl-n-hex-1-yl, 1,1-diethyl-n-hept-1-yl, 1,1-diethyl-n-oct-1-yl, 1,1-diethyl-n-dec-1-yl, 1,1-diethyl-n-dodec-1-yl, 1,1-diethyl-n-tetradec-1-yl, 1,1-diethyln-n-hexadec-1-yl, 1,1-diethyl-n-octadec-1-yl, 1-(n-propyl)-cyclohex-1-yl, 1-(n-butyl)-cyclohex-1-yl, 1-(n-hexyl)-cyclohex-1-yl, 1-(n-octyl)-cyclohex-1-yl und 1-(n-decyl)-cyclohex-1-yl.

As used throughout the present application the term alkenyl comprises linear, branched, and cyclic alkenyl substituents. The term alkenyl group exemplarily comprises the substituents ethenyl, propenyl, butenyl, pentenyl, cyclopentenyl, hexenyl, cyclohexenyl, heptenyl, cycloheptenyl, octenyl, cyclooctenyl or cyclooctadienyl.

As used throughout the present application the term alkynyl comprises linear, branched, and cyclic alkynyl substituents. The term alkynyl group exemplarily comprises ethynyl, propynyl, butynyl, pentynyl, hexynyl, heptynyl or octynyl.

As used throughout the present application the term alkoxy comprises linear, branched, and cyclic alkoxy substituents. The term alkoxy group exemplarily comprises methoxy, ethoxy, n-propoxy, i-propoxy, n-butoxy, i-butoxy, s-butoxy, t-butoxy and 2-methylbutoxy.

As used throughout the present application the term thioalkoxy comprises linear, branched, and cyclic thioalkoxy substituents, in which the O of the exemplarily alkoxy groups is replaced by S.

As used throughout the present application, the terms "halogen" and "halo" may be understood in the broadest sense as being preferably fluorine, chlorine, bromine or iodine.

Whenever hydrogen is mentioned herein, it could also be replaced by deuterium at each occurrence.

It is understood that when a molecular fragment is described as being a substituent or otherwise attached to another moiety, its name may be written as if it were a fragment (e.g. naphtyl, dibenzofuryl) or as if it were the whole molecule (e.g. naphthalene, dibenzofuran). As used herein, these different ways of designating a substituent or attached fragment are considered to be equivalent.

In one embodiment, the organic molecules according to the invention have an excited state lifetime of not more than 150 $\mu$s, of not more than 100 $\mu$s, in particular of not more than 50 $\mu$s, more preferably of not more than 10 $\mu$s or not more than 7 $\mu$s in a film of poly(methyl methacrylate) (PMMA) with 10% by weight of organic molecule at room temperature.

In one embodiment of the invention, the organic molecules according to the invention represent thermally-activated delayed fluorescence (TADF) emitters, which exhibit a $\Delta E_{ST}$ value, which corresponds to the energy difference between the first excited singlet state (S1) and the first excited triplet state (T1), of less than 5000 $cm^{-1}$, preferably less than 3000 $cm^{-1}$, more preferably less than 1500 $cm^{-1}$, even more preferably less than 1000 $cm^{-1}$ or even less than 500 $cm^{-1}$.

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10% by weight of organic molecule at room temperature.

In a further embodiment of the invention, the organic molecules according to the invention have a "blue material

index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

In a further embodiment of the invention, the organic molecules according to the invention have a highest occupied molecular orbital with the energy $E^{HOMO}$, which is higher in energy than -6.2 eV, preferably higher in energy than -6.1 eV and even more preferably higher in energy than -6.0 eV or even -5.9 eV.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is determined as the onset of the absorption spectrum.

The onset of an absorption spectrum is determined by computing the intersection of the tangent to the absorption spectrum with the x-axis. The tangent to the absorption spectrum is

In a further embodiment of the invention, the organic molecules according to the invention have an emission peak in the visible or nearest ultraviolet range, i.e., in the range of a wavelength of from 380 to 800 nm, with a full width at half maximum of less than 0.50 eV, preferably less than 0.48 eV, more preferably less than 0.45 eV, even more preferably less than 0.43 eV or even less than 0.40 eV in a film of poly(methyl methacrylate) (PMMA) with 10% by weight of organic molecule at room temperature.

In a further embodiment of the invention, the organic molecules according to the invention have a "blue material index" (BMI), calculated by dividing the photoluminescence quantum yield (PLQY) in % by the CIEy color coordinate of the emitted light, of more than 150, in particular more than 200, preferably more than 250, more preferably of more than 300 or even more than 500.

In a further embodiment of the invention, the organic molecules according to the invention have a highest occupied molecular orbital with the energy $E^{HOMO}$, which is higher in energy than -6.2 eV, preferably higher in energy than -6.1 eV and even more preferably higher in energy than -6.0 eV or even -5.9 eV.

Orbital and excited state energies can be determined either by means of experimental methods or by calculations employing quantum-chemical methods, in particular density functional theory calculations. The energy of the highest occupied molecular orbital $E^{HOMO}$ is determined by methods known to the person skilled in the art from cyclic voltammetry measurements with an accuracy of 0.1 eV. The energy of the lowest unoccupied molecular orbital $E^{LUMO}$ is determined as the onset of the absorption spectrum.

The onset of an absorption spectrum is determined by computing the intersection of the tangent to the absorption spectrum with the x-axis. The tangent to the absorption spectrum is set at the low-energy side of the absorption band and at the point at half maximum of the maximum intensity of the absorption spectrum.

The energy of the first excited triplet state T1 is determined from the onset of the emission spectrum at low temperature, typically at 77 K. For host compounds, where the first excited singlet state and the lowest triplet state are energetically separated by > 0.4 eV, the phosphorescence is usually visible in a steady-state spectrum in 2-Me-THF. The triplet energy can thus be determined as the onset of the phosphorescence spectrum. For TADF emitter molecules, the energy of the first excited triplet state T1 is determined from the onset of the delayed emission spectrum at 77 K, if not otherwise stated measured in a film of) PMMA with

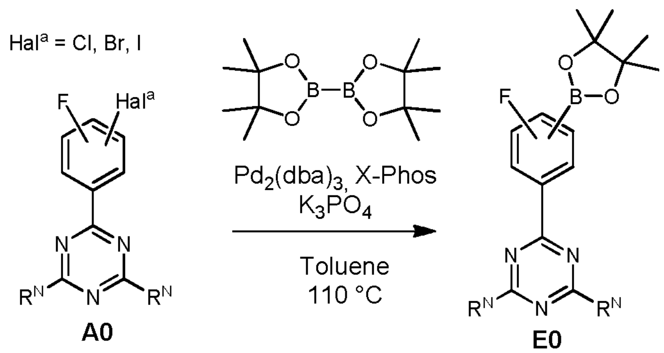


A0 E0

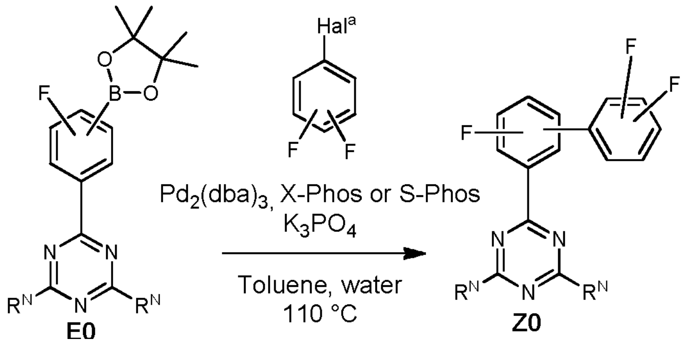


E0 Z0

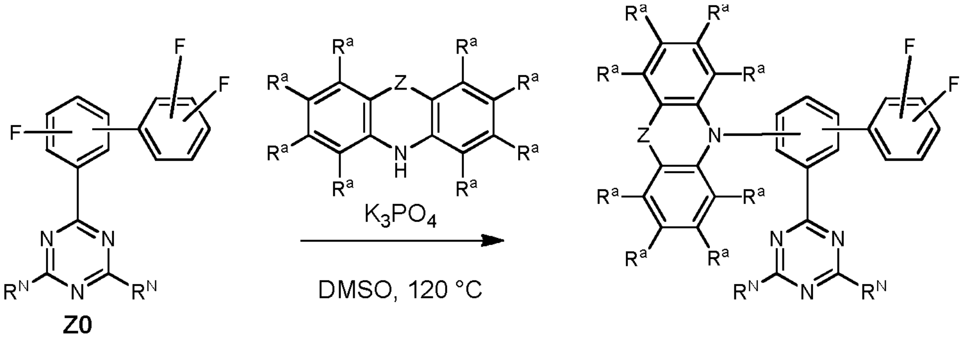


Z0

According to the invention, in the reaction for the synthesis of **Z0** a boronic acid can be used instead of a boronic acid ester.

**A0** can be synthesized via the following synthesis routes:

**Hal$^a$: Cl, Br, I**

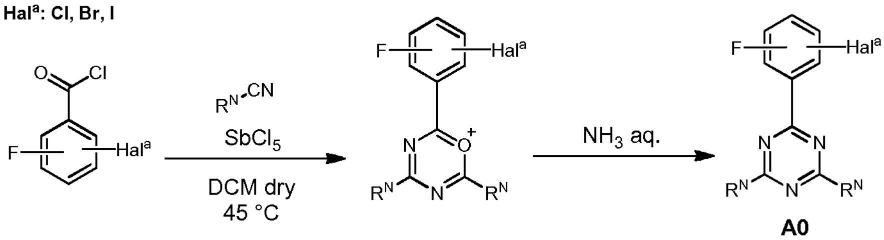


A0

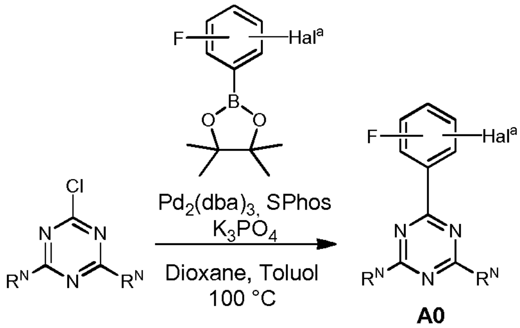


A0

Alternatively, **Z0** can be synthesized via the following synthesis route:

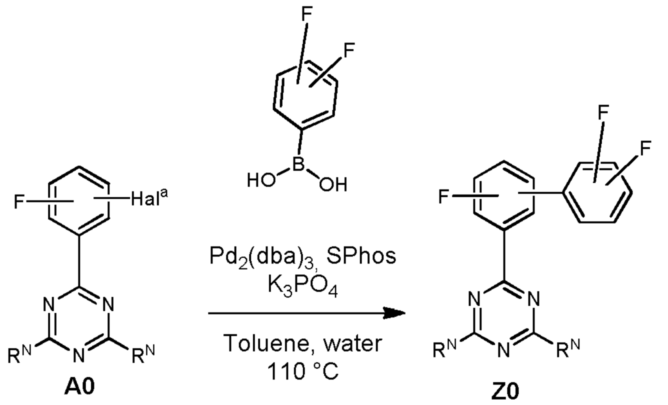


Alternatively, **Z0** can be synthesized via the following synthesis route, wherein a difluorophenyl boronic acid is used as a reactant and wherein $Hal^b$ is selected from the group consisting of Br and I:

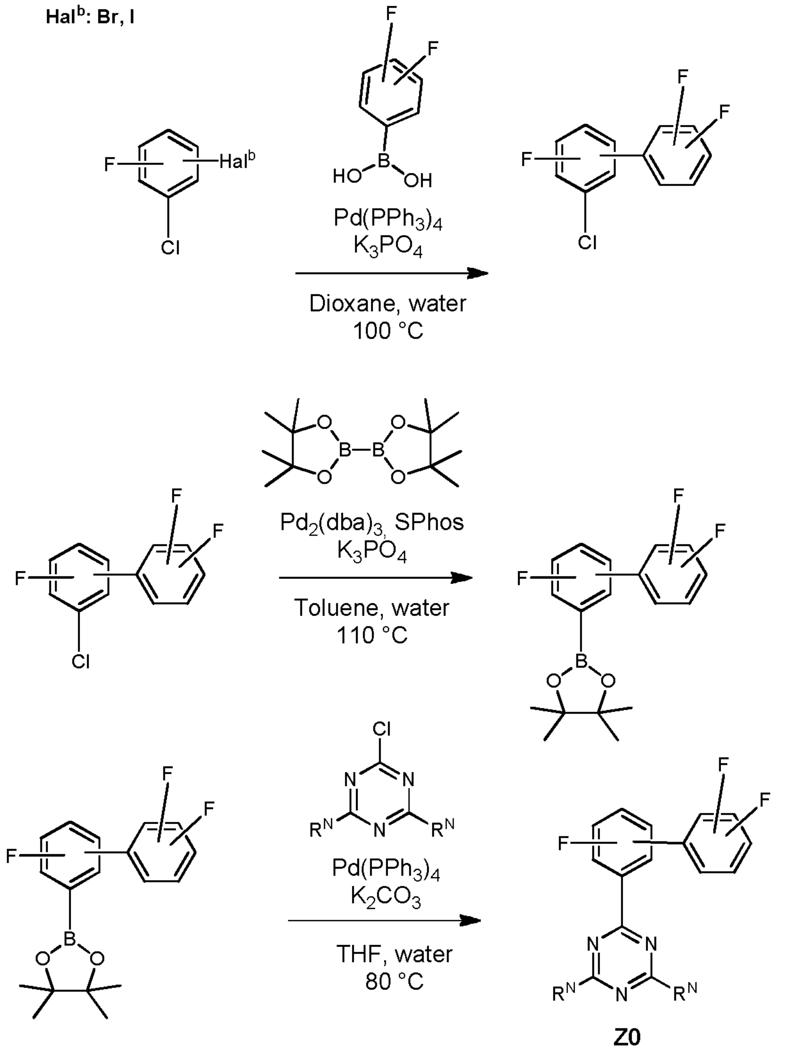


For the reaction of a nitrogen heterocycle in a nucleophilic aromatic substitution with an aryl halide, preferably an aryl fluoride, typical conditions include the use of a base, such as tribasic potassium phosphate or sodium hydride, for example, in an aprotic polar solvent, such as dimethyl sulfoxide (DMSO) or N,N-dimethylformamide (DMF), for example.

A further aspect of the invention relates to the use of an organic molecule according to the invention as a luminescent emitter or as an absorber, and/or as host material and/or as electron transport material, and/or as hole

injection material, and/or as hole blocking material in an organic optoelectronic device.

The optoelectronic device may be understood in the broadest sense as any device based on organic materials that is suitable for emitting light in the visible or nearest ultraviolet (UV) range, i.e., in the range of a wavelength of from 380 to 800 nm. More preferably, the optoelectronic device may be able to emit light in the visible range, i.e., of from 400 to 800 nm.

In the context of such use, the organic optoelectronic device is more particularly selected from the group consisting of:

- organic light-emitting diodes (OLEDs),
- light-emitting electrochemical cells,
- OLED sensors, especially in gas and vapour sensors not hermetically externally shielded,
- organic diodes,
- organic solar cells,
- organic transistors,
- organic field-effect transistors,
- organic lasers and
- down-conversion elements.

In a preferred embodiment in the context of such use, the optoelectronic device is a device selected from the group consisting of an organic light emitting diode (OLED), a light emitting electrochemical cell (LEC), and a light-emitting transistor.

In the case of the use, the fraction of the organic molecule according to the invention in the emission layer in an organic optoelectronic device, more particularly in OLEDs, is 1 % to 99 % by weight, more particularly 5 % to 80 % by weight. In an alternative embodiment, the proportion of the organic molecule in the emission layer is 100 % by weight.

In one embodiment, the light-emitting layer comprises not only the organic molecules according to the invention but also a host material whose triplet (T1) and singlet (S1) energy levels are energetically higher than the triplet (T1) and singlet (S1) energy levels of the organic molecule.

A further aspect of the invention relates to a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

In one embodiment, the light-emitting layer comprises (or (essentially) consists of) a composition comprising or consisting of:

(a) at least one organic molecule according to the invention, in particular in the form of an emitter and/or a host, and
(b) one or more emitter and/or host materials, which differ from the organic molecule according to the invention and
(c) optional one or more dyes and/or one or more solvents.

Particularly preferably the light-emitting layer EML comprises (or (essentially) consists of) a composition comprising or consisting of:

(i) 1-50 % by weight, preferably 5-40 % by weight, in particular 10-30 % by weight, of one or more organic molecules according to the invention;
(ii) 5-99 % by weight, preferably 30-94.9 % by weight, in particular 40-89% by weight, of at least one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

Preferably, energy can be transferred from the host compound H to the one or more organic molecules according to the invention, in particular transferred from the first excited triplet state T1(H) of the host compound H to the first excited triplet state T1(E) of the one or more organic molecules according to the invention and/ or from the first excited singlet state S1(H) of the host compound H to the first excited singlet state S1(E) of the one or more organic molecules according to the invention.

In a further embodiment, the light-emitting layer EML comprises (or (essentially) consists of) a composition comprising or consisting of:

(i) 1-50 % by weight, preferably 5-40 % by weight, in particular 10-30 % by weight, of one organic molecule according to the invention;
(ii) 5-99 % by weight, preferably 30-94.9 % by weight, in particular 40-89% by weight, of one host compound H; and
(iii) optionally 0-94 % by weight, preferably 0.1-65 % by weight, in particular 1-50 % by weight, of at least one further host compound D with a structure differing from the structure of the molecules according to the invention; and
(iv) optionally 0-94 % by weight, preferably 0-65 % by weight, in particular 0-50 % by weight, of a solvent; and
(v) optionally 0-30 % by weight, in particular 0-20 % by weight, preferably 0-5 % by weight, of at least one further emitter molecule F with a structure differing from the structure of the molecules according to the invention.

In one embodiment, the host compound H has a highest occupied molecular orbital HOMO(H) having an energy $E^{HOMO}(H)$ in the range of from -5 to -6.5 eV and the at least one further host compound D has a highest occupied molecular orbital HOMO(D) having an energy $E^{HOMO}(D)$, wherein $E^{HOMO}(H) > E^{HOMO}(D)$.

In a further embodiment, the host compound H has a lowest unoccupied molecular orbital LUMO(H) having an energy $E^{LUMO}(H)$ and the at least one further host compound D has a lowest unoccupied molecular orbital LUMO(D) having an energy $E^{LUMO}(D)$, wherein $E^{LUMO}(H) > E^{LUMO}(D)$.

In one embodiment, the host compound H has a highest occupied molecular orbital HOMO(H) having an energy $E^{HOMO}(H)$ and a lowest unoccupied molecular orbital LUMO(H) having an energy $E^{LUMO}(H)$, and

the at least one further host compound D has a highest occupied molecular orbital HOMO(D) having an energy $E^{HOMO}(D)$ and a lowest unoccupied molecular orbital LUMO(D) having an energy $E^{LUMO}(D)$,
the organic molecule according to the invention has a highest occupied molecular orbital HOMO(E) having an energy $E^{HOMO}(E)$ and a lowest unoccupied molecular orbital LUMO(E) having an energy $E^{LUMO}(E)$,
wherein
$E^{HOMO}(H) > E^{HOMO}(D)$ and the difference between the energy level of the highest occupied molecular orbital HOMO(E) of organic molecule according to the invention ($E^{HOMO}(E)$) and the energy level of the highest occupied molecular orbital HOMO(H) of the host compound H ($E^{HOMO}(H)$) is between -0.5 eV and 0.5 eV, more preferably between -0.3 eV and 0.3 eV, even more preferably between -0.2 eV and 0.2 eV or even between -0.1 eV and 0.1 eV; and $E^{LUMO}(H) > E^{LUMO}(D)$ and the difference between the energy level of the lowest unoccupied molecular orbital LUMO(E) of organic molecule according to the invention ($E^{LUMO}(E)$) and the lowest unoccupied molecular orbital LUMO(D) of the at least one further host compound D ($E^{LUMO}(D)$) is between -0.5 eV and 0.5 eV, more preferably between -0.3 eV and 0.3 eV, even more preferably between -0.2 eV and 0.2 eV or even between -0.1 eV and 0.1 eV.

In a further aspect, the invention relates to an organic optoelectronic device comprising an organic molecule or a composition of the type described here, more particularly in the form of a device selected from the group consisting of organic light-emitting diode (OLED), light-emitting electrochemical cell, OLED sensor, more particularly gas and vapour sensors not hermetically externally shielded, organic diode, organic solar cell, organic transistor, organic field-effect transistor, organic laser and down-conversion element.

In a preferred embodiment, the optoelectronic device is a device selected from the group consisting of an organic light emitting diode (OLED), a light emitting electrochemical cell (LEC), and a light-emitting transistor.

In one embodiment of the organic optoelectronic device of the invention, the organic molecule according to the invention is used as emission material in a light-emitting layer EML.

In one embodiment of the organic optoelectronic device of the invention the light-emitting layer EML consists of the composition according to the invention described here.

Exemplarily, when the optoelectronic device is an OLED, it may exhibit the following layer structure:

1. substrate
2. anode layer A
3. hole injection layer, HIL
4. hole transport layer, HTL
5. electron blocking layer, EBL
6. emitting layer, EML
7. hole blocking layer, HBL
8. electron transport layer, ETL
9. electron injection layer, EIL

10. cathode layer,

wherein the OLED comprises each layer only optionally, different layers may be merged and the OLED may comprise more than one layer of each layer type defined above.

Furthermore, the optoelectronic device may optionally comprise one or more protective layers protecting the device from damaging exposure to harmful species in the environment including, exemplarily moisture, vapor and/or gases.

In one embodiment of the invention, the optoelectronic device is an OLED, which exhibits the following inverted layer structure:

1. substrate
2. cathode layer
3. electron injection layer, EIL
4. electron transport layer, ETL
5. hole blocking layer, HBL
6. emitting layer, B
7. electron blocking layer, EBL
8. hole transport layer, HTL
9. hole injection layer, HIL
10. anode layer A

Wherein the OLED with an inverted layer structure comprises each layer only optionally, different layers may be merged and the OLED may comprise more than one layer of each layer types defined above.

In one embodiment of the invention, the optoelectronic device is an OLED, which may exhibit stacked architecture. In this architecture, contrary to the typical arrangement, where the OLEDs are placed side by side, the individual units are stacked on top of each other. Blended light may be generated with OLEDs exhibiting a stacked architecture, in particular white light may be generated by stacking blue, green and red OLEDs. Furthermore, the OLED exhibiting a stacked architecture may optionally comprise a charge generation layer (CGL), which is typically located between two OLED subunits and typically consists of a n-doped and p-doped layer with the n-doped layer of one CGL being typically located closer to the anode layer.

In one embodiment of the invention, the optoelectronic device is an OLED, which comprises two or more emission layers between anode and cathode. In particular, this so-called tandem OLED comprises three emission layers, wherein one emission layer emits red light, one emission layer emits green light and one emission layer emits blue light, and optionally may comprise further layers such as charge generation layers, blocking or transporting layers between the individual emission layers. In a further embodiment, the emission layers are adjacently stacked. In a further embodiment, the tandem OLED comprises a charge generation layer between each two emission layers. In addition, adjacent emission layers or emission layers separated by a charge generation layer may be merged.

The substrate may be formed by any material or composition of materials. Most frequently, glass slides are used as substrates. Alternatively, thin metal layers (e.g., copper, gold, silver or aluminum films) or plastic films or slides may be used. This may allow a higher degree of flexibility. The anode layer A is mostly composed of materials allowing to obtain an (essentially) transparent film. As at least one of both electrodes should be (essentially) transparent in order to allow light emission from the OLED, either the anode layer A or the cathode layer C is transparent. Preferably, the anode layer A comprises a large content or even consists of transparent conductive oxides (TCOs). Such anode layer A may exemplarily comprise indium tin oxide, aluminum zinc oxide, fluorine doped tin oxide, indium zinc oxide, PbO, SnO, zirconium oxide, molybdenum oxide, vanadium oxide, wolfram oxide, graphite, doped Si, doped Ge, doped GaAs, doped polyaniline, doped polypyrrol and/or doped polythiophene.

Particularly preferably, the anode layer A (essentially) consists of indium tin oxide (ITO) (e.g., (InO3)0.9(SnO2)0.1). The roughness of the anode layer A caused by the transparent conductive oxides (TCOs) may be compensated by using a hole injection layer (HIL). Further, the HIL may facilitate the injection of quasi charge carriers (i.e., holes) in that the transport of the quasi charge carriers from the TCO to the hole transport layer (HTL) is facilitated. The hole injection layer (HIL) may comprise poly-3,4-ethylendioxy thiophene (PEDOT), polystyrene sulfonate (PSS), $MoO_2$, $V_2O_5$, CuPC or CuI, in particular a mixture of PEDOT and PSS. The hole injection layer (HIL) may also prevent the diffusion of metals from the anode layer A into the hole transport layer (HTL). The HIL may exemplarily comprise PEDOT:PSS (poly-3,4-ethylendioxy thiophene: polystyrene sulfonate), PEDOT (poly-3,4-ethylendioxy thiophene), mMTDATA (4,4',4"-tris[phenyl(m-tolyl)amino]triphenylamine), Spiro-TAD (2,2',7,7'-tetrakis(n,n-diphenylamino)-9,9'-spirobifluorene), DNTPD (N1,N1'-(biphenyl-4,4'-diyl)bis(N1-phenyl-N4,N4-di-m-tolylbenzene-1,4-diamine), NPB (N,N'-nis-(1-naphthalenyl)-N,N'-bisphenyl-(1,1'-biphenyl)-4,4'-diamine), NPNPB (N,N'-diphenyl-N,N'-di-[4-(N,N-diphenyl-amino)phenyl]benzidine), MeO-TPD (N,N,N',N'-tetrakis(4-methoxyphenyl)benzidine), HAT-CN (1,4,5,8,9,11-hexaazatriphenylen-hexacarbonitrile) and/or Spiro-NPD (N,N'-diphenyl-N,N'-bis-(1-naphthyl)-9,9'-spirobifluorene-2,7-diamine).

Adjacent to the anode layer A or hole injection layer (HIL) typically a hole transport layer (HTL) is located. Herein, any hole transport compound may be used. Exemplarily, electron-rich heteroaromatic compounds such as triarylamines and/or carbazoles may be used as hole transport compound. The HTL may decrease the energy barrier between the anode layer A and the light-emitting layer EML. The hole transport layer (HTL) may also be an electron blocking layer (EBL). Preferably, hole transport compounds bear comparably high energy levels of their triplet states T1. Exemplarily the hole transport layer (HTL) may comprise a star-shaped heterocycle such as tris(4-carbazoyl-9-ylphenyl)amine (TCTA), poly-TPD (poly(4-butylphenyl-diphenyl-amine)), [alpha]-NPD (poly(4-butylphenyl-diphenyl-amine)), TAPC (4,4'-cyclohexyliden-bis[N,N-bis(4-methylphenyl)benzenamine]), 2-TNATA (4,4',4"-tris[2-naphthyl(phenyl)amino]triphenylamine), Spiro-TAD, DNTPD, NPB, NPNPB, MeO-TPD, HAT-CN and/or TrisPcz (9,9'-diphenyl-6-(9-phenyl-9H-carbazol-3-yl)-9H,9'H-3,3'-bicarbazole). In addition, the HTL may comprise a p-doped layer, which may be composed of an inorganic or organic dopant in an organic hole-transporting matrix. Transition metal oxides such as vanadium oxide, molybdenum oxide or tungsten oxide may exemplarily be used as inorganic dopant. Tetrafluorotetracyanoquinodimethane (F4-TCNQ), copper-pentafluorobenzoate (Cu(I)pFBz) or transition metal complexes may exemplarily be used as organic dopant.

The EBL may exemplarily comprise mCP (1,3-bis(carbazol-9-yl)benzene), TCTA, 2-TNATA, mCBP (3,3-di(9H-carbazol-9-yl)biphenyl), tris-Pcz, CzSi (9-(4-tert-Butylphenyl)-3,6-bis(triphenylsilyl)-9H-carbazole), and/or DCB (N,N'-dicarbazolyl-1,4-dimethylbenzene).

Adjacent to the hole transport layer (HTL), typically, the light-emitting layer EML is located. The light-emitting layer EML comprises at least one light emitting molecule. Particular, the EML comprises at least one light emitting molecule according to the invention. In one embodiment, the light-emitting layer comprises only the organic molecules according to the invention. Typically, the EML additionally comprises one or more host material. Exemplarily, the host material is selected from CBP (4,4'-Bis-(N-carbazolyl)-biphenyl), mCP, mCBP Sif87 (dibenzo[b,d]thiophen-2-yltriphenylsilane), CzSi, SimCP ([3,5-Di(9H-carbazol-9-yl)phenyl]triphenylsilane), Sif88 (dibenzo[b,d]thiophen-2-yl)diphenylsilane), DPEPO (bis[2-(diphenylphosphino)phenyl] ether oxide), 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole, 9-[3,5-bis(2-dibenzothiophenyl)phenyl]-9H-carbazole, T2T (2,4,6-tris(biphenyl-3-yl)-1,3,5-triazine), T3T (2,4,6-tris(triphenyl-3-yl)-1,3,5-triazine) and/or TST (2,4,6-tris(9,9'-spirobifluorene-2-yl)-1,3,5-triazine). The host material typically should be selected to exhibit first triplet (T1) and first singlet (S1) energy levels, which are energetically higher than the first triplet (T1) and first singlet (S1) energy levels of the organic molecule.

In one embodiment of the invention, the EML comprises a so-called mixed-host system with at least one hole-dominant host and one electron-dominant host. In a particular embodiment, the EML comprises exactly one light emitting molecule according to the invention and a mixed-host system comprising T2T as electron-dominant host and a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole as hole-dominant host. In a further embodiment the EML comprises 50-80 % by weight, preferably 60-75 % by weight of a host selected from CBP, mCP, mCBP, 9-[3-(dibenzofuran-2-yl)phenyl]-9H-carbazole, 9-[3-(dibenzofuran-2-yl)phenyl]-9H-carbazole, 9-[3-(dibenzothiophen-2-yl)phenyl]-9H-carbazole, 9-[3,5-bis(2-dibenzofuranyl)phenyl]-9H-carbazole and 9-[3,5-bis(2-dibenzothiophenyl)phenyl]-9H-carbazole; 10-45 % by weight, preferably 15-30 % by weight of T2T and 5-40 % by weight, preferably 10-30 % by weight of light emitting molecule according to the invention.

Adjacent to the light-emitting layer EML an electron transport layer (ETL) may be located. Herein, any electron transporter may be used. Exemplarily, compounds poor of electrons such as, e.g., benzimidazoles, pyridines, triazoles, oxadiazoles (e.g., 1,3,4-oxadiazole), phosphinoxides and sulfone, may be used. An electron transporter may also be a star-shaped heterocycle such as 1,3,5-tri(1-phenyl-1H-benzo[d]imidazol-2-yl)phenyl (TPBi). The ETL may comprise NBphen (2,9-bis(naphthalen-2-yl)-4,7-diphenyl-1,10-phenanthroline), Alq3 (Aluminum-tris(8-hydroxyquinoline)), TSPO1 (diphenyl-4-triphenylsilylphenyl-phosphinoxide), BPyTP2 (2,7-di(2,2'-bipyridin-5-yl)triphenyle), Sif87 (dibenzo[b,d]thiophen-2-yltriphenylsilane), Sif88 (dibenzo[b,d]thiophen-2-yl)diphenylsilane), BmPyPhB (1,3-bis[3,5-di(pyridin-3-yl)phenyl]benzene) and/or BTB (4,4'-bis-[2-(4,6-diphenyl-1,3,5-triazinyl)]-1,1'-biphenyl). Optionally, the ETL may be doped with materials such as Liq. The electron transport layer (ETL) may also block holes or a holeblocking layer (HBL) is introduced.

The HBL may exemplarily comprise BCP (2,9-dimethyl-4,7-diphenyl-1,10-phenanthroline = Bathocuproine), BAlq (bis(8-hydroxy-2-methylquinoline)-(4-phenylphenoxy)aluminum), NBphen (2,9-bis(naphthalen-2-yl)-4,7-diphenyl-1,10-phenanthroline), Alq3 (Aluminum-tris(8-hydroxyquinoline)), TSPO1 (diphenyl-4-triphenylsilylphenyl-phosphinoxide), T2T (2,4,6-tris(biphenyl-3-yl)-1,3,5-triazine), T3T (2,4,6-tris(triphenyl-3-yl)-1,3,5-triazine), TST (2,4,6-tris(9,9'-spirobifluorene-2-yl)-1,3,5-triazine), and/or TCB/TCP (1,3,5-tris(N-carbazolyl)benzol/ 1,3,5-tris(carbazol)-9-yl) benzene).

Adjacent to the electron transport layer (ETL), a cathode layer C may be located. Exemplarily, the cathode

layer C may comprise or may consist of a metal (e.g., Al, Au, Ag, Pt, Cu, Zn, Ni, Fe, Pb, LiF, Ca, Ba, Mg, In, W, or Pd) or a metal alloy. For practical reasons, the cathode layer may also consist of (essentially) intransparent metals such as Mg, Ca or Al. Alternatively or additionally, the cathode layer C may also comprise graphite and or carbon nanotubes (CNTs). Alternatively, the cathode layer C may also consist of nanoscalic silver wires.

An OLED may further, optionally, comprise a protection layer between the electron transport layer (ETL) and the cathode layer C (which may be designated as electron injection layer (EIL)). This layer may comprise lithium fluoride, cesium fluoride, silver, Liq (8-hydroxyquinolinolatolithium), $Li_2O$, $BaF_2$, MgO and/or NaF.

Optionally, also the electron transport layer (ETL) and/or a hole blocking layer (HBL) may comprise one or more host compounds.

In order to modify the emission spectrum and/or the absorption spectrum of the light-emitting layer EML further, the light-emitting layer EML may further comprise one or more further emitter molecule F. Such an emitter molecule F may be any emitter molecule known in the art. Preferably such an emitter molecule F is a molecule with a structure differing from the structure of the molecules according to the invention. The emitter molecule F may optionally be a TADF emitter. Alternatively, the emitter molecule F may optionally be a fluorescent and/or phosphorescent emitter molecule which is able to shift the emission spectrum and/or the absorption spectrum of the light-emitting layer EML. Exemplarily, the triplet and/or singlet excitons may be transferred from the emitter molecule according to the invention to the emitter molecule F before relaxing to the ground state S0 by emitting light typically red-shifted in comparison to the light emitted by emitter molecule E. Optionally, the emitter molecule F may also provoke two-photon effects (i.e., the absorption of two photons of half the energy of the absorption maximum).

Optionally, an optoelectronic device (e.g., an OLED) may, for example, be an essentially white optoelectronic device. Exemplarily such white optoelectronic device may comprise at least one (deep) blue emitter molecule and one or more emitter molecules emitting green and/or red light. Then, there may also optionally be energy transmittance between two or more molecules as described above.

As used herein, if not defined more specifically in the particular context, the designation of the colors of emitted and/or absorbed light is as follows:

| | |
|---|---|
| violet: | wavelength range of >380-420 nm; |
| deep blue: | wavelength range of >420-480 nm; |
| sky blue: | wavelength range of >480-500 nm; |
| green: | wavelength range of >500-560 nm; |
| yellow: | wavelength range of >560-580 nm; |
| orange: | wavelength range of >580-620 nm; |
| red: | wavelength range of >620-800 nm. |

With respect to emitter molecules, such colors refer to the emission maximum. Therefore, exemplarily, a deep blue emitter has an emission maximum in the range of from >420 to 480 nm, a sky blue emitter has an emission maximum in the range of from >480 to 500 nm, a green emitter has an emission maximum in a range of from >500 to 560 nm, a red emitter has an emission maximum in a range of from >620 to 800 nm.

A deep blue emitter may preferably have an emission maximum of below 480 nm, more preferably below 470 nm, even more preferably below 465 nm or even below 460 nm. It will typically be above 420 nm, preferably above 430 nm, more preferably above 440 nm or even above 450 nm.

Accordingly, a further aspect of the present invention relates to an OLED, which exhibits an external quantum efficiency at 1000 cd/m2 of more than 8%, more preferably of more than 10%, more preferably of more than 13%, even more preferably of more than 15% or even more than 20% and/or exhibits an emission maximum between 420 nm and 500 nm, preferably between 430 nm and 490 nm, more preferably between 440 nm and 480 nm, even more preferably between 450 nm and 470 nm and/or exhibits a LT80 value at 500 cd/m2 of more than 100 h, preferably more than 200 h, more preferably more than 400 h, even more preferably more than 750 h or even more than 1000 h. Accordingly, a further aspect of the present invention relates to an OLED, whose emission exhibits a CIEy color coordinate of less than 0.45, preferably less than 0.30, more preferably less than 0.20 or even more preferably less than 0.15 or even less than 0.10.

A further aspect of the present invention relates to an OLED, which emits light at a distinct color point. According to the present invention, the OLED emits light with a narrow emission band (small full width at half maximum (FWHM)). In one aspect, the OLED according to the invention emits light with a FWHM of the main emission peak of less than 0.50 eV, preferably less than 0.48 eV, more preferably less than 0.45 eV, even more preferably less than 0.43 eV or even less than 0.40 eV.

A further aspect of the present invention relates to an OLED, which emits light with CIEx and CIEy color

coordinates close to the CIEx (= 0.131) and CIEy (= 0.046) color coordinates of the primary color blue (CIEx = 0.131 and CIEy = 0.046) as defined by ITU-R Recommendation BT.2020 (Rec. 2020) and thus is suited for the use in Ultra High Definition (UHD) displays, e.g. UHD-TVs. In commercial applications, typically top-emitting (top-electrode is transparent) devices are used, whereas test devices as used throughout the present application represent bottom-emitting devices (bottom-electrode and substrate are transparent). The CIEy color coordinate of a blue device can be reduced by up to a factor of two, when changing from a bottom- to a top-emitting device, while the CIEx remains nearly unchanged (Okinaka et al. (2015), 22.1: Invited Paper: New Fluorescent Blue Host Materials for Achieving Low Voltage in OLEDs, SID Symposium Digest of Technical Papers, 46; doi:10.1002/sdtp.10480). Accordingly, a further aspect of the present invention relates to an OLED, whose emission exhibits a CIEx color coordinate of between 0.02 and 0.30, preferably between 0.03 and 0.25, more preferably between 0.05 and 0.20 or even more preferably between 0.08 and 0.18 or even between 0.10 and 0.15 and/ or a CIEy color coordinate of between 0.00 and 0.45, preferably between 0.01 and 0.30, more preferably between 0.02 and 0.20 or even more preferably between 0.03 and 0.15 or even between 0.04 and 0.10.

In a further aspect, the invention relates to a method for producing an optoelectronic component. In this case an organic molecule of the invention is used.

The optoelectronic device, in particular the OLED according to the present invention can be produced by any means of vapor deposition and/ or liquid processing. Accordingly, at least one layer is

- prepared by means of a sublimation process,
- prepared by means of an organic vapor phase deposition process,
- prepared by means of a carrier gas sublimation process,
- solution processed or printed.

The methods used to produce the optoelectronic device, in particular the OLED according to the present invention are known in the art. The different layers are individually and successively deposited on a suitable substrate by means of subsequent deposition processes. The individual layers may be deposited using the same or differing deposition methods.

Vapor deposition processes exemplarily comprise thermal (co)evaporation, chemical vapor deposition and physical vapor deposition. For active matrix OLED display, an AMOLED backplane is used as substrate. The individual layer may be processed from solutions or dispersions employing adequate solvents. Solution deposition process exemplarily comprise spin coating, dip coating and jet printing. Liquid processing may optionally be carried out in an inert atmosphere (e.g., in a nitrogen atmosphere) and the solvent may optionally be completely or partially removed by means known in the state of the art.

## Examples

*General synthesis scheme I*

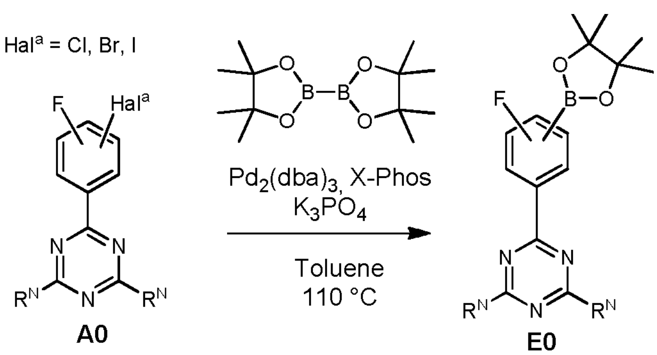

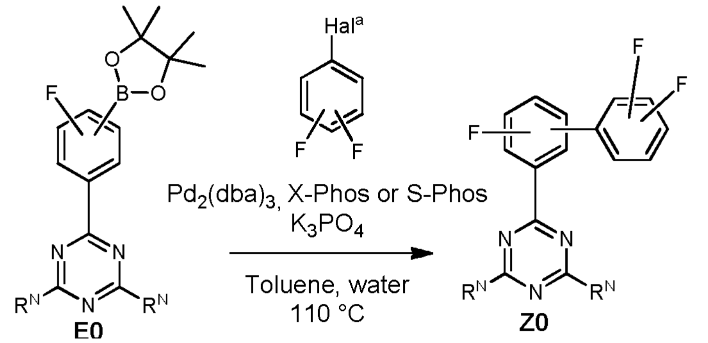


E0 Z0

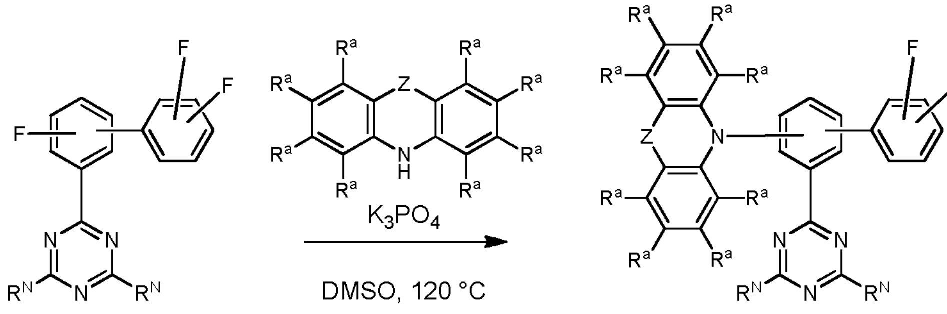


Z0

*General procedure for synthesis **AAV1***:

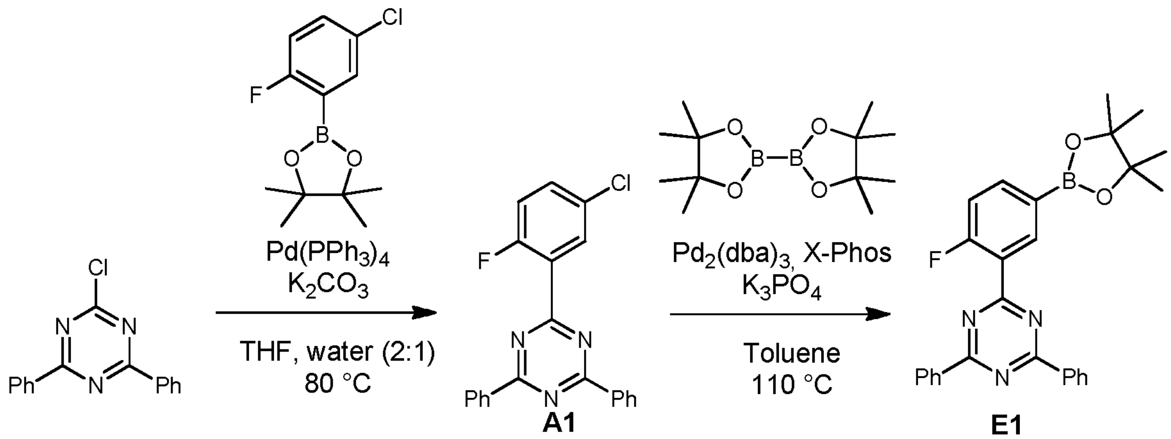


A1 E1

2-Chloro-4,6-diphenyl-1,3,5-triazine (1.00 equivalent), 5-chloro-2-fluoro-phenylboronic acid pinacol ester (1.20 equivalents), $Pd(PPh_3)_4$ (0.03 equivalent), potassium carbonate (3.00 equivalents) are stirred under nitrogen atmosphere in a THF/water (2:1) mixture at 80 °C until completion of the reaction (monitoring with GC/MS, usually finished within 2-15 h). After cooling down to room temperature (rt), the phases are separated and the aqueous layer is extracted with dichloromethane. The combined organic phase is then washed with brine, dried over $MgSO_4$, and the solvent evaporated under reduced pressure. The obtained crude product is purified via flash chromatography or recrystallization, yielding **A1** as a solid.

Subsequently, under nitrogen atmosphere, **A1** (1.00 equivalent), bis(pinacolato)diboron (1.20 equivalents), $Pd_2(dba)_3$ (0.01 equivalent), X-Phos (0.04 equivalents) and potassium acetate (2.6 equivalents) are reacted in dry toluene at 110 °C until full conversion (monitoring with LC/MS, usually finished within 2-15 h). After cooling down to rt, the mixture is filtered, the filter cake washed with dichloromethane and the filtrate concentrated in vacuo. The resulting crude product is purified via flash chromatography or recrystallization, yielding the pure product **E1** as a solid.

*General procedure for synthesis **AAV1-2:***

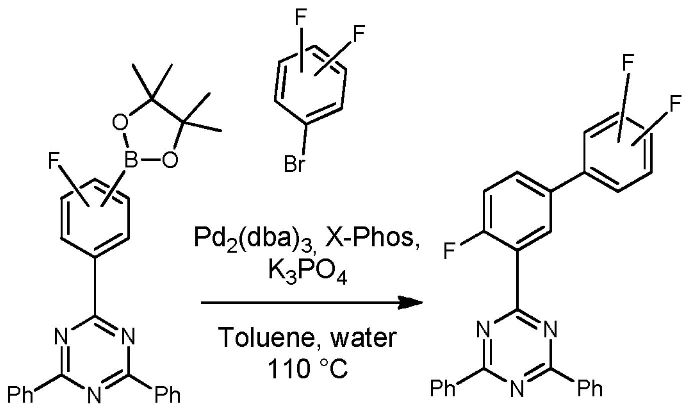


**E1** **Z1**

**E1** (1.00 equivalents), 1-bromo-difluorobenzene (1.10 equivalents), $Pd_2(dba)_3$ (0.02 equivalents), 2-(dicyclohexylphosphino)-2",4",6"-triisopropylbiphenyl (X-Phos) (0.08 equivalents) and tribasic potassium phosphate (2.50 equivalents) are stirred under nitrogen atmosphere in a toluene/water mixture (ratio of 10:1) at 110 °C until completion of the reaction (monitoring with GC/MS, usually finished within 12 h). After cooling down to rt, the reaction mixture is filtered through a fiber glass filter and the filter cake washed with dichloromethane. The filtrate is transferred to a separating funnel and the phases are separated. The organic phase is washed with brine, dried over $MgSO_4$, and the solvent evaporated under reduced pressure. The obtained crude product is purified via flash chromatography or recrystallization, giving the product as a solid.

*General procedure for synthesis **AAV2**:*

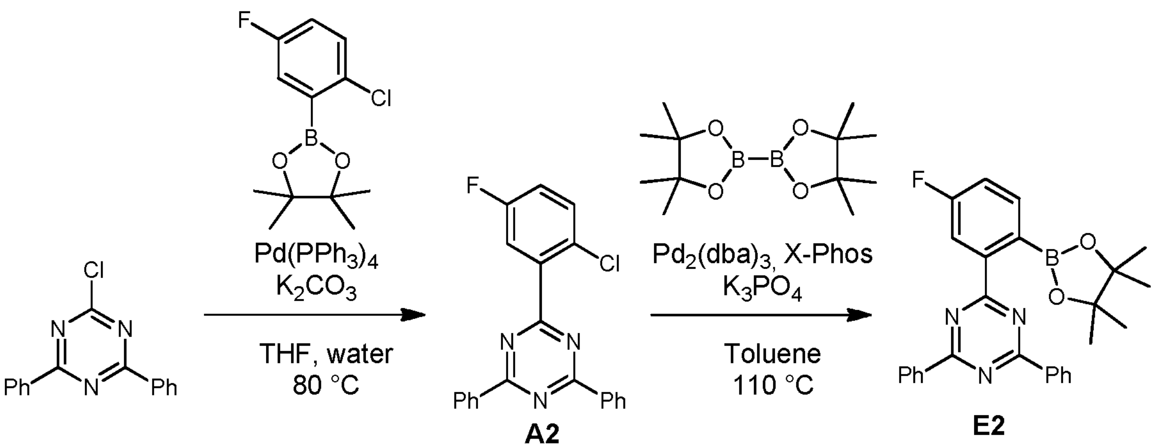


**A2** **E2**

The synthesis of **E2** is carried out according to ***AAV1,*** wherein 2-chloro-4,6-diphenyl-1,3,5-triazine reacts with 2-chloro-5-fluoro-phenylboronic acid pinacol ester.

*General procedure for synthesis **AAV2-2**:*

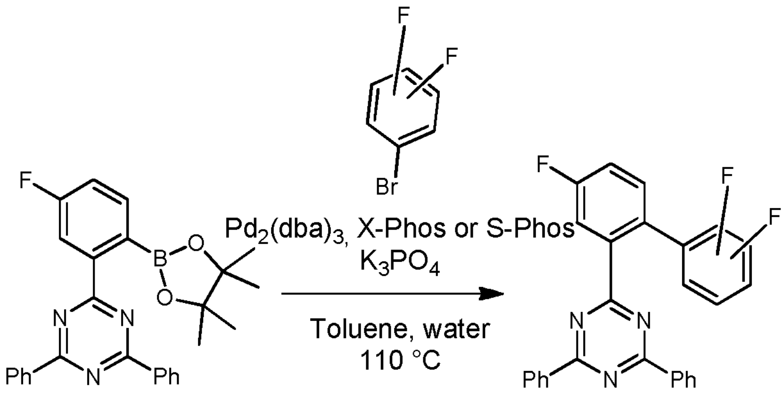


E2 Z2

The synthesis of **Z2** is carried out according to ***AAV1-2,*** wherein **E2** reacts with a 1-bromo-difluorobenzene.

*General procedure for synthesis **AAV3**:*

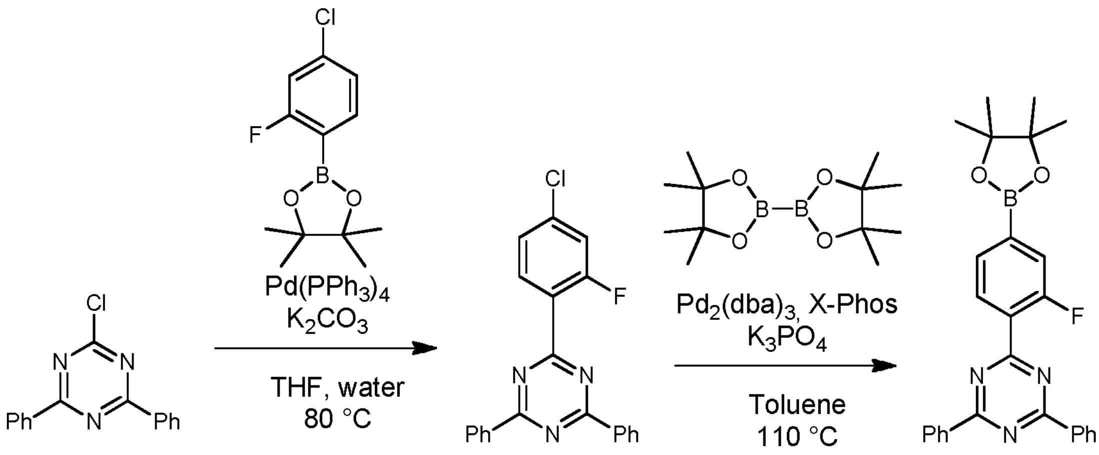


A3 E3

The synthesis of E3 is carried out according to *AAV1,* wherein 2-chloro-4,6-diphenyl-1,3,5-triazine reacts with 4-chloro-2-fluoro-phenylboronic acid pinacol ester.

*General procedure for synthesis **AAV3-2**:*

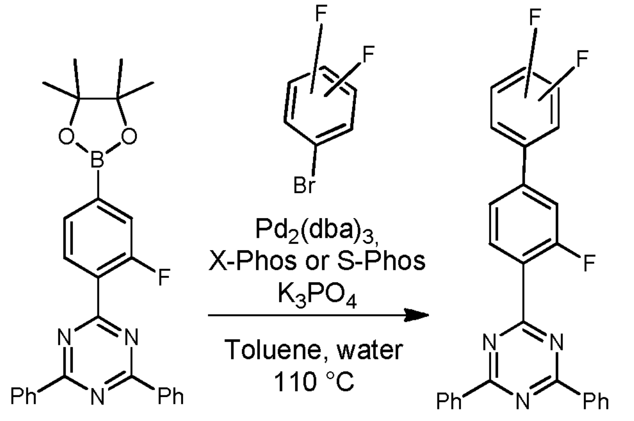


E3 Z3

The synthesis of Z3 is carried out according to *AAV1-2,* wherein E3 reacts with a 1-bromo-difluorobenzene.

*General procedure for synthesis AAV4:*

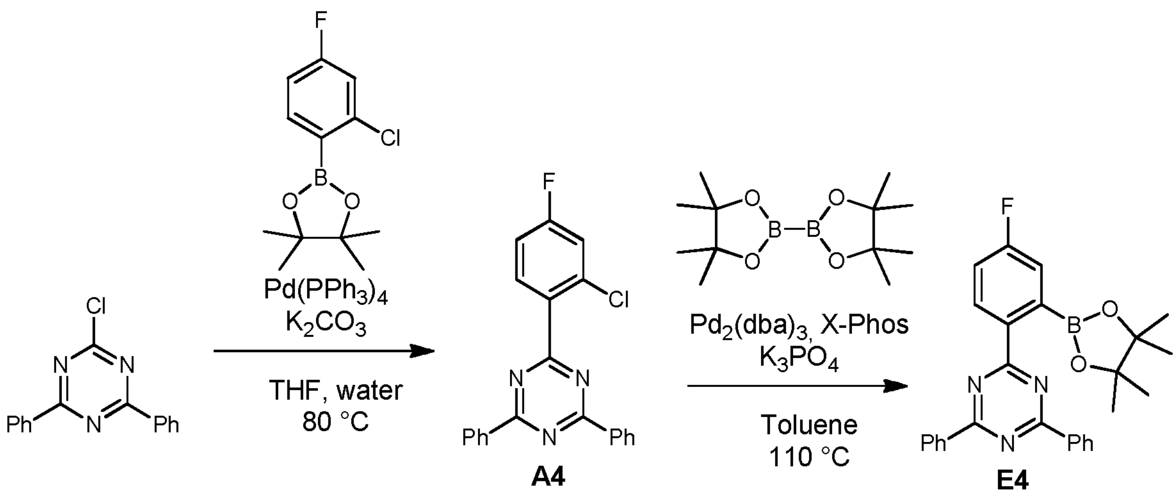


A4 E4

The synthesis of E4 is carried out according to *AAV1,* wherein 2-chloro-4,6-diphenyl-1,3,5-triazine reacts with 2-chloro-4-fluoro-phenylboronic acid pinacol ester.

*General procedure for synthesis* ***AAV4-2:***

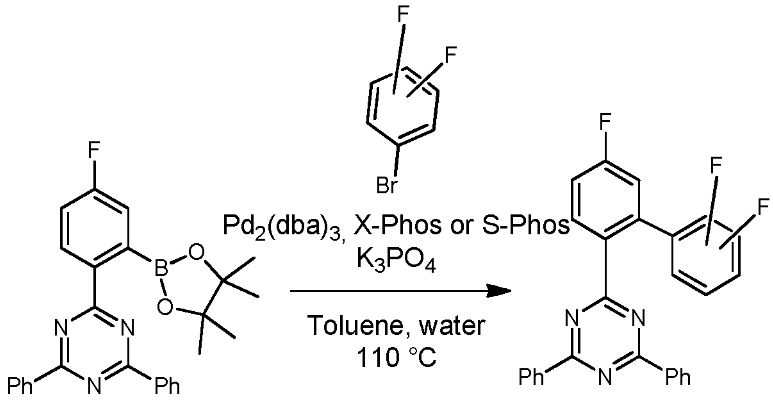


E4 Z4

The synthesis of **Z4** is carried out according to ***AAV1-2,*** wherein **E4** reacts with a 1-bromo-difluorobenzene.

*General procedure for synthesis* ***AAV5*****:**

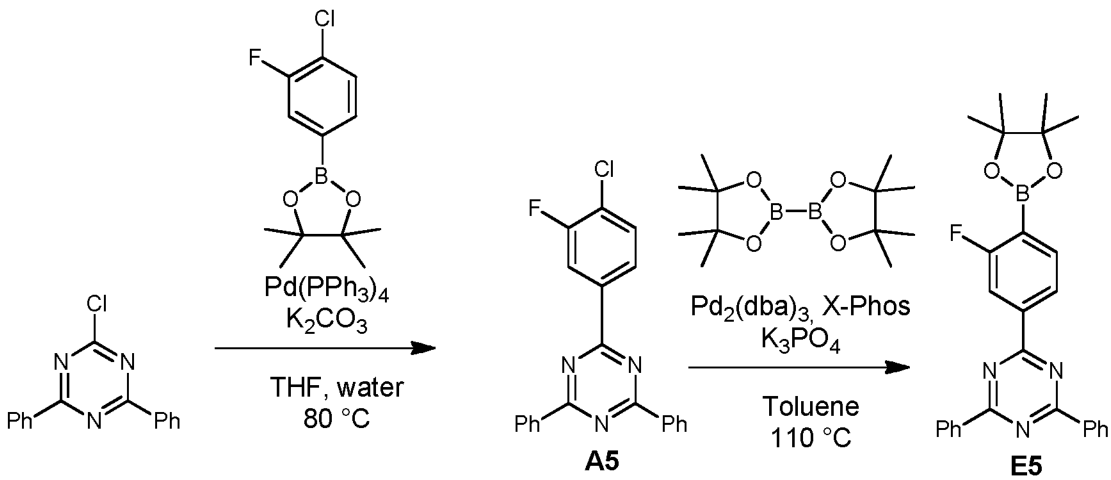


A5 E5

The synthesis of **E5** is carried out according to ***AAV1,*** wherein 2-chloro-4,6-diphenyl-1,3,5-triazine reacts with 4-chloro-3-fluoro-phenylboronic acid pinacol ester.

*General procedure for synthesis* ***AAV5-2*****:**

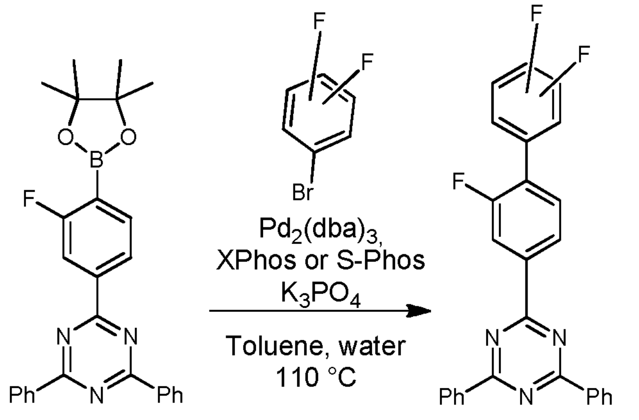


E5 Z5

The synthesis of **Z5** is carried out according to ***AAV1-2,*** wherein **E5** reacts a 1-bromo-difluorobenzene.

*General procedure for synthesis **AAV6***:

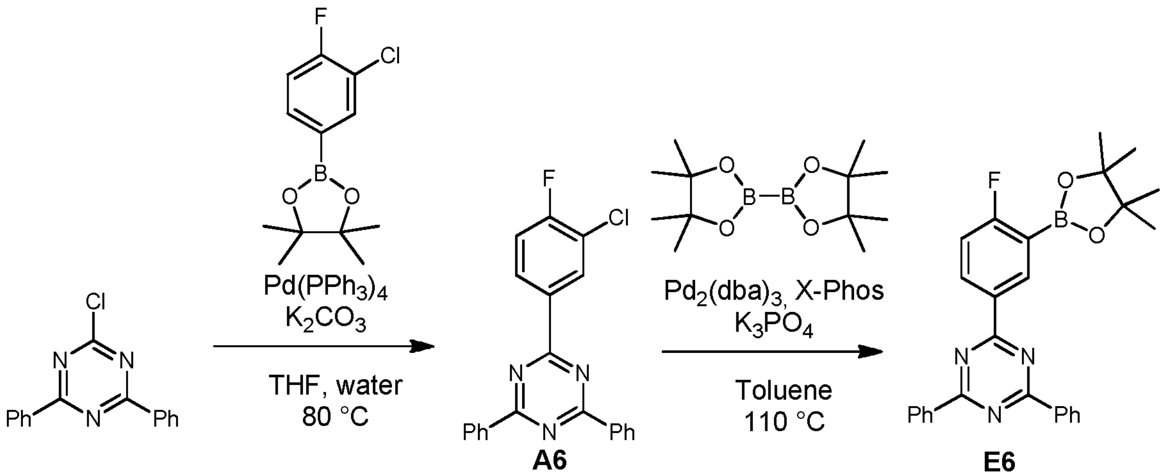


A6 E6

The synthesis of E6 is carried out according to *AAV1,* wherein 2-chloro-4,6-diphenyl-1,3,5-triazine reacts with 3-chloro-4-fluoro-phenylboronic acid pinacol ester.

*General procedure for synthesis **AAV6-2**:*

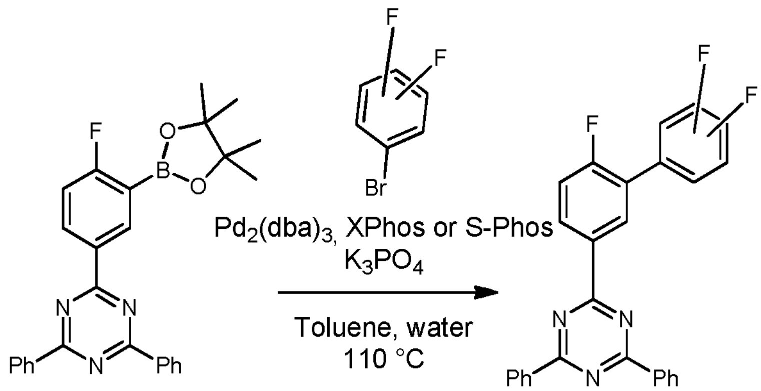


E6 Z6

The synthesis of **Z6** is carried out according to ***AAV1-2,*** wherein **E6** reacts with a 1-bromo-difluorobenzene.

*General procedure for synthesis* ***AAV7*:**

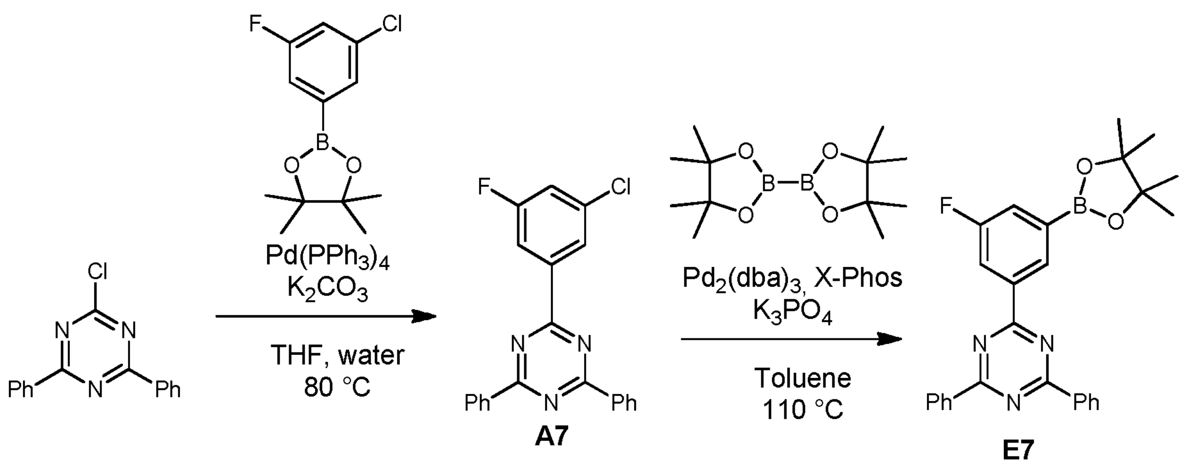


A7 E7

The synthesis of **E7** is carried out according to ***AAV1,*** wherein 2-Chloro-4,6-diphenyl-1,3,5-triazine reacts with 3-chloro-5-fluoro-phenylboronic acid pinacol ester.

*General procedure for synthesis* ***AAV7-2*:**

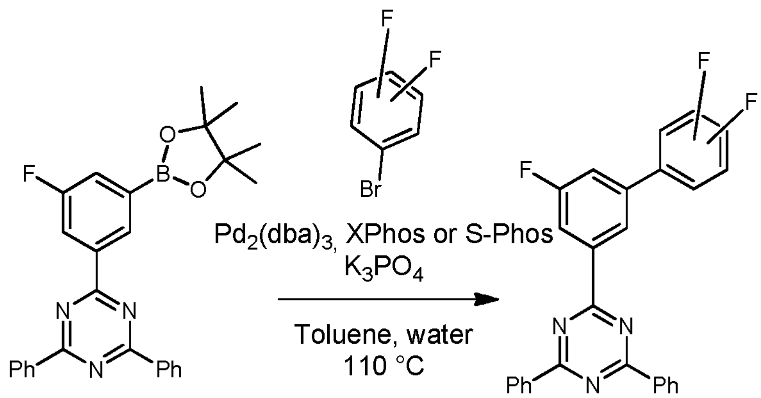


E7 Z7

The synthesis of **Z7** is carried out according to ***AAV1-2,*** wherein **E7** reacts with a 1-bromo-difluorobenzene.

*General procedure for synthesis **AAV8**:*

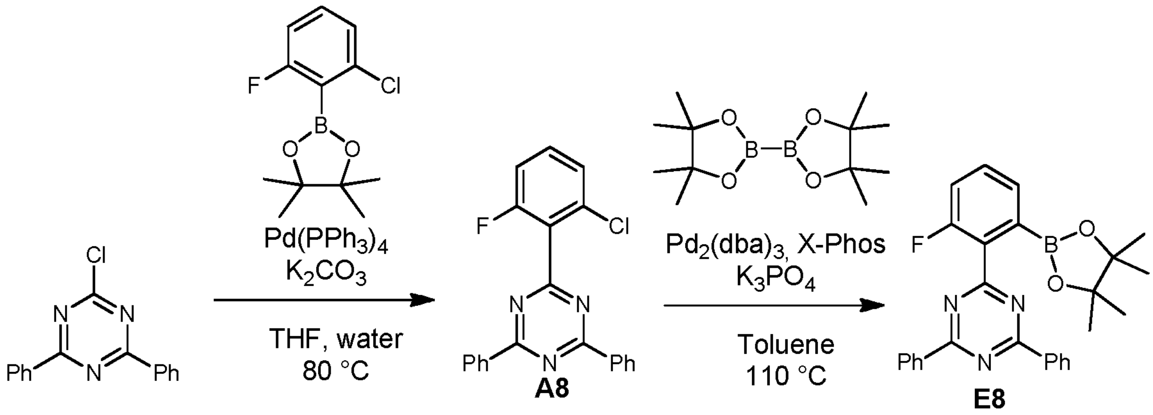


The synthesis of **E8** is carried out according to ***AAV1,*** wherein 2-chloro-4,6-diphenyl-1,3,5-triazine reacts with 2-chloro-6-fluoro-phenylboronic acid pinacol ester.

*General procedure for synthesis **AAV8-2**:*

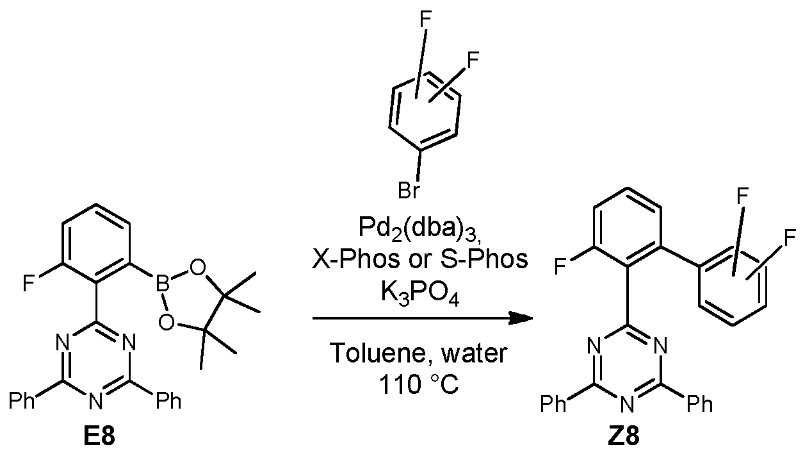


The synthesis of **Z8** is carried out according to ***AAV1-2,*** wherein **E8** reacts with a 1-bromo-difluorobenzene.

*General procedure for synthesis **AAV9**:*

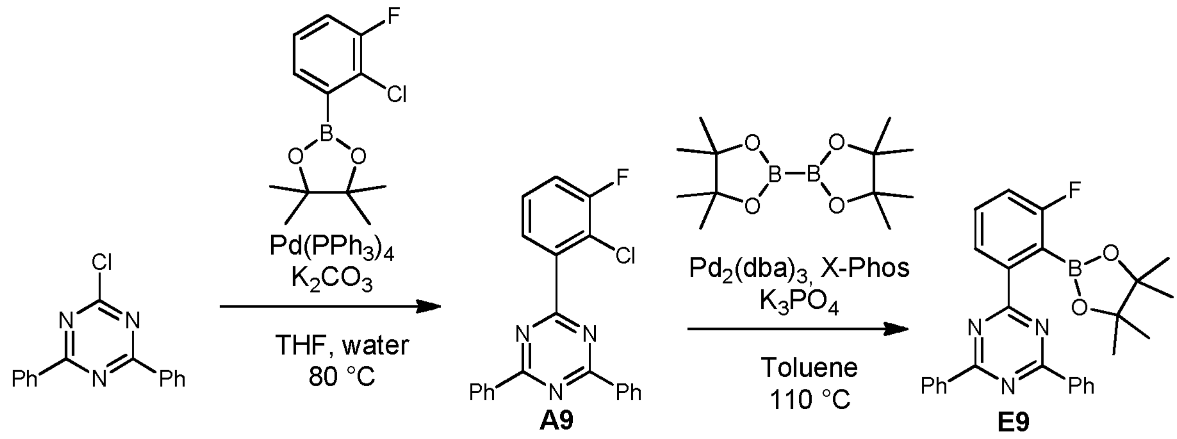


The synthesis of **E9** is carried out according to ***AAV1,*** wherein 2-chloro-4,6-diphenyl-1,3,5-triazine reacts with 2-chloro-3-fluoro-phenylboronic acid pinacol ester.

*General procedure for synthesis **AAV9-2**:*

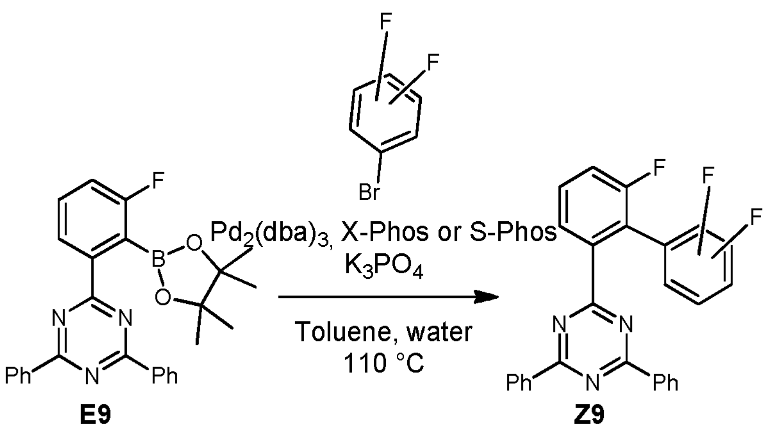


The synthesis of **Z9** is carried out according to ***AAV1-2,*** wherein **E9** reacts with a 1-bromo-difluorobenzene.

*General procedure for synthesis **AAV10**:*

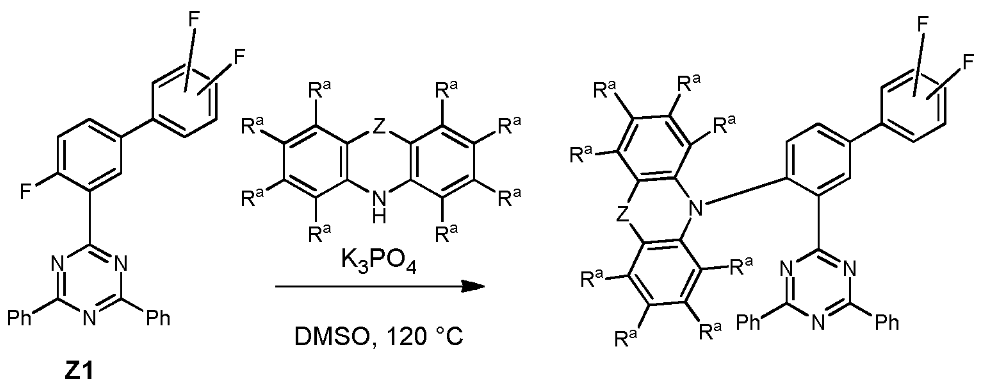

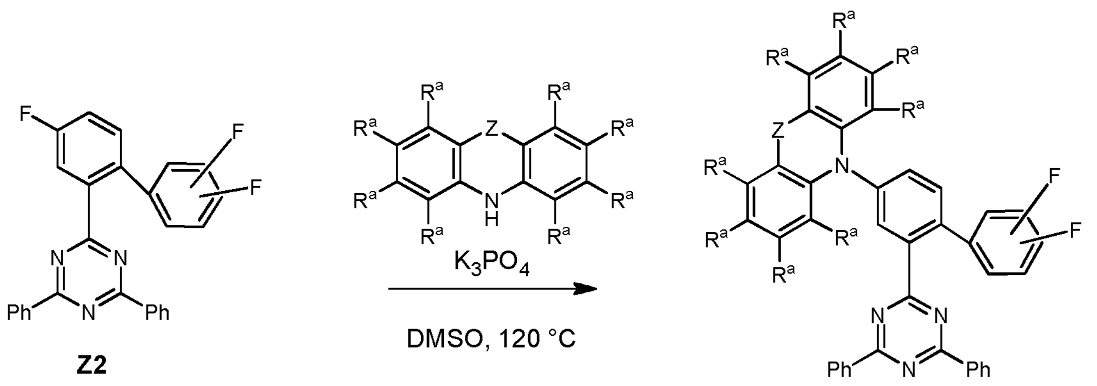

$K_3PO_4$
DMSO, 120 °C


Z2

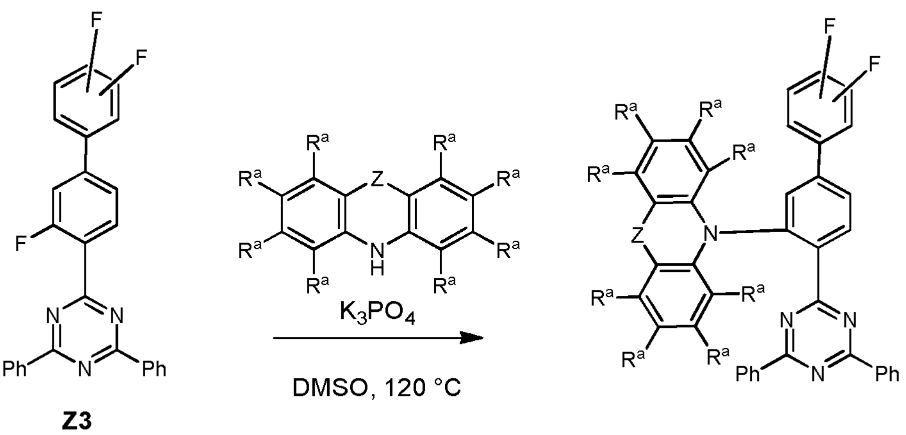

$K_3PO_4$
DMSO, 120 °C


Z3

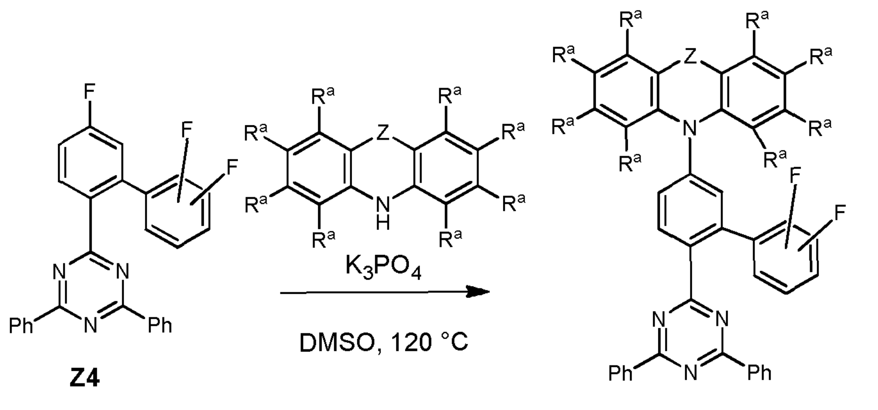

$K_3PO_4$
DMSO, 120 °C


Z4

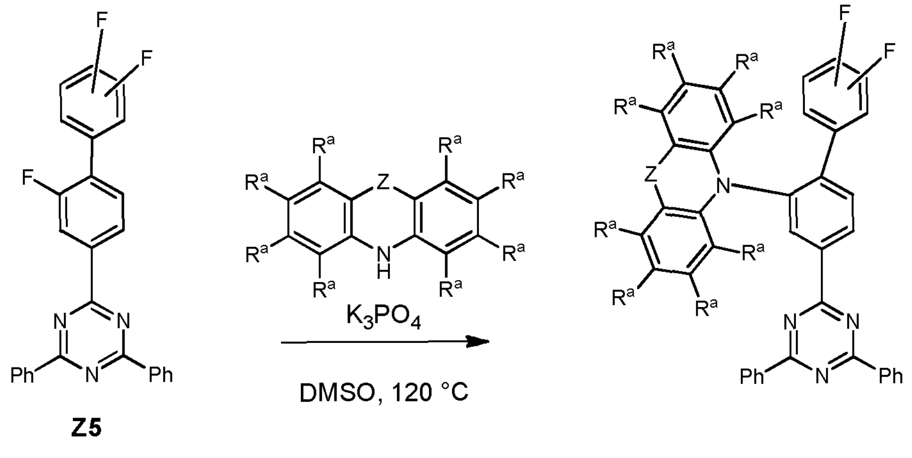

$K_3PO_4$
DMSO, 120 °C


Z5

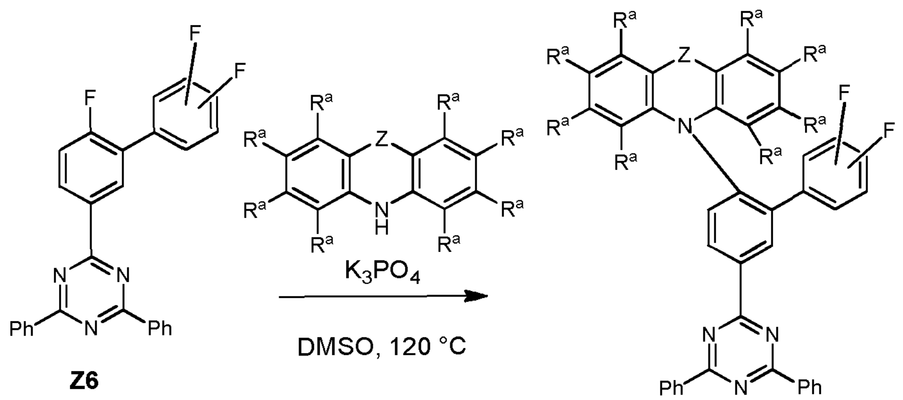


**Z6**

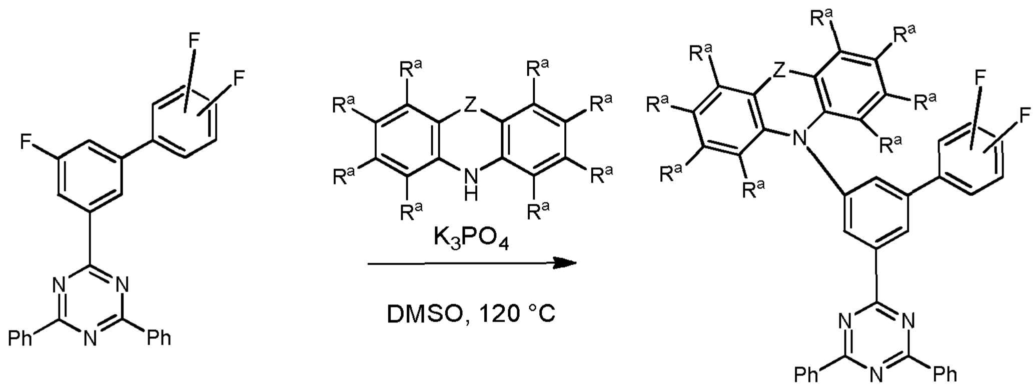


**Z7**

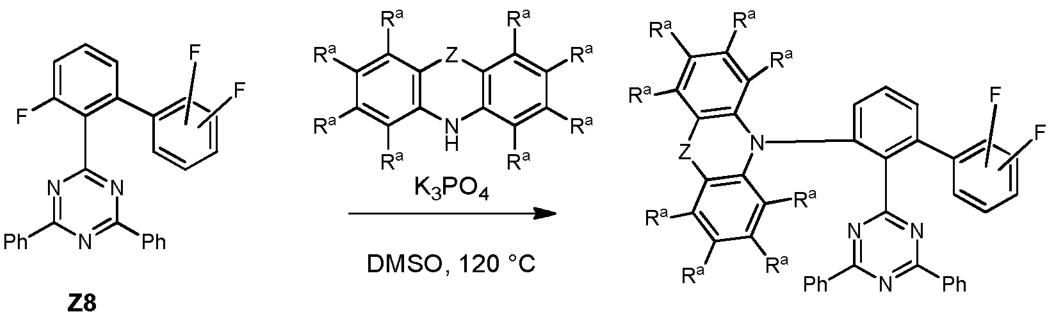


**Z8**

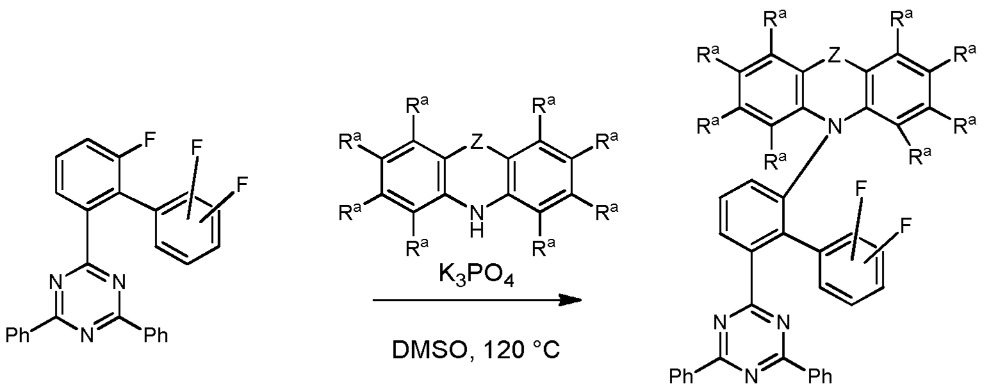


**Z9**

**Z1, Z2, Z3, Z4, Z5, Z6, Z7, Z8, or Z9** (1 equivalent each), the corresponding donor molecule D-H (1.00 equivalents) and tribasic potassium phosphate (2.00 equivalents) are suspended under nitrogen atmosphere in DMSO and stirred at 120 °C (12-16 h). Subsequently the reaction mixture is poured into an excess of water in order to precipitate

the product. The precipitate is filtered off, washed with water and dried under vacuum. The crude product is purified by recrystallization or by flash chromatography. The product is obtained as a solid.

In particular, the donor molecule D-H is a 3,6-substituted carbazole (e.g., 3,6-dimethylcarbazole, 3,6-diphenylcarbazole, 3,6-di-tert-butylcarbazole), a 2,7-substituted carbazole (e.g., 2,7-dimethylcarbazole, 2,7-diphenylcarbazole, 2,7-di-tert-butylcarbazole), a 1,8-substituted carbazole (e.g., 1,8-dimethylcarbazole, 1,8-diphenylcarbazole, 1,8-di-tert-butylcarbazole), a 1-substituted carbazole (e.g., 1-methylcarbazole, 1-phenylcarbazole, 1-tert-butylcarbazole), a 2-substituted carbazole (e.g., 2-methylcarbazole, 2-phenylcarbazole, 2-tert-butylcarbazole), or a 3-substituted carbazole (e.g., 3-methylcarbazole, 3-phenylcarbazole, 3-tert-butylcarbazole).

Exemplarily a halogen-substituted carbazole, particularly 3-bromocarbazole, can be used as D-H.
In a subsequent reaction a boronic acid ester functional group or boronic acid functional group may be exemplarily introduced at the position of the one or more halogen substituents, which was introduced via D-H, to yield the corresponding carbazol-3-ylboronic acid ester or carbazol-3-ylboronic acid, e.g., via the reaction with bis(pinacolato)diboron (CAS No. 73183-34-3). Subsequently, one or more substituents $R^a$ may be introduced in place of the boronic acid ester group or the boronic acid group via a coupling reaction with the corresponding halogenated reactant $R^a$-Hal, preferably $R^a$-Cl and $R^a$-Br.

Alternatively, one or more substituents $R^a$ may be introduced at the position of the one or more halogen substituents, which was introduced via D-H, via the reaction with a boronic acid of the substituent $R^a$[$R^a$-$B(OH)_2$] or a corresponding boronic acid ester.

**HPLC-MS:**

HPLC-MS spectroscopy is performed on a HPLC by Agilent (1100 series) with MS-detector (Thermo LTQ XL). A reverse phase column 4,6mm x 150mm, particle size 5,0μm from Waters (without pre-column) is used in the HPLC. The HPLC-MS measurements are performed at room temperature (rt) with the solvents acetonitrile, water and THF in the following concentrations:

| | | |
|---|---|---|
| solvent A: | $H_2O$ (90%) | MeCN (10%) |
| solvent B: | $H_2O$ (10%) | MeCN (90%) |
| solvent C: | THF (50%) | MeCN (50%) |

From a solution with a concentration of 0.5mg/ml an injection volume of 15 μL is taken for the measurements. The following gradient is used:

| Flow rate [ml/min] | time [min] | A[%] | B[%] | C[%] |
|---|---|---|---|---|
| 3 | 0 | 40 | 50 | 10 |
| 3 | 10 | 15 | 25 | 60 |
| 3 | 14 | 15 | 25 | 60 |
| 3 | 14.01 | 40 | 50 | 10 |
| 3 | 18 | 40 | 50 | 10 |
| 3 | 19 | 40 | 50 | 10 |

Ionisation of the probe is performed by APCI (atmospheric pressure chemical ionization).

*Cyclic voltammetry*

Cyclic voltammograms are measured from solutions having concentration of $10^{-3}$ mol/l of the organic molecules in dichloromethane or a suitable solvent and a suitable supporting electrolyte (e.g. 0.1 mol/l of tetrabutylammonium hexafluorophosphate). The measurements are conducted at room temperature under nitrogen atmosphere with a three-electrode assembly (Working and counter electrodes: Pt wire, reference electrode: Pt wire) and calibrated using $FeCp_2/FeCp_2^+$ as internal standard. The HOMO data was corrected using ferrocene as internal standard against SCE.

*Density functional theory calculation*

Molecular structures are optimized employing the BP86 functional and the resolution of identity approach (RI). Excitation energies are calculated using the (BP86) optimized structures employing Time-Dependent DFT (TD-DFT)

methods. Orbital and excited state energies are calculated with the B3LYP functional. Def2-SVP basis sets (and a m4-grid for numerical integration are used. The Turbomole program package is used for all calculations.

*Photophysical measurements*

Sample pretreatment: Spin-coating
Apparatus: Spin150, SPS euro.
The sample concentration is 10 mg/ml, dissolved in a suitable solvent.
Program: 1) 3 s at 400 U/min; 20 s at 1000 U/min at 1000 Upm/s. 3) 10 s at 4000 U/min at 1000 Upm/s. After coating, the films are tried at 70 °C for 1 min.
Photoluminescence spectroscopy and TCSPC (*Time-correlated single-photon counting*) Steady-state emission spectroscopy is measured by a Horiba Scientific, Modell FluoroMax-4 equipped with a 150 W Xenon-Arc lamp, excitation- and emissions monochromators and a Hamamatsu R928 photomultiplier and a time-correlated single-photon counting option. Emissions and excitation spectra are corrected using standard correction fits.
Excited state lifetimes are determined employing the same system using the TCSPC method with FM-2013 equipment and a Horiba Yvon TCSPC hub.
Excitation sources:

NanoLED 370 (wavelength: 371 nm, puls duration: 1,1 ns)
NanoLED 290 (wavelength: 294 nm, puls duration: <1 ns)
SpectraLED 310 (wavelength: 314 nm)
SpectraLED 355 (wavelength: 355 nm).

Data analysis (exponential fit) is done using the software suite DataStation and DAS6 analysis software. The fit is specified using the chi-squared-test.

Photoluminescence quantum yield measurements

For photoluminescence quantum yield (PLQY) measurements an *Absolute PL Quantum Yield Measurement C9920-03G* system (*Hamamatsu Photonics*) is used. Quantum yields and CIE coordinates are determined using the software U6039-05 version 3.6.0.
Emission maxima are given in nm, quantum yields Φ in % and CIE coordinates as x,y values. PLQY is determined using the following protocol:

1) Quality assurance: Anthracene in ethanol (known concentration) is used as reference
2) Excitation wavelength: the absorption maximum of the organic molecule is determined and the molecule is excited using this wavelength
3) Measurement
Quantum yields are measured for sample of solutions or films under nitrogen atmosphere. The yield is calculated using the equation:

$$\Phi_{PL} = \frac{n_{photon}, emited}{n_{photon}, absorbed} = \frac{\int \frac{\lambda}{hc}\left[Int_{emitted}^{sample}(\lambda) - Int_{absorbed}^{sample}(\lambda)\right]d\lambda}{\int \frac{\lambda}{hc}\left[Int_{emitted}^{reference}(\lambda) - Int_{absorbed}^{reference}(\lambda)\right]d\lambda}$$

wherein $n_{photon}$ denotes the photon count and Int. the intensity.

**Production and characterization of optoelectronic devices**

OLED devices comprising organic molecules according to the invention can be produced via vacuum-deposition methods. If a layer contains more than one compound, the weight-percentage of one or more compounds is given in %. The total weight-percentage values amount to 100 %, thus if a value is not given, the fraction of this compound equals to the difference between the given values and 100%.
The not fully optimized OLEDs are characterized using standard methods and measuring electroluminescence spectra, the external quantum efficiency (in %) in dependency on the intensity, calculated using the light detected by the photodiode, and the current. The OLED device lifetime is extracted from the change of the luminance during operation at constant current density. The LT50 value corresponds to the time, where the measured luminance decreased to 50

% of the initial luminance, analogously LT80 corresponds to the time point, at which the measured luminance decreased to 80 % of the initial luminance, LT 95 to the time point, at which the measured luminance decreased to 95 % of the initial luminance etc.

Accelerated lifetime measurements are performed (e.g. applying increased current densities). Exemplarily LT80 values at 500 cd/m$^2$ are determined using the following equation:

$$\mathrm{LT80}\left(500\,\frac{cd^2}{m^2}\right) = \mathrm{LT80}(L_0)\left(\frac{L_0}{500\,\frac{cd^2}{m^2}}\right)^{1.6}$$

wherein $L_0$ denotes the initial luminance at the applied current density.

The values correspond to the average of several pixels (typically two to eight), the standard deviation between these pixels is given. Figures show the data series for one OLED pixel.

**Example 1**

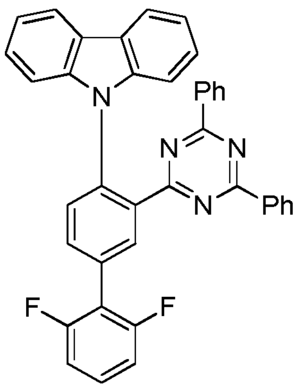

Example 1 was synthesized according to ***AAV1*** (91% yield over 2 steps), ***AAV1-2*** (31% yield), and ***AAV10*** (37% yield).

MS (HPLC-MS), m/z (retention time): 838.39 (12.16 min).

Figure 1 depicts the emission spectrum of example 1 (10% by weight in PMMA). The emission maximum is at 463 nm. The photoluminescence quantum yield (PLQY) is 83%, the full width at half maximum is 0.41 eV and the emission lifetime is 36 µs.

**Additional Examples of organic molecules of the invention**

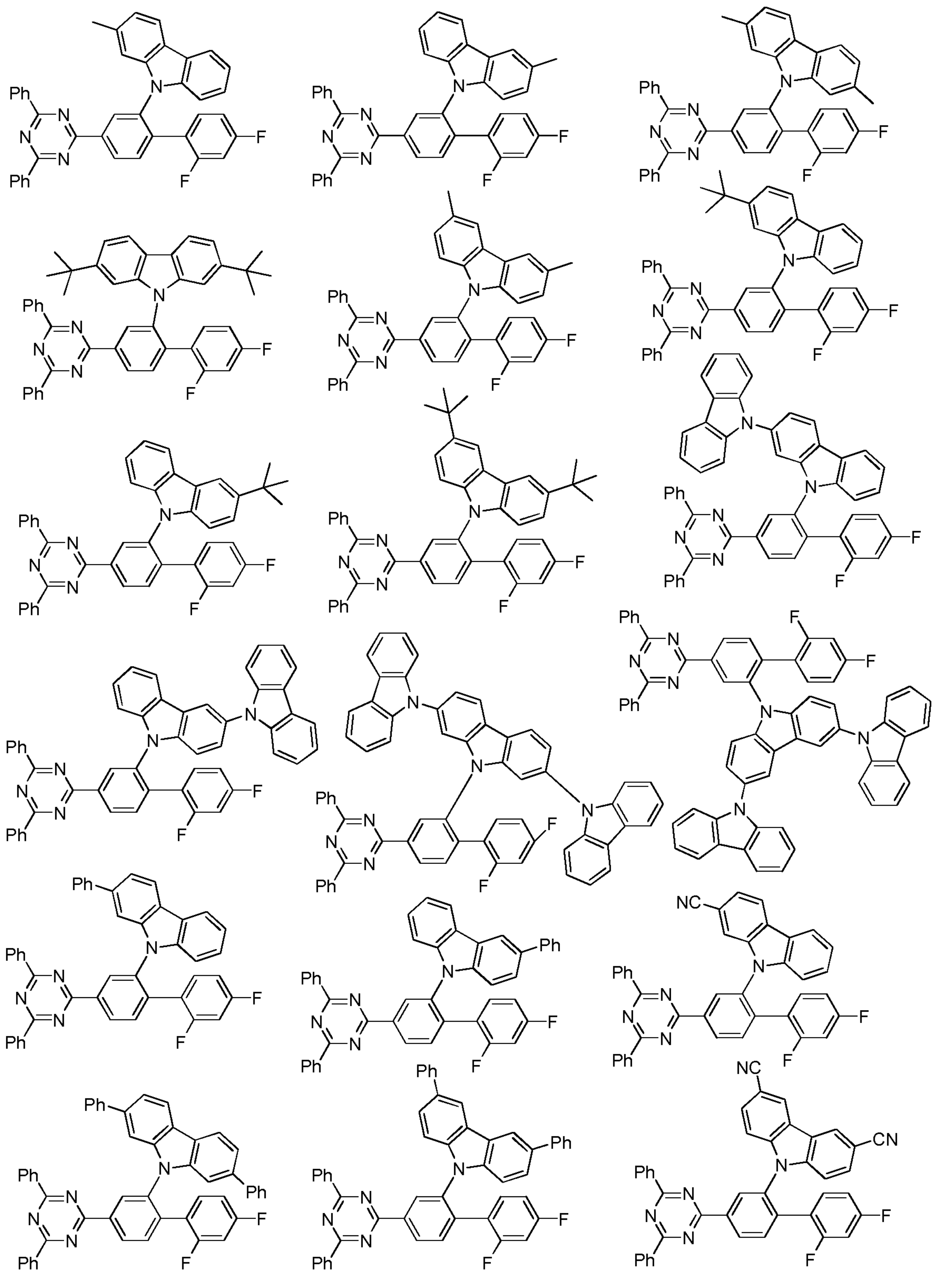

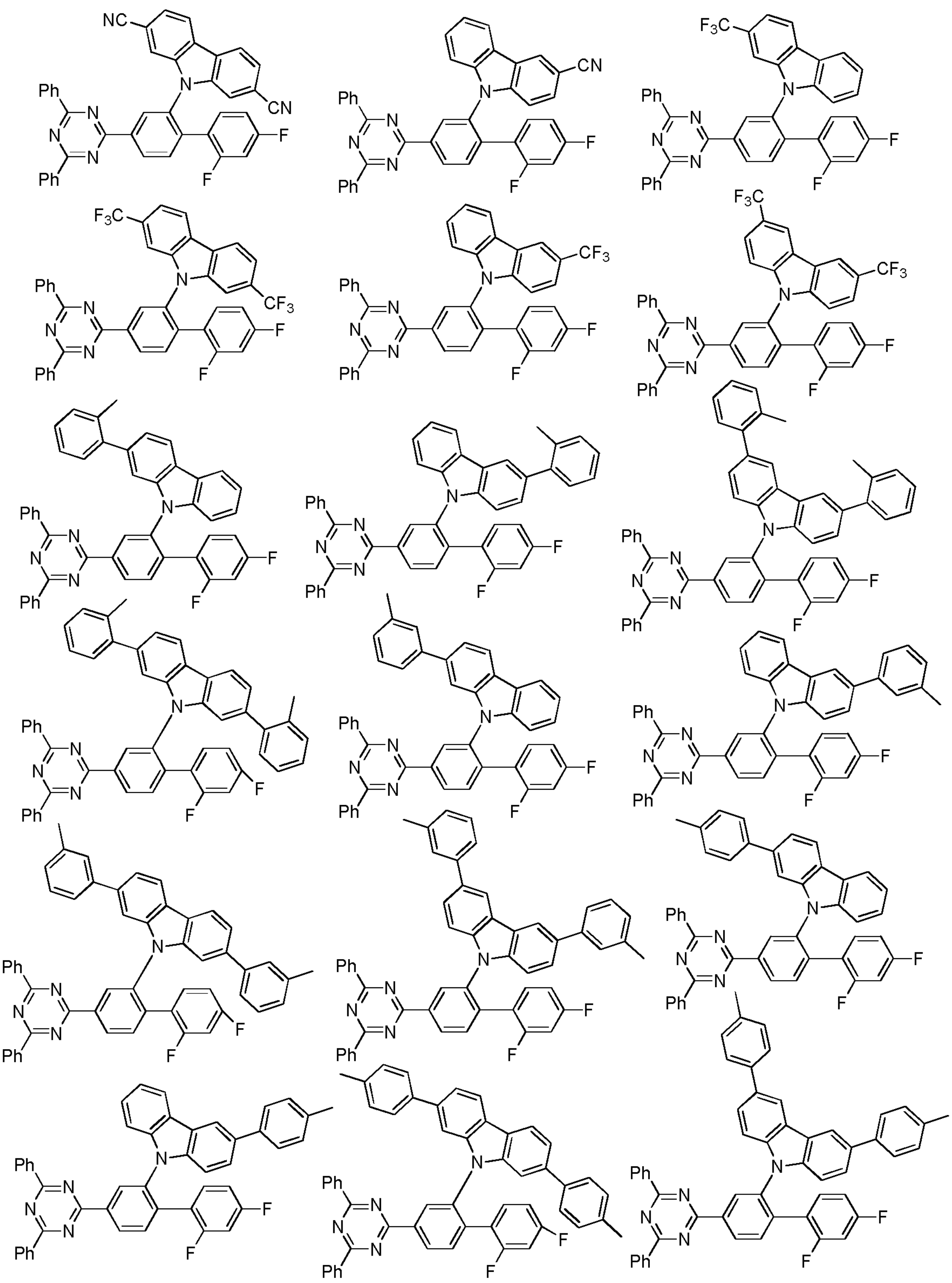

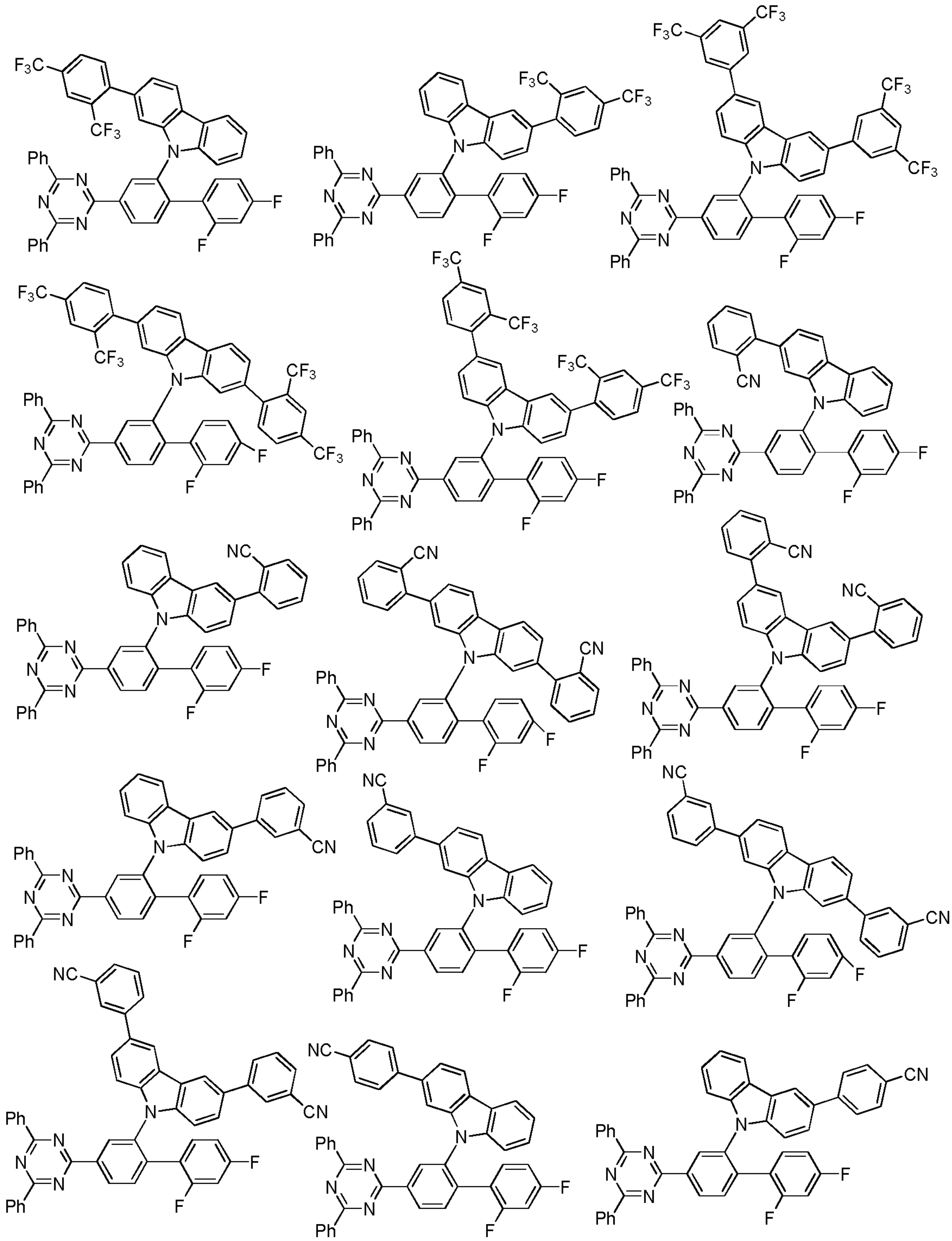

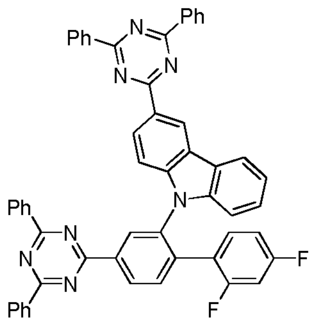
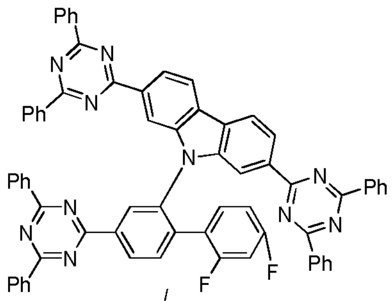
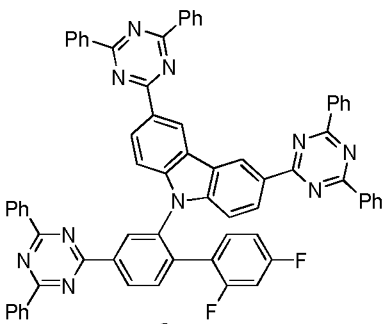

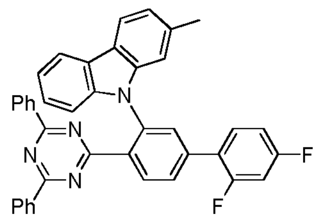
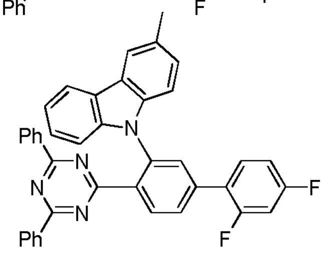
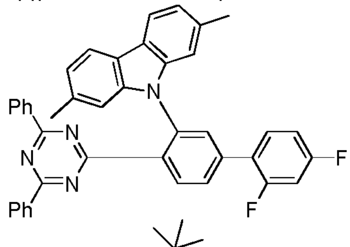

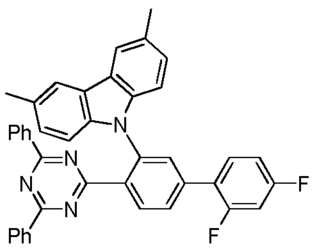
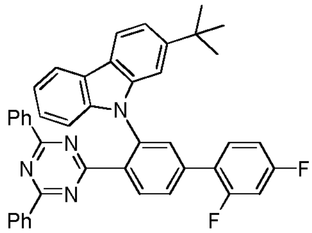
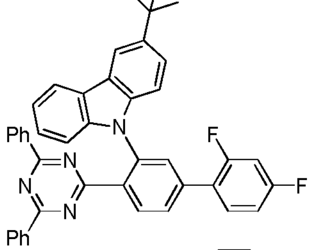

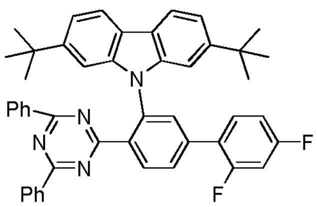
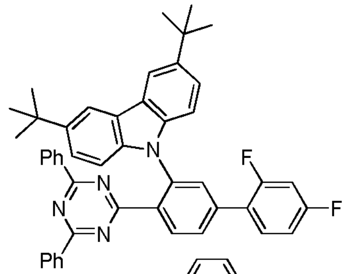
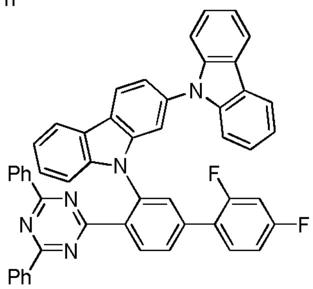

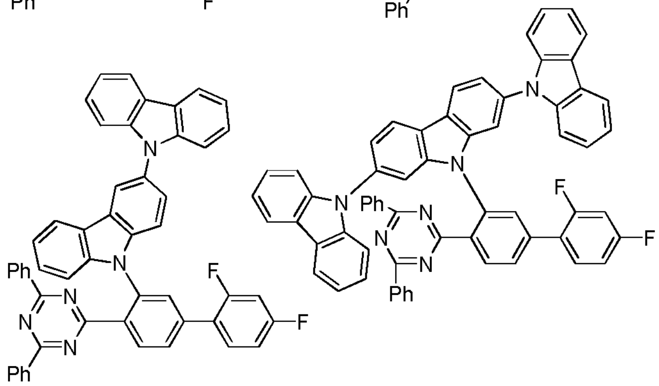
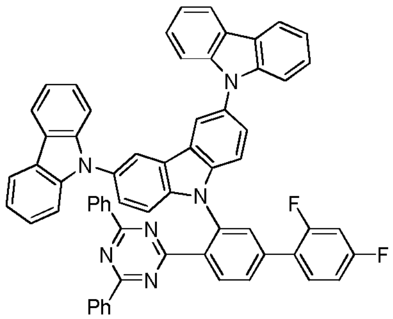

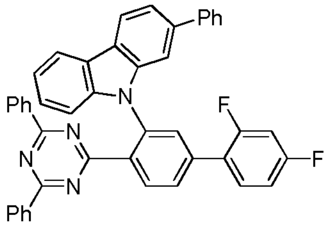
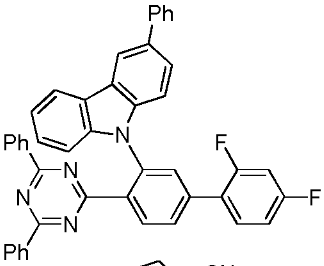
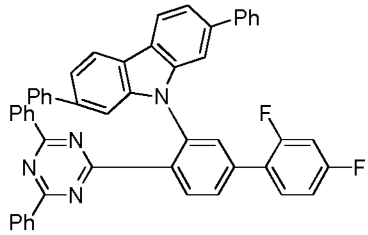
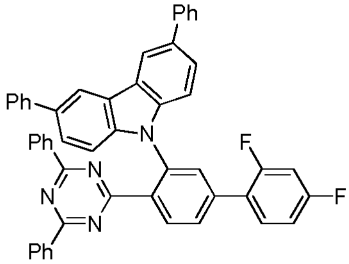
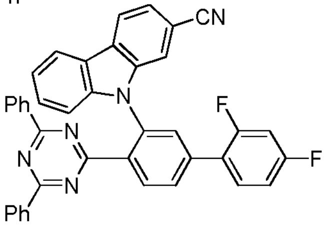
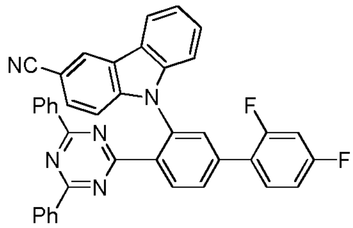
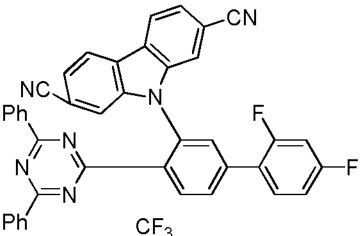
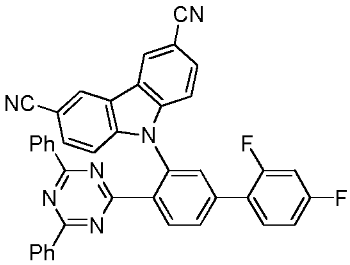
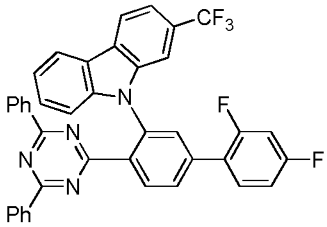
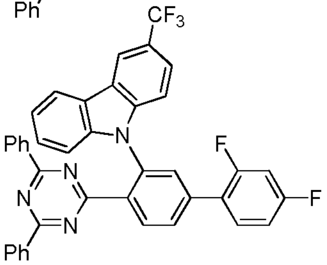
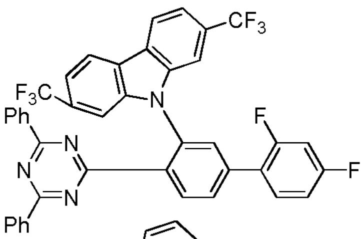
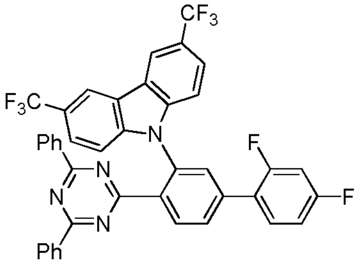
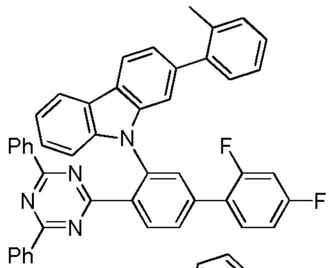
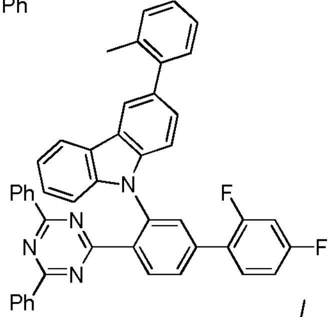
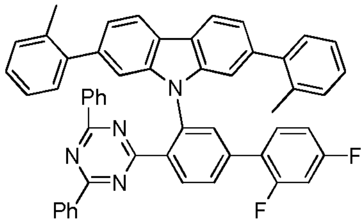
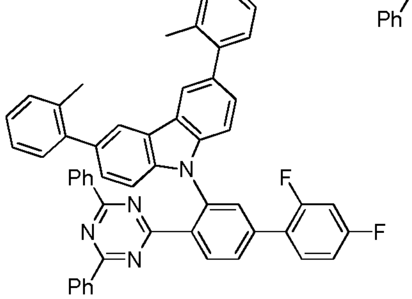
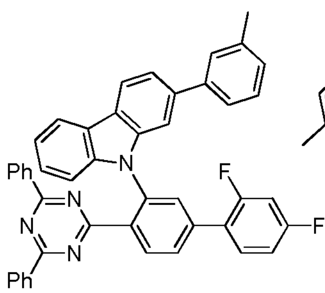
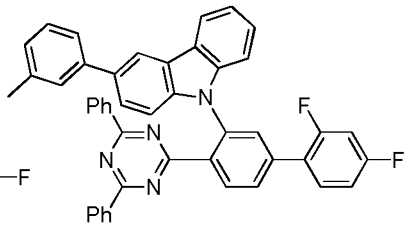

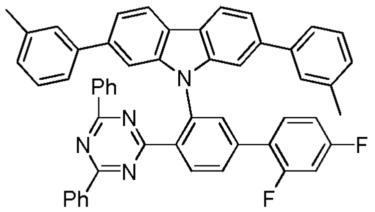

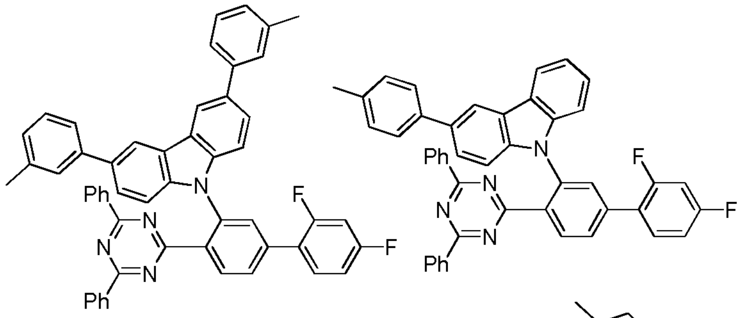

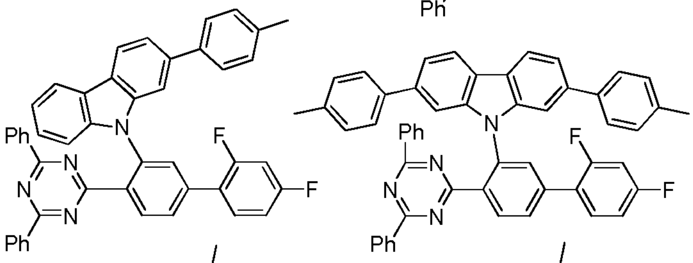

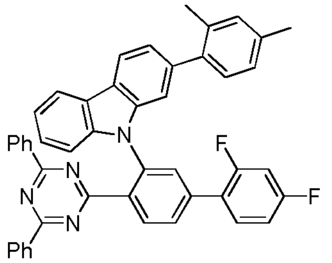

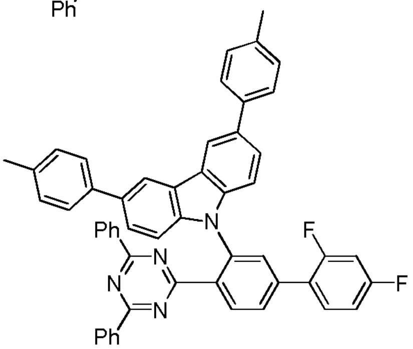

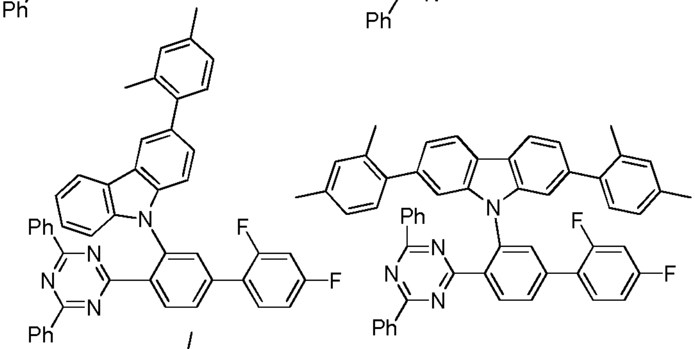

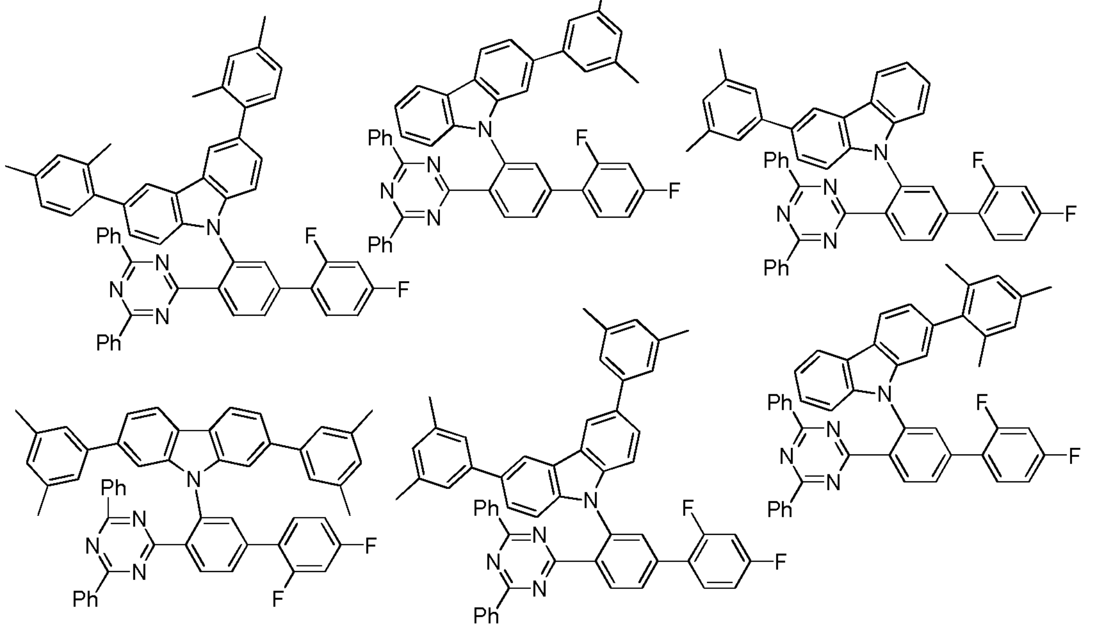

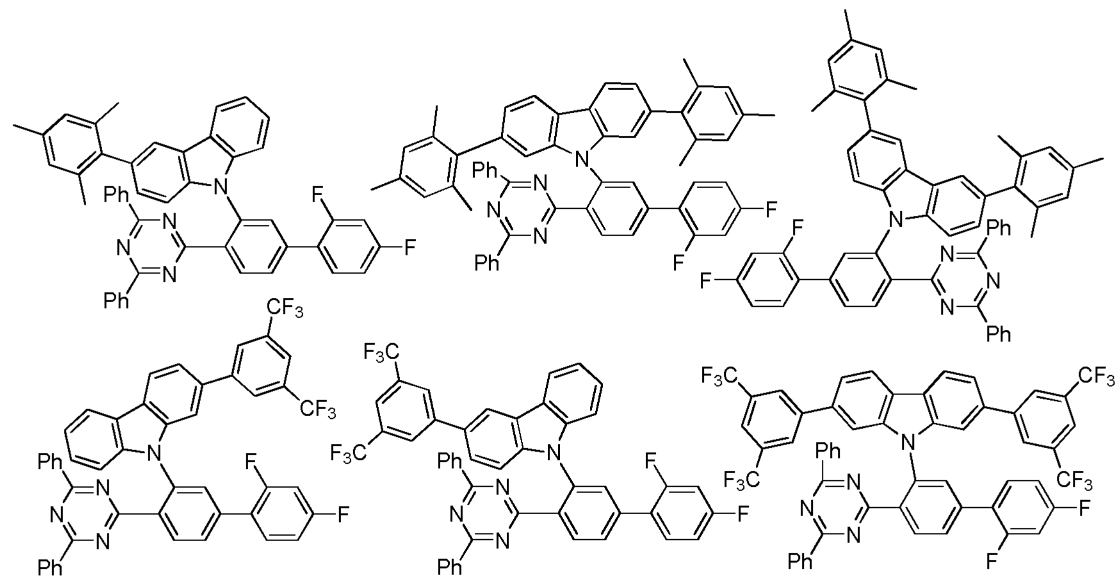

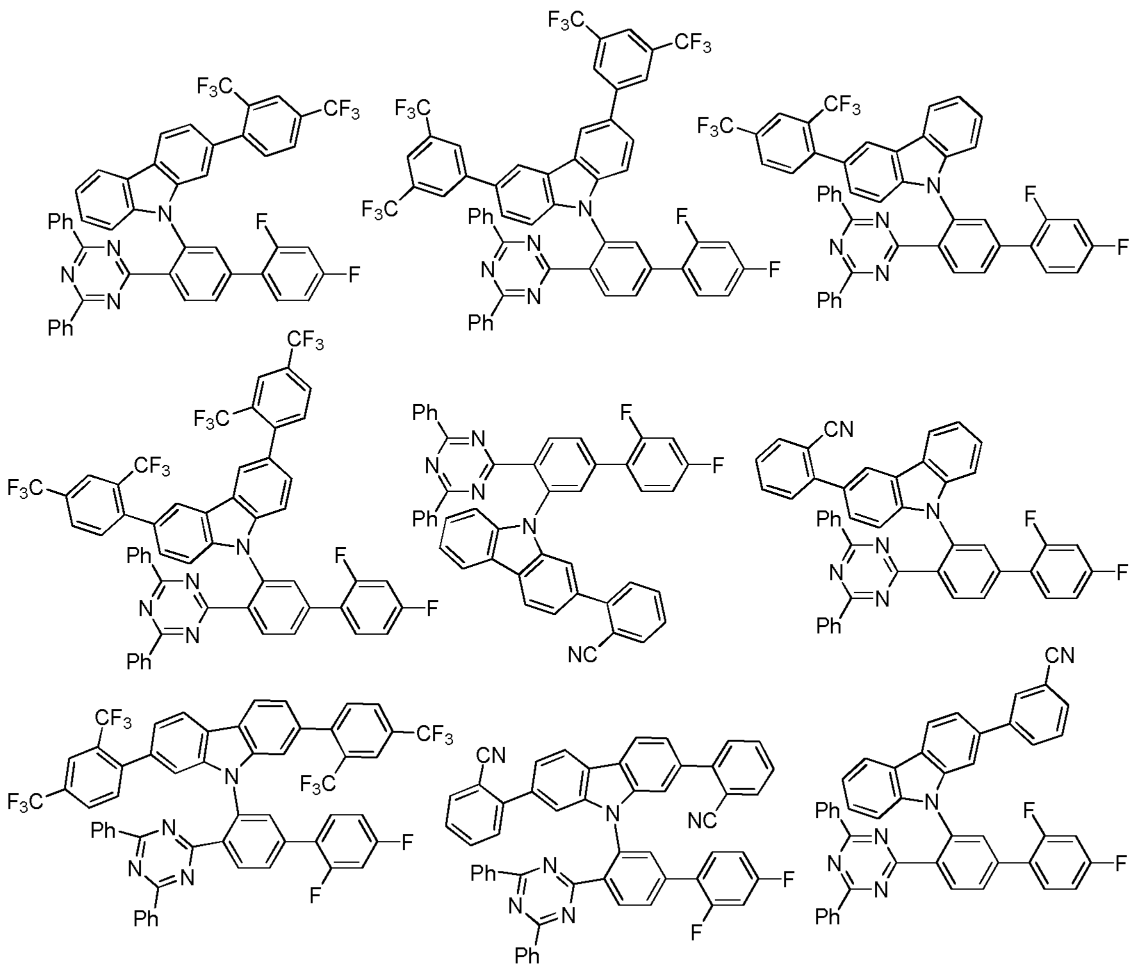

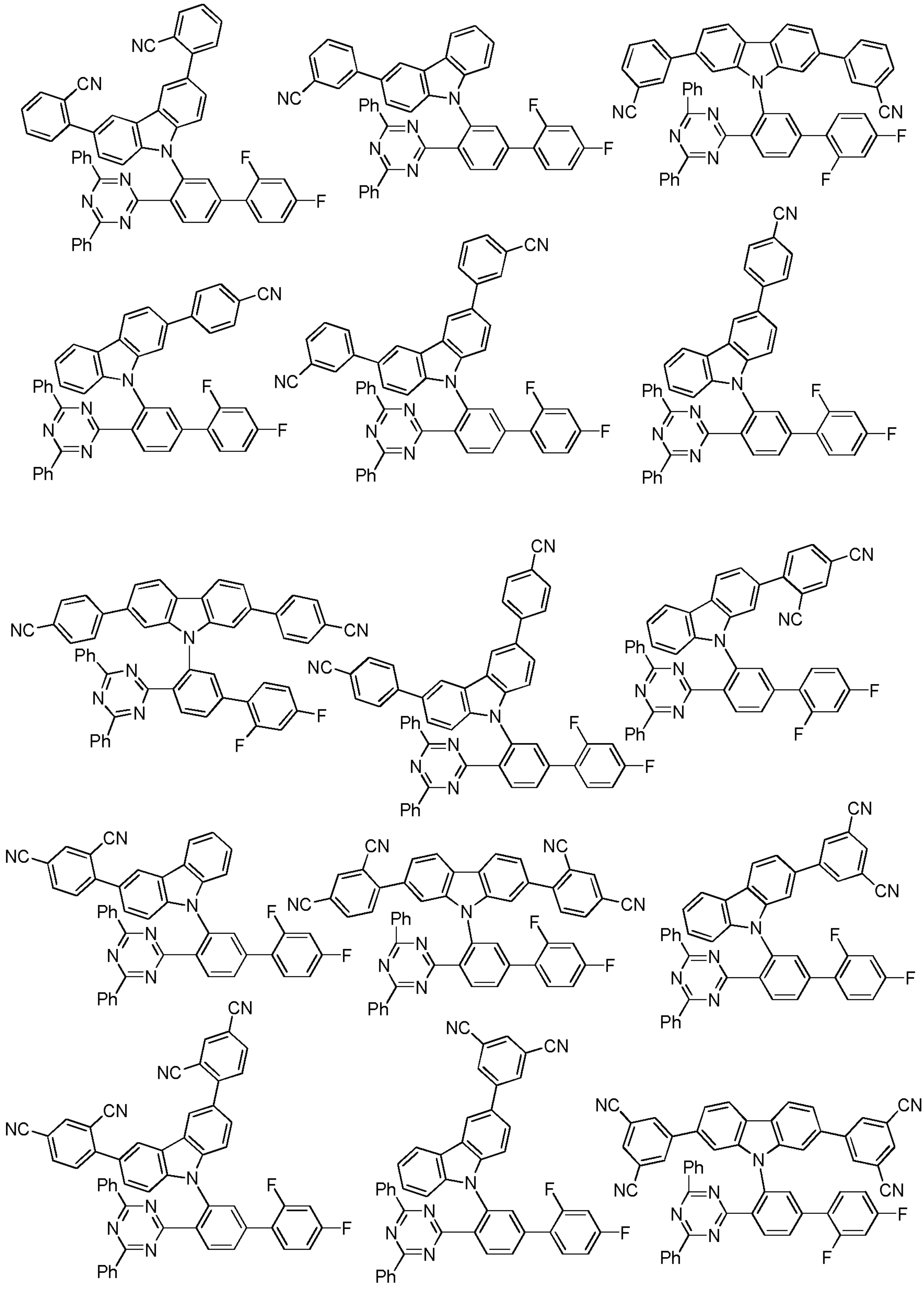

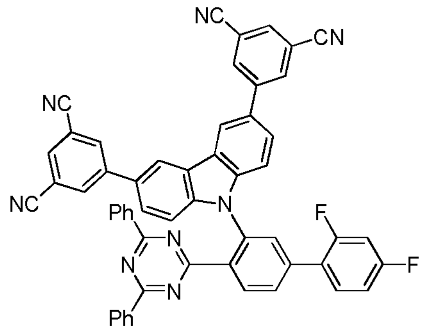
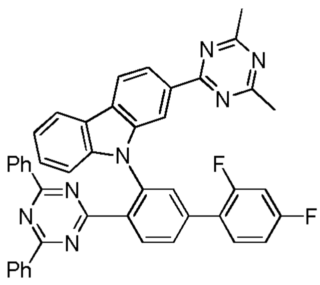
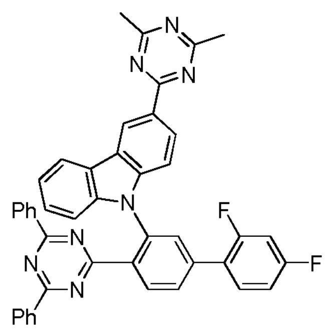

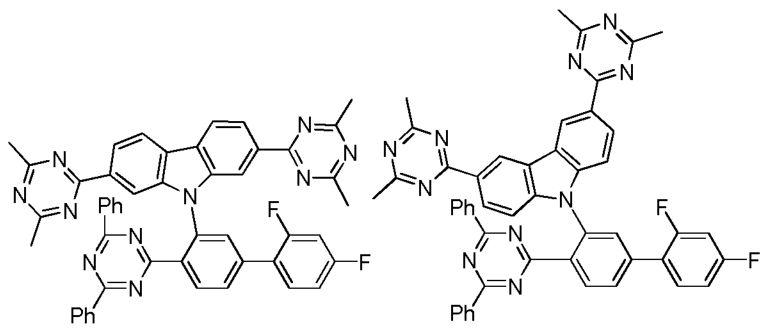
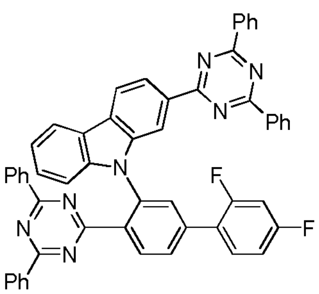

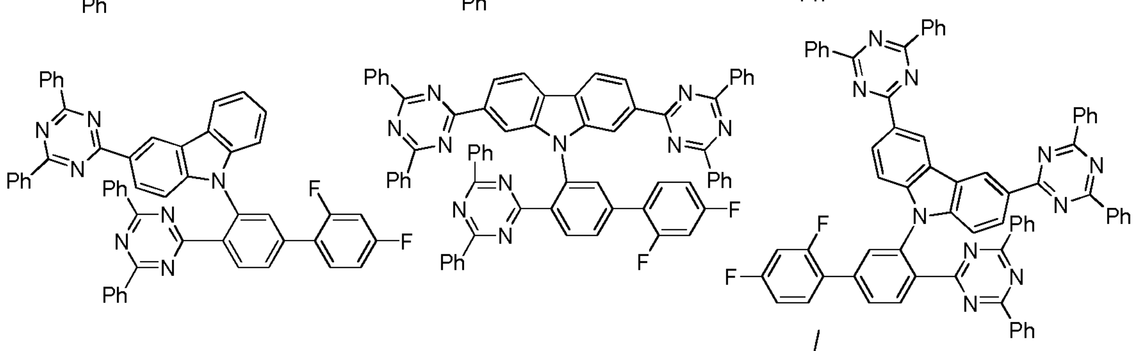

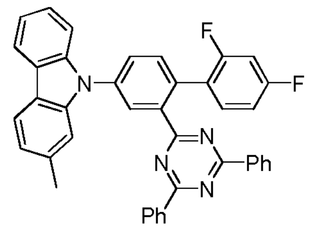
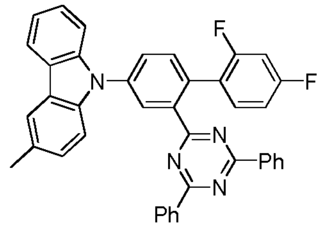
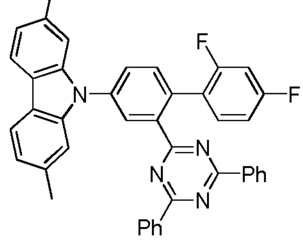

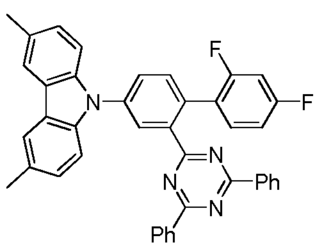
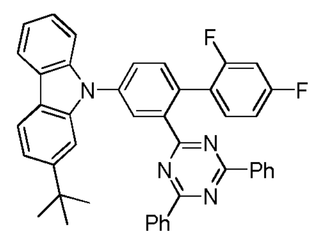
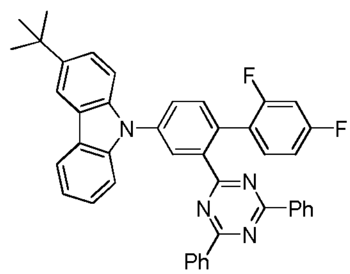

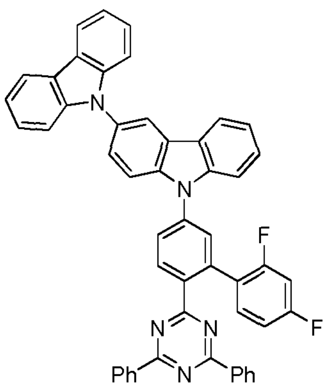

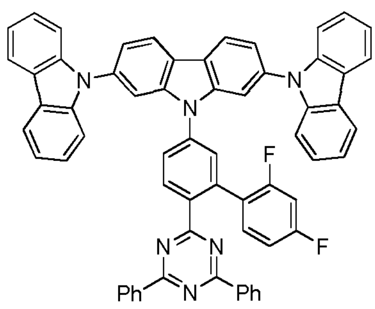

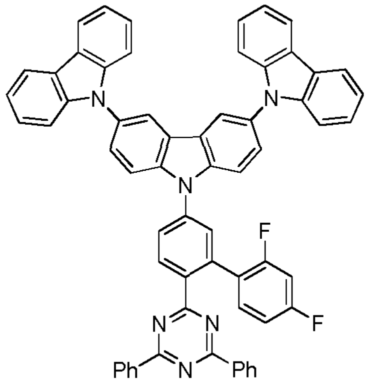

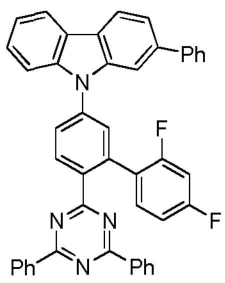

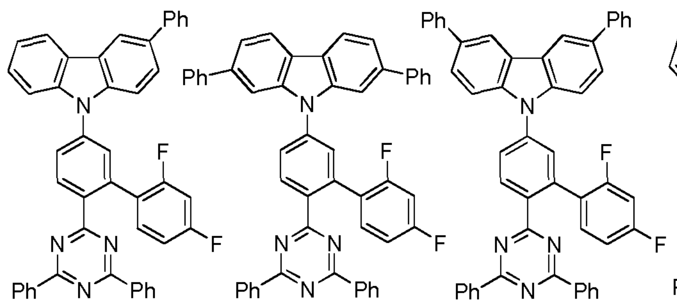

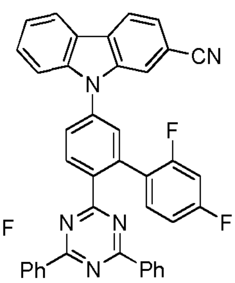

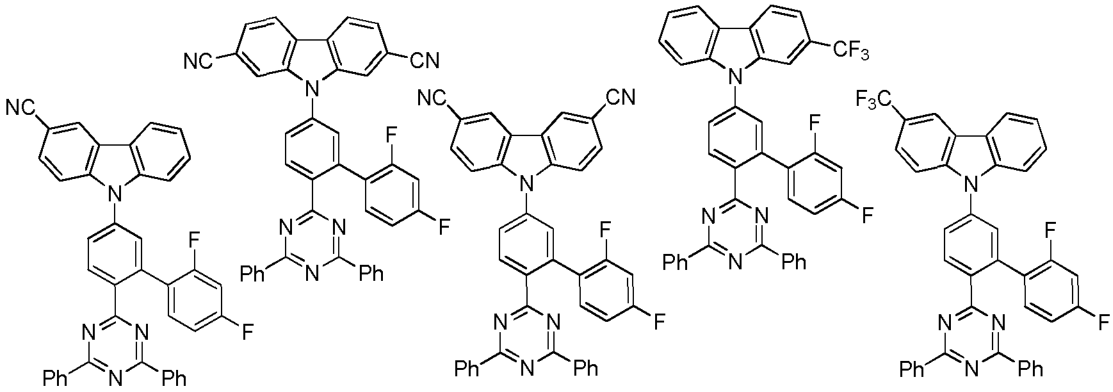

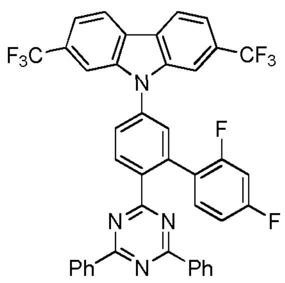

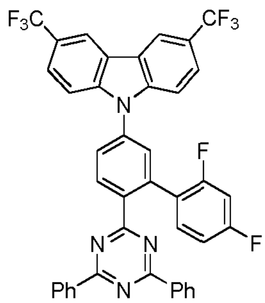

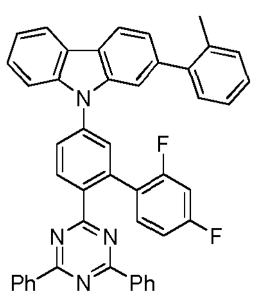

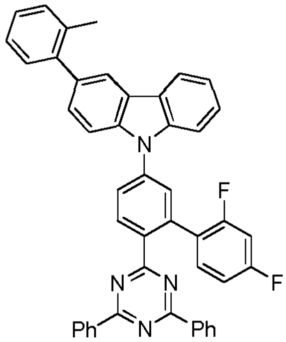

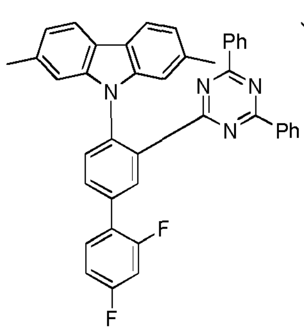

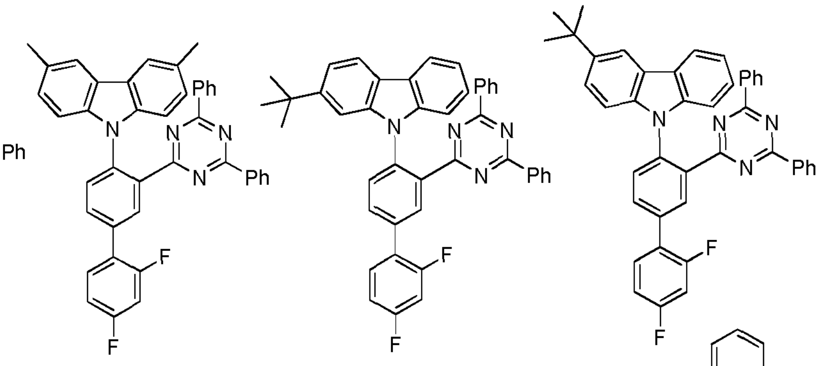

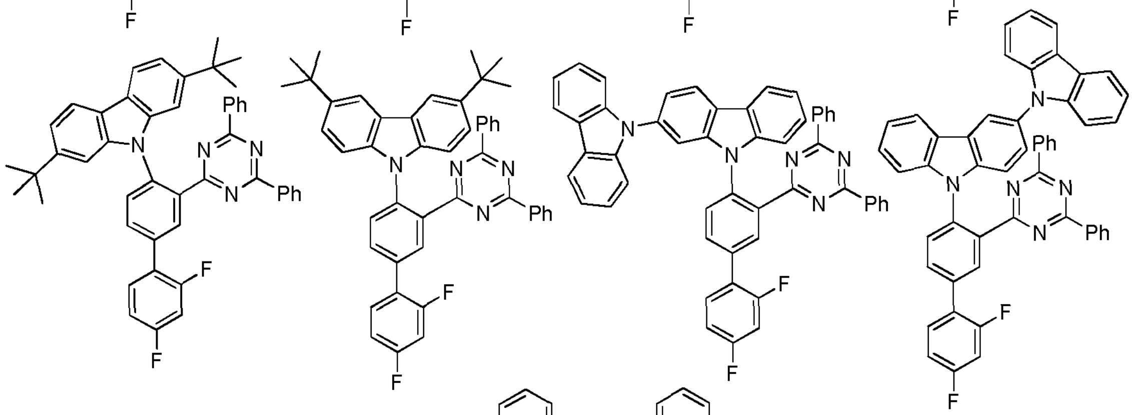

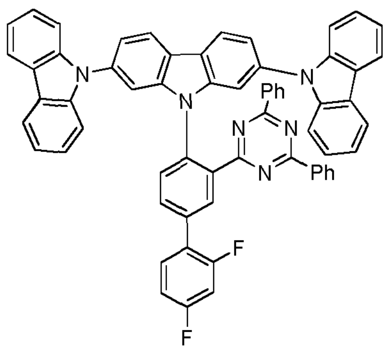

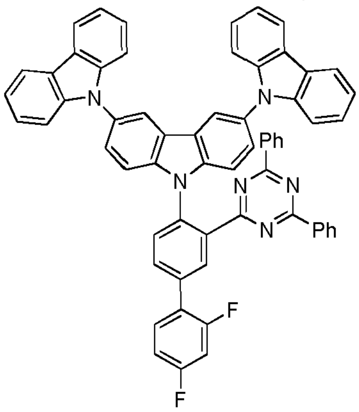

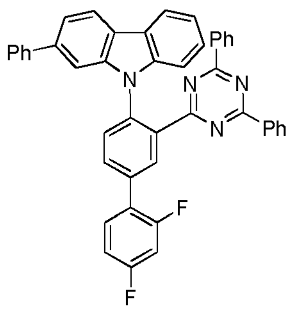

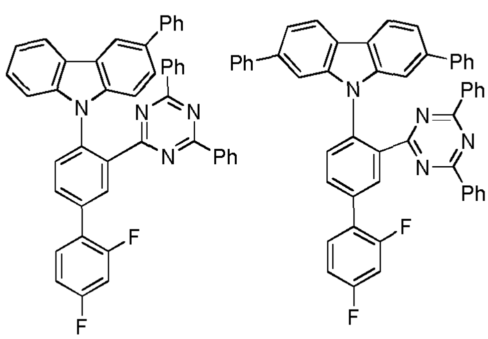

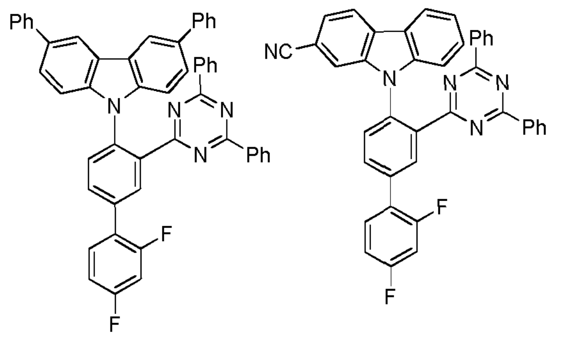

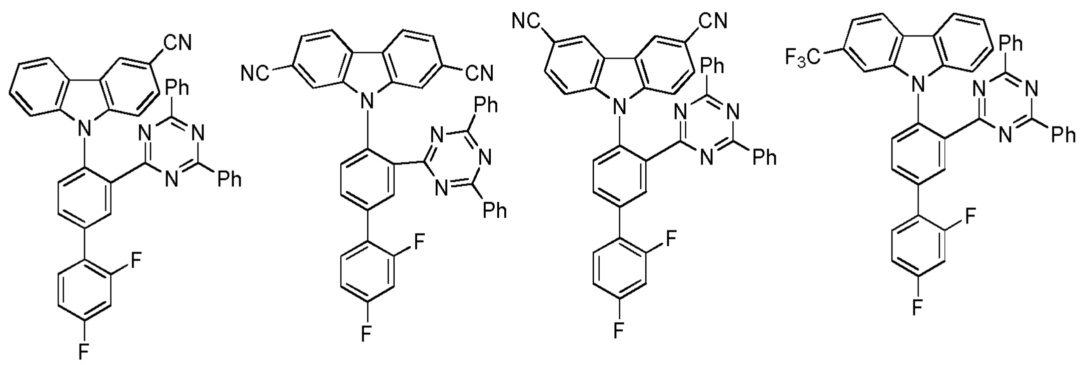

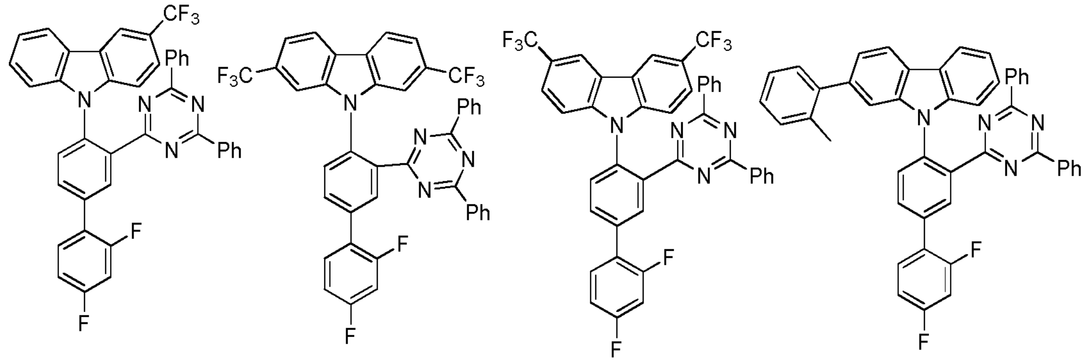

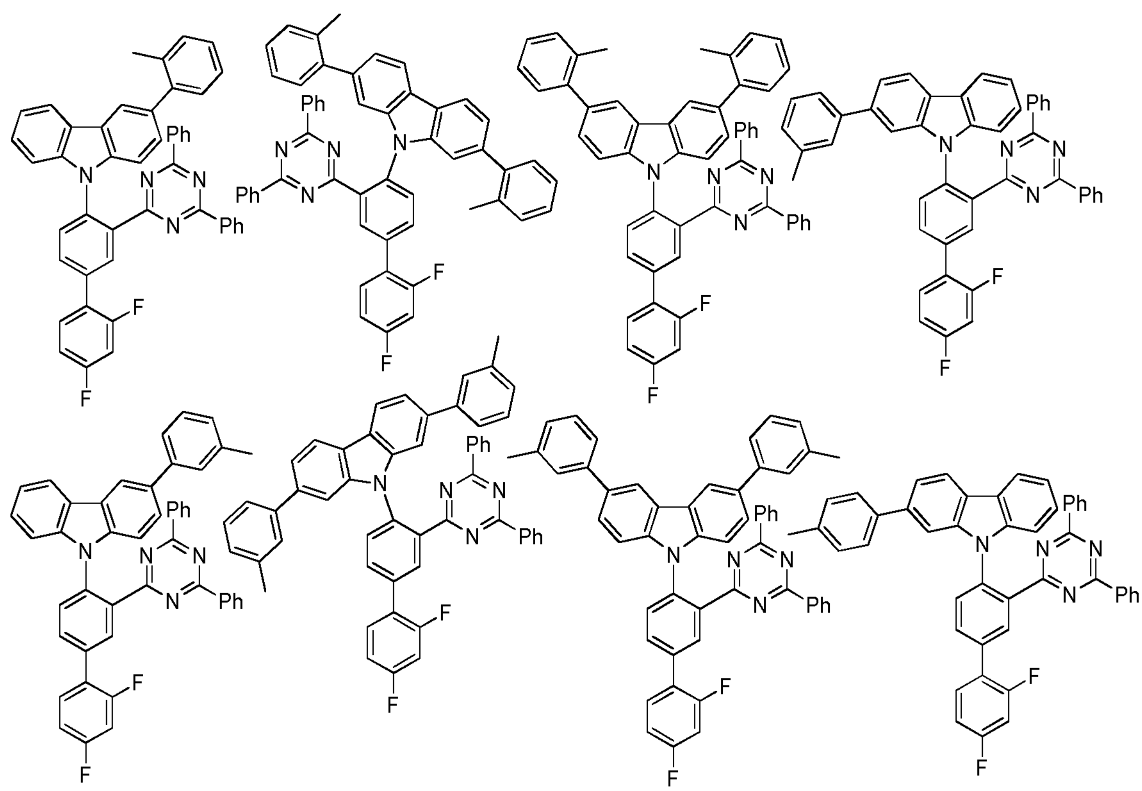

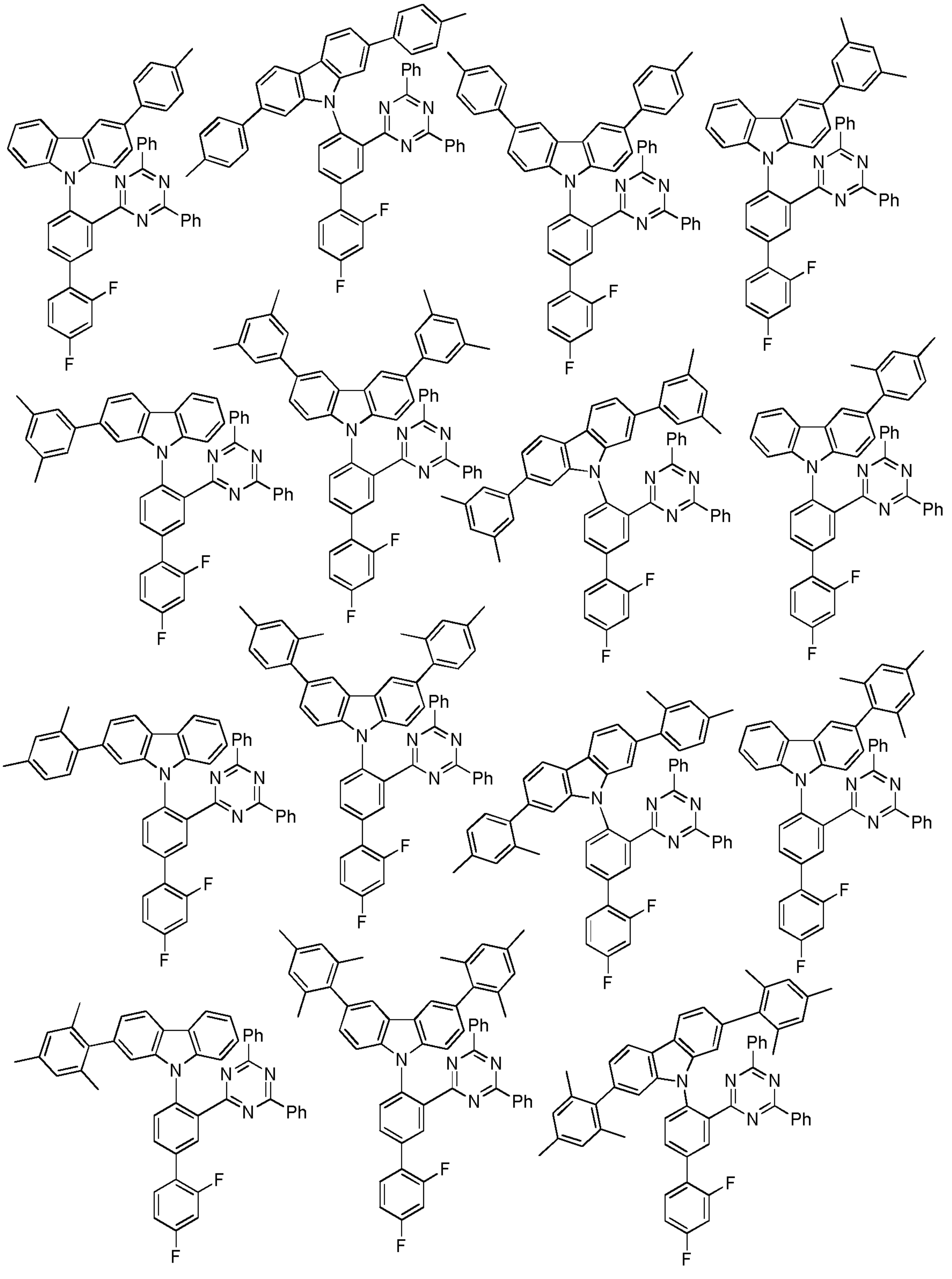

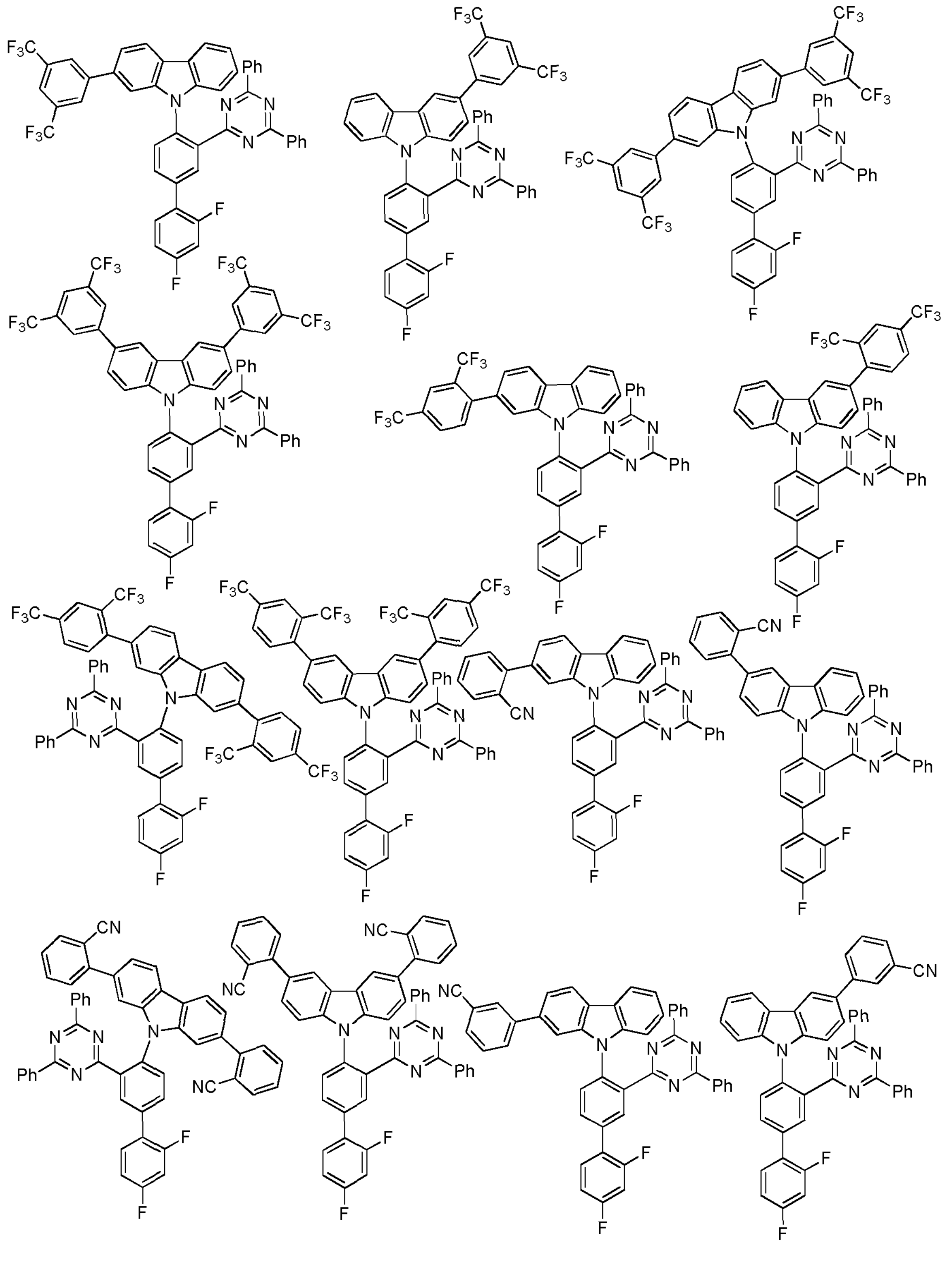

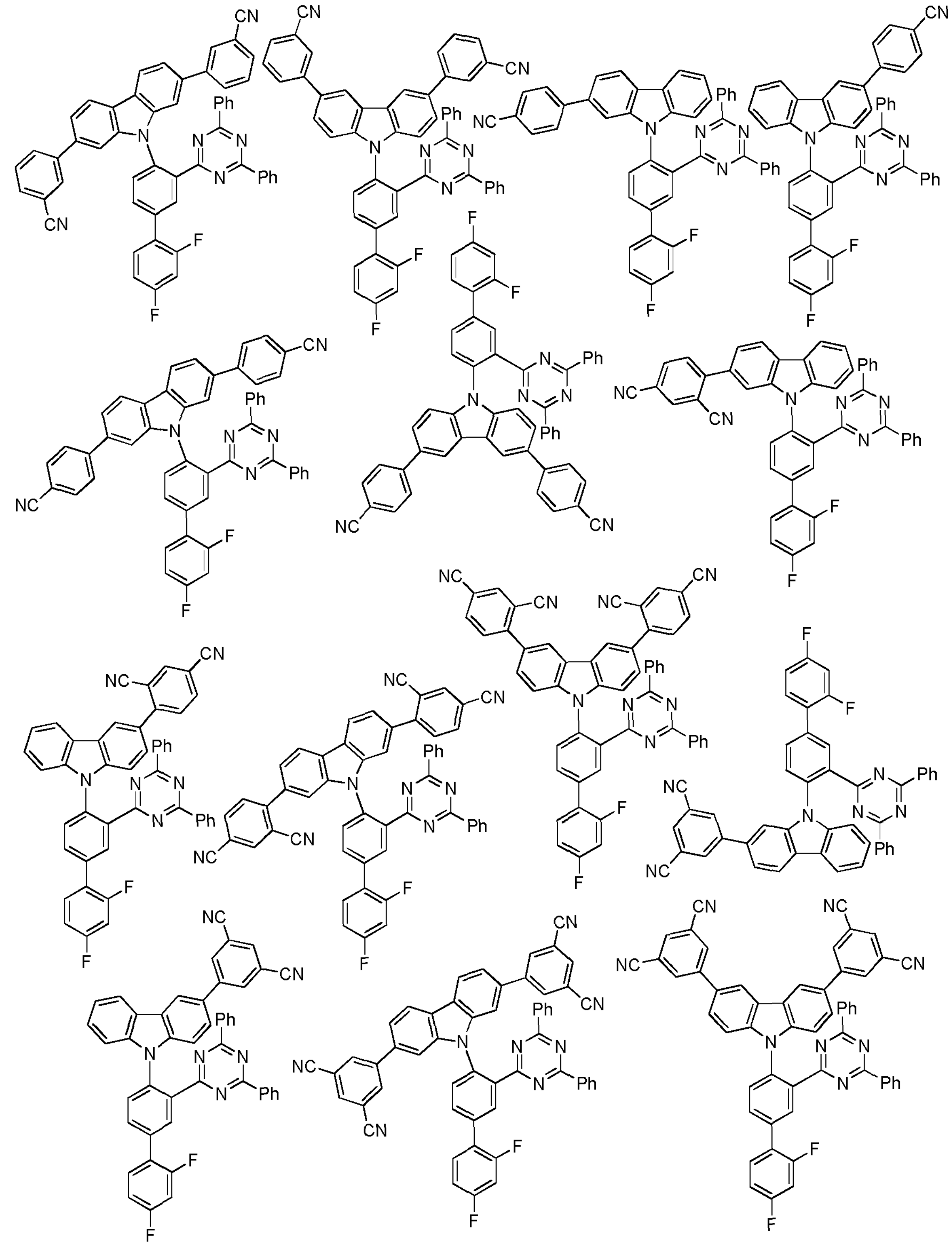

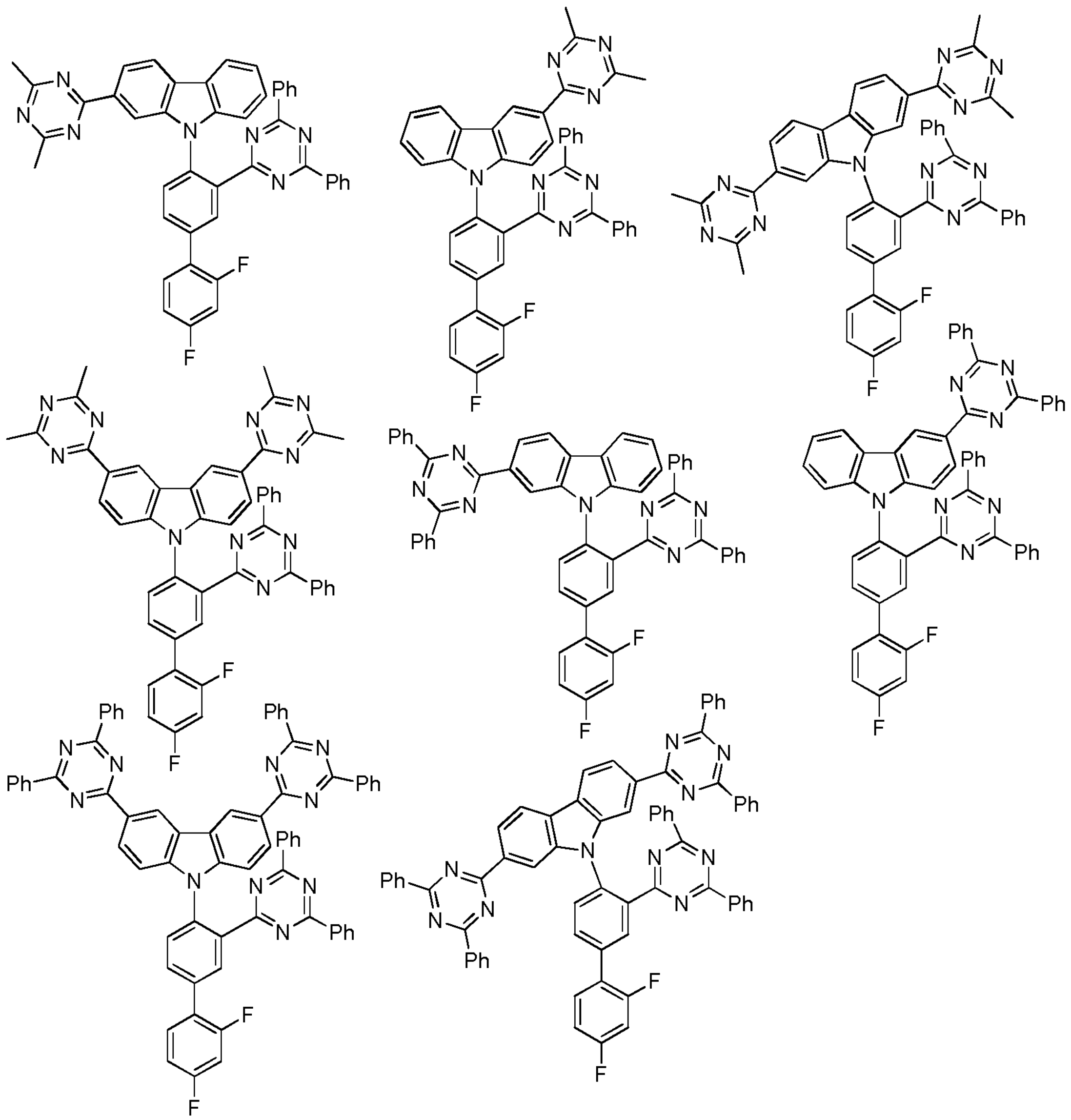

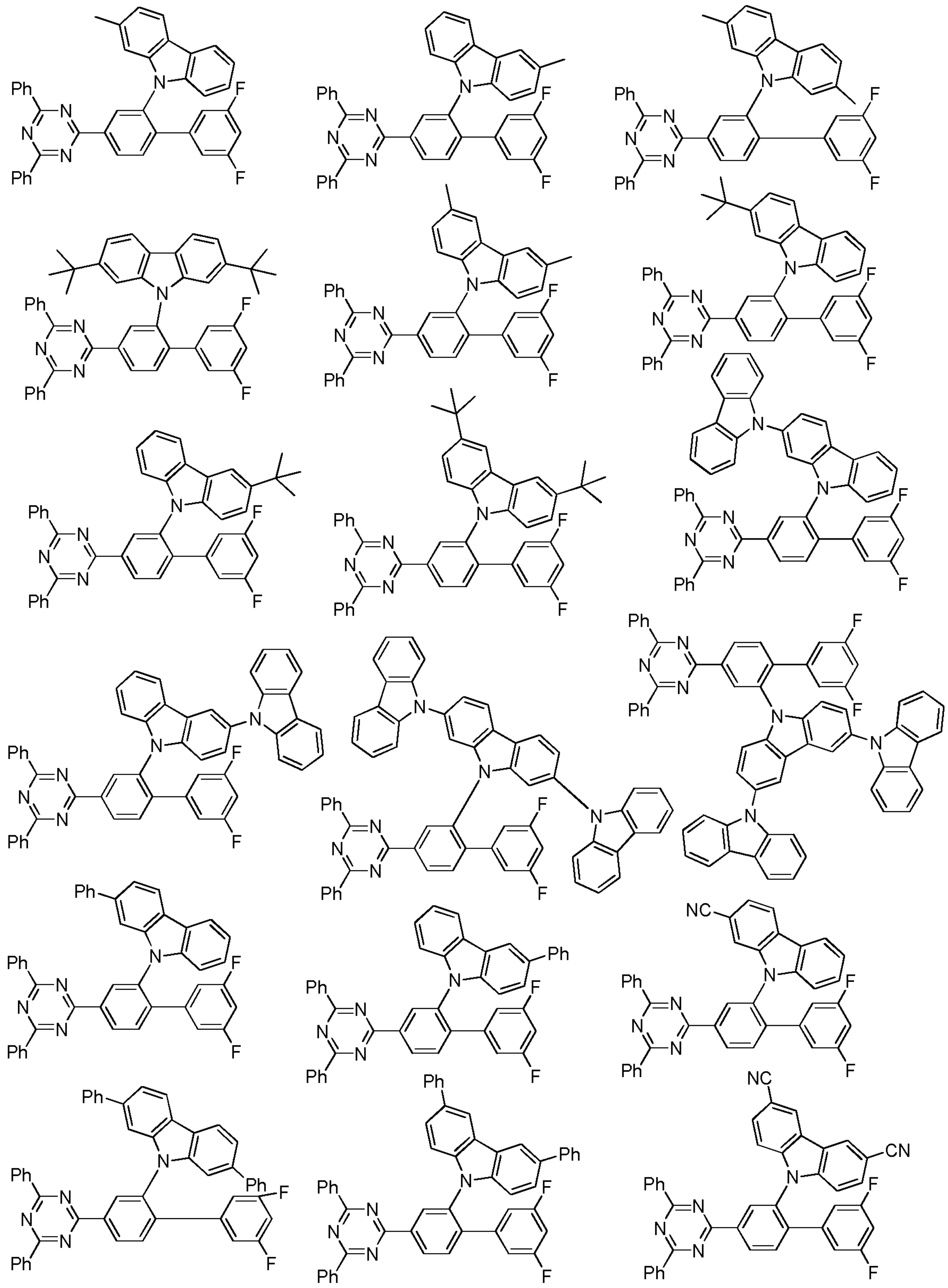

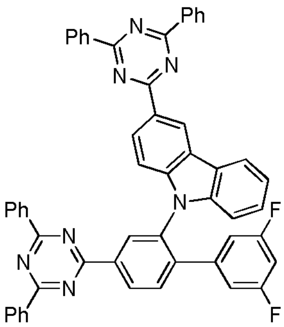
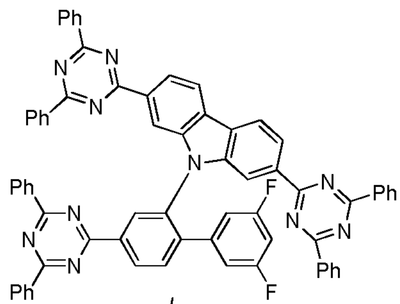
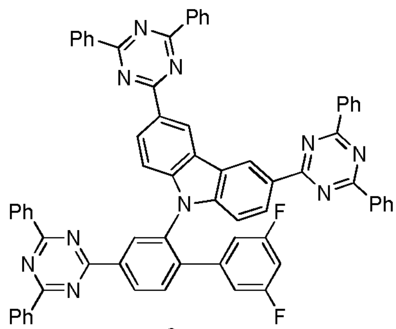
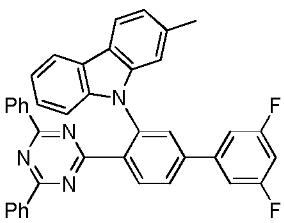
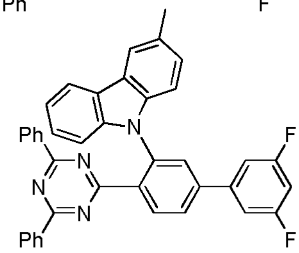
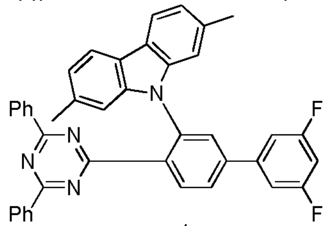
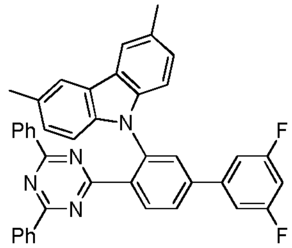
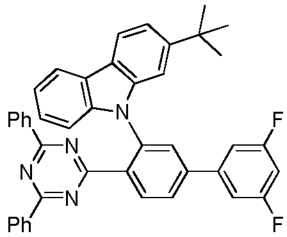
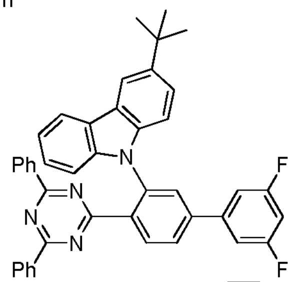
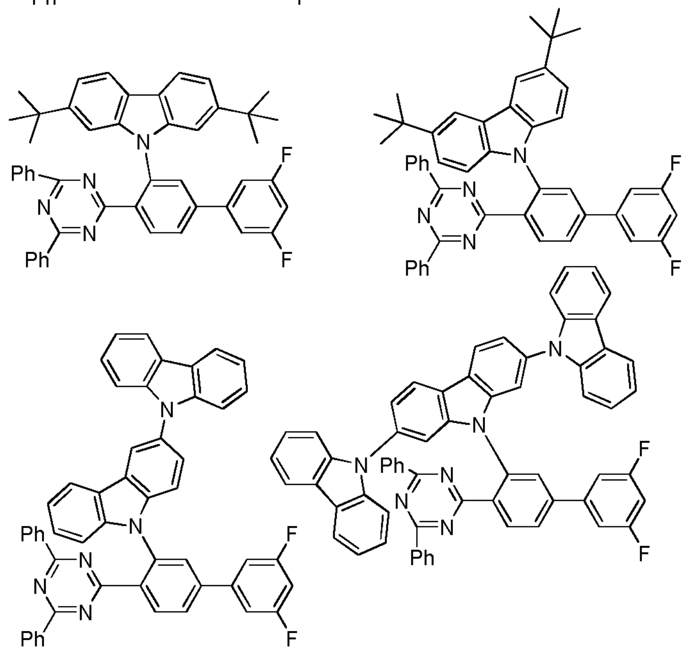
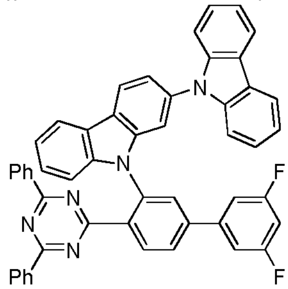
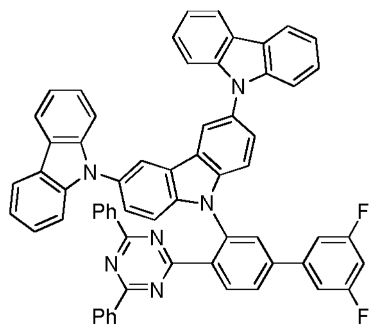

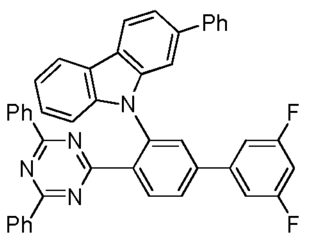
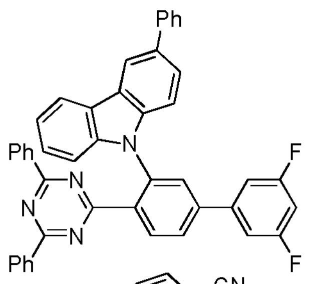
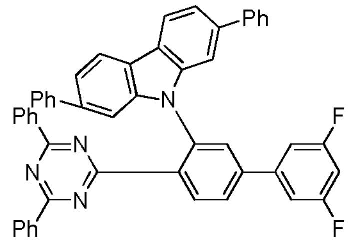
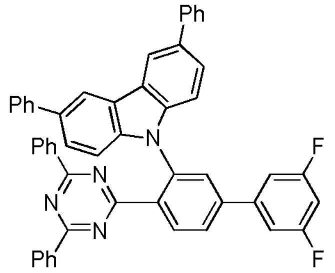
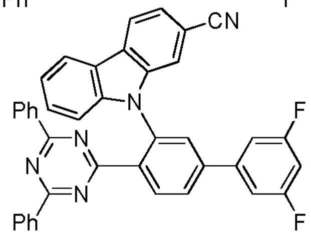
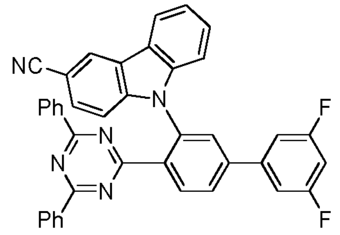
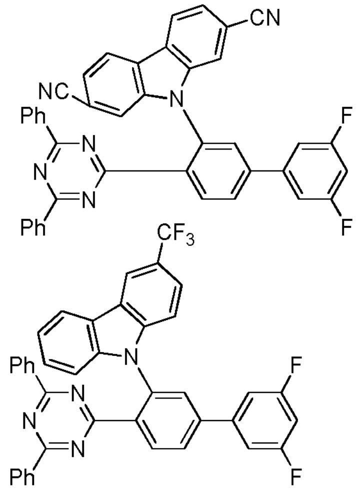
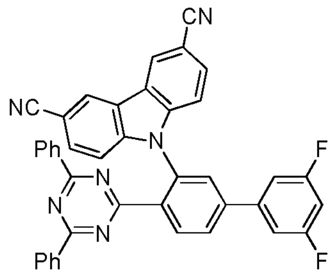
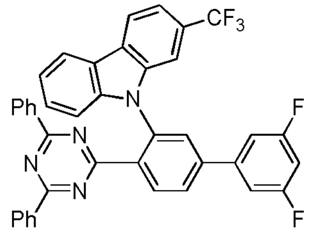
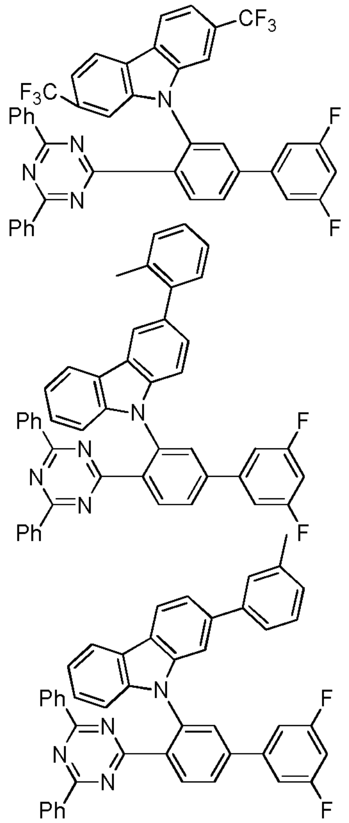
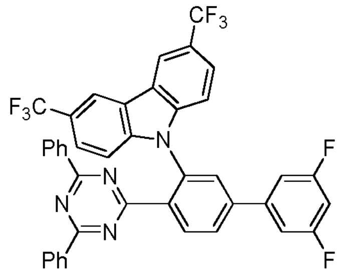
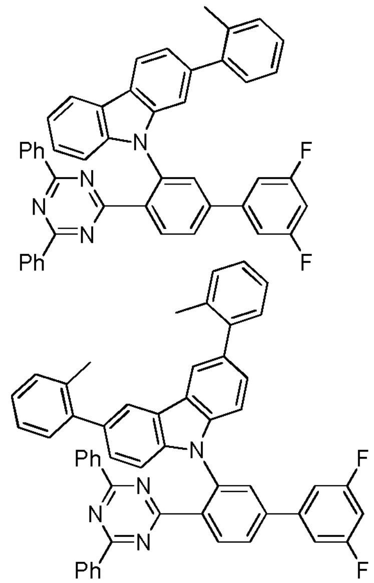
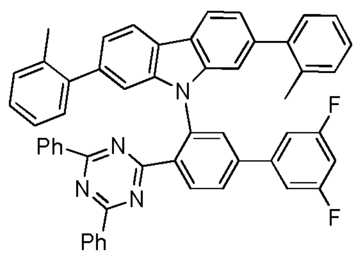
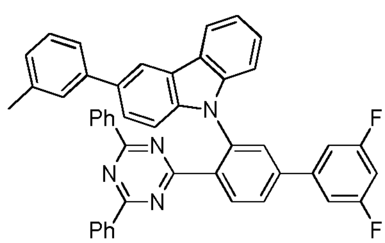

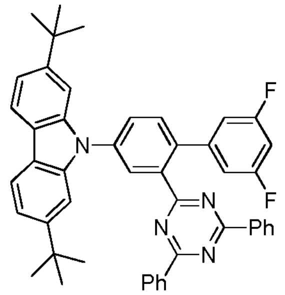
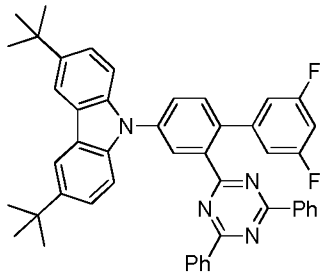
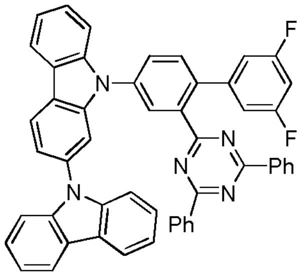
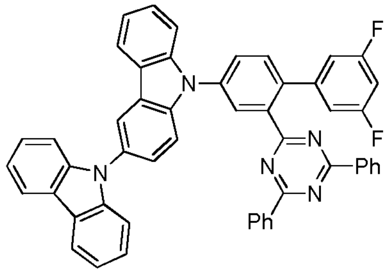
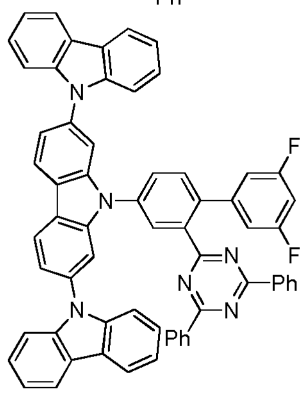
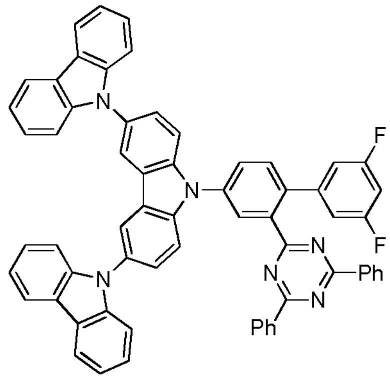
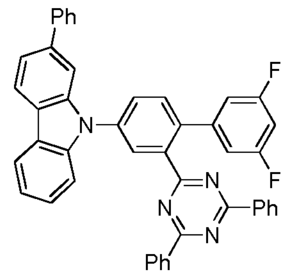
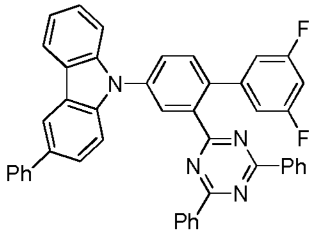
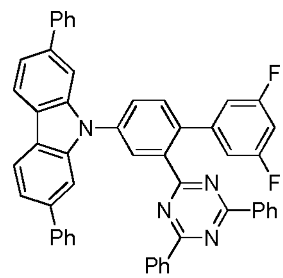
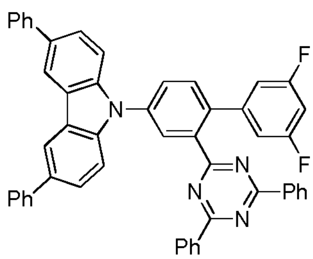
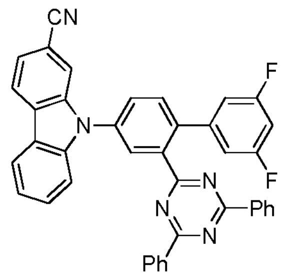
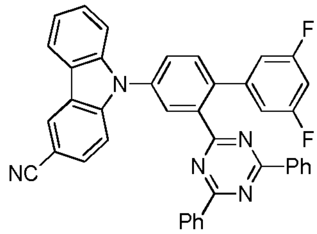
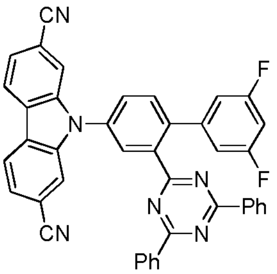
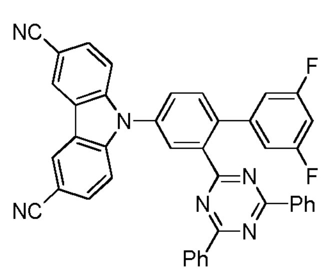
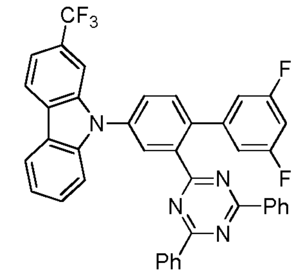

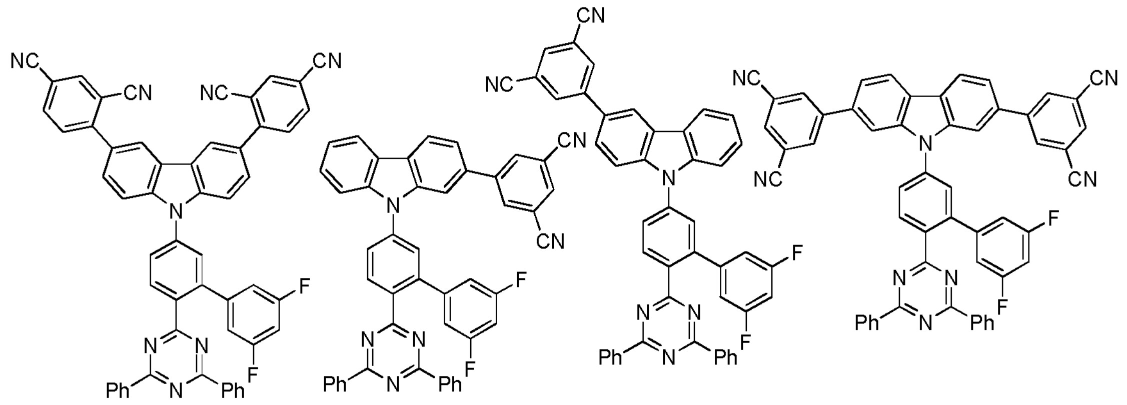

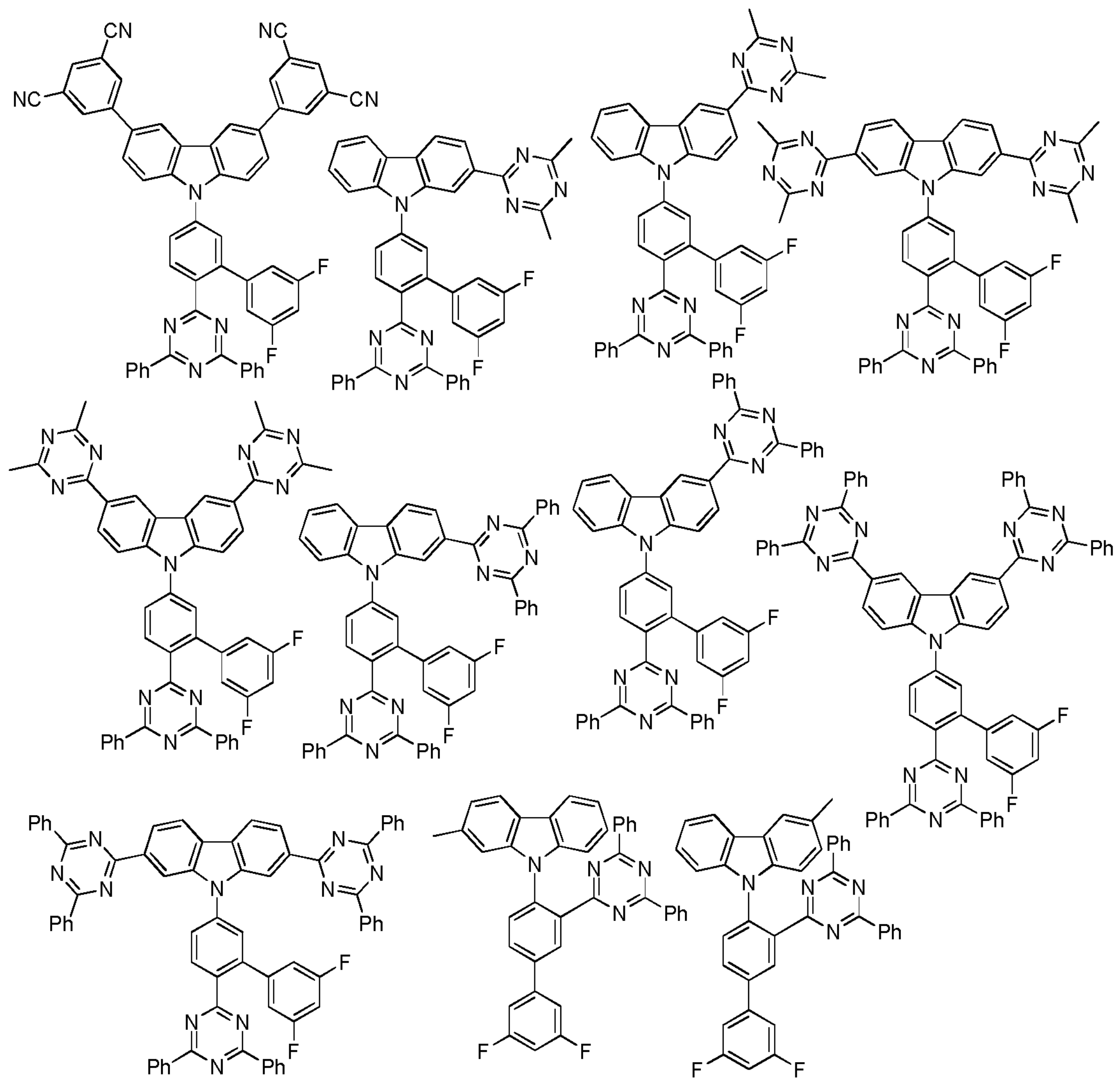

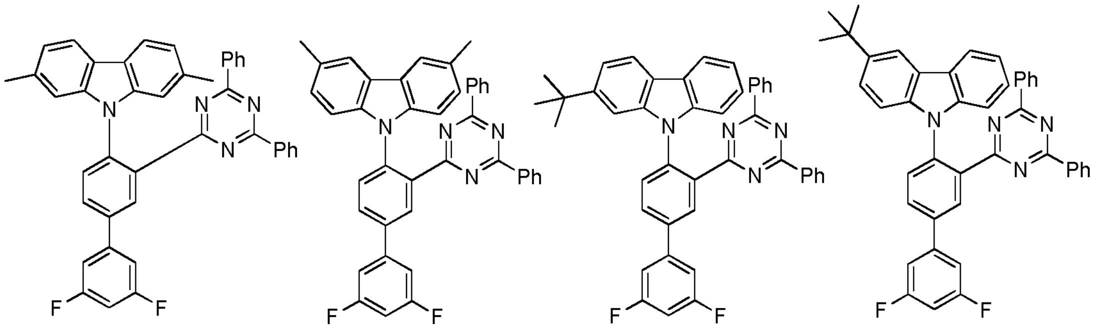

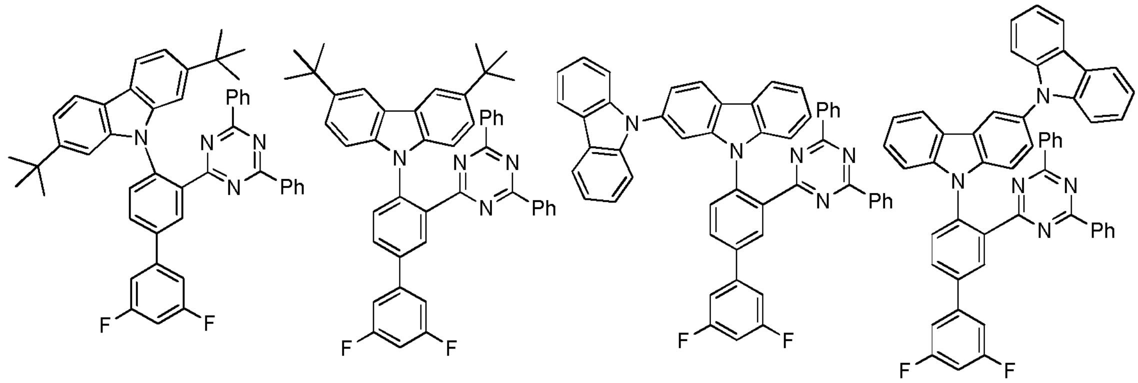

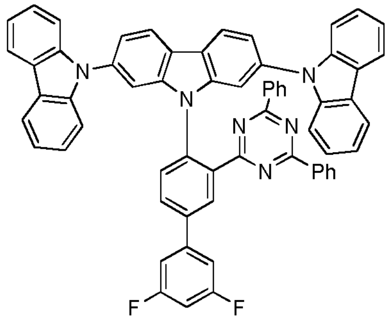

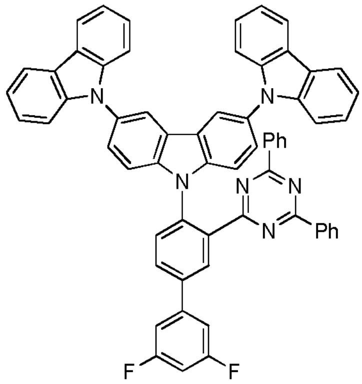

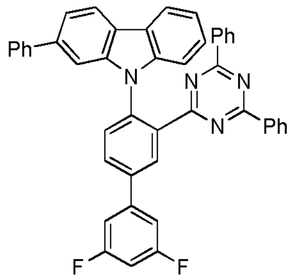

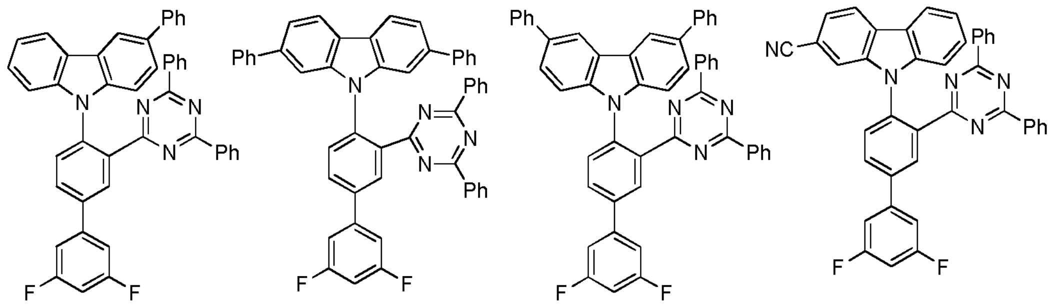

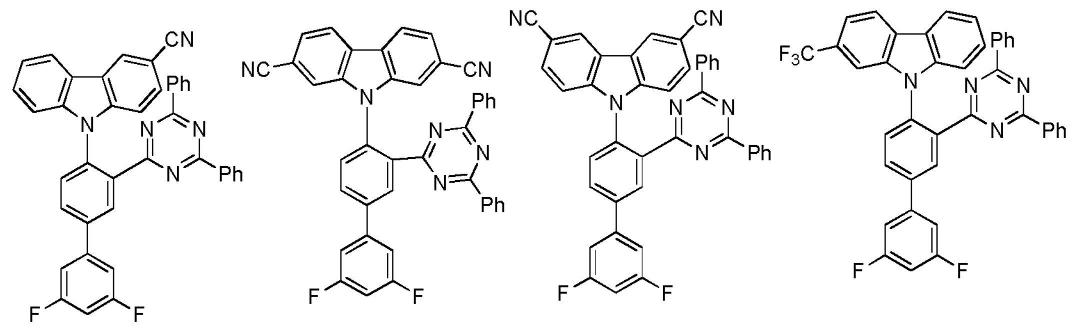

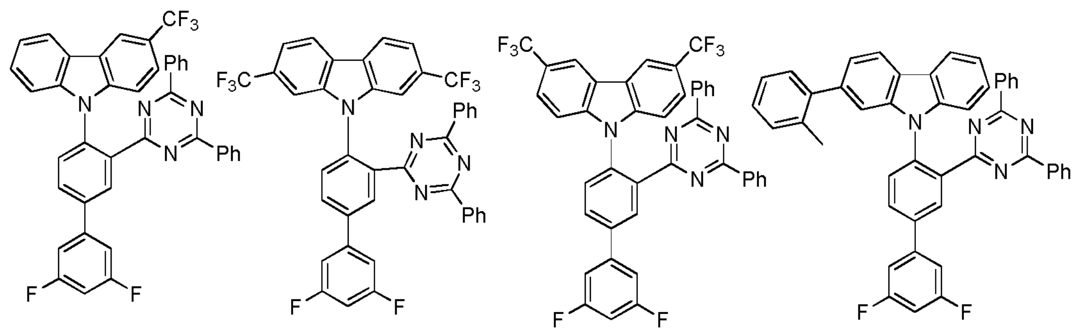

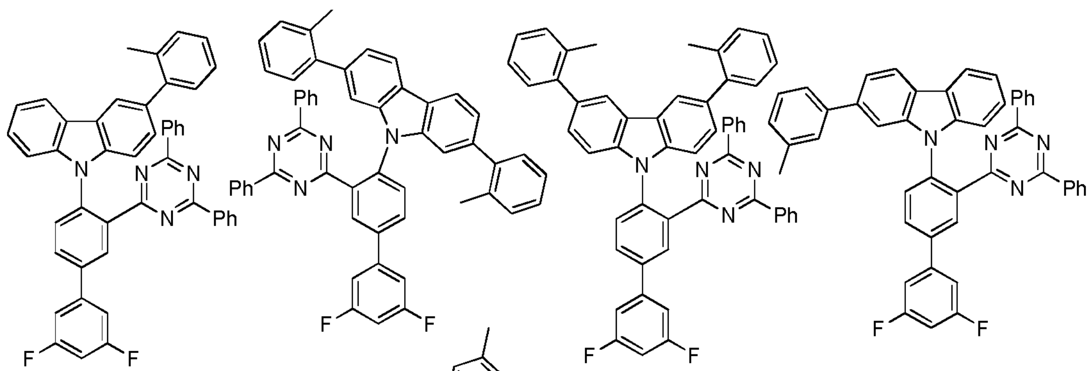

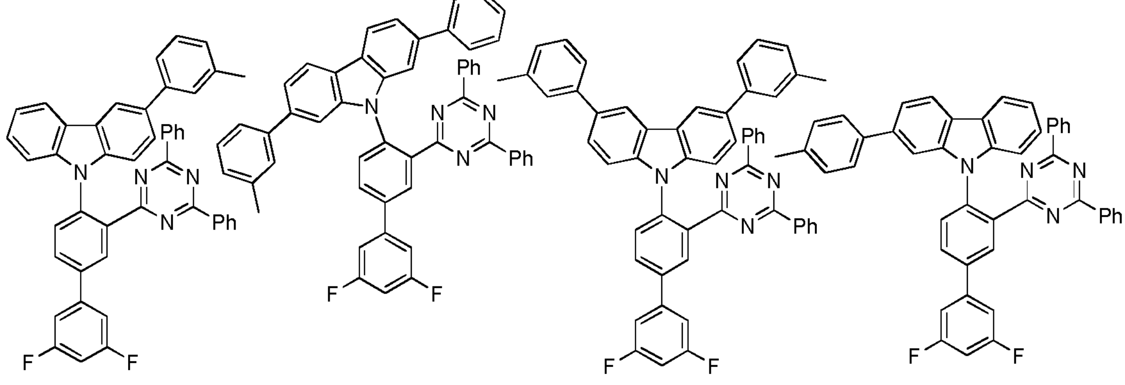

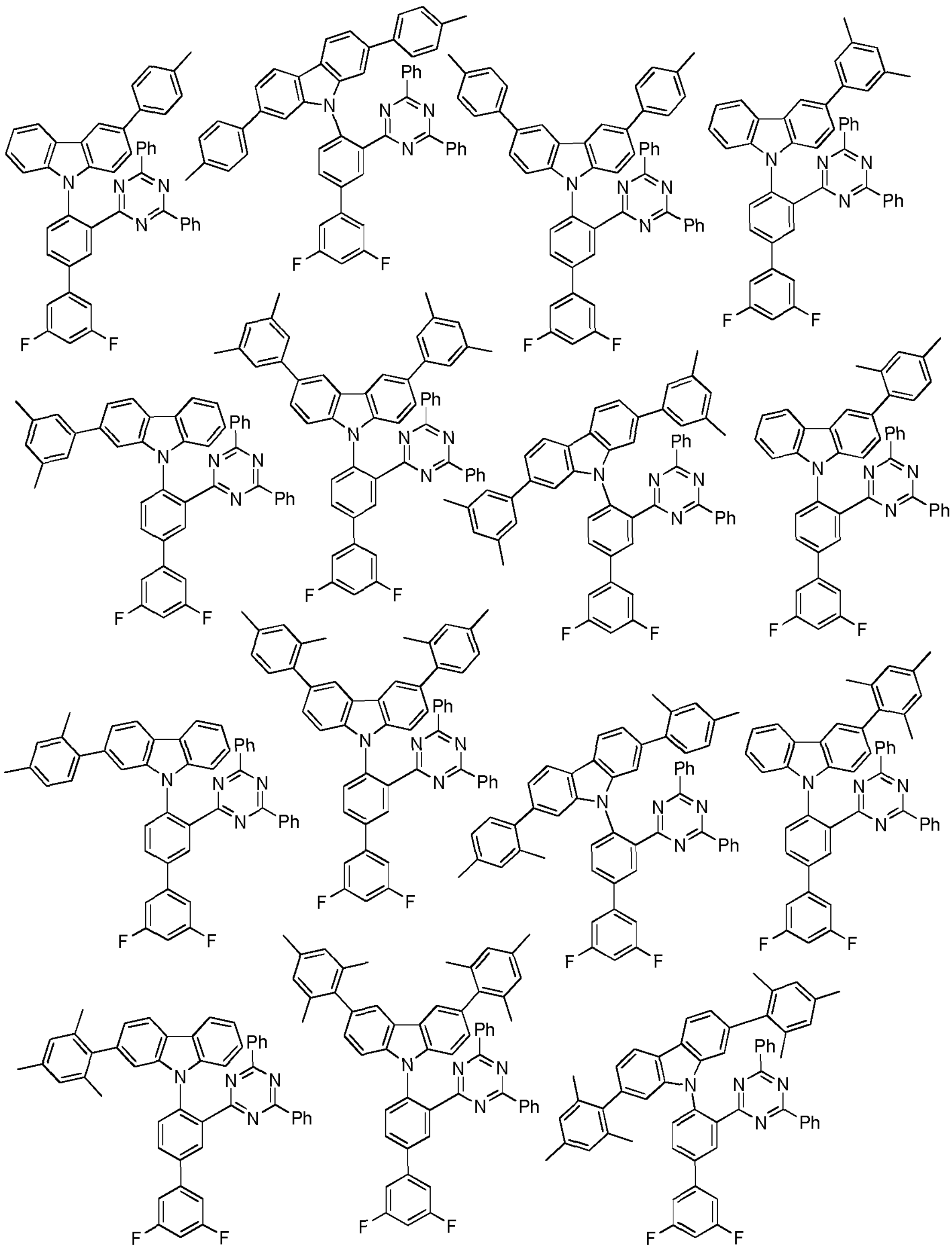

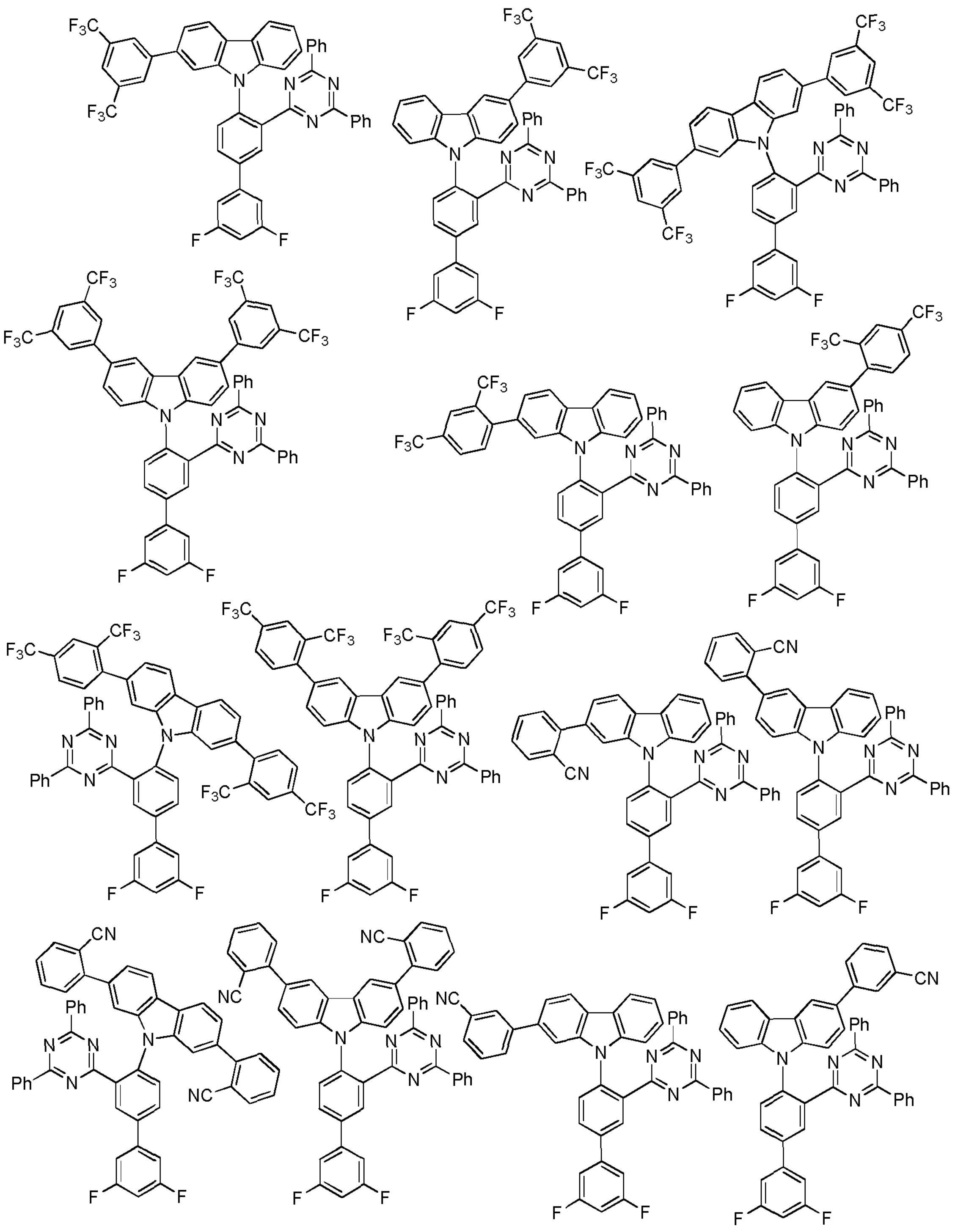

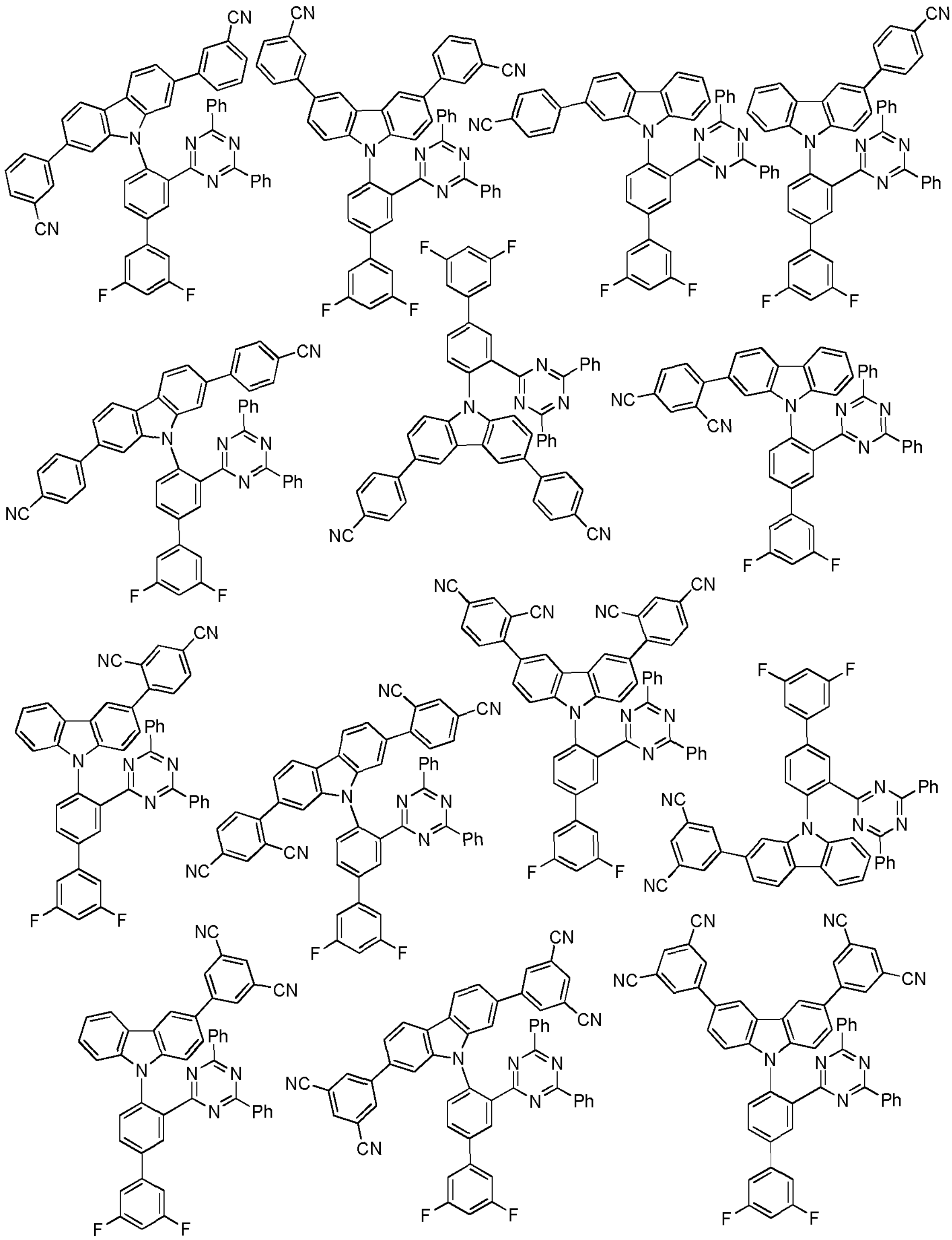

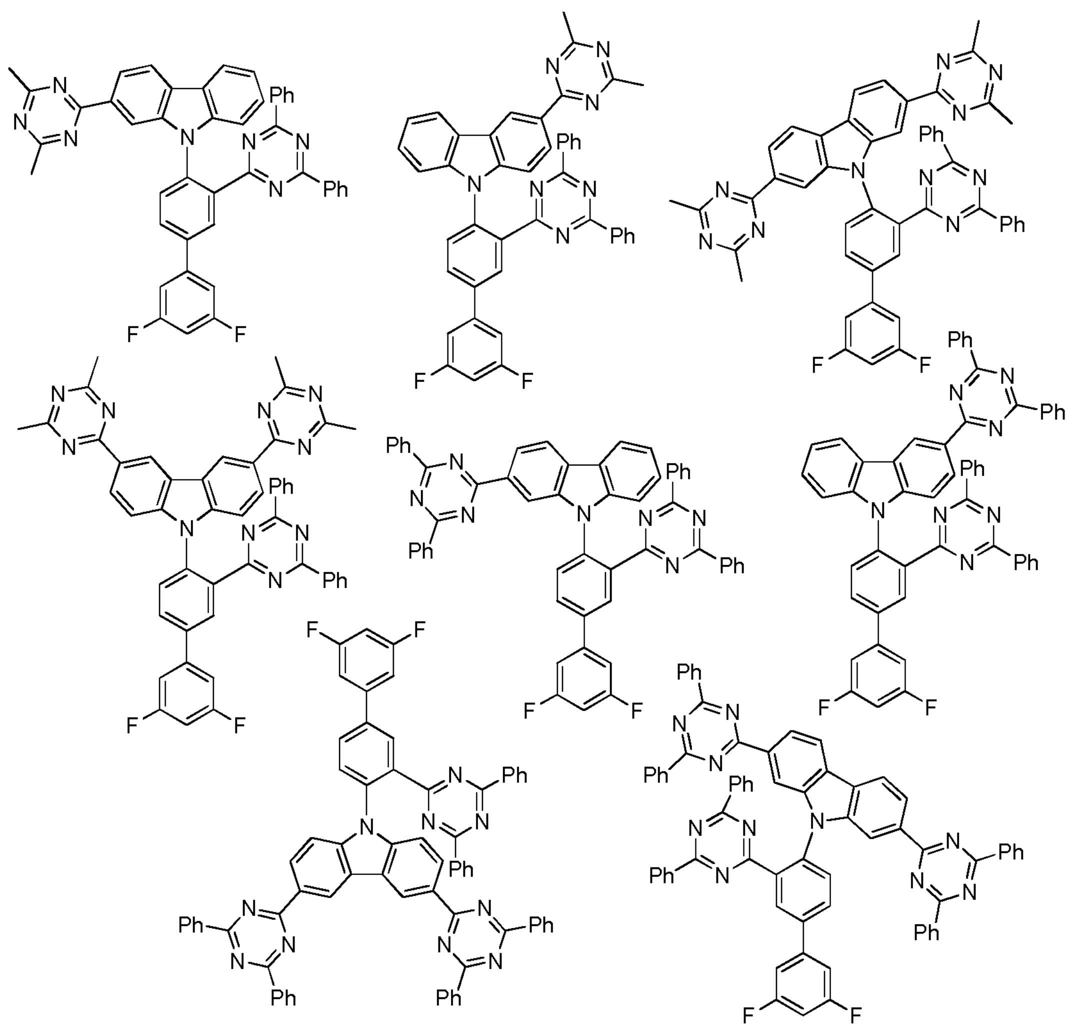

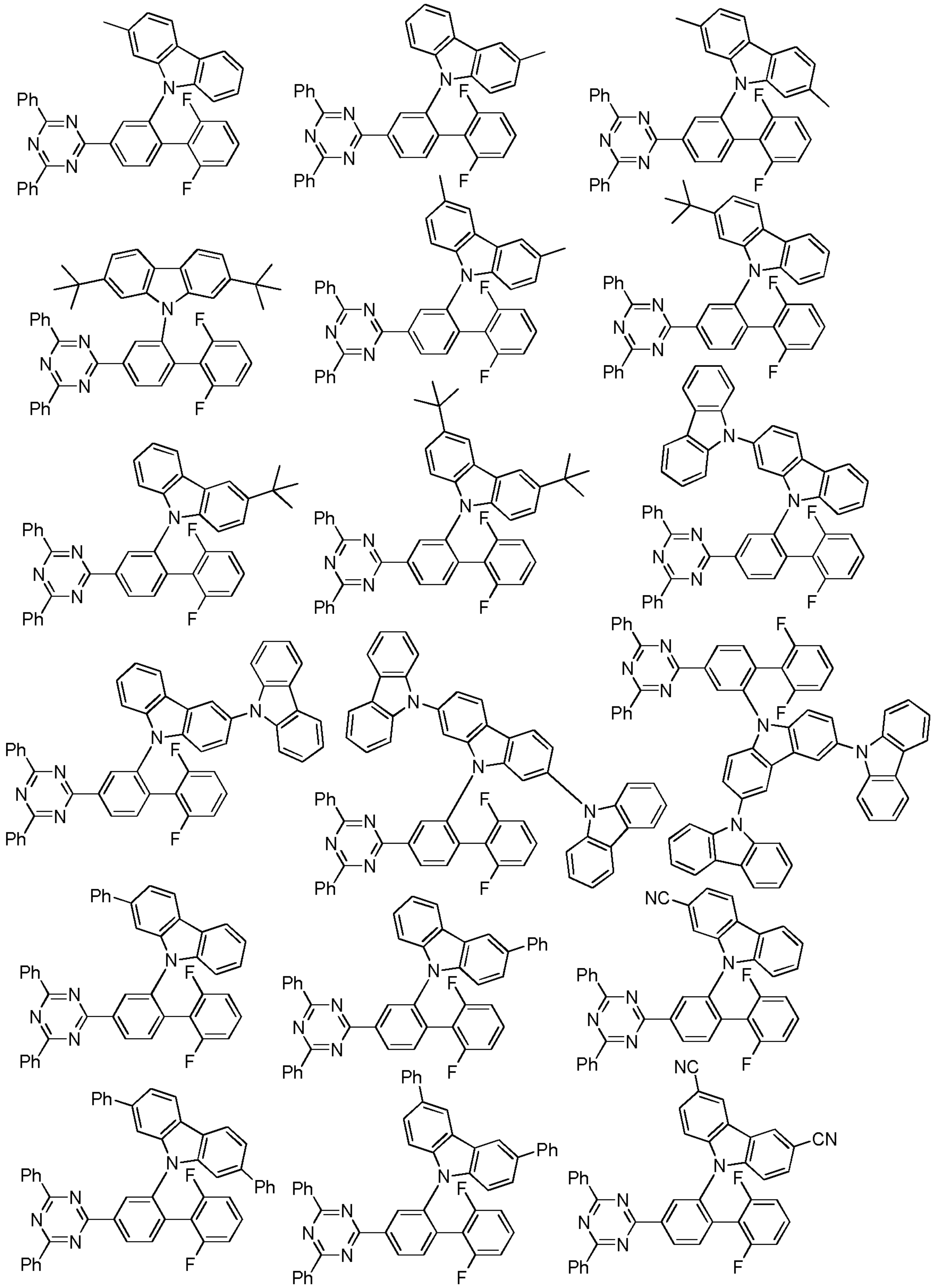

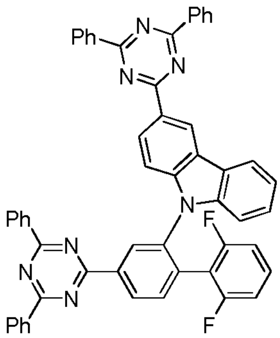
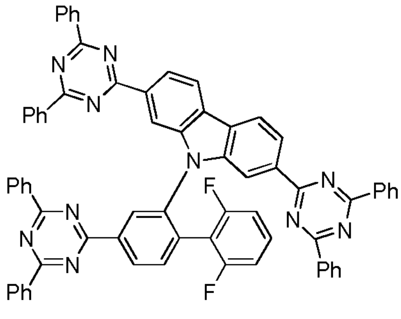
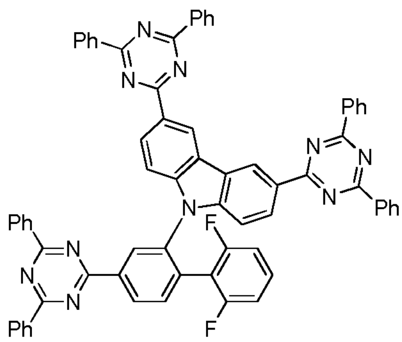
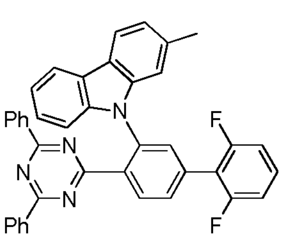
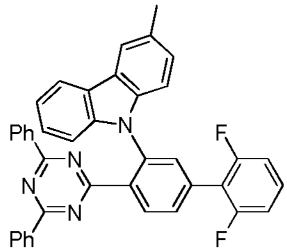
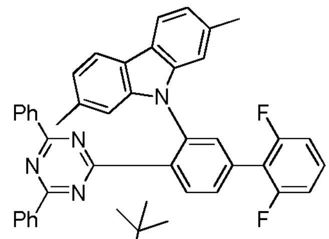
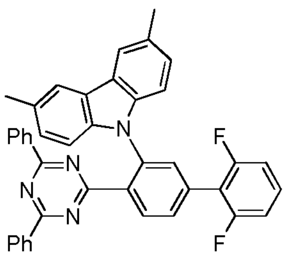
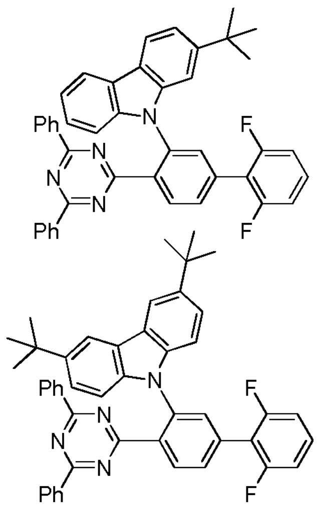
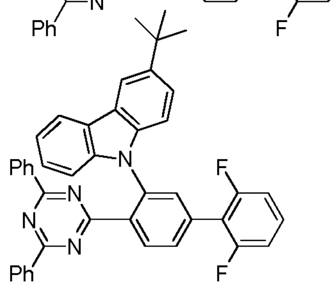
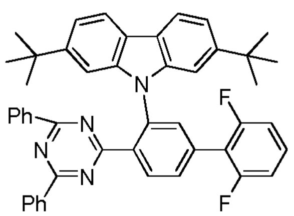
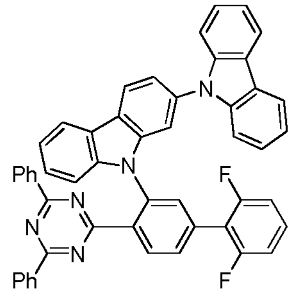
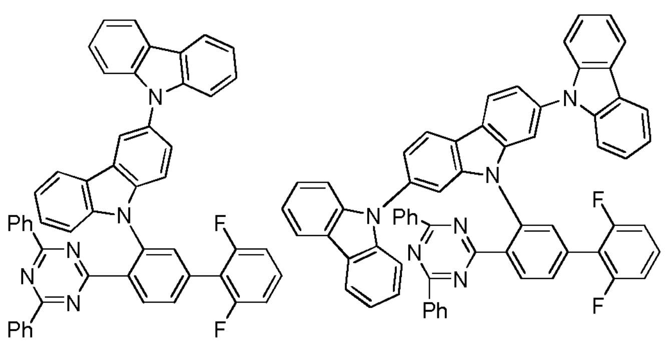
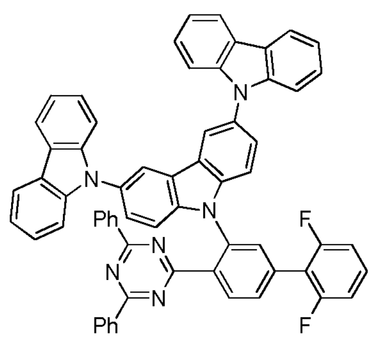

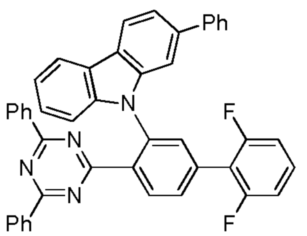
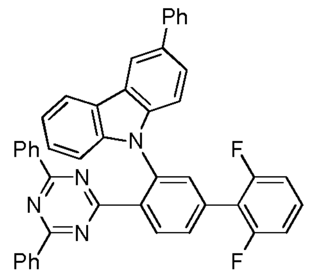
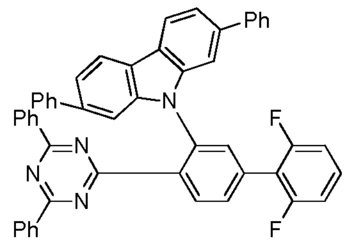
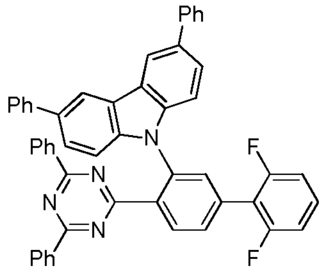
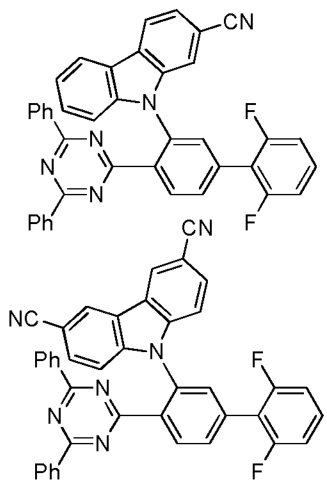
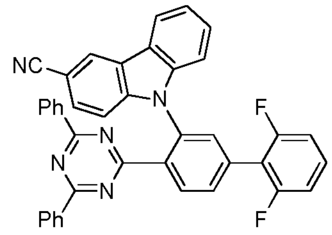
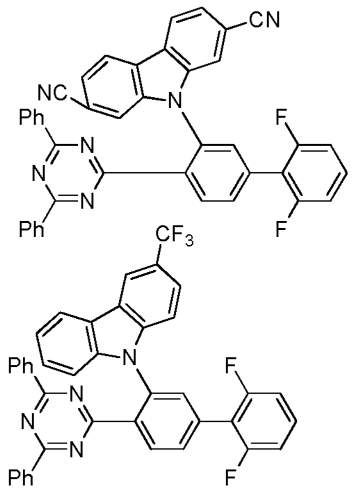
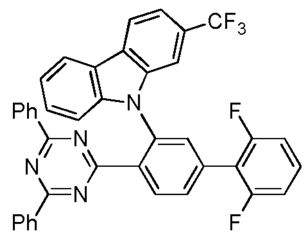
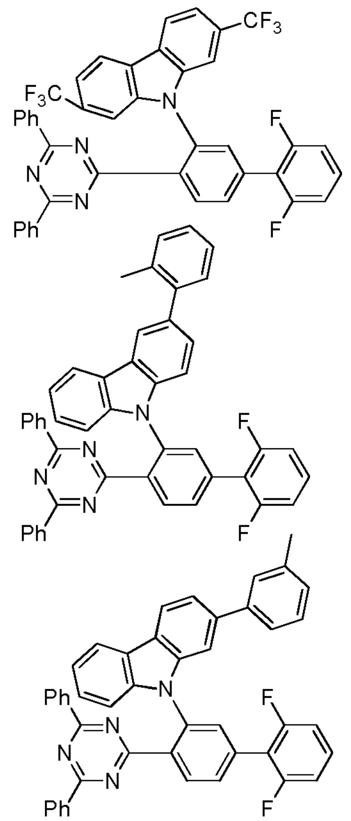
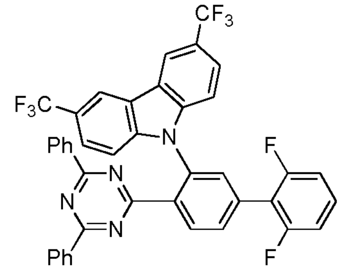
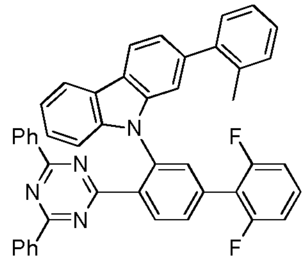
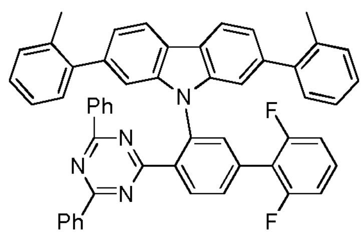
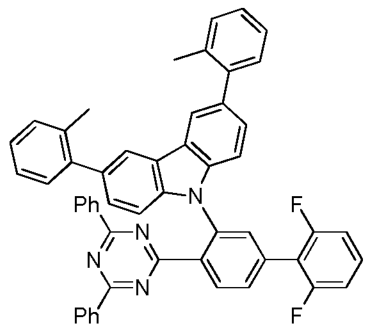
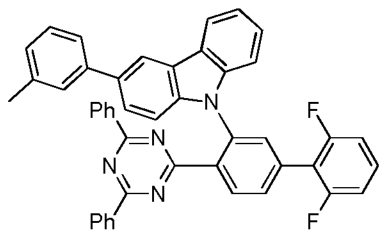

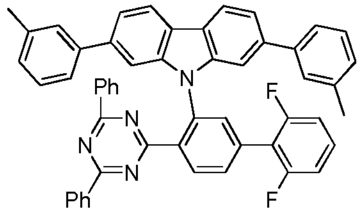

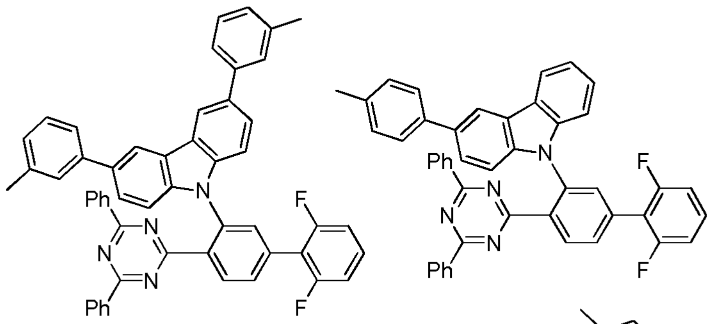

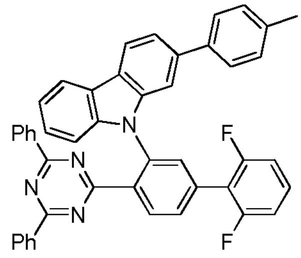

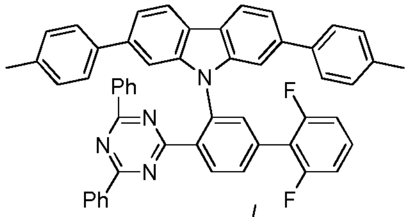

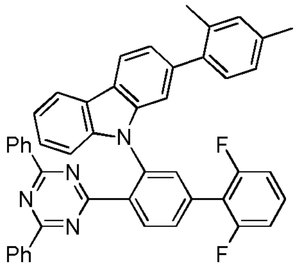

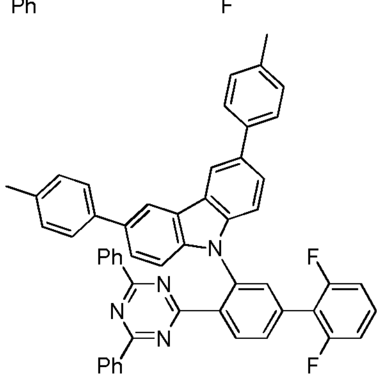

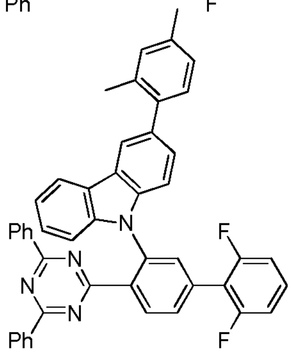

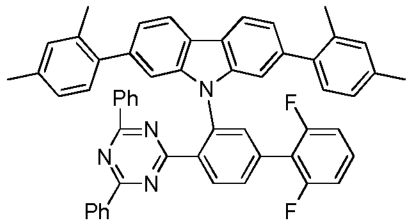

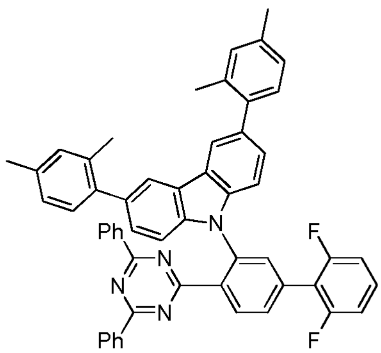

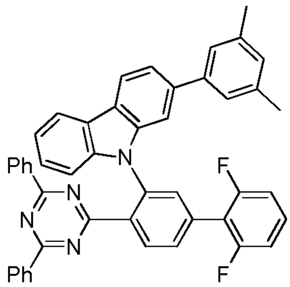

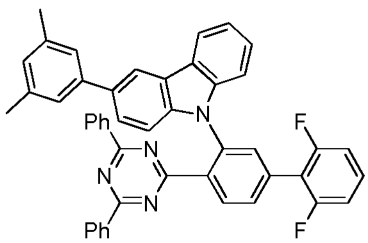

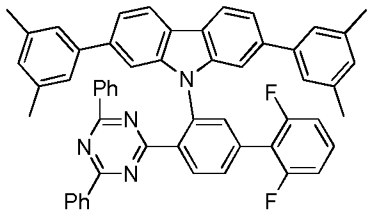

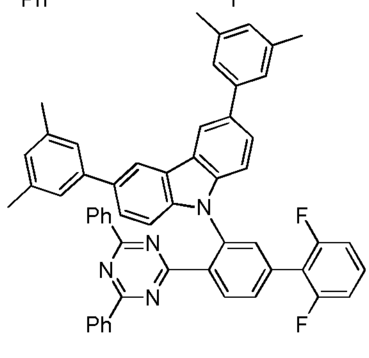

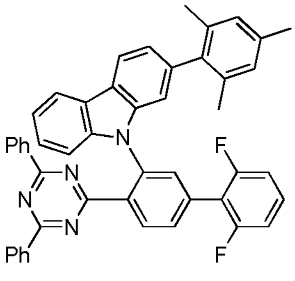

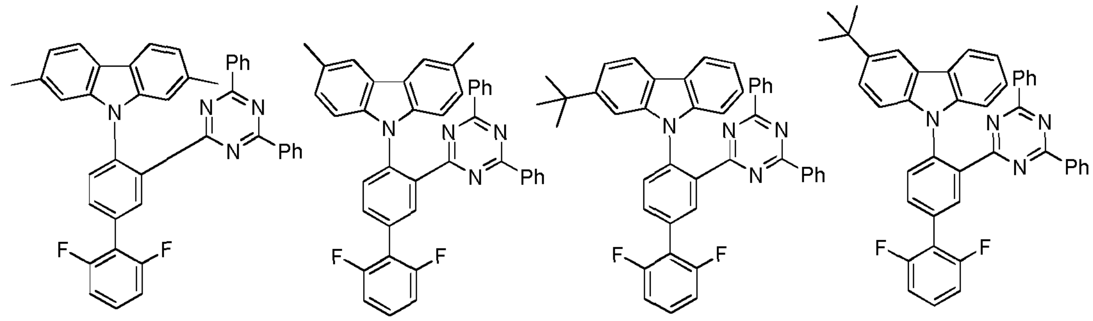

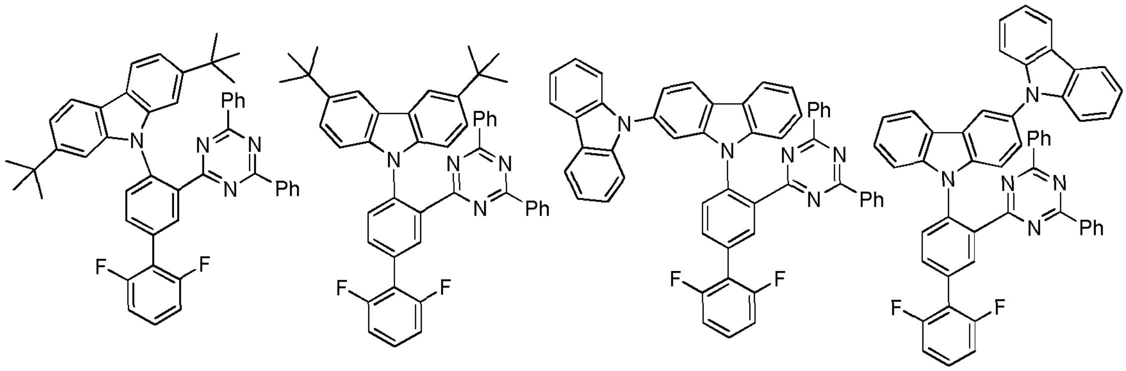

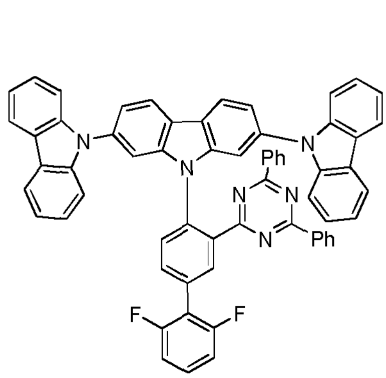

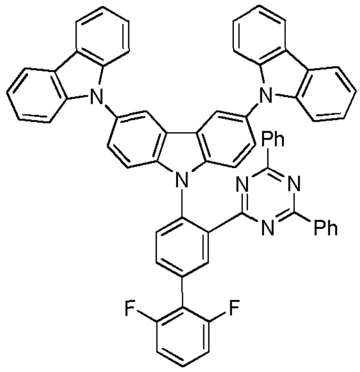

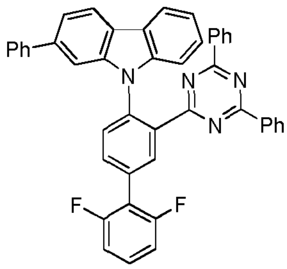

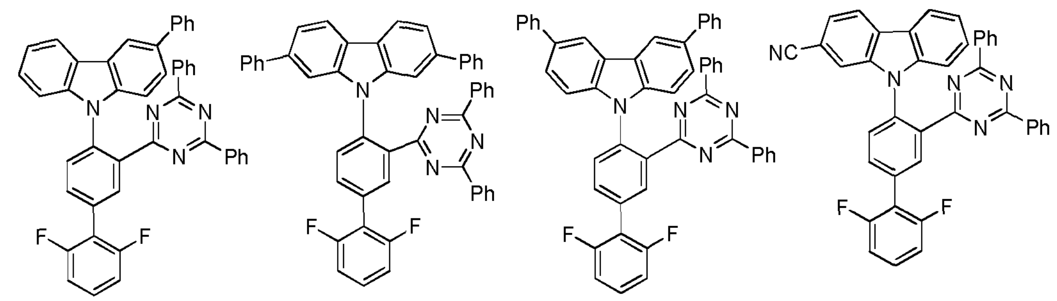

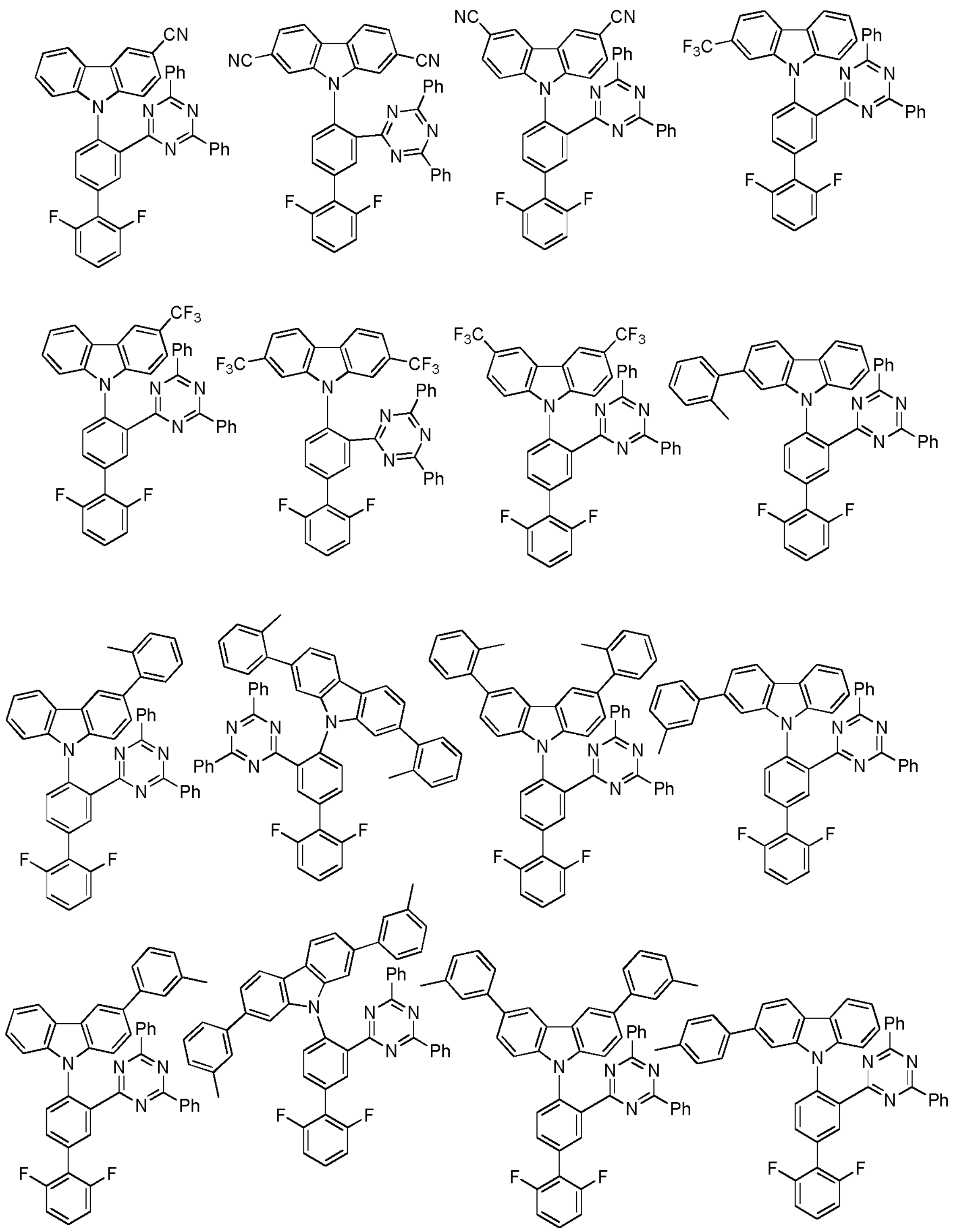

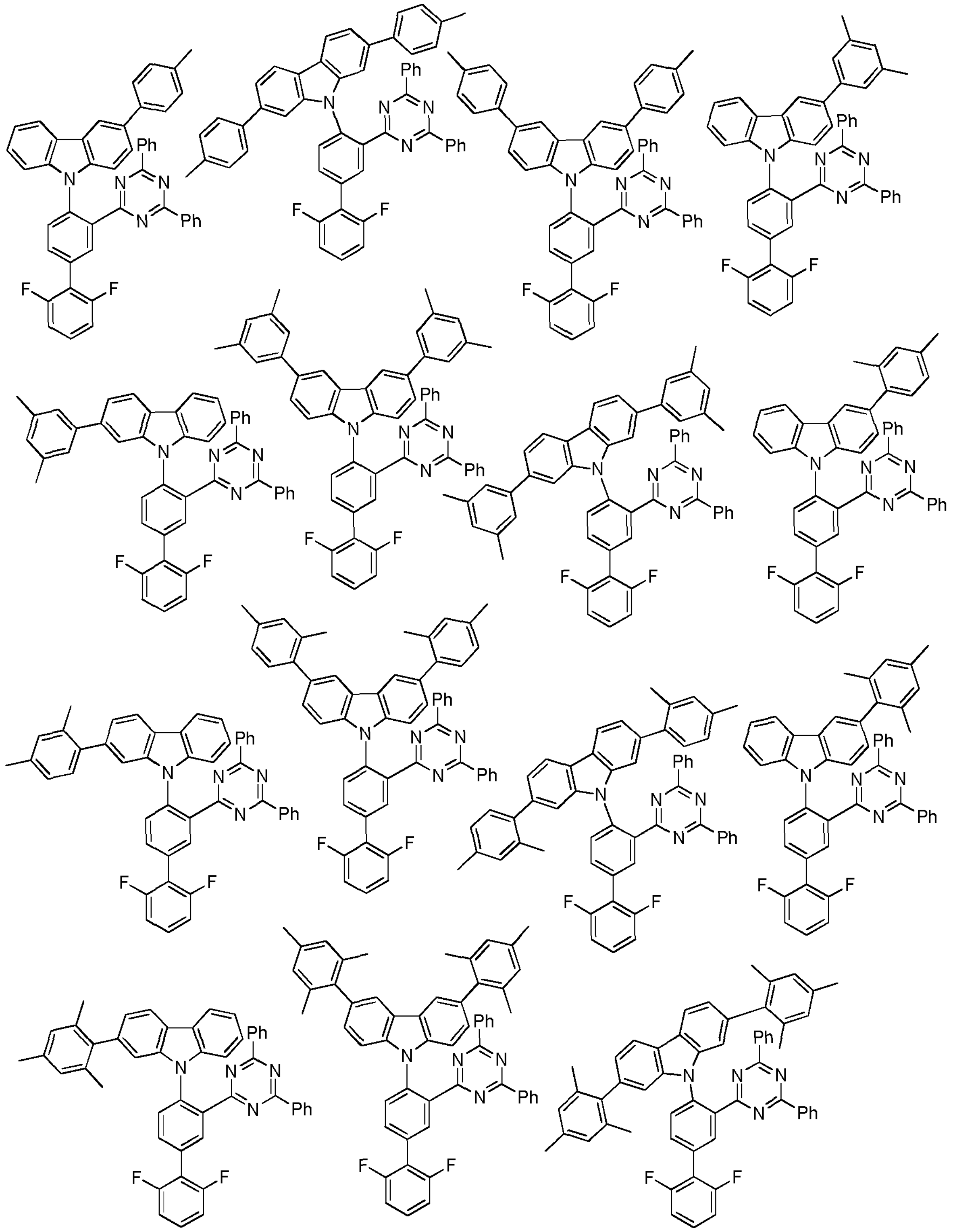

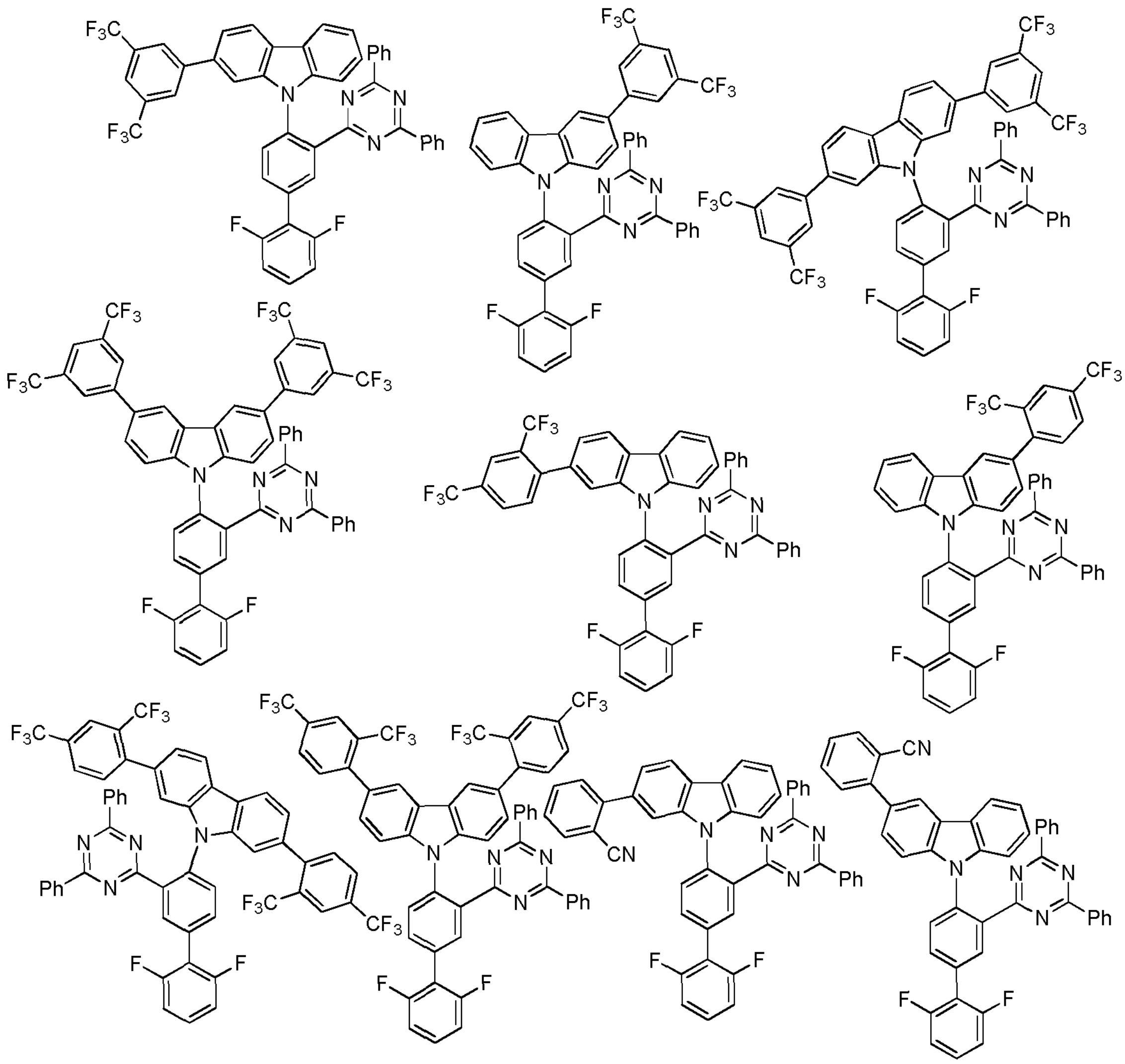

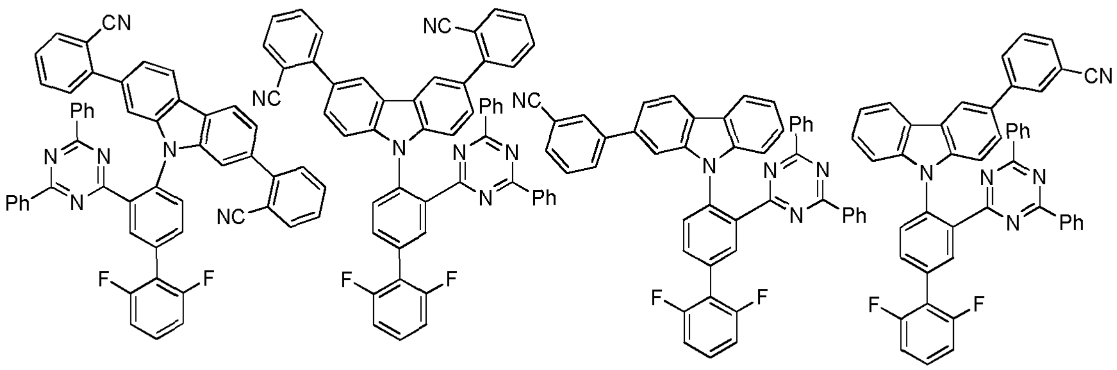

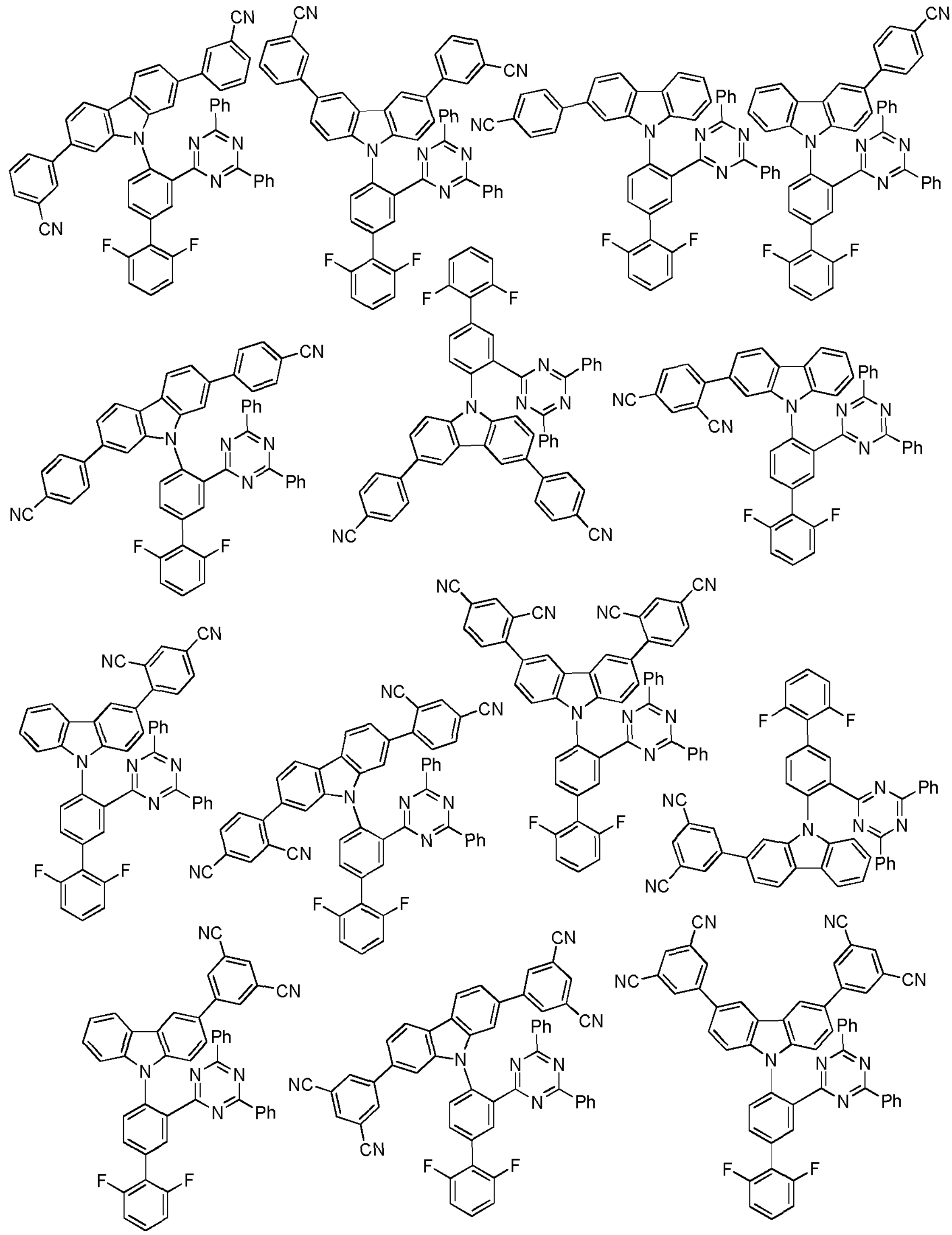

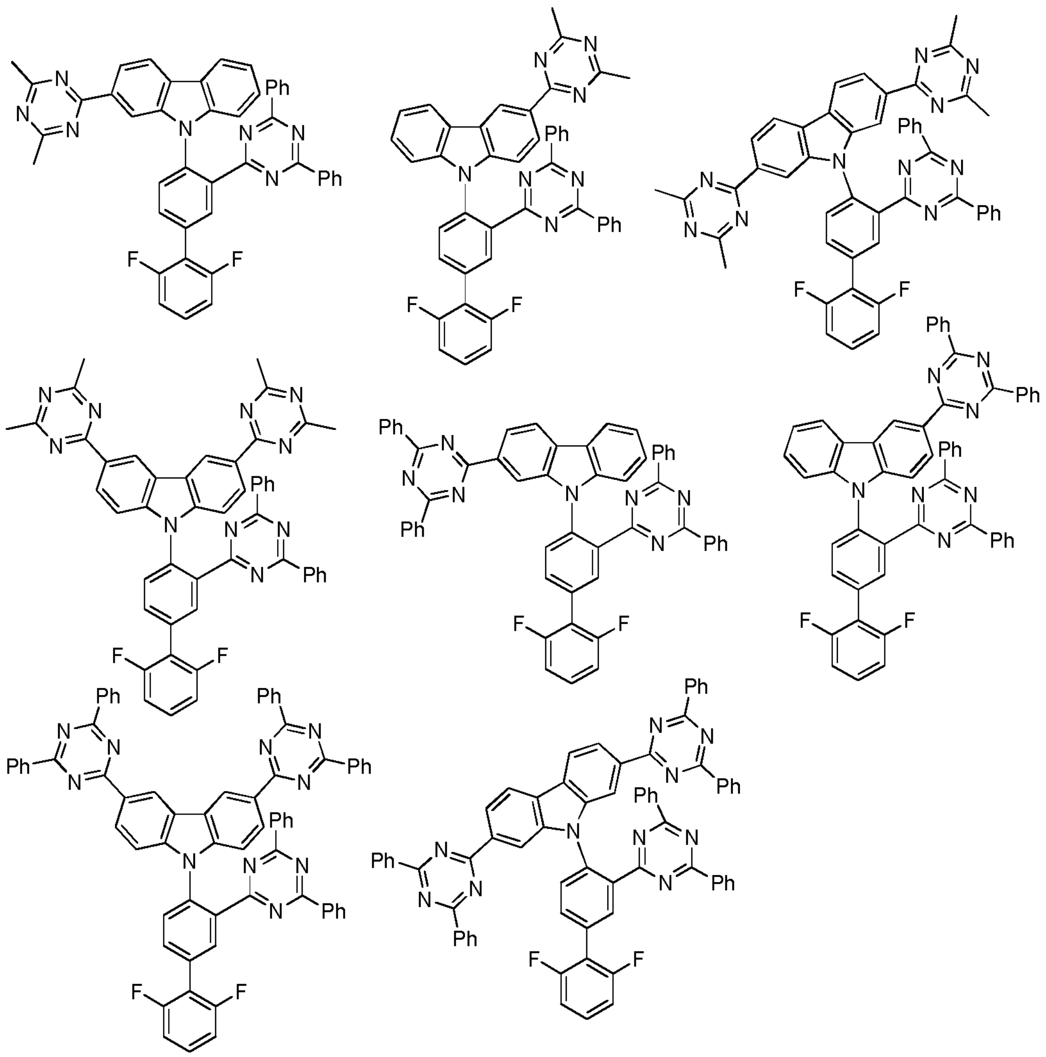

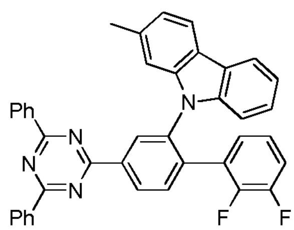
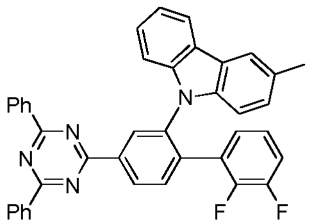
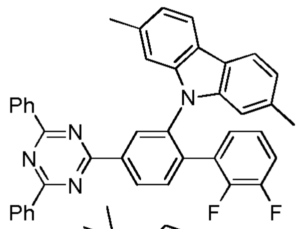
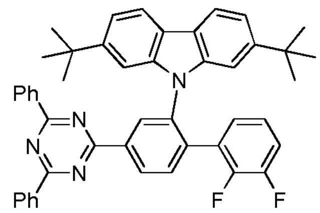
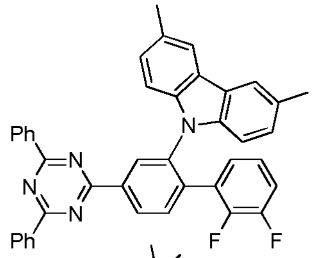
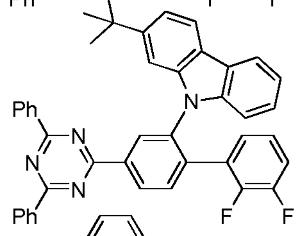
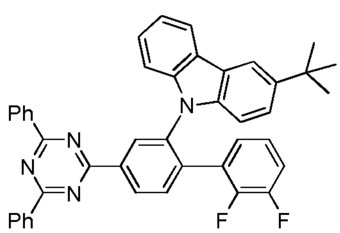
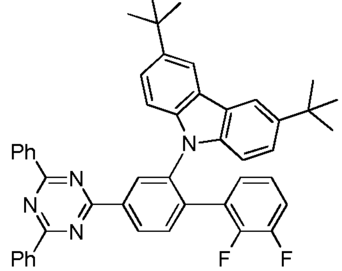
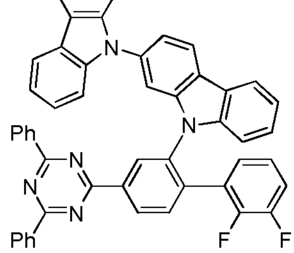
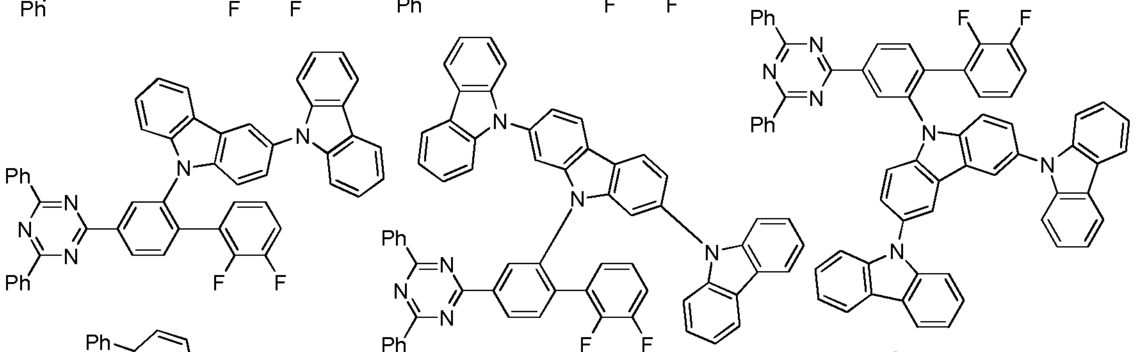
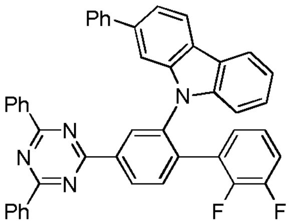
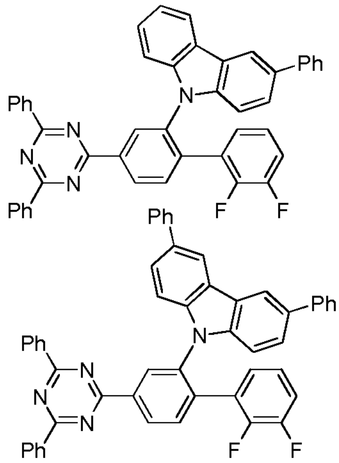
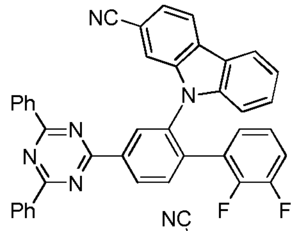
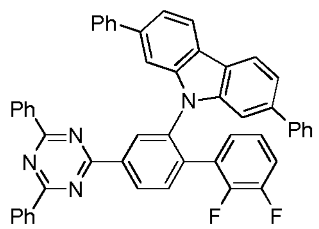
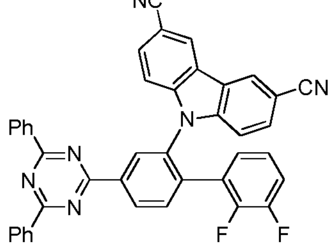

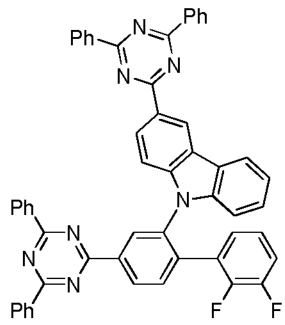
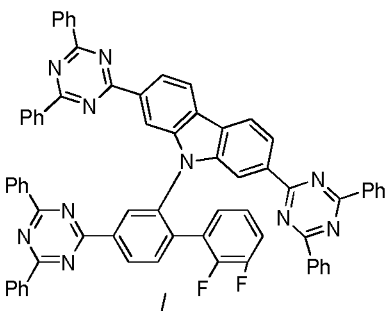
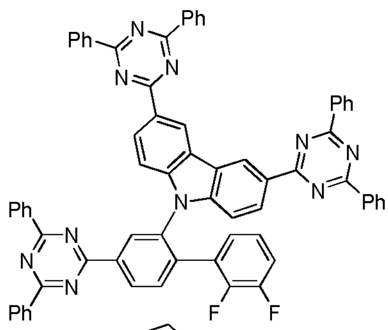
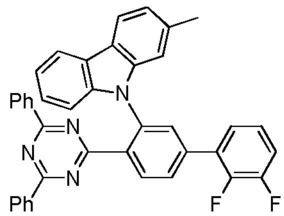
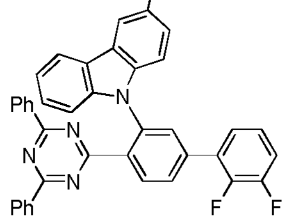
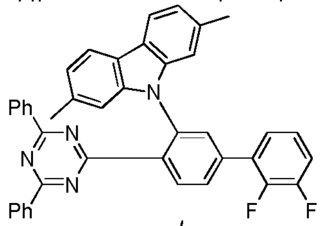
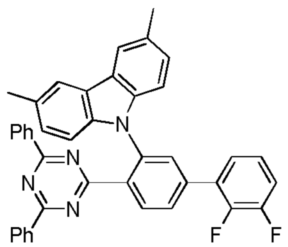
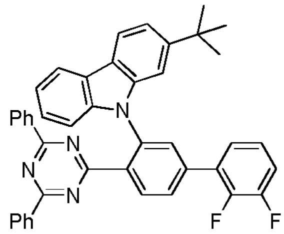
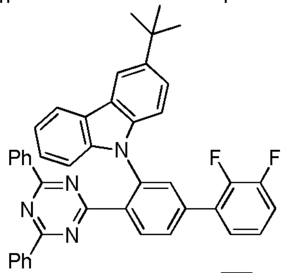
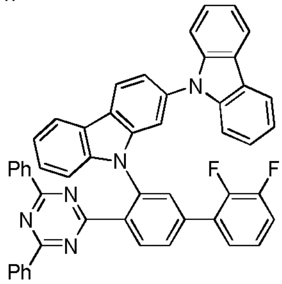
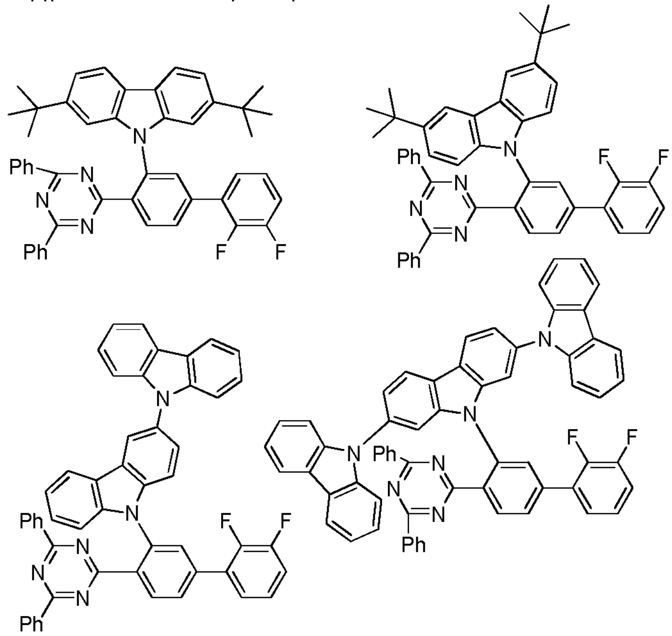
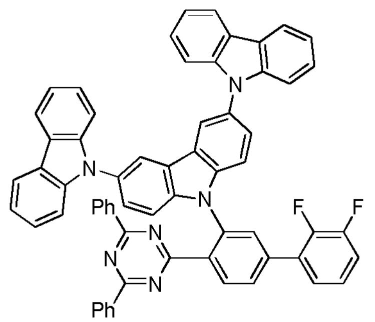

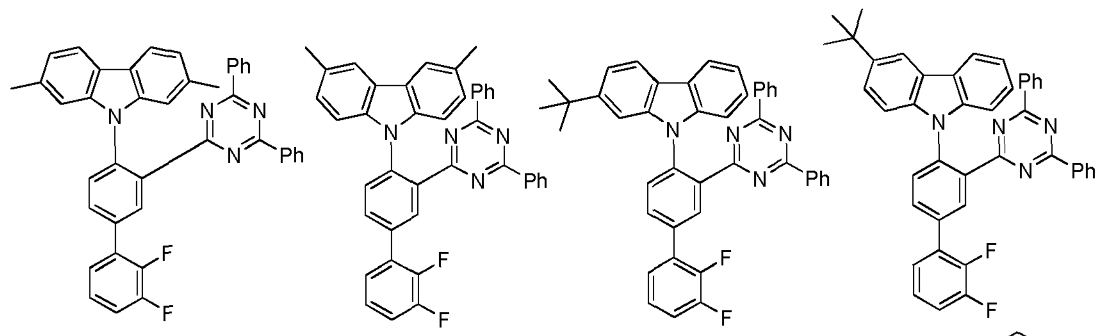

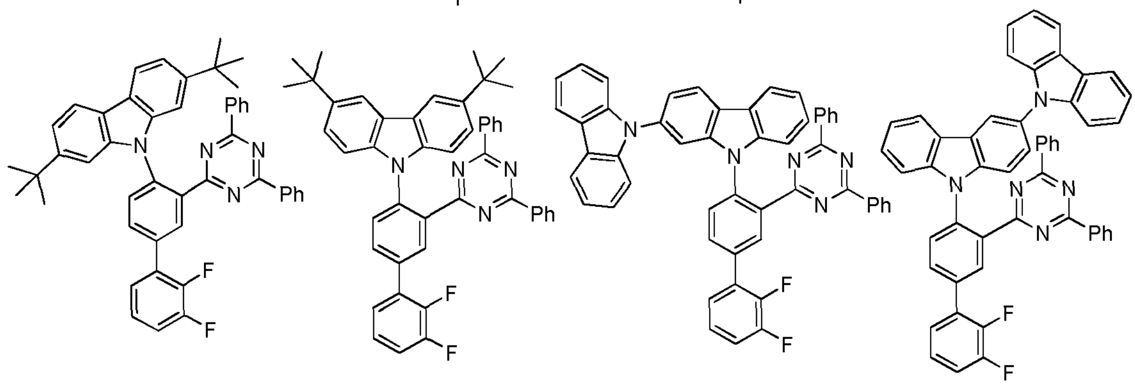

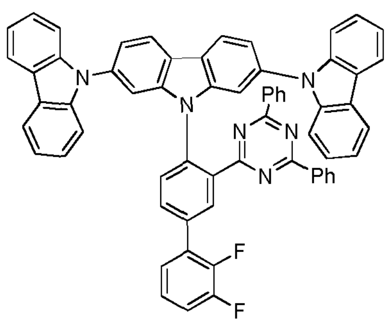

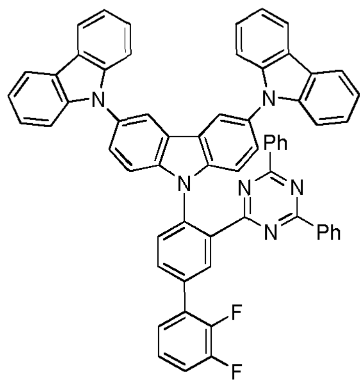

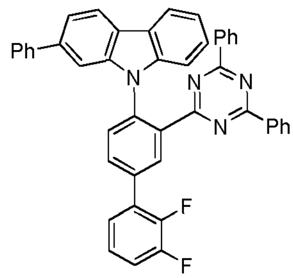

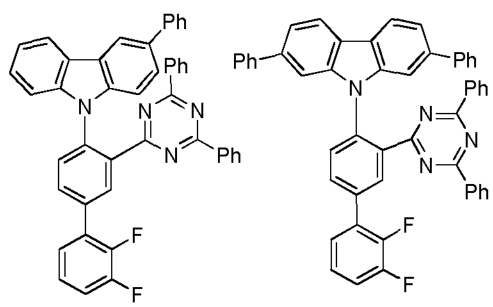

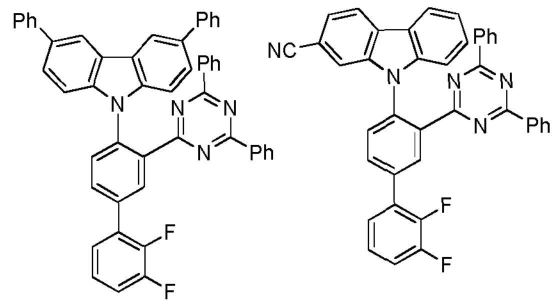

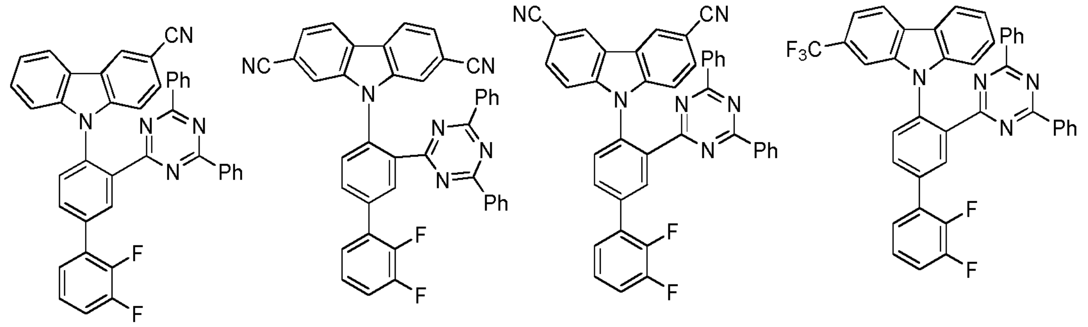

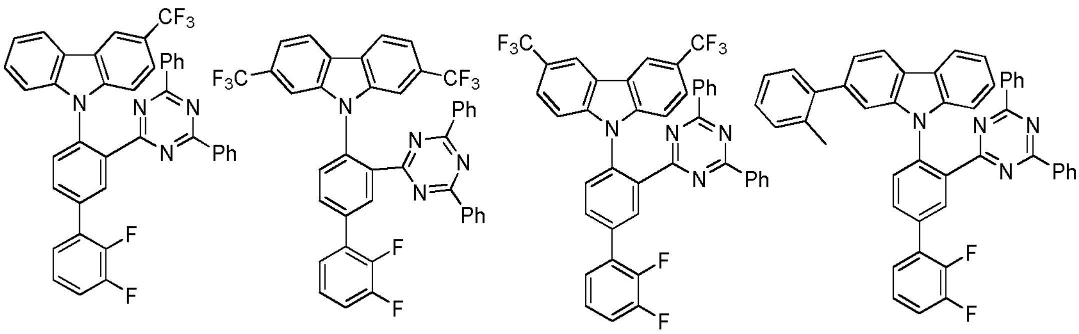

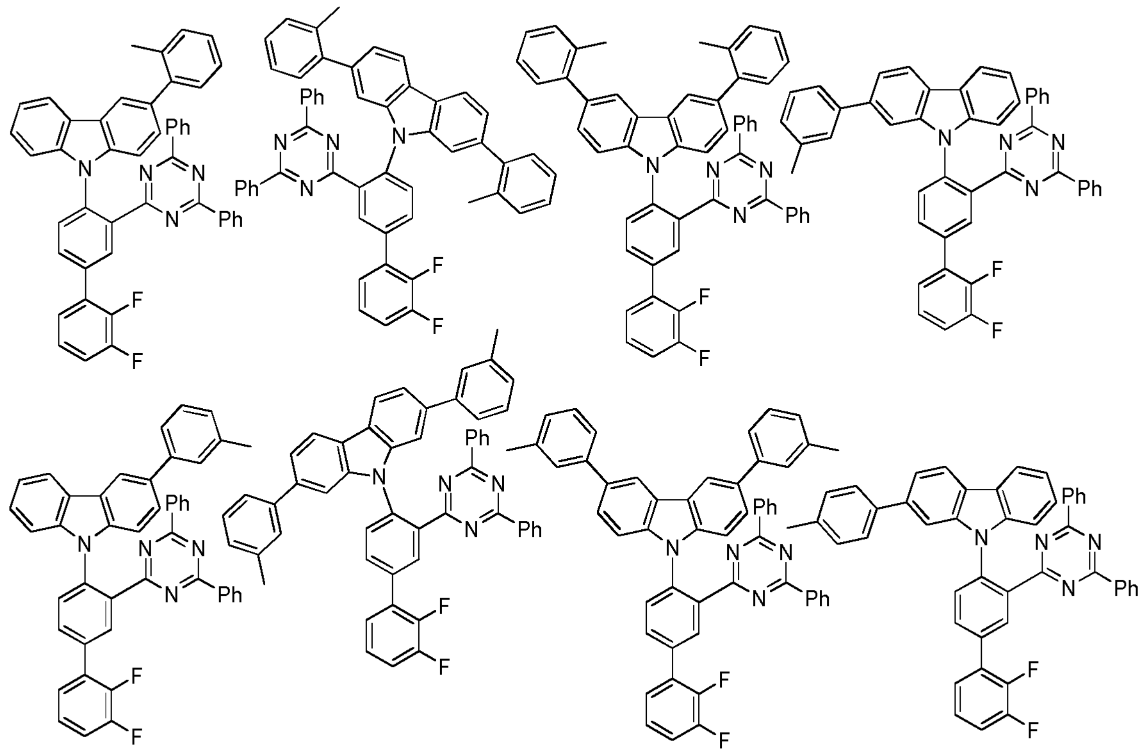

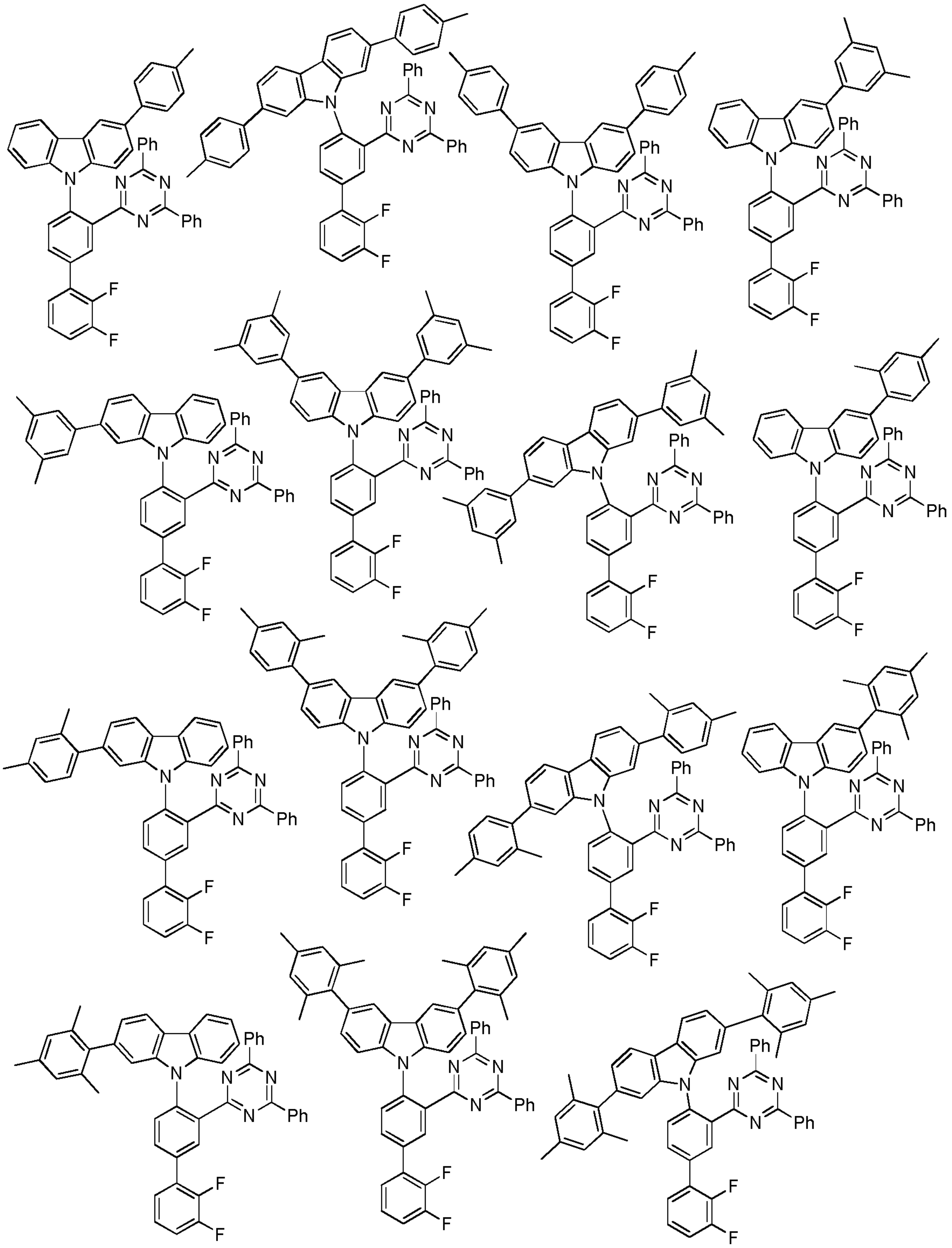

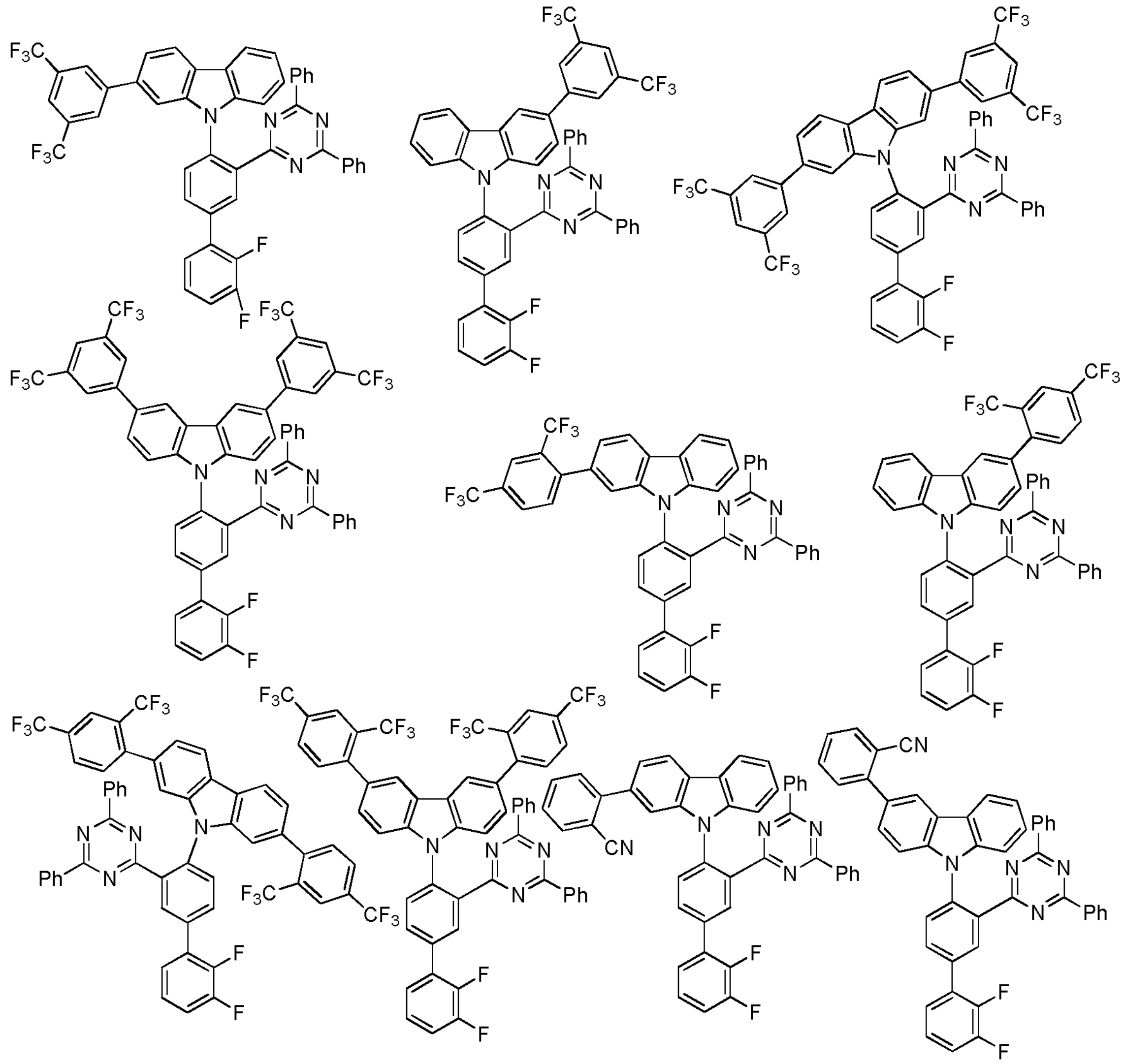

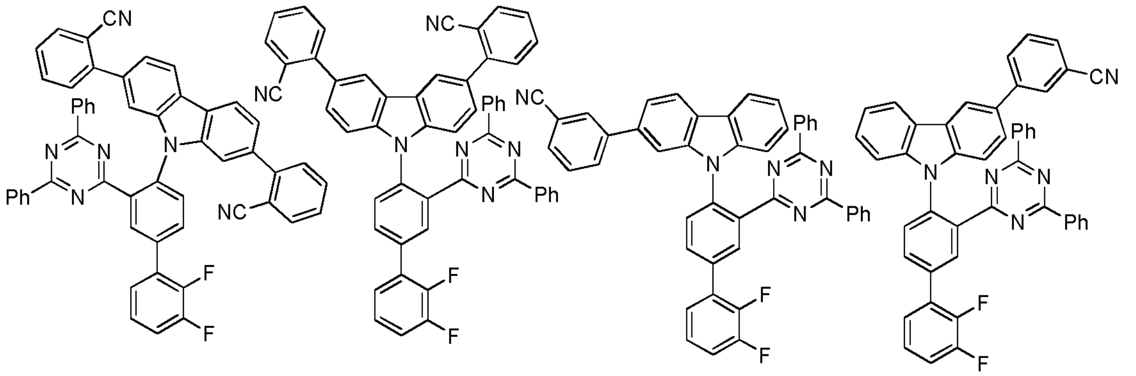

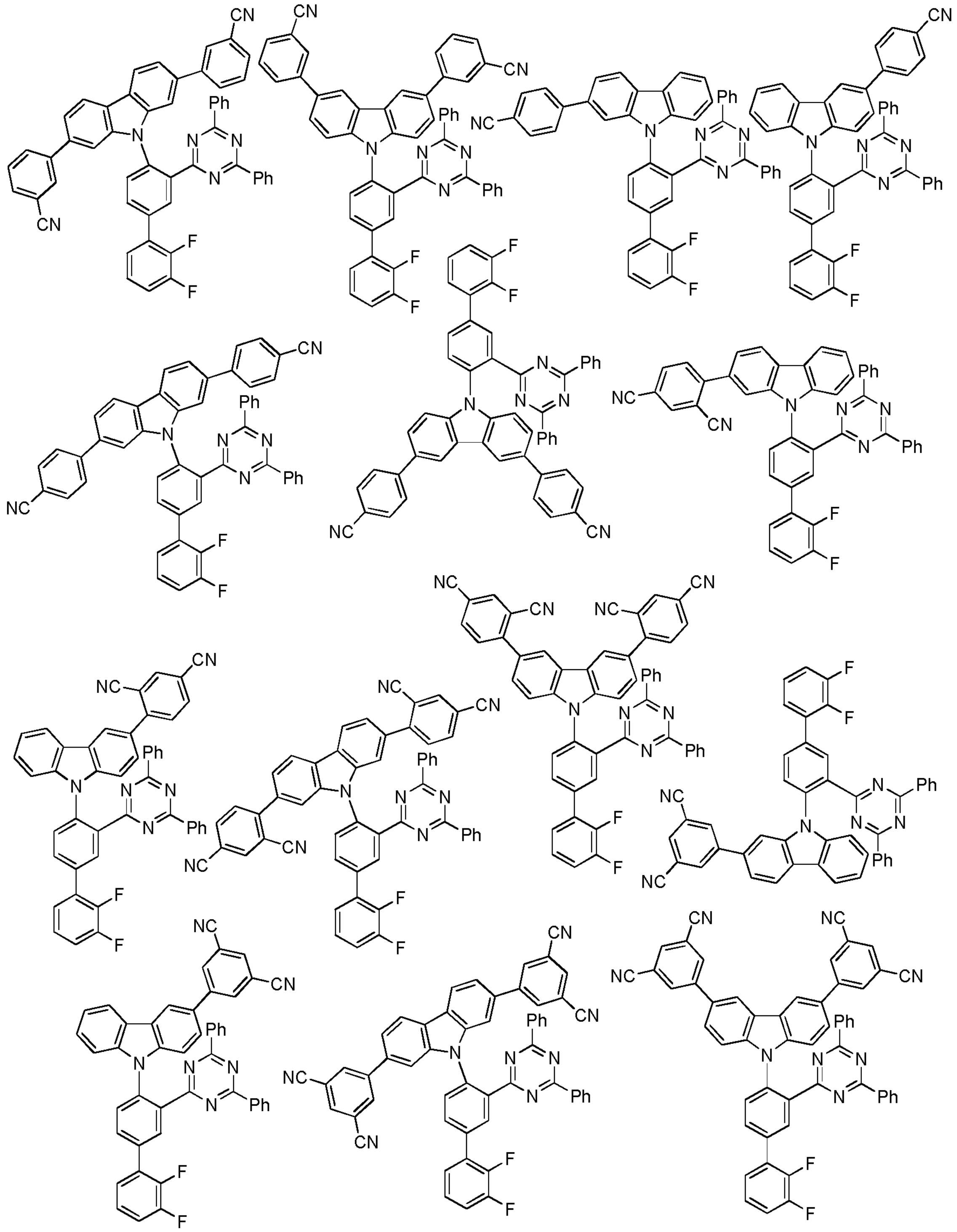

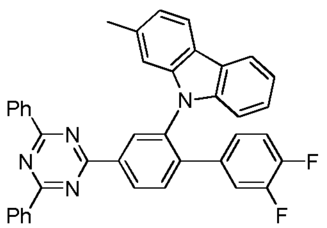
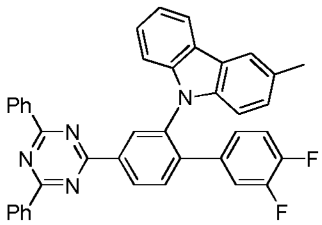
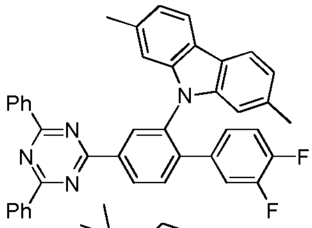
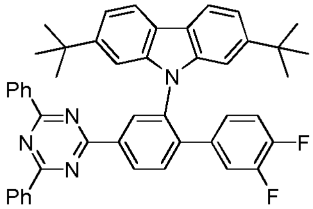
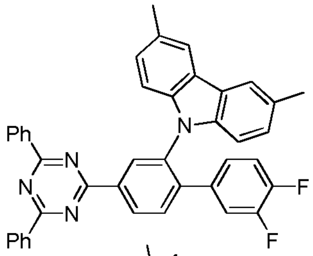
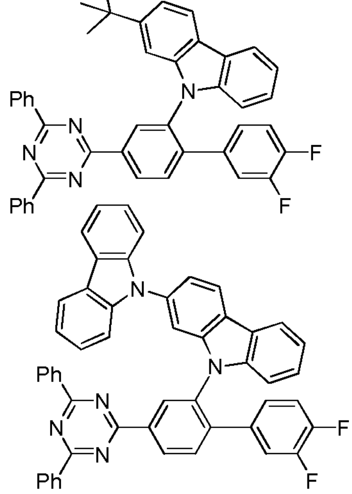
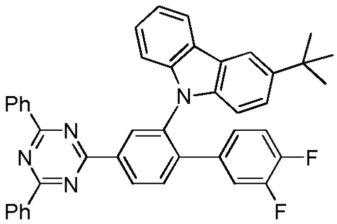
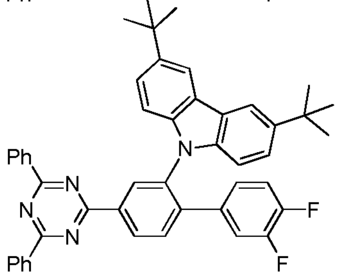
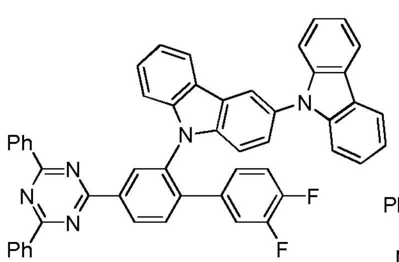
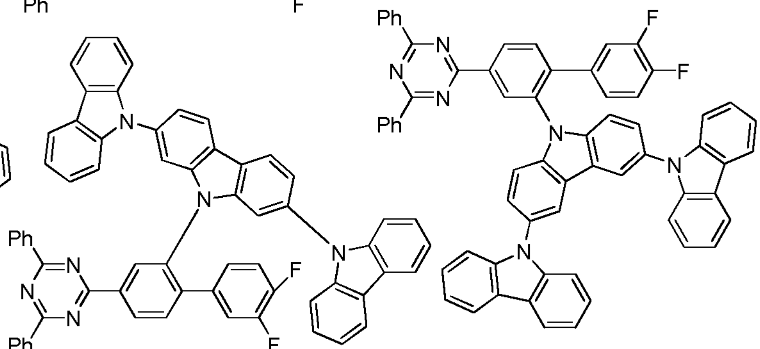
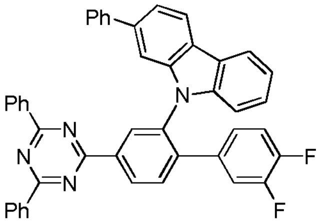
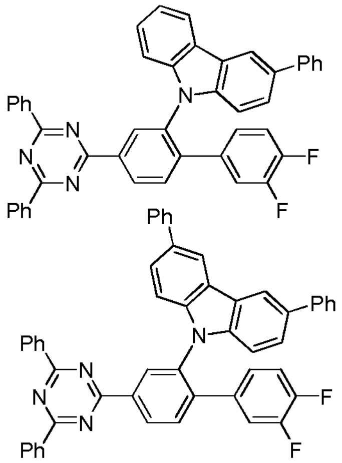
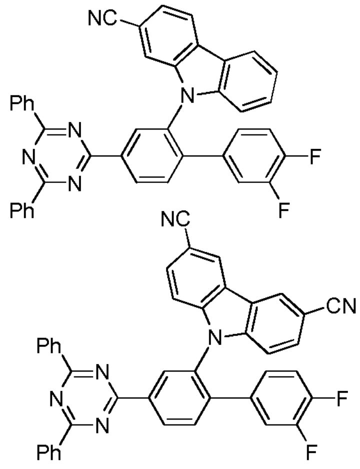
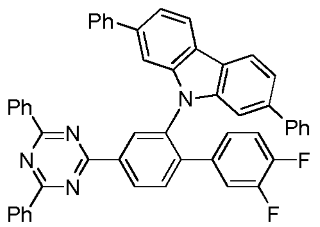

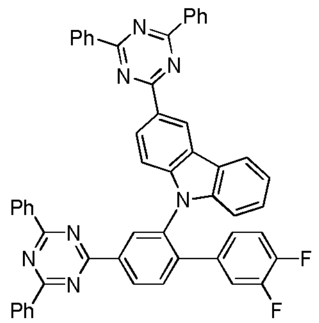
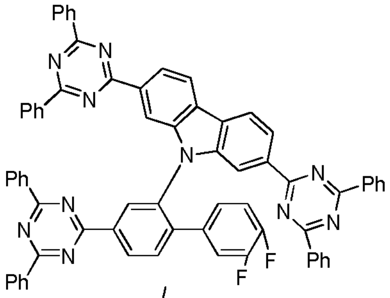
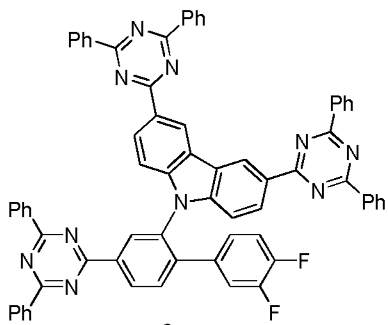
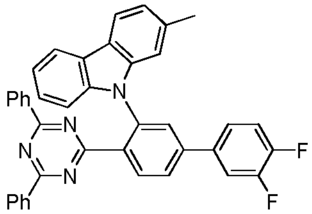
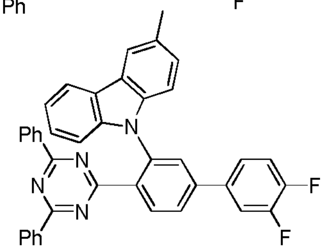
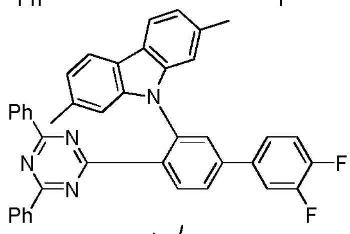
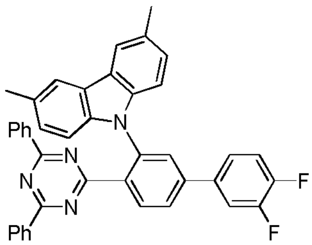
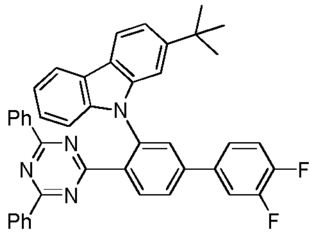
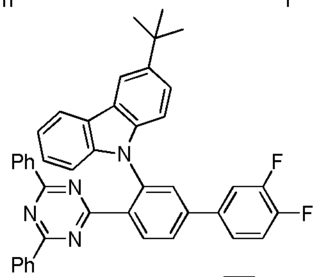
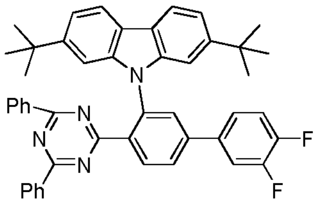
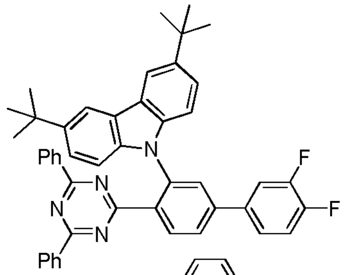
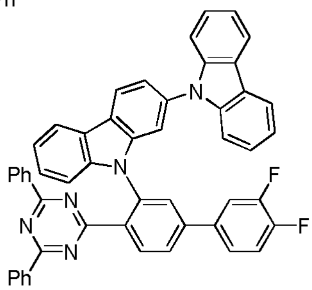
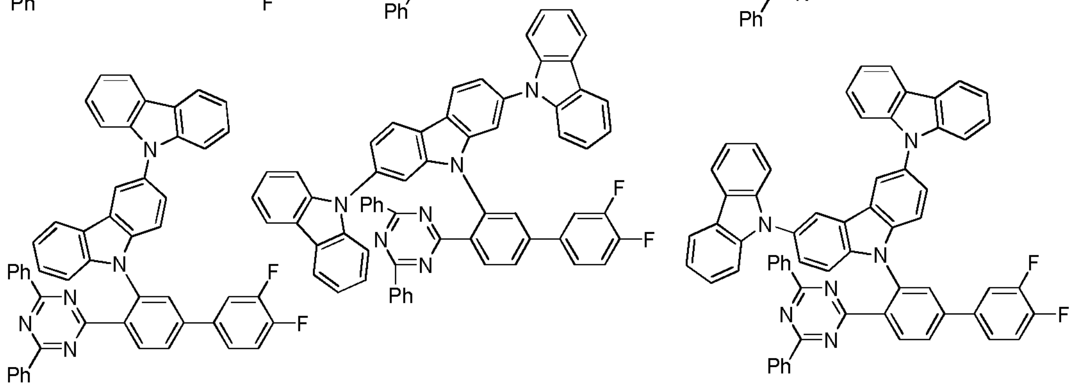

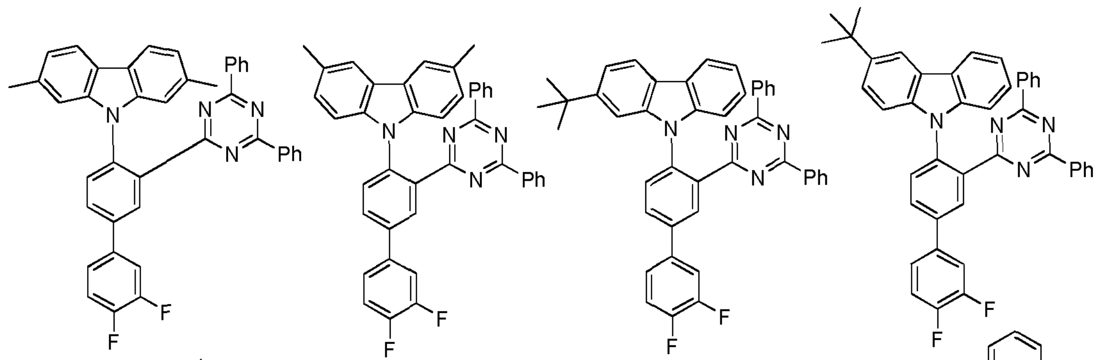

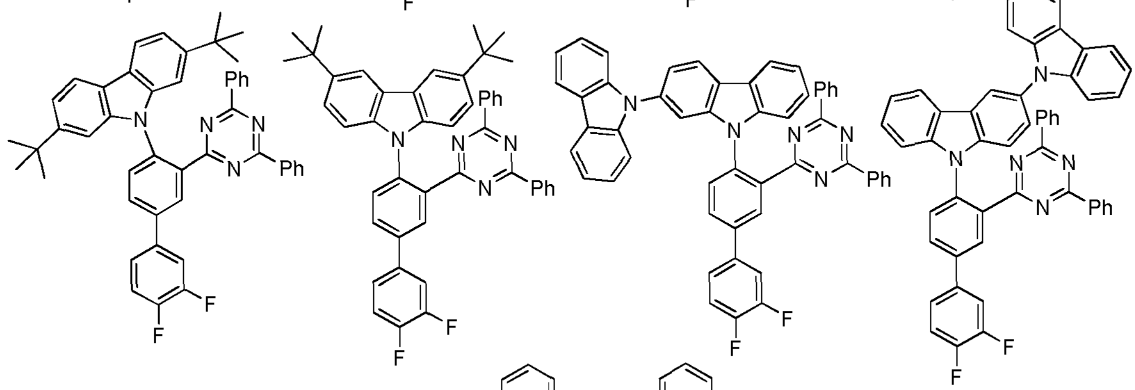

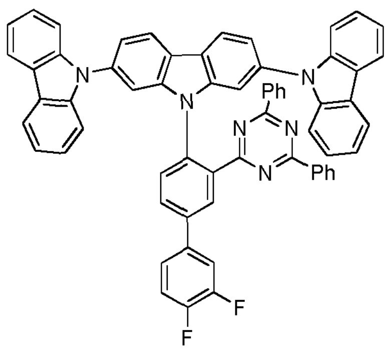

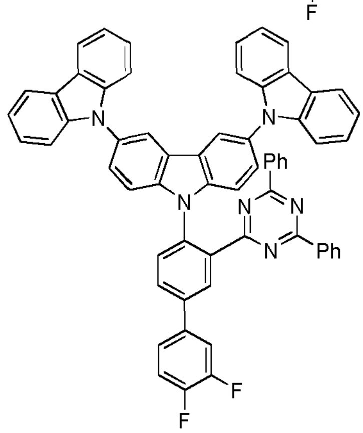

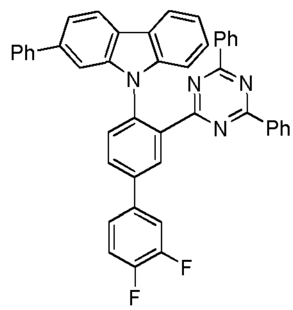

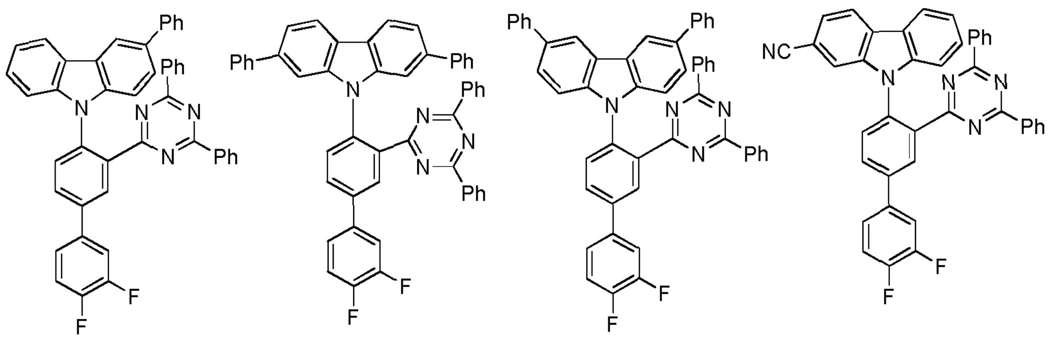

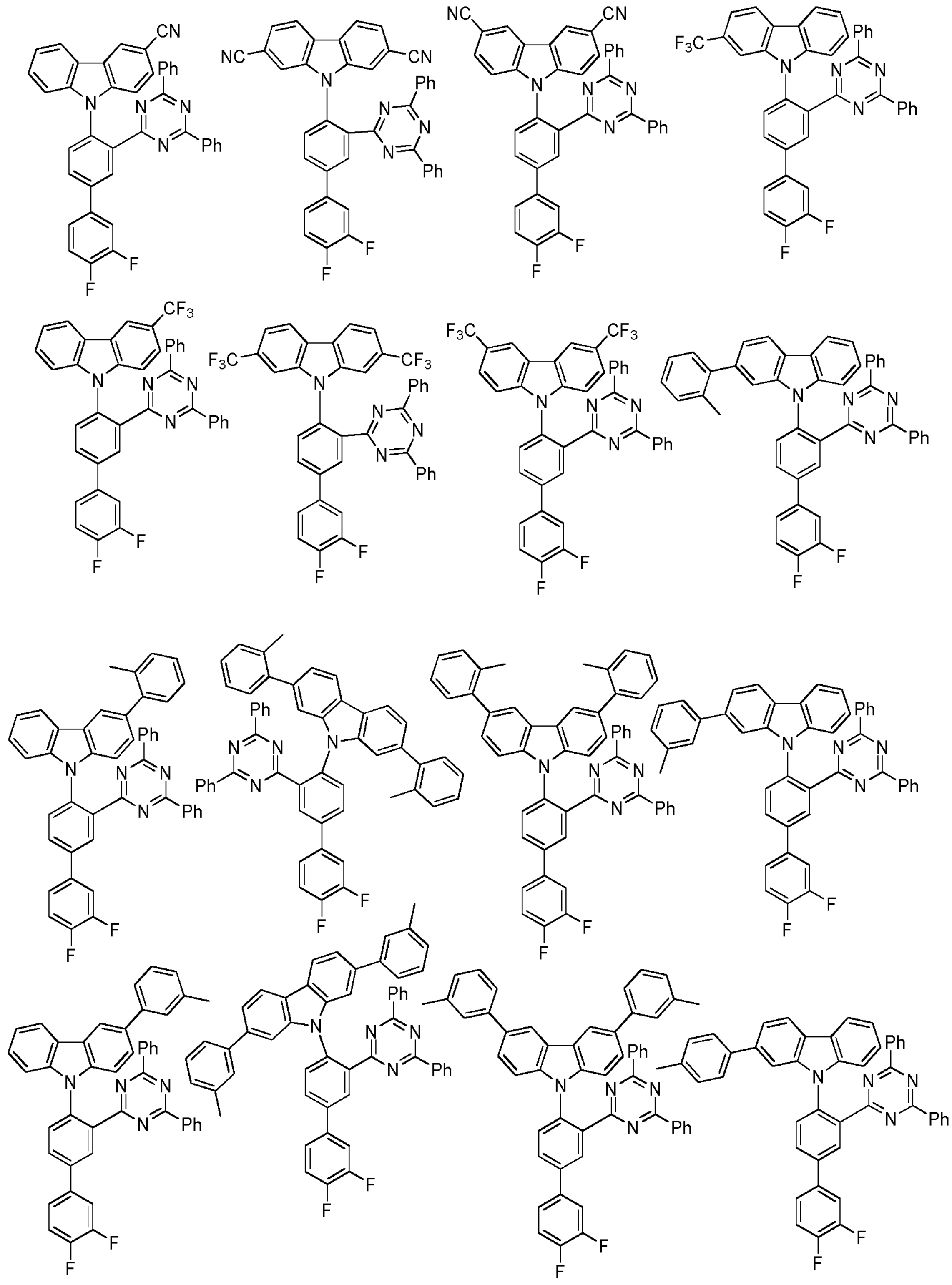

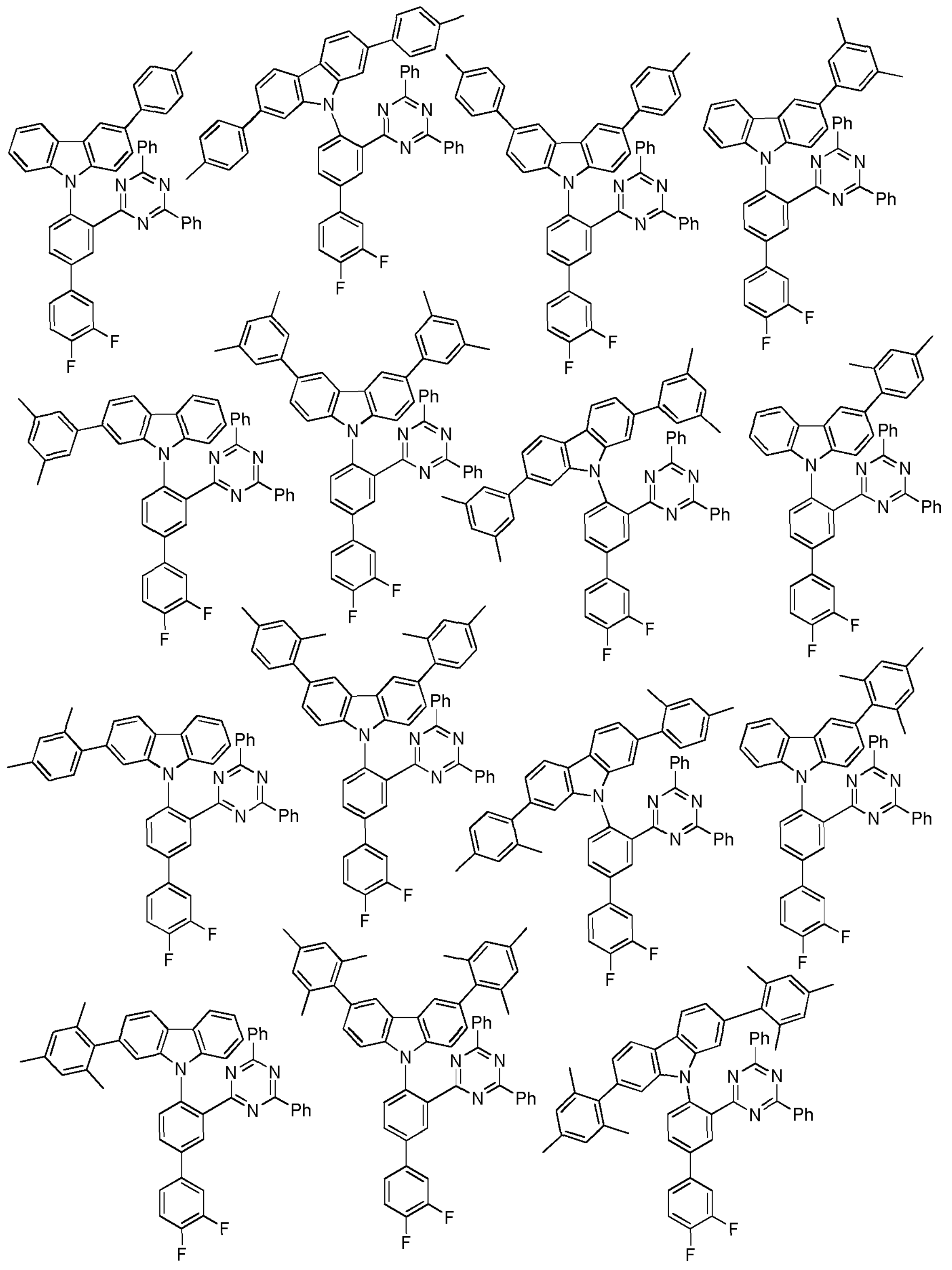

**Figure**

Figure 1 Emission spectrum of example 1 (10% by weight) in PMMA.

## Claims

**1.** Organic molecule consisting of

- a first chemical moiety consisting of a structure of Formula I,

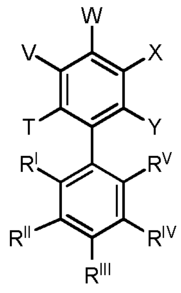

Formula I

and

- one second chemical moiety consisting of a structure of Formula IIa,

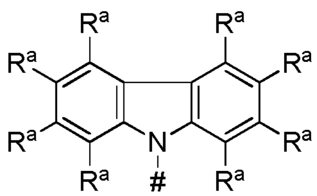

Formula IIa

wherein the first chemical moiety is linked to the second chemical moiety via a single bond;
wherein

T is the binding site of a single bond linking the first chemical moiety to the second chemical moiety, or is selected from the group consisting of $R^1$ and $R^T$;
V is the binding site of a single bond linking the first chemical moiety to the second chemical moiety or is hydrogen (H);
W is the binding site of a single bond linking the first chemical moiety to the second chemical moiety, or is selected from the group consisting of $R^1$ and $R^T$;
X is selected from the group consisting of $R^1$ and $R^T$;
Y is selected from the group consisting of $R^1$ and $R^T$;
# represents the binding site of a single bond linking the first chemical moiety to the second chemical moiety;
$R^T$ is 1,3,5-triazinyl, substituted with two substituents $R^N$:

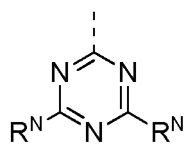,

wherein the dotted bond represents the binding site of $R^T$ to the single bond linking the first chemical moiety and $R^T$;
$R^N$ is at each occurrence independently from another selected from the group consisting of hydrogen, deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;

C2-C8-alkynyl,
wherein one or more hydrogen atoms are optionally substituted by deuterium;
$C_6$-$C_{18}$-aryl,
which is optionally substituted with one or more substituents $R^6$; and
$C_3$-$C_{17}$-heteroaryl,
which is optionally substituted with one or more substituents $R^6$;
$R^I$, $R^{II}$, $R^{III}$, $R^{IV}$, and $R^V$ is at each occurrence independently from another selected from the group consisting of
hydrogen,
deuterium,
F,
$C_1$-$C_5$-alkyl,
wherein one or more hydrogen atoms are optionally substituted by deuterium;
C2-C8-alkenyl,
wherein one or more hydrogen atoms are optionally substituted by deuterium;
C2-C8-alkynyl,
wherein one or more hydrogen atoms are optionally substituted by deuterium; and
$C_6$-$C_{18}$-aryl,
which is optionally substituted with one or more substituents $R^6$;
$R^1$ is at each occurrence independently from another selected from the group consisting of hydrogen,
deuterium,
$C_1$-$C_5$-alkyl,
wherein one or more hydrogen atoms are optionally substituted by deuterium;
C2-C8-alkenyl,
wherein one or more hydrogen atoms are optionally substituted by deuterium;
$C_2$-$C_8$-alkynyl,
wherein one or more hydrogen atoms are optionally substituted by deuterium; and
$C_6$-$C_{18}$-aryl,
which is optionally substituted with one or more substituents independently from another selected from the group consisting of

> $C_1$-$C_5$-alkyl,
> wherein optionally one or more hydrogen atoms are independently from each other substituted by deuterium, CN, $CF_3$, or F;
> and
> $C_6$-$C_{18}$-aryl,
> which is optionally substituted with one or more $C_1$-$C_5$-alkyl substituents;

$R^a$, $R^3$ and $R^4$ is at each occurrence independently from another selected from the group consisting of
hydrogen,
deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-alkyl,

> which is optionally substituted with one or more substituents $R^5$ and
> wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-alkoxy,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and wherein one or more non-adjacent $CH_2$-groups are optionally substituted by $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
$R^5$ is at each occurrence independently from another selected from the group consisting of hydrogen, deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-alkyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-alkoxy,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_1$-$C_{40}$-thioalkoxy,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkenyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_2$-$C_{40}$-alkynyl,

which is optionally substituted with one or more substituents $R^6$ and wherein one or more non-adjacent $CH_2$-groups are optionally substituted by $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S or $CONR^6$;

$C_6$-$C_{60}$-aryl,
which is optionally substituted with one or more substituents $R^6$; and
$C_3$-$C_{57}$-heteroaryl,
which is optionally substituted with one or more substituents $R^6$;
$R^6$ is at each occurrence independently from another selected from the group consisting of hydrogen, deuterium, OPh, $CF_3$, CN, F,
$C_1$-$C_5$-alkyl,
wherein one or more hydrogen atoms are optionally, independently from each other substituted by deuterium, CN, $CF_3$, or F;
$C_1$-$C_5$-alkoxy,
wherein one or more hydrogen atoms are optionally, independently from each other substituted by deuterium, CN, $CF_3$, or F;
$C_1$-$C_5$-thioalkoxy,
wherein one or more hydrogen atoms are optionally, independently from each other substituted by deuterium, CN, $CF_3$, or F;
C2-C5-alkenyl,
wherein one or more hydrogen atoms are optionally, independently from each other substituted by deuterium, CN, $CF_3$, or F;
C2-C5-alkynyl,
wherein one or more hydrogen atoms are optionally, independently from each other substituted by deuterium, CN, $CF_3$, or F;
$C_6$-$C_{18}$-aryl,
which is optionally substituted with one or more $C_1$-$C_5$-alkyl substituents;
$C_3$-$C_{17}$-heteroaryl,
which is optionally substituted with one or more $C_1$-$C_5$-alkyl substituents;
$N(C_6$-$C_{18}$-aryl$)_2$;
$N(C_3$-$C_{17}$-heteroaryl$)_2$,
and $N(C_3$-$C_{17}$-heteroaryl$)(C_6$-$C_{18}$-aryl$)$;
wherein the substituents $R^a$, $R^3$, $R^4$ or $R^5$ independently from each other optionally form a mono- or polycyclic, aliphatic, aromatic and/or benzo-fused ring system with one or more substituents $R^a$, $R^3$, $R^4$ or $R^5$;
wherein the terms alkyl comprises linear, branched, and cyclic alkyl substituents, the term alkenyl comprises linear, branched, and cyclic alkenyl substituents and the term alkynyl comprises linear, branched, and cyclic alkynyl substituents;
wherein exactly one substituent selected from the group consisting of T, W, X, and Y is $R^T$, and exactly one substituent selected from the group consisting of T, V and W represents the binding site of a single bond linking the first chemical moiety and the second chemical moiety;
wherein T is hydrogen in case W is $R^T$ and V represents the binding site of a single bond linking the first chemical moiety and the second chemical moiety;
and W is hydrogen in case T is $R^T$ and V represents the binding site of a single bond linking the first chemical moiety and the second chemical moiety;
and wherein exactly two substituents selected from the group consisting of $R^I$, $R^{II}$, $R^{III}$, $R^{IV}$ and $R^V$ are F.

2. Organic molecule according to claim 1, wherein $R^1$ and $R^N$ is independently from each other and at each occurrence independently from another selected from the group consisting of H, methyl and phenyl.

3. Organic molecule according to claim 1 or 2, wherein X is $R^T$ and W represents the binding site of a single bond linking the first chemical moiety and the second chemical moiety.

4. Organic molecule according to one or more of claims 1 to 3, wherein the second chemical moiety consists of a structure of Formula IIb:

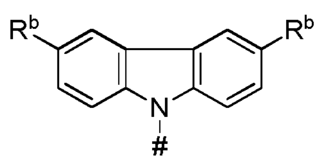

Formula IIb

wherein

$R^b$ is at each occurrence independently from another selected from the group consisting of deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C$=$CR^5$, C≡C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**5.** Organic molecule according to one or more of claims 1 to 3, wherein the second chemical moiety consists of a structure of formula IIc:

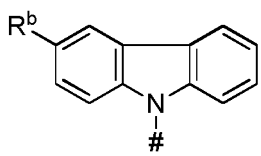

Formula IIc

wherein

$R^b$ is at each occurrence independently from another selected from the group consisting of deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-alkyl,

which is optionally substituted with one or more substituents $R^5$ and

wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-alkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_1$-$C_{40}$-thioalkoxy,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkenyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_2$-$C_{40}$-alkynyl,

which is optionally substituted with one or more substituents $R^5$ and
wherein one or more non-adjacent $CH_2$-groups are optionally substituted by $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S or $CONR^5$;

$C_6$-$C_{60}$-aryl,
which is optionally substituted with one or more substituents $R^5$; and
$C_3$-$C_{57}$-heteroaryl,
which is optionally substituted with one or more substituents $R^5$;
and wherein apart from that the definitions in claim 1 apply.

**6.** Organic molecule according to claim 4 or 5, wherein $R^b$ is at each occurrence independently from another selected from the group consisting of

- Me, $^i$Pr, $^t$Bu, CN, $CF_3$,
- Ph, which is optionally substituted with one or more substituents independently from each other selected from the group consisting of Me, $^i$Pr, $^t$Bu, CN, $CF_3$ and Ph;
- pyridinyl, which is optionally substituted with one or more substituents independently from each other selected from the group consisting of Me, $^i$Pr, $^t$Bu, CN, $CF_3$ and Ph;
- pyrimidinyl, which is optionally substituted with one or more substituents independently from each other selected from the group consisting of Me, $^i$Pr, $^t$Bu, CN, $CF_3$ and Ph;
- carbazolyl, which is optionally substituted with one or more substituents independently from each other selected from the group consisting of Me, $^i$Pr, $^t$Bu, CN, $CF_3$ and Ph;
- triazinyl, which is optionally substituted with one or more substituents independently from each other selected from the group consisting of Me, $^i$Pr, $^t$Bu, CN, $CF_3$, and Ph;

and
- $N(Ph)_2$.

**7.** Use of a molecule according to one or more of claims 1 to 6 as luminescent emitter and/or host material and or electron transport material and/or hole injection material and/or hole blocking material in an organic optoelectronic device.

**8.** Use according to claim 7, wherein the organic optoelectronic device is selected from the group consisting of:

- organic light-emitting diodes (OLEDs),
- light-emitting electrochemical cells,

- OLED-sensors, in particular in non-hermetically shielded gas and vapor sensors,
- organic diodes,
- organic solar cells,
- organic transistors,
- organic field-effect transistors,
- organic lasers and
- down-conversion elements.

**9.** Composition, comprising or consisting of:

(a) at least one organic molecule according to one or more of claims 1 to 6, in particular in the form of an emitter and/or a host, and
(b) one or more emitter and/or host materials, which differ from the organic molecule of one or more of claims 1 to 6 and
(c) optional one or more dyes and/or one or more solvents.

**10.** Optoelectronic device, comprising one organic molecule according to one or more of claims 1 to 6 or a composition according to claim 9, in particular in form of a device selected from the group consisting of organic light-emitting diode (OLED), light-emitting electrochemical cell OLED-sensor, in particular in non-hermetically shielded gas and vapor sensors, organic diode, organic solar cell, organic transistor, organic field-effect transistor, organic laser and down-conversion element.

**11.** Optoelectronic device according to claim 10, comprising

- a substrate,
- an anode and
- a cathode, wherein the anode or the cathode are disposed on the substrate and
- at least a light-emitting layer, which is arranged between anode and cathode and which comprises the organic molecule according to claims 1 to 6 or a composition according to claim 9.

**12.** Process for producing an optoelectronic device, wherein an organic molecule according to one of claims 1 to 6 or a composition according to claim 9 is used.

**13.** Process according to claim 12, comprising the processing of the organic molecule by vacuum evaporation method or from a solution.

**Patentansprüche**

**1.** Organisches Molekül, bestehend aus

- einer ersten chemischen Einheit, die aus einer Struktur der Formel I besteht

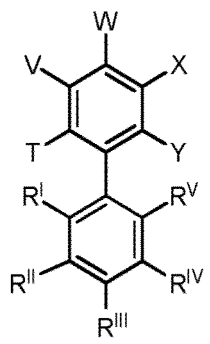

Formel I

und

- einer zweiten chemischen Einheit, die aus einer Struktur der Formel IIa besteht

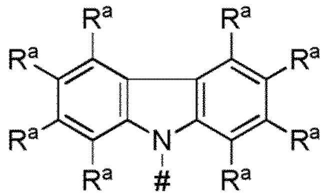

Formel IIa

wobei die erste chemische Einheit über eine Einfachbindung mit der zweiten chemischen Einheit verknüpft ist;
wobei
T ist die Bindungsstelle einer Einfachbindung, die die erste chemische Einheit mit der zweiten chemischen Einheit verknüpft, oder ausgewählt ist aus der Gruppe bestehend aus $R^1$ und $R^T$;
V ist die Bindungsstelle einer Einfachbindung, die die erste chemische Einheit mit der zweiten chemischen Einheit verknüpft, oder ist Wasserstoff (H);
W ist die Bindungsstelle einer Einfachbindung, die die erste chemische Einheit mit der zweiten chemischen Einheit verknüpft, oder ausgewählt ist aus der Gruppe bestehend aus $R^1$ und $R^T$ ist;
X ist ausgewählt aus der Gruppe bestehend aus $R^1$ und $R^T$;
Y ist ausgewählt aus der Gruppe bestehend aus $R^1$ und $R^T$;
# ist die Bindungsstelle einer Einfachbindung, die die erste chemische Einheit mit der zweiten chemischen Einheit verknüpft;
$R^T$ ist 1,3,5-Triazinyl, substituiert mit zwei Substituenten $R^N$:

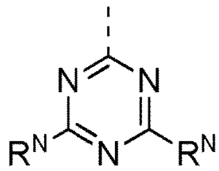
,

wobei die gestrichelte Bindung die Bindungsstelle von $R^T$ an die Einfachbindung steht, die die erste chemische Einheit
und $R^T$ verknüpft;
$R^N$ ist bei jedem Auftreten unabhängig voneinander ausgewählt aus der Gruppe bestehend aus:

Wasserstoff, Deuterium,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind;
$C_6$-$C_{18}$-Aryl,
das optional mit einem oder mehreren Substituenten $R^6$ substituiert ist; und
$C_3$-$C_{17}$-Heteroaryl,
das optional mit einem oder mehreren Substituenten $R^6$ substituiert ist;

$R^I$, $R^{II}$, $R^{III}$, $R^{IV}$ und $R^V$ sind bei jedem Auftreten unabhängig voneinander ausgewählt aus der Gruppe bestehend aus:

Wasserstoff,
Deuterium,
F,

$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,
das optional mit einem oder mehreren Substituenten $R^6$ substituiert ist;
$R^1$ ist bei jedem Auftreten unabhängig voneinander ausgewählt aus der Gruppe bestehend aus:

Wasserstoff,
Deuterium,
$C_1$-$C_5$-Alkyl,
wobei ein oder mehrere Wasserstoffatome optional durch Deuterium substituiert sind;
$C_2$-$C_8$-Alkenyl,
wobei ein oder mehrere Wasserstoffatome optional durch Deuterium substituiert sind;
$C_2$-$C_8$-Alkinyl,
wobei ein oder mehrere Wasserstoffatome optional durch Deuterium substituiert sind; und
$C_6$-$C_{18}$-Aryl,

das optional mit einem oder mehreren Substituenten substituiert ist, die unabhängig voneinander ausgewählt sind aus der Gruppe bestehend aus
$C_1$-$C_5$-Alkyl,
wobei optional ein oder mehrere Wasserstoffatome unabhängig voneinander durch Deuterium, CN, $CF_3$ oder F substituiert sind;
und
$C_6$-$C_{18}$-Aryl,
das optional mit einem oder mehreren $C_1$-$C_5$-Alkylsubstituenten substituiert ist.

$R^a$, $R^3$ und $R^4$ sind bei jedem Auftreten unabhängig voneinander ausgewählt aus der Gruppe bestehend aus:

Wasserstoff,
Deuterium,
$N(R^5)_2$,
$OR^5$,
$Si(R^5)_3$,
$B(OR^5)_2$,
$OSO_2R^5$,
$CF_3$,
CN,
F,
Br,
I,
$C_1$-$C_{40}$-Alkyl,

das optional mit einem oder mehreren Substituenten $R^5$ substituiert ist und wobei eine oder mehrere nicht benachbarte $CH_2$-Gruppen optional durch $R^5C$=$CR^5$, C=C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$ ersetzt sind;

$C_1$-$C_{40}$-Alkoxy,

das optional mit einem oder mehreren Substituenten $R^5$ substituiert ist und wobei eine oder mehrere nicht benachbarte $CH_2$-Gruppen optional durch $R^5C$=$CR^5$, C=C, $Si(R^5)_2$, $Ge(R^5)_2$, $Sn(R^5)_2$, C=O, C=S, C=Se, C=$NR^5$, P(=O)($R^5$), SO, $SO_2$, $NR^5$, O, S oder $CONR^5$ ersetzt sind;

$C_1$-$C_{40}$-Thioalkoxy,

das optional mit einem oder mehreren Substituenten $R^5$ substituiert ist und wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sind;

$C_2$-$C_{40}$-Alkenyl,

das optional mit einem oder mehreren Substituenten $R^5$ substituiert ist und wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sind;

$C_2$-$C_{40}$-Alkinyl,

das optional mit einem oder mehreren Substituenten $R^5$ substituiert ist und wobei eine oder mehrere nicht benachbarte $CH_2$-Gruppen optional durch $R^5C{=}CR^5$, $C{\equiv}C$, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S oder $CONR^5$ ersetzt sind;

$C_6$-$C_{60}$-Aryl,
das optional mit einem oder mehreren Substituenten $R^5$ substituiert ist; und
$C_3$-$C_{57}$-Heteroaryl,
das optional mit einem oder mehreren Substituenten $R^5$ substituiert ist;
$R^5$ ist bei jedem Auftreten unabhängig voneinander ausgewählt aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-Alkyl,

das optional mit einem oder mehreren Substituenten $R^6$ substituiert ist und wobei eine oder mehrere nicht benachbarte $CH_2$-Gruppen optional durch $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S oder $CONR^6$ ersetzt sind;

$C_1$-$C_{40}$-Alkoxy,

das optional mit einem oder mehreren Substituenten $R^6$ substituiert ist und wobei eine oder mehrere nicht benachbarte $CH_2$-Gruppen optional durch $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S oder $CONR^6$ ersetzt sind;

$C_1$-$C_{40}$-Thioalkoxy,

das optional mit einem oder mehreren Substituenten $R^6$ substituiert ist und wobei eine oder mehrere nicht benachbarte $CH_2$-Gruppen optional durch $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S oder $CONR^6$ ersetzt sind;

$C_2$-$C_{40}$-Alkenyl,

das optional mit einem oder mehreren Substituenten $R^6$ substituiert ist und wobei eine oder mehrere nicht benachbarte $CH_2$-Gruppen optional durch $R^6C{=}CR^6$, $C{\equiv}C$, $Si(R^6)_2$, $Ge(R^6)_2$, $Sn(R^6)_2$, C=O, C=S, C=Se, $C{=}NR^6$, $P({=}O)(R^6)$, SO, $SO_2$, $NR^6$, O, S oder $CONR^6$ ersetzt sind;

$C_2$-$C_{40}$-Alkinyl,

das optional mit einem oder mehreren Substituenten $R^6$ substituiert ist und

wobei eine oder mehrere nicht benachbarte $CH_2$-Gruppen optional durch $R^6C=CR^6$, C≡C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$ ersetzt sind;

$C_6$-$C_{60}$-Aryl,
das optional mit einem oder mehreren Substituenten $R^6$ substituiert ist; und
$C_3$-$C_{57}$-Heteroaryl,
das optional mit einem oder mehreren Substituenten $R^6$ substituiert ist;
$R^6$ ist bei jedem Auftreten unabhängig voneinander ausgewählt aus der Gruppe bestehend aus:

Wasserstoff, Deuterium, OPh, $CF_3$, CN, F,
$C_1$-$C_5$-Alkyl,
wobei ein oder mehrere Wasserstoffatome optional unabhängig voneinander durch Deuterium, CN, $CF_3$ oder F ersetzt sind;
$C_1$-$C_5$-Alkoxy,
wobei ein oder mehrere Wasserstoffatome optional unabhängig voneinander durch Deuterium, CN, $CF_3$ oder F ersetzt sind;
$C_1$-$C_5$-Thioalkoxy,
wobei ein oder mehrere Wasserstoffatome optional unabhängig voneinander durch Deuterium, CN, $CF_3$ oder F ersetzt sind;
$C_2$-$C_5$-Alkenyl,
wobei ein oder mehrere Wasserstoffatome optional unabhängig voneinander durch Deuterium, CN, $CF_3$ oder F ersetzt sind;
$C_2$-$C_5$-Alkinyl,
wobei ein oder mehrere Wasserstoffatome optional unabhängig voneinander durch Deuterium, CN, $CF_3$ oder F ersetzt sind;
$C_6$-$C_{18}$-Aryl,
das optional mit einem oder mehreren $C_1$-$C_5$-Alkylsubstituenten substituiert ist;
$C_3$-$C_{17}$-Heteroaryl,
das optional mit einem oder mehreren $C_1$-$C_5$-Alkylsubstituenten substituiert ist;
$N(C_6$-$C_{18}$-$Aryl)_2$,
$N(C_3$-$C_{17}$-$Heteroaryl)_2$
und $N(C_3$-$C_{17}$-Heteroaryl)($C_6$-$C_{18}$-aryl);
wobei die Substituenten $R^a$, $R^3$, $R^4$ oder $R^5$ unabhängig voneinander optional mit einem oder mehreren Substituenten $R^a$, $R^3$, $R^4$ oder $R^5$ ein mono- oder polycyclisches, aliphatisches, aromatisches und/oder benzoanneliertes Ringsystem bilden;

wobei der Begriff Alkyl lineare, verzweigte und cyclische Alkylsubstituenten umfasst, der Begriff Alkenyl lineare, verzweigte und cyclische Alkenylsubstituenten umfasst und der Begriff Alkinyl lineare, verzweigte und cyclische Alkinylsubstituenten umfasst;
wobei genau ein Substituent aus der Gruppe bestehend aus T, W, X und Y für $R^T$ steht, und genau ein Substituent aus der Gruppe bestehend aus T, V und W für die Bindungsstelle einer Einfachbindung steht, die die erste chemische Einheit mit der zweiten chemischen Einheit verknüpft;
wobei T für Wasserstoff steht, wenn W für $R^T$ steht und V für die Bindungsstelle einer Einfachbindung steht, die die erste chemische Einheit mit der zweiten chemischen Einheit verknüpft;
und W für Wasserstoff steht, wenn T für $R^T$ steht und V für die Bindungsstelle einer Einfachbindung steht, die die erste chemische Einheit mit der zweiten chemischen Einheit verknüpft;
und wobei genau zwei Substituenten aus der Gruppe bestehend aus $R^I$, $R^{II}$, $R^{III}$, $R^{IV}$ und $R^V$ für F stehen.

2. Organisches Molekül nach Anspruch 1, wobei $R^1$ und $R^N$ unabhängig voneinander und bei jedem Auftreten unabhängig voneinander aus der Gruppe ausgewählt sind bestehend aus H, Methyl und Phenyl.

3. Organisches Molekül nach Anspruch 1 oder 2, wobei X für $R^T$ steht und W für die Bindungsstelle einer Einfachbindung steht, die die erste chemische Einheit mit der zweiten chemischen Einheit verknüpft.

4. Organisches Molekül nach einem oder mehreren der Ansprüche 1 bis 3, wobei die zweite chemische Einheit aus einer Struktur der Formel IIb:

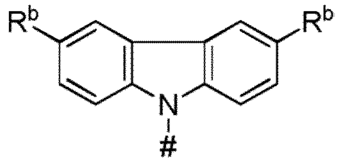

Formel IIb

besteht, wobei
$R^b$ bei jedem Auftreten unabhängig voneinander ausgewählt ist aus der Gruppe bestehend aus:

Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,

das optional mit einem oder mehreren Substituenten $R^5$ substituiert ist und
wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Alkoxy,

das optional mit einem oder mehreren Substituenten $R^5$ substituiert ist und
wobei eine oder mehrere nicht benachbarte $CH_2$-Gruppen optional durch $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_1$-$C_{40}$-Thioalkoxy,

das optional mit einem oder mehreren Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkenyl,

das optional mit einem oder mehreren Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_2$-$C_{40}$-Alkinyl,

das optional mit einem oder mehreren Substituenten $R^5$ substituiert ist und
wobei eine oder mehrere nicht benachbarte $CH_2$-Gruppen optional durch $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S oder $CONR^5$ ersetzt sind;

$C_6$-$C_{60}$-Aryl,
das optional mit einem oder mehreren Substituenten $R^5$ substituiert ist; und
$C_3$-$C_{57}$-Heteroaryl,
das optional mit einem oder mehreren Substituenten $R^5$ substituiert ist;
und wobei davon abgesehen die Definitionen von Anspruch 1 gelten.

5. Organisches Molekül nach einem oder mehreren der Ansprüche 1 bis 3, wobei die zweite chemische Einheit aus einer Struktur der Formel IIc besteht:

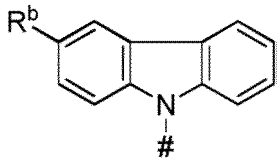

Formel IIc

wobei

$R^b$ bei jedem Auftreten unabhängig voneinander ausgewählt ist aus der Gruppe bestehend aus Deuterium, $N(R^5)_2$, $OR^5$, $Si(R^5)_3$, $B(OR^5)_2$, $OSO_2R^5$, $CF_3$, CN, F, Br, I,
$C_1$-$C_{40}$-Alkyl,

das optional mit einem oder mehreren Substituenten $R^5$ substituiert ist und
wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sind;

$C_1$-$C_{40}$-Alkoxy,

das optional mit einem oder mehreren Substituenten $R^5$ substituiert ist und
wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sind;

$C_1$-$C_{40}$-Thioalkoxy,

das optional mit einem oder mehreren Substituenten $R^5$ substituiert ist und
wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sind;

$C_2$-$C_{40}$-Alkenyl,

das optional mit einem oder mehreren Substituenten $R^5$ substituiert ist und
wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sind;

$C_2$-$C_{40}$-Alkinyl,

das optional mit einem oder mehreren Substituenten $R^5$ substituiert ist und
wobei eine oder mehrere nicht benachbarte $CH_2$-Gruppen optional durch $R^5C{=}CR^5$, C=C, $Si(R^5)_2$, $Ge(R^5)_2$, $Sn(R^5)_2$, C=O, C=S, C=Se, $C{=}NR^5$, $P(=O)(R^5)$, SO, $SO_2$, $NR^5$, O, S oder $CONR^5$ ersetzt sind;

$C_6$-$C_{60}$-Aryl,
das optional mit einem oder mehreren Substituenten $R^5$ substituiert ist; und
$C_3$-$C_{57}$-Heteroaryl,
das optional mit einem oder mehreren Substituenten $R^5$ substituiert ist;
und wobei davon abgesehen die Definitionen von Anspruch 1 gelten.

**6.** Organisches Molekül nach Anspruch 4 oder 5, wobei $R^b$ bei jedem Auftreten unabhängig voneinander ausgewählt ist aus der Gruppe bestehend aus:

- Me, $^i$Pr, $^t$Bu, CN, $CF_3$,
- Ph, das optional mit einem oder mehreren Substituenten substituiert ist, die unabhängig voneinander aus der Gruppe bestehend aus Me, $^i$Pr, $^t$Bu, CN, $CF_3$ und Ph ausgewählt sind;
- Pyridinyl, das optional mit einem oder mehreren Substituenten substituiert ist, die unabhängig voneinander aus der Gruppe bestehend aus Me, $^i$Pr, $^t$Bu, CN, $CF_3$ und Ph ausgewählt sind;
- Pyrimidinyl, das optional mit einem oder mehreren Substituenten substituiert ist, die unabhängig voneinander aus der Gruppe bestehend aus Me, $^i$Pr, $^t$Bu, CN, $CF_3$ und Ph ausgewählt sind;

- Carbazolyl, das optional mit einem oder mehreren Substituenten substituiert ist, die unabhängig voneinander aus der Gruppe bestehend aus Me, $^{i}$Pr, $^{t}$Bu, CN, $CF_3$ und Ph ausgewählt sind;
- Triazinyl, das optional mit einem oder mehreren Substituenten substituiert ist, die unabhängig voneinander aus der Gruppe bestehend aus Me, $^{i}$Pr, $^{t}$Bu, CN, $CF_3$ und Ph ausgewählt sind; und
- $N(Ph)_2$.

**7.** Verwendung eines Moleküls nach einem oder mehreren der Ansprüche 1 bis 6 als lumineszierender Emitter und/oder Wirtsmaterial und/oder Elektronentransportmaterial und/oder Lochinjektionsmaterial und/oder Lochblockiermaterial in einer organischen optoelektronischen Vorrichtung.

**8.** Verwendung nach Anspruch 7, wobei die organische optoelektronische Vorrichtung ausgewählt ist aus der Gruppe bestehend aus:

• organischen lichtemittierenden Dioden (OLEDs),
• lichtemittierenden elektrochemischen Zellen,
• OLED-Sensoren, insbesondere in nicht hermetisch nach außen abgeschirmten Gas- und Dampf-Sensoren,
• organischen Dioden,
• organischen Solarzellen,
• organischen Transistoren,
• organischen Feldeffekttransistoren,
• organischen Lasern und
• Down-Konversions-Elementen.

**9.** Zusammensetzung, aufweisend oder bestehend aus:

(a) mindestens einem organisches Molekül nach einem oder mehreren der Ansprüche 1 bis 6, insbesondere in Form eines Emitters und/oder eines Wirts, und
(b) einem oder mehreren Emitter- und/oder Wirtsmaterialien, die von dem organischen Molekül nach einem oder mehreren der Ansprüche 1 bis 6 verschieden sind, und
(c) optional einem oder mehreren Farbstoffe und/oder einem oder mehreren Lösungsmittel.

**10.** Optoelektronische Vorrichtung, aufweisend ein organisches Molekül nach einem oder mehreren der Ansprüche 1 bis 6 oder eine Zusammensetzung nach Anspruch 9, insbesondere in Form einer Vorrichtung ausgewählt aus der Gruppe bestehend aus: einer organischen lichtemittierenden Diode (OLED), einer lichtemittierenden elektrochemischen Zelle, einem OLED-Sensor, insbesondere in nicht hermetisch nach außen abgeschirmten Gas- und Dampf-Sensoren, einer organischen Diode, einer organischen Solarzelle, einem organischen Transistor, einem organischen Feldeffekttransistor, einem organischen Laser, und einem Down-Konversions-Element.

**11.** Optoelektronische Vorrichtung nach Anspruch 10, aufweisend:

- ein Substrat,
- eine Anode und
- eine Kathode, wobei die Anode oder die Kathode auf das Substrat aufgebracht ist, und
- mindestens eine lichtemittierende Schicht, die zwischen der Anode und der Kathode angeordnet ist und die das organische Molekül nach den Ansprüchen 1 bis 6 oder eine Zusammensetzung nach Anspruch 9 aufweist.

**12.** Verfahren zur Herstellung einer optoelektronischen Vorrichtung, bei dem ein organisches Molekül nach einem der Ansprüche 1 bis 6 oder eine Zusammensetzung nach Anspruch 9 verwendet wird.

**13.** Verfahren nach Anspruch 12, bei dem das organische Molekül durch ein Vakuumverdampfungsverfahren oder aus einer Lösung verarbeitet wird.

## Revendications

**1.** Molécule organique constituée par

- un premier fragment chimique constitué par une structure de formule I,

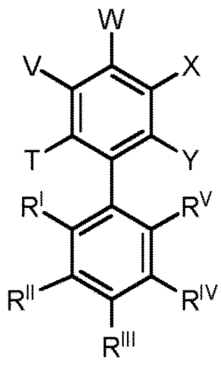

Formule I

et

- un deuxième fragment chimique constitué par une structure de formule IIa,

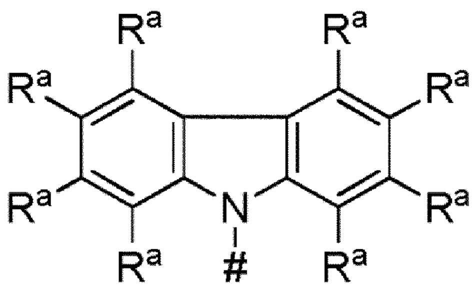

Formule IIa

le premier fragment chimique étant lié au deuxième fragment chimique via une simple liaison ;

T étant le point de liaison d'une simple liaison liant le premier fragment chimique au deuxième fragment chimique ou étant choisi dans le groupe constitué par $R^1$ et $R^T$ ;

V étant le point de liaison d'une simple liaison liant le premier fragment chimique au deuxième fragment chimique ou étant hydrogène (H) ;

W étant le point de liaison d'une simple liaison liant le premier fragment chimique au deuxième fragment chimique ou étant choisi dans le groupe constitué par $R^1$ et $R^T$ ;

X étant choisi dans le groupe constitué par $R^1$ et $R^T$ ;

Y étant choisi dans le groupe constitué par $R^1$ et $R^T$ ;

# représentant le site de liaison d'une simple liaison liant le premier fragment chimique au deuxième fragment chimique ;

$R^T$ étant 1,3,5-triazinyle, substitué par deux substituants $R^N$ :

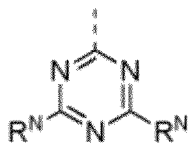

,

la liaison pointillée représentant le site de liaison de $R^T$ à la simple liaison reliant le premier fragment chimique et $R^T$ ;

$R^N$ étant en chaque occurrence indépendamment d'un autre choisi dans le groupe constitué par hydrogène, deutérium,

$C_{1-5}$-alkyle,

un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcynyle,
un ou plusieurs atomes d'hydrogène étant éventuellement substitués par deutérium ;
$C_{6-18}$-aryle,
qui est éventuellement substitué par un ou plusieurs substituants $R^6$ ; et
$C_{3-17}$-hétéroaryle,
qui est éventuellement substitué par un ou plusieurs substituants $R^6$ ; $R^I$, $R^{II}$, $R^{III}$, $R^{IV}$ et $R^V$ étant en chaque occurrence indépendamment d'un autre choisi dans le groupe constitué par hydrogène, deutérium,
F,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcynyle,
un ou plusieurs atomes d'hydrogène étant éventuellement substitués par deutérium ; et
$C_{6-18}$-aryle,
qui est éventuellement substitué par un ou plusieurs substituants R6 ;
$R^1$ étant en chaque occurrence indépendamment d'un autre choisi dans le groupe constitué par hydrogène, deutérium,
$C_{1-5}$-alkyle,
un ou plusieurs atomes d'hydrogène étant éventuellement substitués par deutérium ;
$C_{2-8}$-alcényle,
un ou plusieurs atomes d'hydrogène étant éventuellement substitués par deutérium ;
$C_{2-8}$-alcynyle,
un ou plusieurs atomes d'hydrogène étant éventuellement substitués par deutérium ; et
$C_{6-18}$-aryle,
qui est éventuellement substitué par un ou plusieurs substituants indépendamment d'un autre choisi dans le groupe constitué par ;

> $C_{1-5}$-alkyle,
> éventuellement un ou plusieurs atomes d'hydrogène étant indépendamment l'un de l'autre substitués par deutérium, CN, $CF_3$ ou F ;
> et
> $C_{6-18}$-aryle,
> qui est éventuellement substitué par un ou plusieurs substituants $C_{1-5}$-alkyle ;
> $R^a$, $R^3$ et $R^4$ étant en chaque occurrence indépendamment d'un autre choisi dans le groupe constitué par hydrogène, deutérium,
> $N(R^5)_2$,
> $OR^5$,
> $Si(R^5)_3$,
> $B(OR^5)_2$,
> $OSO_2R^5$,
> $CF_3$,
> CN,
> F,
> Br,
> I,

$C_{1-40}$-alkyle,

> qui est éventuellement substitué par un ou plusieurs substituants $R^5$ et
> un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{1-40}$-alcoxyle,

qui est éventuellement substitué par un ou plusieurs substituants $R^5$ et
un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{1-40}$-thioalcoxyle,

qui est éventuellement substitué par un ou plusieurs substituants $R^5$ et
un ou plusieurs groupes $CH_2$ non adjacents étant éventuellement substitués par $R^5C=CR^5$, C=C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{2-40}$-alcényle,

qui est éventuellement substitué par un ou plusieurs substituants $R^5$ et
un ou plusieurs groupes $CH_2$ non adjacents étant éventuellement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{2-40}$-alcynyle,

qui est éventuellement substitué par un ou plusieurs substituants $R^5$ et
un ou plusieurs groupes $CH_2$ non adjacents étant éventuellement substitués par $R^5C=CR^5$, $C\equiv C$, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{6-60}$-aryle,
qui est éventuellement substitué par un ou plusieurs substituants $R^5$ ; et
$C_{3-57}$-hétéroaryle,
qui est éventuellement substitué par un ou plusieurs substituants $R^5$ ;
R5 étant en chaque occurrence indépendamment d'un autre choisi dans le groupe constitué par hydrogène, deutérium, $N(R^6)_2$, $OR^6$, $Si(R^6)_3$, $B(OR^6)_2$, $OSO2R^6$, $CF_3$, CN, F, Br, I,

$C_{1-40}$-alkyle,

qui est éventuellement substitué par un ou plusieurs substituants $R^6$ et
un ou plusieurs groupes $CH_2$ non adjacents étant éventuellement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;

$C_{1-40}$-alcoxyle,

qui est éventuellement substitué par un ou plusieurs substituants $R^6$ et
un ou plusieurs groupes $CH_2$ non adjacents étant éventuellement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;

$C_{1-40}$-thioalcoxyle,

qui est éventuellement substitué par un ou plusieurs substituants $R^6$ et
un ou plusieurs groupes $CH_2$ non adjacents étant éventuellement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;

$C_{2-40}$-alcényle,

qui est éventuellement substitué par un ou plusieurs substituants $R^6$ et
un ou plusieurs groupes $CH_2$ non adjacents étant éventuellement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;

$C_{2-40}$-alcynyle,

qui est éventuellement substitué par un ou plusieurs substituants $R^6$ et
un ou plusieurs groupes $CH_2$ non adjacents étant éventuellement substitués par $R^6C=CR^6$, $C\equiv C$, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;

$C_{6-60}$-aryle,
qui est éventuellement substitué par un ou plusieurs substituants $R^6$; et
$C_{3-57}$-hétéroaryle,
qui est éventuellement substitué par un ou plusieurs substituants $R^6$ ;

$R^6$ étant en chaque occurrence indépendamment d'un autre choisi dans le groupe constitué par hydrogène, deutérium, OPh, $CF_3$, CN, F,
$C_{1-5}$-alkyle,
un ou plusieurs atomes d'hydrogène étant éventuellement, indépendamment l'un de l'autre substitués par deutérium, CN, $CF_3$, ou F ;

$C_{1-5}$-alcoxyle,
un ou plusieurs atomes d'hydrogène étant éventuellement, indépendamment l'un de l'autre substitués par deutérium, CN, $CF_3$, ou F ;
$C_{1-5}$-thioalcoxyle,
un ou plusieurs atomes d'hydrogène étant éventuellement, indépendamment l'un de l'autre substitués par deutérium, CN, $CF_3$, ou F ;
$C_{2-5}$-alcényle,
un ou plusieurs atomes d'hydrogène étant éventuellement, indépendamment l'un de l'autre substitués par deutérium, CN, $CF_3$, ou F ;
$C_{2-5}$-alcynyle,
un ou plusieurs atomes d'hydrogène étant éventuellement, indépendamment l'un de l'autre substitués par deutérium, CN, $CF_3$, ou F ;
$C_{6-18}$-aryle,
qui est éventuellement substitué par un ou plusieurs substituants $C_{1-5}$-alkyle ;
$C_{3-17}$-hétéroaryle,
qui est éventuellement substitué par un ou plusieurs substituants $C_{1-5}$-alkyle ;
$N(C_{6-18}\text{-aryl})_2$ ;
$N(C_{3-17}\text{-hétéroaryl})_2$,
et $N(C_{3-17}\text{-hétéroaryl})(C_{6-18}\text{-aryl})$ ;

les substituants $R^a$, $R^3$, $R^4$ ou $R^5$ indépendamment les uns des autres formant éventuellement un système cyclique monocyclique ou polycyclique, aliphatique, aromatique et/ou benzo-fusionné compo$R^T$ant un ou plusieurs substituants $R^a$, $R^3$, $R^4$ ou $R^5$ ;
les termes alkyle comprenant des substituants alkyle linéaires, ramifiés et cycliques, le terme alcényle comprenant des substituants alcényle linéaires, ramifiés et cycliques et le terme alcynyle comprenant des substituants alcynyle linéaires, ramifiés et cycliques ;
exactement un substituant choisi dans le groupe constitué par T, W, X, et Y étant $R^T$, et exactement un substituant choisi dans le groupe constitué par T, V et W représentant le site de liaison d'une simple liaison liant le premier fragment chimique et le deuxième fragment chimique.
T étant hydrogène dans le cas où W est $R^T$ et V représentant le site de liaison d'une simple liaison liant le premier fragment chimique et le deuxième fragment chimique ;
et W étant hydrogène dans le cas où T est $R^T$ et V représentant le site de liaison d'une simple liaison liant le premier fragment chimique et le deuxième fragment chimique ;
et exactement deux substituants choisis dans le groupe constitué par $R^I$, $R^{II}$, $R^{III}$, $R^{IV}$ et $R^V$ étant F.

**2.** Molécule organique selon la revendication 1, $R^1$ et $R^N$ étant indépendamment l'un de l'autre et en chaque occurrence indépendamment d'un autre choisis dans le groupe constitué par H, méthyle et phényle.

**3.** Molécule organique selon la revendication 1 ou 2, X étant $R^T$ et W représentant le site de liaison d'une simple liaison liant le premier fragment chimique et le deuxième fragment chimique.

**4.** Molécule organique selon une ou plusieurs des revendications 1 à 3, le deuxième fragment chimique étant constitué par une structure de formule IIb :

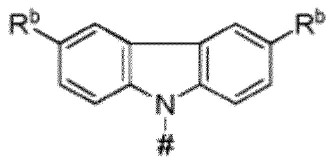

Formule IIb

$R^b$ étant en chaque occurrence indépendamment d'un autre choisi dans le groupe constitué par deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{1-40}$-alcoxyle,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{1-40}$-thioalcoxyle,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{2-40}$-alcényle,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{2-40}$-alcynyle,

qui est éventuellement substitué par un ou plusieurs substituants $R^5$ et
un ou plusieurs groupes $CH_2$ non adjacents étant éventuellement substitués par $R^5C=CR^5$, C≡C, $Si(R^5)_2$, $Ge(R^5)_2$, $Sn(R^5)_2$, C=O, C=S, C=Se, $C=NR^5$, $P(=O)(R^5)$, SO, $SO_2$, $NR^5$, O, S ou $CONR^5$ ;

$C_{6-60}$-aryle,
qui est éventuellement substitué par un ou plusieurs substituants $R^5$ ; et
$C_{3-57}$-hétéroaryle,
qui est éventuellement substitué par un ou plusieurs substituants $R^5$ ;

et les définitions dans la revendication 1 s'appliquant par ailleurs.

**5.** Molécule organique selon une ou plusieurs des revendications 1 à 3, le deuxième fragment chimique étant constitué par une structure de formule IIc :

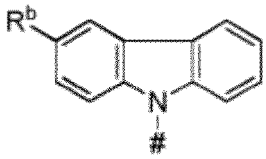

Formule IIc

$R^b$ étant en chaque occurrence indépendamment d'un autre choisi dans le groupe constitué par deutérium, $N(R^5)_2$, $OR^5$, $Si(R^5)_3$, $B(OR^5)_2$, $OSO_2R^5$, $CF_3$, CN, F, Br, I,
$C_{1-40}$-alkyle,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S ou $CONR^5$ ;

$C_{1-40}$-alcoxyle,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S ou $CONR^5$ ;

$C_{1-40}$-thioalcoxyle,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S ou $CONR^5$ ;

$C_{2-40}$-alcényle,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S ou $CONR^5$ ;

$C_{2-40}$-alcynyle,

qui est éventuellement substitué par un ou plusieurs substituants $R^5$ et
un ou plusieurs groupes $CH_2$ non adjacents étant éventuellement substitués par $R^5C{=}CR^5$, C≡C, $Si(R^5)_2$, $Ge(R^5)_2$, $Sn(R^5)_2$, C=O, C=S, C=Se, $C{=}NR^5$, $P({=}O)(R^5)$, SO, $SO_2$, $NR^5$, O, S ou $CONR^5$ ;

$C_{6-60}$-aryle,
qui est éventuellement substitué par un ou plusieurs substituants $R^5$ ; et
$C_{3-57}$-hétéroaryle,
qui est éventuellement substitué par un ou plusieurs substituants $R^5$ ;
et les définitions dans la revendication 1 s'appliquant par ailleurs.

**6.** Molécule organique selon la revendication 4 ou 5, $R^b$ étant en chaque occurrence indépendamment d'un autre choisi dans le groupe constitué par

- Me, $^i$Pr, $^t$Bu, CN, $CF_3$,
- Ph, qui est éventuellement substitué par un ou plusieurs substituants choisis indépendamment l'un de l'autre dans le groupe constitué par Me, $^i$Pr, $^t$Bu, CN, $CF_3$ et Ph ;
- pyridinyle, qui est éventuellement substitué par un ou plusieurs substituants choisis indépendamment l'un de l'autre dans le groupe constitué par Me, $^i$Pr, $^t$Bu, CN, $CF_3$ et Ph ;
- pyrimidinyle, qui est éventuellement substitué par un ou plusieurs substituants choisis indépendamment l'un de l'autre dans le groupe constitué par Me, $^i$Pr, $^t$Bu, CN, $CF_3$ et Ph ;

- carbazolyle, qui est éventuellement substitué par un ou plusieurs substituants choisis indépendamment l'un de l'autre dans le groupe constitué par Me, $^{i}Pr$, $^{t}Bu$, CN, $CF_3$ et Ph ;
- triazinyle, qui est éventuellement substitué par un ou plusieurs substituants choisis indépendamment l'un de l'autre dans le groupe constitué par Me, $^{i}Pr$, $^{t}Bu$, CN, $CF_3$ et Ph ;

et
- $N(Ph)_2$.

**7.** Utilisation d'une molécule selon l'une quelconque des revendications 1 à 6 en tant qu'émetteur luminescent et/ou matériau hôte et/ou matériau de transport d'électrons et/ou matériau d'injection de trous et/ou matériau de blocage de trous dans un dispositif optoélectronique organique.

**8.** Utilisation selon la revendication 7, le dispositif optoélectronique organique étant choisi dans le groupe constitué par :

- des diodes électroluminescentes organiques (OLED),
- des cellules électrochimiques électroluminescentes,
- des capteurs OLED, en particulier dans des capteurs de gaz et de vapeur non hermétiquement scellés,
- des diodes organiques,
- des cellules solaires organiques,
- des transistors organiques,
- des transistors à effet de champ organiques,
- des lasers organiques et
- des éléments de conversion descendante.

**9.** Composition comprenant ou constituée par :

(a) au moins une molécule organique selon une ou plusieurs des revendications 1 à 6, en particulier sous forme d'un émetteur et/ou d'un hôte, et
(b) un ou plusieurs matériaux émetteurs et/ou hôtes, qui diffèrent de la molécule organique selon une ou plusieurs des revendications 1 à 6 et
(c) éventuellement un ou plusieurs colorants et/ou un ou plusieurs solvants.

**10.** Dispositif optoélectronique, comprenant une molécule organique selon une ou plusieurs des revendications 1 à 6 ou une composition selon la revendication 9, en particulier sous forme d'un dispositif choisi dans le groupe constitué par une diode électroluminescente organique (OLED), une cellule électrochimique électroluminescente, un capteur OLED, en particulier dans des capteurs de gaz et de vapeur non hermétiquement scellés, une diode organique, une cellule solaire organique, un transistor organique, un transistor à effet de champ organique, un laser organique et un élément de conversion descendante.

**11.** Dispositif optoélectronique selon la revendication 10, comprenant

- un substrat,
- une anode et
- une cathode, l'anode et la cathode étant disposées sur le substrat et
- au moins une couche luminescente, qui est disposée entre anode et cathode et qui comprend la molécule organique selon les revendications 1 à 6 ou une composition selon la revendication 9.

**12.** Procédé pour la production d'un dispositif optoélectronique, une molécule organique selon l'une des revendications 1 à 6 ou une composition selon la revendication 9 étant utilisée.

**13.** Procédé selon la revendication 12, comprenant le traitement de la molécule organique par un procédé d'évaporation sous vide ou à partir d'une solution.

**Figure 1**

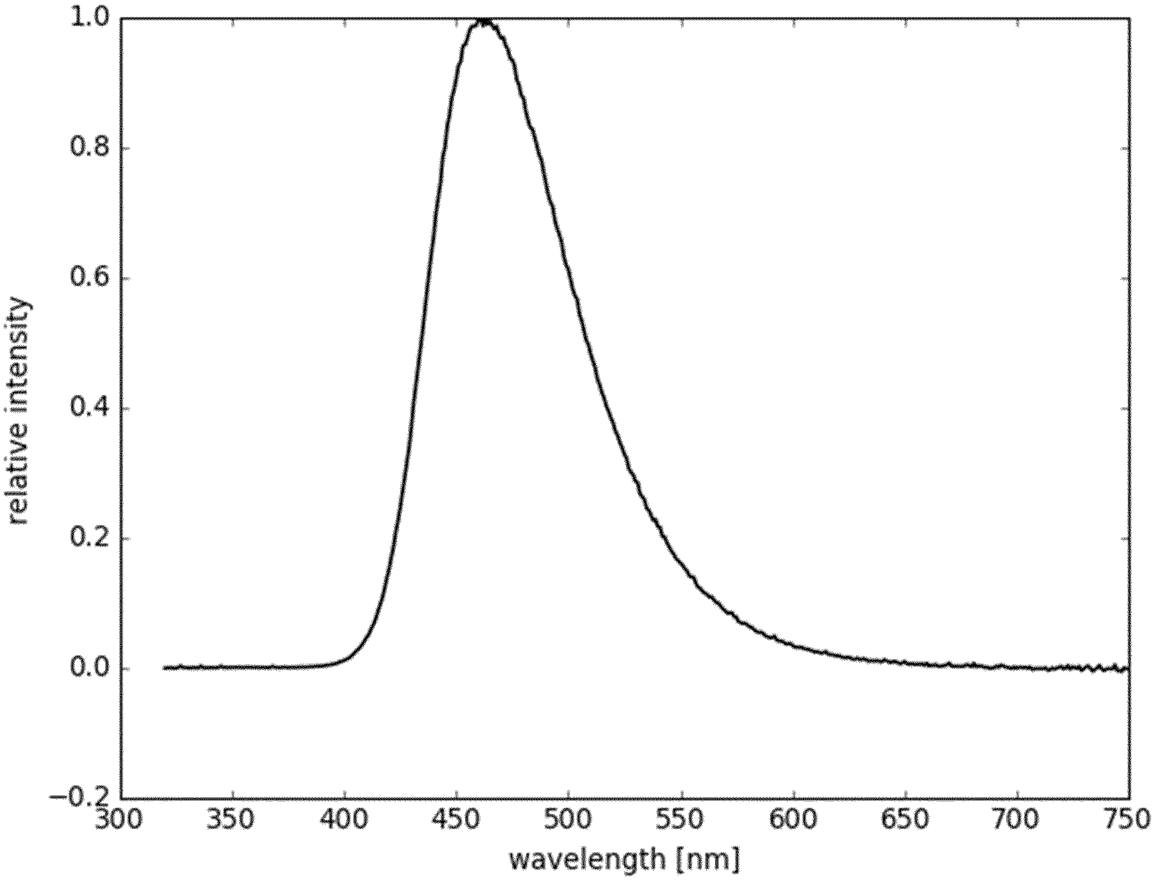
1.0
0.8
0.6
0.4
0.2
0.0
−0.2
relative intensity
300
350
400
450
500
550
600
650
700
750
wavelength [nm]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150349273 A1
- WO 2014146752 A1


### Non-patent literature cited in the description


- **OKINAKA et al.** Invited Paper: New Fluorescent Blue Host Materials for Achieving Low Voltage. *OLEDs, SID Symposium Digest of Technical Papers,* 2015, vol. 22.1, 46
- *CHEMICAL ABSTRACTS,* 73183-34-3